# EUROPEAN PATENT SPECIFICATION

(11) **EP 1 651 232 B1**
(45) Date of publication and mention of the grant of the patent: **22.12.2010**
(21) Application number: 04786102.6
(22) Date of filing: 22.07.2004
(51) Int. Cl.: A61K 31/535, A61K 31/41, A61K 31/42, A61K 31/415, A61K 31/40, A61P 29/00, A61P 25/00, A61P 37/00

(54) **COMPOUNDS FOR INFLAMMATION AND IMMUNE-RELATED USES**
VERBINDUNGEN GEGEN ENTZÜNDUNGEN UND IMMUN-RELEVANTE VERWENDUNGEN
COMPOSES POUR L'INFLAMMATION ET UTILISATIONS LIEES AU SYSTEME IMMUNITAIRE

(30) Priority: 23.07.2003 US 489711 P
(43) Date of publication of application: 03.05.2006
(60) Divisional application: 10075464.7
(73) Proprietor: Synta Pharmaceuticals Corporation, Lexington, MA 02421 (US)
(72) Inventor: XIE, Yu, Natick, MA 01760 (US); HOLMQVIST, Mats, Malden, MA 02148 (US); MAHIOU, Jerome, Lexington, MA 02421 (US); ONO, Mitsunori, Lexington, MA 02421 (US); SUN, Lijun, Harvard, MA 01451 (US); CHEN, Shoujun, Bedford, MA 01730 (US); ZHANG, Shijie, Nashua, NH 03062 (US); JIANG, Jun, Acton, MA 01720 (US); CHIMMANAMADA, Dinesh, Waltham, MA 02452 (US); YU, Chih-Yi, Malden, MA 02148 (US)
(74) Representative: Snodin, Michael D.
(86) International application number: PCT/US2004/023895
(87) International publication number: WO 2005/009539

(56) References cited:
- EP-A- 1 099 701
- EP-A1- 1 277 729
- WO-A-01/55146
- WO-A-96/40640
- WO-A-03/068749
- WO-A-2004/056774
- WO-A-2005/009539
- WO-A-2005/073193
- WO-A1-97/08135
- WO-A1-99/01127
- US-A- 5 348 968
- US-B2- 6 620 811

## Description

### RELATED APPLICATIONS

This application claims the benefit of U.S. Provisional Application No. 60/489,711, filed July 23, 2003.

### FIELD OF THE INVENTION

This invention relates to biologically active chemical compounds, namely phenyl and pyridyl derivatives that may be used for immunosuppression or to treat or prevent inflammatory conditions and immune disorders.

### BACKGROUND OF THE INVENTION

Inflammation is a mechanism that protects mammals from invading pathogens. However, while transient inflammation is necessary to protect a mammal from infection, uncontrolled inflammation causes tissue damage and is the underlying cause of many illnesses. Inflammation is typically initiated by binding of an antigen to T-cell antigen receptor. Antigen binding by a T-cell initiates calcium influx into the cell via calcium ion channels, such as Ca²⁺-release-activated Ca²⁺ channels (CRAC). Calcium ion influx in turn initiates a signaling cascade that leads to activation of these cells and an inflammatory response characterized by cytokine production.

Interleukin 2 (IL-2) is a cytokine that is secreted by T cells in response to calcium ion influx into the cell. IL-2 modulates immunological effects on many cells of the immune system. For example, It is a potent T cell mitogen that is required for the T cell proliferation, promoting their progression from G1 to S phase of the cell cycle; it stimulates the growth of NK cells; and it acts as a growth factor to B cells and stimulates antibody synthesis.

IL-2, although useful in the immune response, can cause a variety of problems. IL-2 damages the blood-brain barrier and the endothelium of brain vessels. These effects may be the underlying causes of neuropsychiatric side effects observed under IL-2 therapy, e.g. fatigue, disorientation and depression. It also alters the electrophysiological behaviour of neurons.

Due to its effects on both T and B cells, IL-2 is a major central regulator of immune responses. It plays a role in inflammatory reactions, tumour surveillance, and hematopoiesis. It also affects the production of other cytokines, inducing IL-1, TNF-α and TNF-β secretion, as well as stimulating the synthesis of IFN-γ in peripheral leukocytes.

T cells that are unable to produce IL-2 become inactive (anergic). This renders them potentially inert to any antigenic stimulation they might receive in the future. As a result, agents which inhibit IL-2 production can be used for immunosupression or to treat or prevent inflammation and immune disorders. This approach has been clinically validated with immunosuppressive drugs such as cyclosporin, FK506, and RS61443. Despite this proof of concept, agents that inhibit IL-2 production remain far from ideal. Among other problems, efficacy limitations and unwanted side effects (including dose-dependant nephrotoxicity and hypertension) hinder their use.

Over production of proinflammatory cytokines other than IL-2 has also been implicated in many autoimmune diseases. For example, Interleukin 5 (IL-5), a cytokine that increases the production of eosinophils, is increased in asthma. Overproduction of IL-5 is associated with accumulation of eosinophils in the asthmatic bronchial mucosa, a hall mark of allergic inflammation. Thus, patients with asthma and other inflammatory disorders involving the accumulation of eosinophils would benefit from the development of new drugs that inhibit the production of IL-5.

Interleukin 4 (IL-4) and interleukin 13 (IL-13) have been identified as mediators of the hypercontractility of smooth muscle found in inflammatory bowel disease and asthma. Thus, patients with athsma and inflammatory bowel disease would benefit from the development of new drugs that inhibit IL-4 and IL-13 production.

Granulocyte macrophage-colony stimulating factor (GM-CSF) is a regulator of maturation of granulocyte and macrophage lineage population and has been implicated as a key factor in inflammatory and autoimmune diseases. Anti-GM-CSF antibody blockade has been shown to ameliorate autoimmune disease. Thus, development of new drugs that inhibit the production of GM-CSF would be beneficial to patients with an inflammatory or autoimmune disease.

There is therefore a continuing need for new drugs which overcome one or more of the shortcomings of drugs currently used for immunosuppression or in the treatment or prevention of inflammatory disorders and autoimmune disorders. Desirable properties of new drugs include efficacy against diseases or disorders that are currently untreatable or poorly treatable, new mechanism of action, oral bioavailability and/or reduced side effects.

Among the prior art, WO 2004/056774 A2 describes substituted biphenyl-4-carboxylic acid arylamide analogues capable of modulating receptor activity. Further, WO 96/40640 A describes biphenyl-2-carboxylic acid-tetrahydroisoquinoline-6-yl amides, their preparation and their use as inhibitors of microsomal triglyceride transfer protein and/or apolipoprotein B secretion. Among further prior art, WO 03/068749 A1 relates to vanilloid receptor modulators having potency and selectivity as VR-1 antagonists. EP 1 099 701 A1 discloses 7-[(4'-trifluoromethyl-biphenyl-2-carbonyl)amino]-quinoline-3-carboxylic acid amides and methods of inhibiting the secretion of apolipoprotein B. Further, WO 01/55146 A1 describes factor Xa inhibitors with aryl amidines and derivatives for use as an anticoagulant agent and prevention of thrombosis disorders. International patent application WO 2005/073193 A1 describes compounds useful in the treatment of vanillin receptor mediated diseases and other maladies.

Further, US 5 348 948 A describes indole, indazole and benzisoxazole compounds useful as agonists at 5-HT₁-like receptors in the treatment of depression, anxiety, migraine, pain and illnesses associated with a deficiency of serotoninergenic neurotransmission. Among further prior art, US 6 620 811 B2 describes isonicotin-and nicotinamide derivatives of benzothiazoles useful as adenosine receptor ligands. Further, WO 99/01127 relates to substituted benzanilides which are ligands, agonists or antagonists of the CCR5 receptor. Further, EP 1 127 729 relates to 2-chloro-5-nitrocarboxamides derivatives having PPAR γ modulating activities and their use. Among further prior art, WO 97/08135 describes aminosalicylic acid amides useful in pest control.

### SUMMARY OF THE INVENTION

This invention meets the above-mentioned needs by providing certain phenyl and pyridyl derivatives that inhibit the production of IL-2, IL-4, IL-5, IL-13, GM-CSF, TNF-α, and IFNγ. These compounds are particularly useful for immunosuppression and/or to treat or prevent inflammatory conditions and immune disorders.

The invention relates to an in vitro method of inhibiting immune cell activation comprising administering to the cell a compound of formula (I): 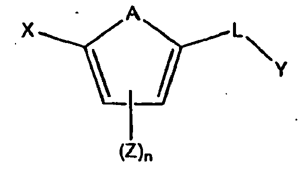 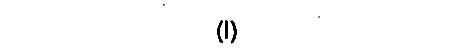 or a pharmaceutically acceptable salt, solvate or clathrate thereof, wherein:
X is an optionally substituted phenyl, an optionally substituted triazolyl, an optionally substituted pyridyl, or an optionally substituted indolizinyl;
Y is an optionally substituted aryl or an optionally substituted heteroaryl, which may be optionally substituted with 1-3 substituents independently selected from halo, C₁₋₄ alkyl, halogenated C₁-C₄ alkyl, or C₁₋₄ alkoxy;
A is -CH=CH-, -CZ=CH- or -CH=CZ-;
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of
   -NRCH₂- and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is O or an integer from 1 to 4; and
p is 0, 1, or 2.

In another embodiment, the invention relates to an in vitro method of inhibiting cytokine production in a cell, comprising administering to the cell a compound represented by the following structural formula: 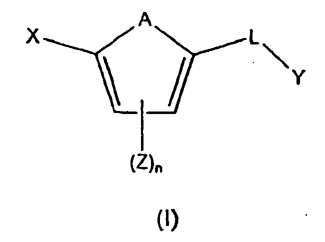 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
A is -CH=CH-, -CZ=CH-, or -CH=CZ-;
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group-consisting of-NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4; and
p is 0, 1, or 2.

In a further embodiment, the invention relates to an in vitro method of modulating an ion channel in a cell, wherein the ion channel
is involved in immune cell activation, comprising administering to the cell a compound represented by the following structural formula: 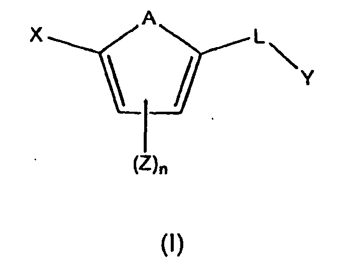 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
A is -CH=CH-, -CZ=CH-, or -CH=CZ-;
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or
R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is an integer selected from 0-4; and
p is 0, 1, or 2.

In another embodiment, the invention relates to an in vitro method of inhibiting T-cell and/or B-cell proliferation in response to an
antigen, comprising administering to the cell a compound represented by the following structural formula: 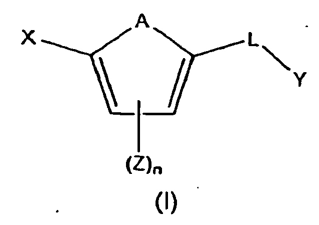 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
A is -CH=CH-, -CZ=CH-, or -CH=CZ-.
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or
R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4; and
p is 0, 1, or 2.

In another embodiment, the invention relates to compounds represented by the following structural formula (II): 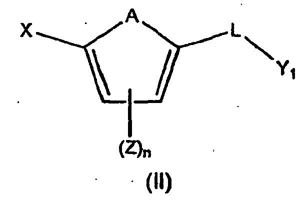 and pharmaceutically acceptable salts, solvates or clathrates thereof wherein:
X, A, Z, L, R, R₁, R₂, R₄, R₅, P and n are defined as for formula (I); and
Y₁ is an optionally substituted aryl or an optionally substituted heteroaryl, which may be substituted with 1-3 substituents independently selected from halo, -C₁₋₄ alkyl, halogenated C₁-C₄ alkyl, or C₁₋₄ alkoxy.

In this embodiment, the following conditions are met:
1) Y₁ is not a heteroaryl that is further substituted with a substituted or unsubstituted aryl or a substituted or unsubstituted heteroaryl;
2) when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y₁ is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydronaphthyl, a substituted pyrazinyl, or a substituted or unsubstituted pyridinyl;
3) when X is m-nitrophenyl or m-(trifluoromethyl)phenyl, Y₁ is not a substituted or unsubstituted phenyl;
4) X is not a phenyl that is substituted in the ortho position with - S(O)₂NH₂; and/or
5) X is not a nitrophenyl when Y₁ is a substituted 1H-pyrazolyl.

In another embodiment, the invention is related to compounds represented by formula (III): 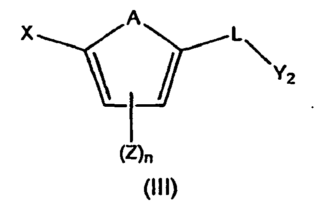 and pharmaceutically acceptable salts, solvates or clathrates thereof wherein:
X, A, Z, L, R, R₁, R₂, R₄, R₅, P and n are defined as for formula (I); and
Y₂ is an optionally substituted cycloalkyl, an optionally substituted cycloalkenyl, or an optionally substituted heterocyclyl.

In this embodiment, the following conditions are met:
1) X is not a phenyl that is substituted in the ortho position with -CN or - S(O)₂NH₂:
2) Y₂ is not a 4,5-dihydroisoxazlyl that is further substituted with a substituted or unsubstituted aryl or a substituted or unsubstituted heteroaryl; and/or
3) Y₂ is not a substituted cyclopentenyl.

In another embodiment, the invention relates to compounds represented by formula (IV): 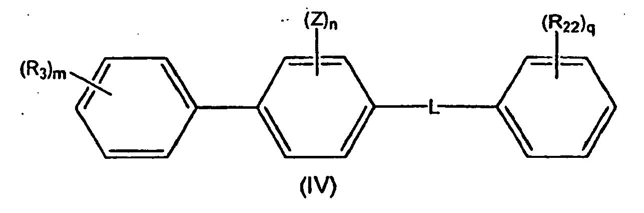 and pharmaceutically acceptable salts, solvates or clathrates thereof, wherein:
L, Z and n are defined as in formula (I);
R₃ and R₂₂, for each occurrence, are, independently, selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
R₁, R₂, R₄, R₅ and p are defined as above;and
m and q are each independently, 0 or an integer from 1 to 5.

In this embodiment, the following conditions are met:
1) when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y or Y₁ is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydrortaphthyl, a substituted pyrazinyl, or a substituted or unsubstituted pyridinyl;
2) when X is m-nitrophenyl or m-(trifluoromethyl)phenyl, Y or Y₁ is not a substituted or unsubstituted phenyl; and/or
3) X is not a phenyl that is substituted in the ortho position with -S(O)₂NH₂.

In another embodiment, the invention relates to compounds represented by formula (V): 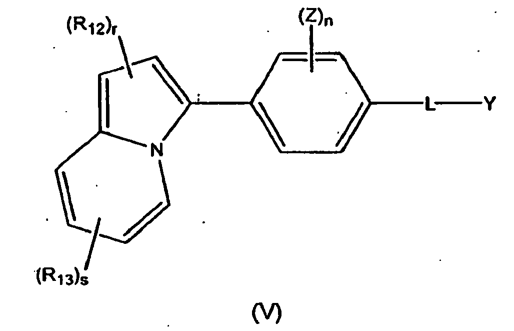 and pharmaceutically acceptable salts, solvates or clathrates thereof, wherein:
Y, L, Z and n are defined as in formula (1);
R₁₂ and R₁₃, for each occurrence, is, independently, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
R₁, R₂, R₄, R₅ and p are defined as above;
r is 0.1 or 2; and
s is 0 or an integer from 1 to 4.

In another embodiment, the invention relates to compounds represented by formula (VI): 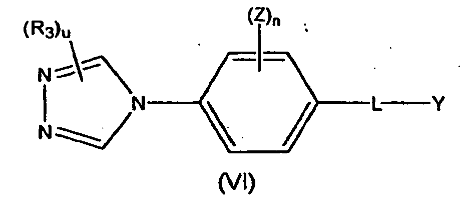 and pharmaceutically acceptable salts, solvates or clathrates thereof, wherein:
Y, Z, L, and n are defined as for formula (I);
R₃, is defined as for formula (IV); and
u is 0, 1, or 2.

A compound of the invention or a pharmaceutically acceptable salt, solvate or clathrate thereof is particularly useful inhibiting immune cell (e.g., T-cells and/or B-cells) activation (e.g., activation in response to an antigen). In particular, a compound of the invention or a pharmaceutically acceptable salt, solvate or clathrate thereof can inhibit the production of certain cytokines that regulate immune cell activation. For example, a compound of the invention or a pharmaceutically acceptable salt, solvate or clathrate thereof can inhibit the production of IL-2, IL-4, IL-5, IL-13, GM-CSF, TNF-α, INF-γ and combinations thereof. Moreover, a compound of the invention or a pharmaceutically acceptable salt, solvate or clathrate thereof can modulate the activity of one or more ion channel involved in activation of immune cells, such as CRAC ion channels, TRPM4 ion channels and Kv1.3 ion channels.

A compound of the invention or a pharmaceutically acceptable salt, solvate or clathrate thereof is particularly useful for immunosuppression or for treating or preventing inflammatory conditions and immune disorders.

The invention also encompasses pharmaceutical compositions comprising an effective amount of a compound of the invention or a pharmaceutically acceptable salt, solvate, clathrate, or prodrug thereof; and a pharmaceutically acceptable carrier or vehicle. These compositions may further comprise additional agents. These compositions are useful for treating or preventing inflammatory conditions and immune disorders.

The invention further encompasses methods for inhibiting immune cell activation, including inhibiting proliferation of t cells and/or B cells, *in vitro* using an effective amount of a compound of the invention or a pharmaceutically acceptable salt, solvate or clathrate thereof or a pharmaceutical composition comprising an effective amount of a compound of the invention or a pharmaceutically acceptable salt, solvate, clathrate, or prodrug thereof.

The invention further encompasses methods for inhibiting cytokine production, (e.g., IL-2, IL-4, IL-5, IL-13, GM-CSF, TNF-α, and/or INF-γ production) *in vitro* using an effective amount of a compound of the invention or a pharmaceutically acceptable salt, solvate or clathrate thereof or a pharmaceutical composition comprising an effective amount of a compound of the invention or a pharmaceutically acceptable salt, solvate or clathrate thereof.

The invention further encompasses methods for modulating ion channel activity (e.g., CRAC, TRPM4, and/or Kv1.3) *in vitro* using an effective amount of a compound of the invention or a pharmaceutically acceptable salt, solvate or clathrate thereof or a pharmaceutical composition comprising an effective amount of a compound of the invention or a pharmaceutically acceptable salt, solvate or clathrate thereof.

All of the methods of this invention may be practiced with a compound of the invention alone, or in combination with other agents, such as other immunosuppressive, anti-inflammatory or immune disorder agents.

### Description of the Drawings

Figure 1 is a graph of % inhibition of I_{CRAC} current in RBL cells and primary human T cells versus concentration of compound 31. Inhibition of I_{CRAC} current by known I_{CRAC} inhibitor, SKF96365, is graphed as a positive control.
Figure 2 is a graph of % inhibition of degranulation in RBL cells by compounds 31 and 66 at various concentrations. The concentration at which 50% of degranulation is inhibited is 0.38µM for compound 31 and 0.43µM for compound 66. Percent inhibition of degranulation at various concentrations of SKF96365 is shown as a positive control.
Figure 3 is a graph of IL-2 production upon stimulation with PMA/ionomycin in blood samples taken from cynomolgus monkeys before and 1, 2, and 4 hours after receiving an IV infusion of CsA (control), compound 31 or compound 75.
Figure 4 is a graph of TNF-α production upon stimulation with PMA/ionomycin in blood samples taken from cynomolgus monkeys before and 1, 2, and 4 hours after receiving an IV infusion of CsA (control), compound 31 or compound 75.
Figure 5 is a graph of IL-2 production upon stimulation with PMA/ionomycin in blood samples taken from cynomolgus monkeys before and 1, 2, and 4 hours after receiving an oral dose of CsA (control), compound 31 or compound 75.
Figure 6 is a graph of TNF-α production upon stimulation with PMA/ionomycin in blood samples taken from cynomolgus monkeys before and 1, 2, and 4 hours after receiving an oral dose of CsA (control), compound 31 or compound 75.

### DETAILED DESCRIPTION OF THE INVENTION

### DEFINITIONS

Unless otherwise specified, the below terms used herein are defined as follows:
As used herein, the term an "aromatic ring" or "aryl" means a monocyclic or polycyclic-aromatic ring or ring radical comprising carbon and hydrogen atoms.
Examples of suitable aryl groups include, but are not limited to, phenyl, tolyl, anthacenyl, fluorenyl, indenyl, azulenyl, and naphthyl, as well as benzo-fused carbocyclic moieties such as 5,6,7,8-tetrahydronaphthyl. An aryl group can be unsubstituted or substituted with one or more substituents (including without limitation alkyl (preferably, lower alkyl or alkyl substituted with one or more halo), hydroxy, alkoxy (preferably, lower alkoxy), alkylthio, cyano, halo, amino, and nitro. In certain embodiments, the aryl group is a monocyclic ring, wherein the ring comprises 6 carbon atoms.

As used herein, the term "alkyl" means a saturated straight chain or branched non-cyclic hydrocarbon typically having from 1 to 10 carbon atoms. Representative saturated straight chain alkyls include methyl, ethyl, n-propyl, n-butyl, n-pentyl, n-hexyl, n-heptyl, n-octyl, n-nonyl and n-decyl; while saturated branched alkyls include isopropyl, sec-butyl, isobutyl, tert-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like. Alkyl groups included in compounds of this invention may be optionally substituted with one or more substituents, such as amino, alkylamino, alkoxy, alkylthio, oxo, halo, acyl, nitro, hydroxyl, cyano, aryl, alkylaryl, aryloxy, arylthio, arylamino, carbocyclyl, carbocyclyloxy, carbocyclylthio, carbocyclylamino, heterocyclyl, heterocyclyloxy, heterocyclylamino, heterocyclylthio, and the like. In addition, any carbon in the alkyl segment may be substituted with oxygen (=O), sulfur (=S), or nitrogen (=NR²³, wherein R²³ is -H, an alkyl, acetyl, or aralkyl). Lower alkyls are typically preferred for the compounds of this invention.

The term alkylene refers to an alkyl group that has at least two points of attachment to at least two moieties (e.g., {-CH₂-}, -{CH₂CH₂-}, 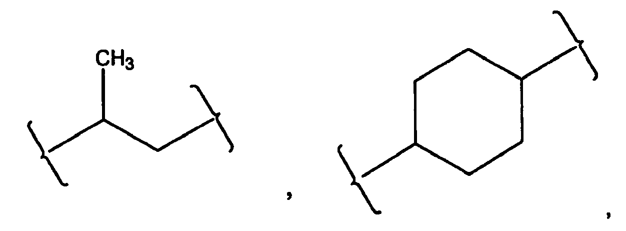 etc., wherein the brackets indicate the points of attachement). Alkylene groups may be substituted or unsubstituted.

An aralkyl group refers to an aryl group that is attached to another moiety via an alkylene linker. Aralkyl groups can be substituted or unsubstituted.

The term "alkoxy," as used herein, refers to an alkyl group which is linked to another moiety though an oxygen atom. Alkoxy groups can be substituted or unsubstituted.

As used herein, the term "alkenyl" means an alkyl radical typically having from 2 to 10 carbon atoms and having at least one carbon-carbon double bond. Representative straight chain and branched alkenyls include vinyl, allyl, 1-butenyl, 2-butenyl, isobutylenyl, 1-pentenyl, 2-pentenyl, 3-methyl-1-butenyl,--methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl, 3-decenyl and the like. Alkenyl groups can be substituted or unsubstituted.

As used herein, the term "alkynyl" means an alkyl radical typically having from 2 to 10 carbon atoms and having at lease one carbon-carbon triple bond. Representative straight chain and branched alkynyls include acetylenyl, propynyl, 1-butynyl, 2-butynyl, 1-pentynyl, 2-pentynyl, 3-methyl-1-butynyl, 4-pentynyl,-1-hexynyl, 2-hexynyl, 5-hexynyl, 1-heptynyl, 2-heptynyl, 6-heptynyl, 1-octynyl, 2-octynyl, 7-octynyl, 1-nonynyl, 2-nonynyl, 8-nonynyl, 1-decynyl, 2-decynyl, 9-decynyl and the like. Alkynyl groups can be substituted or unsubstituted.

As used herein, the term "cycloalkyl" means a saturated cyclic alkyl radical typically having from 3 to 10 carbon atoms. Representative cycloalkyls include cyclopropyl, cyclobutyl, cyclopentyl, cyclohexyl, cycloheptyl, cyclooctyl, cyclononyl, and cyclodecyl. Cycloalkyl groups can be substituted or unsubstituted.

As used herein, the term "bicycloalkyl" means a bi-cyclic alkyl system typically having from 8 to 14 carbon atoms and at least one saturated cyclic alkyl ring. Representative bicyclocycloalkyls include indanyl, 1,2,3,4-tetrahydronaphthyl, 5,6,7,8-tetrahydronaphthyl, perhydronaphthyl and the like. Bicycloalkyl groups can be substituted or unsubstituted.

As used herein, the term "cycloalkenyl" means a cyclic non-aromatic alkyl radical having at least one carbon-carbon double bond in the cyclic system and typically having from 5 to 10 carbon atoms. Representative cycloalkenyls include cyclopentenyl, cyclopentadienyl, cyclohexenyl, cyclohexadienyl, cycloheptenyl, cycloheptadienyl, cycloheptatrienyl, cyclooctenyl, cyclooctadienyl, cyclooctatrienyl, cyclooctatetraenyl, cyclononenyl, cyclononadienyl, cyclodecenyl, cyclodecadienyl and the like. Cycloalkenyl groups can be substituted or unsubstituted.

As used herein, the term "heterocycle" or "heterocyclyl" means a monocyclic heterocyclic ring (typically having 3- to 10-members) which is either a saturated ring or a unsaturated non-aromatic ring. A 3-membered heterocycle can contain up to 3 heteroatoms, and a 4- to 10-membered heterocycle can contain up to 4 heteroatoms. Each heteroatom is independently selected from nitrogen, which can be quaternized; oxygen; and sulfur, including sulfoxide and sulfone. The heterocycle may be attached via any heteroatom or carbon atom. Representative heterocycles include morpholinyl, thiomorpholinyl, pyrrolidinonyl, pyrrolidinyl, piperidinyl, piperazinyl, benzo[1,3]dioxolyl, hydantoinyl, valerolactamyl, oxiranyl, oxetanyl, tetrahydrofuranyl, tetrahydropyranyl, tetrahydropyrindinyl, tetrahydropyrimidinyl, tetrahydrothiophenyl, tetrahydrothiopyranyl, and the like. A heteroatom may be substituted with a protecting group known to those of ordinary skill in the art, for example, the hydrogen on a nitrogen may be substituted with a tert-butoxycarbonyl group. Furthermore, the heterocyclyl may be optionally substituted with one or more substituents (including without limitation a halogen atom, an alkyl radical, or aryl radical). Only stable isomers of such substituted heterocyclic groups are contemplated in this definition. Heterocyclyl groups can be substituted or unsubstituted.

As used herein, the term "heteroaromatic" or "heteroaryl" means a monocyclic or polycyclic heteroaromatic ring (or radical thereof) comprising carbon atom ring members and one or more heteroatom ring members (such as, for example, oxygen, sulfur or nitrogen). In one embodiment, the heteroaromatic ring is selected from 5-8 membered heteroaryl rings. In another embodiment, the heteroaromatic ring is a 5 or 6 membered ring. In another embodiment, the heteroaromatic ring has from 1 to about 4 heteroatoms selected from oxygen, sulfur and nitrogen. Representative heteroaryls include pyridyl, furyl, thiophenyl, pyrrolyl, oxazolyl, imidazolyl, indolizinyl, thiazolyl, isoxazolyl, pyrazolyl, isothiazolyl, pyridazinyl, pyrimidinyl, pyrazinyl, triazinyl, triazolyl, pyridinyl, thiadiazolyl, pyrazinyl, quinolyl, isoquniolyl, indazolyl, benzoxazolyl, benzofuryl, benzothiazolyl, indolizinyl, imidazopyridinyl, isothiazolyl, tetrazolyl, benzimidazolyl, benzoxazolyl, benzothiazolyl, benzothiadiazolyl, benzoxadiazolyl, indolyl, tetrahydroindolyl, azaindolyl, imidazopyridyl, qunizaolinyl, purinyl, pyrrolo[2,3]pyrimidyl, pyrazolo[3,4]pyrimidyl or benzo(b)thienyl and the like. These heteroaryl groups (including indolizinyl when mentioned alone) may be optionally substituted with one or more substituents including (but not limited to amino, alkylamino, alkoxy, alkylthio, oxo, halo, acyl, nitro, hydroxyl, cyano, aryl, alkylaryl, aryloxy, arylthio, arylamino, carbocyclyl, carbocyclyloxy, carbocyclylthio, carbocyclylamino, heterocyclyl, heterocyclyloxy, heterocyclylamino, heterocyclylthio, and the like. Particular heteroaryl substituents include halo and lower alkyl optionally substituted with one or more halo.

A heteroaralkyl group refers to a heteroaryl group that is attached to another moiety via an alkylene linker. Heteroaralkyl groups can be substituted or unsubstituted.

As used herein, the term "halogen" or "halo" means -F, -Cl, -Br or -I.

As used herein, the term "haloalkyl" means an alkyl group in which one or more -H is replaced with a halo group. Examples of haloalkyl groups include -CF₃, -CHF₂, -CCl₃, -CH₂CH₂Br, -CH₂CH(CH₂CH₂Br)CH₃, -CHlCH₃, and the like.

As used herein, the term "haloalkyloxy" means an alkyl group in which one or more -H is replaced with a halo group. Examples of haloalkoxy groups include -OCF₃ and -OCHF₂.

As used herein, the term "lower" refers to a group having up to four atoms. For example, a "lower alkyl" refers to an alkyl radical having from 1 to 4 carbon atoms, and a "lower alkenyl" or "lower alkynyl" refers to an alkenyl or alkynyl radical having from 2 to 4 carbon atoms, respectively. Lower substituents are typically preferred.

Where a particular substituent occurs multiple times in a given structure or moeity, the identity of the substitutent is independent in each case and may be the same as or different from other occurrences of that substituent in the structure or moiety. Furthermore, individual substituents in the specific embodiments and exemplary compounds of this invention are preferred in combination with other such substituents in the compounds of this invention, even if such individual substituents are not expressly noted as being preferred or not expressly shown in combination with other substituents.

The compounds of the invention are defined herein by their chemical structures and/or chemical names. Where a compound is referred to by both a chemical structure and a chemical name, and the chemical structure and chemical name conflict, the chemical structure is determinative of the compound's identity.

Suitable substituents for an alkyl, alkylene, alkenyl, alkynyl, cycloalkyl, cycloalkenyl, heterocyclyl, aryl, aralkyl, heteroaryl, and heteroarylalkyl groups include any substituent which will form a stable compound of the invention. Examples of substituents for an alkyl, alkylene, alkenyl, alkynyl, cycloalkyl, cycloalkenyl, heterocyclyl, aryl, aralkyl, heteroaryl, and heteroarylalkyl include an alkyl, an alkenyl, an alkynyl, an cycloalkyl, an cycloalkenyl, an heterocyclyl, an aryl, an heteroaryl, an aralkyl, an heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂, wherein R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl; and R₄ and R₅ for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;

In addition, alkyl, cycloalkyl, alkylene, a heterocyclyl, and any saturated portion of a alkenyl, cycloalkenyl, alkynyl, aralkyl, and heteroaralkyl groups, may also be substituted with =O, =S, =N-R₄.

When a heterocyclyl, heteroaryl, or heteroaralkyl group contains a nitrogen atom, it may be substituted or unsubstituted. When a nitrogen atom in the aromatic ring of a heteroaryl group has a substituent the nitrogen may be a quaternary nitrogen.

Choices and combinations of substituents and variables envisioned by this invention are only those that result in the formation of stable compounds. The term "stable", as used herein, refers to compounds which possess stability sufficient to allow manufacture and which maintains the integrity of the compound for a sufficient period of time to be useful for the purposes detailed herein (e.g., therapeutic or prophylactic administration to a subject). Typically, such compounds are stable at a temperature of 40°C or less, in the absence of excessive moisture, for at least one week. Such choices and combinations will be apparent to those of ordinary skill in the art and may be determined without undue experimentation.

Unless indicated otherwise, the compounds of the invention containing reactive functional groups (such as (without limitation) carboxy, hydroxy, and amino moieties) also include protected derivatives thereof. "Protected derivatives" are those compounds in which a reactive site or sites are blocked with one ore more protecting groups. Suitable protecting groups for carboxy moieties include benzyl, tert-butyl, and the like. Suitable protecting groups for amino and amido groups include acetyl, tert-butoxycarbonyl, benzyloxycarbonyl, and the like. Suitable proetecting groups for hydroxy include benzyl and the like. Other suitable protecting groups are well known to those of ordinary skill in the art and include those found in T. W. Greene, Protecting Groups in Organic Synthesis, John Wiley & Sons, Inc. 1981, the entire teachings of which are incorporated herein by reference.

As used herein, the term "compound(s) of this invention" and similar terms refers to a compound of any one of formulas (I) through(XVIII) or a pharmaceutically acceptable salt, solvate or clathrate thereof and also include protected derivatives thereof.

Usually, the term "prodrug" means a derivative of a compound that can hydrolyze, oxidize, or otherwise react under biological conditions (*in vitro* or *in vivo*) to provide a compound of this invention. Prodrugs may only become active upon such reaction under biological conditions, but they may have activity in their unreacted forms. Examples of prodrugs include, but are not limited to, analogs or derivatives of compounds of any one of formulas (I) through (XXI) that comprise biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Other examples of prodrugs include derivatives of compounds of any one of formulas (I) through (XXI) that comprise -NO, -NO₂, -ONO, or -ONO₂ moieties. Prodrugs can typically be prepared using well-known methods, such as those described by 1 BURGER'S MEDICINAL CHEMISTRY AND DRUG DISCOVERY (1995) 172-178, 949-982 (Manfred E. Wolff ed., 5th ed).

As used herein and unless otherwise indicated, the terms "biohydrolyzable amide", "biohydrolyzable ester", "biohydrolyzable carbamate", "biohydrolyzable carbonate", "biohydrolyzable ureide" and "biohydrolyzable phosphate analogue" mean an amide, ester, carbamate, carbonate, ureide, or phosphate analogue, respectively, that either: 1) does not destroy the biological activity of the compound and confers upon that compound advantageous properties *in vivo,* such as uptake, duration of action, or onset of action; or 2) is itself biologically inactive but is converted *in vivo* to a biologically active compound. Examples of biohydrolyzable amides include, but are not limited to, lower alkyl amides, α-amino acid amides, alkoxyacyl amides, and alkylaminoalkylcarbonyl amides. Examples of biohydrolyzable esters include, but are not limited to, lower alkyl esters, alkoxyacyloxy esters, alkyl acylamino alkyl esters, and choline esters. Examples of biohydrolyzable carbamates include, but are not limited to, lower alkylamines, substituted ethylenediamines, aminoacids, hydroxyalkylamines, heterocyclic and heteroaromatic amines, and polyether amines.

As used herein, the term "pharmaceutically acceptable salt," is a salt formed from an acid and a basic group of one of the compounds of any one of formulas (I) through (XVIII). Illustrative salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (*i.e*., 1,1'-methylene-bis-(2-hydroxy-3-naphthoate)) salts. The term "pharmaceutically acceptable salt" also refers to a salt prepared from a compound of any one of formulas (I) through(XVIII) having an acidic functional group, such as a carboxylic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl,N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N, N,-di-lower alkyl-N-(hydroxy lower alkyl)-amines, such as N,N-dimethyl-N-(2-hydroxyethyl)amine, or tri-(2-hydroxyethyl)amine; N-methyl-D-glucamine; and amino acids such as arginine, lysine, and the like.

As used herein, the term "pharmaceutically acceptable solvate," is a solvate formed from the association of one or more solvent molecules to one or more molecules of a compound of any one of formulas (I) through (XVIII). The term solvate includes hydrates (*e.g*., hemi-hydrate, mono-hydrate, dihydrate, trihydrate, tetrahydrate, and the like).

As used herein, the term "asthma" means a pulmonary disease, disorder or condition characterized by reversible airway obstruction, airway inflammation, and increased airway responsiveness to a variety of stimuli.

"Immunosuppression" refers to impairment of any component of the immune system resulting in decreased immune function. This impairment may be measured by any conventional means including whole blood assays of lymphocyte function, detection of lymphocyte proliferation and assessment of the expression of T cell surface antigens. The antisheep red blood cell (SRBC) primary (IgM) antibody response assay (usually referred to as the plaque assay) is one specific method. This and other methods are described in Luster, M.I., Portier, C., Pait, D.G., White, K.L., Jr., Gennings, C., Munson, A.E., and Rosenthal, G.J. (1992). "Risk Assessment in Immunotoxicology I: Sensitivity and Predictability of Immune Tests." Fundam. Appl. Toxicol., 18, 200-210. Measuring the immune response to a T-cell dependent immunogen is another particularly useful assay (Dean, J.H., House, R.V., and Luster, M.I. (2001). "Immunotoxicology: Effects of, and Responses to, Drugs and Chemicals." In Principles and Methods of Toxicology: Fourth Edition (A.W. Hayes, Ed.), pp. 1415-1450, Taylor & Francis, Philadelphia, Pennsylvania).

The compounds of this invention may be useful for treating immune disorders. As used herein, the term "immune disorder" and like terms means a disease, disorder or condition caused by the immune system of an animal, including autoimmune disorders. Immune disorders include those diseases, disorders or conditions that have an immune component and those that are substantially or entirely immune system-mediated. Autoimmune disorders are those wherein the animal's own immune system mistakenly attacks itself, thereby targeting the cells, tissues, and/or organs of the animal's own body. For example, the autoimmune reaction is directed against the brain in multiple sclerosis and the gut in Crohn's disease. In other autoimmune disorders such as systemic lupus erythematosus (lupus), affected tissues and organs may vary among individuals with the same disease. One person with lupus may have affected skin and joints whereas another may have affected skin, kidney, and lungs. Ultimately, damage to certain tissues by the immune system may be permanent, as with destruction of insulin-producing cells of the pancreas in Type 1 diabetes mellitus. Specific autoimmune disorders that may be ameliorated using the compounds and methods of this invention include without limitation, autoimmune disorders of the nervous system (e.g., multiple sclerosis, myasthenia gravis, autoimmune neuropathies such as Guillain-Barré, and autoimmune uveitis), autoimmune disorders of the blood (e.g., autoimmune hemolytic anemia, pernicious anemia, and autoimmune thrombocytopenia), autoimmune disorders of the blood vessels (e.g., temporal arteritis, anti-phospholipid syndrome, vasculitides such as Wegener's granulomatosis, and Behcet's disease), autoimmune disorders of the skin (e.g., psoriasis, dermatitis herpetiformis, pemphigus vulgaris, and vitiligo), autoimmune disorders of the gastrointestinal system (e.g., Crohn's disease, ulcerative colitis, primary biliary cirrhosis, and autoimmune hepatitis), autoimmune disorders of the endocrine glands (e.g., Type 1 or immune-mediated diabetes mellitus, Grave's disease. Hashimoto's thyroiditis, autoimmune oophoritis and orchitis, and autoimmune disorder of the adrenal gland); and autoimmune disorders of multiple organs (including connective tissue and musculoskeletal system diseases) (e.g., rheumatoid arthritis, systemic lupus erythematosus, scleroderma, polymyositis, dermatomyositis, spondyloarthropathies such as ankylosing spondylitis, and Sjogren's syndrome). In addition, other immune system mediated diseases, such as graft-versus-host disease and allergic disorders, are also included in the definition of immune disorders herein. Because a number of immune disorders are caused by inflammation, there is some overlap between disorders that are considered immune disorders and inflammatory disorders. For the purpose of this invention, in the case of such an overlapping disorder, it may be considered either an immune disorder or an inflammatory disorder. "Treatment of an immune disorder" usually refers to administering a compound or a composition of the invention to a subject, who has an immune disorder, a symptom of such a disease or a predisposition towards such a disease, with the purpose to cure, relieve, alter, affect, or prevent the autoimmune disorder, the symptom of it, or the predisposition towards it.

As used herein, the term "allergic disorder" means a disease, condition or disorder associated with an allergic response against normally innocuous substances. These substances may be found in the environment (such as indoor air pollutants and aeroallergens) or they may be non-environmental (such as those causing dermatological or food allergies). Allergens can enter the body through a number of routes, including by inhalation, ingestion, contact with the skin or injection (including by insect sting). Many allergic disorders are linked to atopy, a predisposition to generate the allergic antibody IgE. Because IgE is able to sensitize mast cells anywhere in the body, atopic individuals often express disease in more than one organ. Generally, allergic disorders include any hypersensitivity that occurs upon re-exposure to the sensitizing allergen, which in turn causes the release of inflammatory mediators. Allergic disorders include without limitation, allergic rhinitis (e.g., hay fever), sinusitis, rhinosinusitis, chronic or recurrent otitis media, drug reactions, insect sting reactions, latex reactions, conjunctivitis, urticaria, anaphylaxis and anaphylactoid reactions, atopic dermatitis, asthma and food allergies.

The compounds of this invention may be used for treating inflammatory disorders. As used herein, an "inflammatory disorder" means a disease, disorder or condition characterized by inflammation of body tissue or having an inflammatory component. These include local inflammatory responses and systemic inflammation. Examples of such inflammatory disorders include: transplant rejection; chronic inflammatory disorders of the joints, including arthritis, rheumatoid arthritis, osteoarthritis and bone diseases associated with increased bone resorption; inflammatory bowel diseases such as ileitis, ulcerative colitis, Barrett's syndrome, and Crohn's disease; inflammatory lung disorders such as asthma, adult respiratory distress syndrome, and chronic obstructive airway disease; inflammatory disorders of the eye including corneal dystrophy, trachoma, onchocerciasis, uveitis, sympathetic ophthalmitis and endophthalmitis; chronic inflammatory disorders of the gums, including gingivitis and periodontitis; tuberculosis; leprosy; inflammatory diseases of the kidney including uremic complications, glomerulonephritis and nephrosis; inflammatory disorders of the skin including sclerodermatitis, psoriasis and eczema; inflammatory diseases of the central nervous system, including chronic demyelinating diseases of the nervous system, multiple sclerosis, AIDS-related neurodegeneration and Alzheimer's disease, infectious meningitis, encephalomyelitis, Parkinson's disease, Huntington's disease, amyotrophic lateral sclerosis and viral or autoimmune encephalitis; autoimmune disorders, immune-complex vasculitis, systemic lupus and erythematodes; systemic lupus erythematosus (SLE); and inflammatory diseases of the heart such as cardiomyopathy, ischemic heart disease hypercholesterolemia, atherosclerosis); as well as various other diseases with significant inflammatory components, including preeclampsia; chronic liver failure, brain and spinal cord trauma, cancer). There may also be a systemic inflammation of the body, exemplified by gram-positive or gram negative shock, hemorrhagic or anaphylactic shock, or shock induced by cancer chemotherapy in response to pro-inflammatory cytokines, e.g., shock associated with pro-inflammatory cytokines. Such shock can be induced, e.g., by a chemotherapeutic agent used in cancer chemotherapy. "Treatment of an inflammatory disorder" usually refers to administering a compound or a composition of the invention to a subject, who has an inflammatory disorder, a symptom of such a disorder or a predisposition towards such a disorder, with the purpose to cure, relieve, alter, affect, or prevent the inflammatory disorder, the symptom of it, or the predisposition towards it.

An "effective amount" is the quantity of compound in which a beneficial outcome is achieved when the compound is administered to a subject or alternatively, the quantity of compound that possess a desired activity in-vivo or in-vitro. In the case of inflammatory disorders and autoimmune disorders, a beneficial clinical outcome includes reduction in the extent or severity of the symptoms associated with the disease or disorder and/or an increase in the longevity and/or quality of life of the subject compared with the absence of the treatment. The precise amount of compound administered to a subject will depend on the type and severity of the disease or condition and on the characteristics of the subject, such as general health, age, sex, body weight and tolerance to drugs. It will also depend on the degree, severity and type of inflammatory disorder or autoimmune disorder or the degree of immunosuppression sought. The skilled artisan will be able to determine appropriate dosages depending on these and other factors. Effective amounts of the disclosed compounds typically range between about 1 mg/mm² per day and about 10 grams/mm² per day, and preferably between 10 mg/mm² per day and about 1 gram/mm².

The compounds of the invention may contain one or more chiral centers and/or double bonds and, therefore, exist as stereoisomers, such as double-bond isomers (*i.e*., geometric isomers), enantiomers, or diastereomers. According to this invention, the chemical structures depicted herein, including the compounds of this invention, encompass all of the corresponding compounds' enantiomers and stereoisomers, that is, both the stereomerically pure form (*e.g*., geometrically pure, enantiomerically pure, or diastereomerically pure) and enantiomeric, diastereomeric, and geometric isomeric mixtures. In some cases, one enantiomer, diastereomer, or geometric isomer will possess superior activity or an improved toxicity or kinetic profile compared to others. In those cases, such enantiomers, diastereomers, and geometric isomers of a compound of this invention is preferred.

The term "inhibit production of IL-2" and like terms means inhibiting IL-2 synthesis (e.g. by inhibiting transcription (mRNA expression), or translation (protein expression)) and/or inhibiting IL-2 secretion in a cell that has the ability to produce and/or secrete IL-2 (e.g., T lymphocyte). Likewise, the term "inhibiting production of IL-2, IL-4, IL-5, IL-13, GM-CSF, TNF-α or INF-γ means inhibiting the synthesis (e.g. by inhibiting transcription, or translation) and/or inhibiting the secretion in a cell that has the ability to produce and/or secrete these cytokines.

As used herein, a composition that "substantially" comprises a compound means that the composition contains more than about 80% by weight, more preferably more than about 90% by weight, even more preferably more than about 95% by weight, and most preferably more than about 97% by weight of the compound.

As used herein, a composition that is "substantially free" of a compound means that the composition contains less than about 20% by weight, more preferably less than about 10% by weight, even more preferably less than about 5% by weight, and most preferably less than about 3% by weight of the compound.

As used herein, a reaction that is "substantially complete" means that the reaction contains more than about 80% by weight of the desired product, more preferably more than about 90% by weight of the desired product, even more preferably more than about 95% by weight of the desired product, and most preferably more than about 97% by weight of the desired product.

As used herein, a racemic mixture means about 50% of one enantiomer and about 50% of is corresponding enantiomer relative to all chiral centers in the molecule. The invention encompasses all enantiomerically-pure, enantiomerically-enriched, diastereomerically pure, diastereomerically enriched, and racemic mixtures of the compounds of any one of formulas (I) through (XXI).

Enantiomeric and diastereomeric mixtures can be resolved into their component enantiomers or stereoisomers by well known methods, such as chiral-phase gas chromatography, chiral-phase high performance liquid chromatography, crystallizing the compound as a chiral salt complex, or crystallizing the compound in a chiral solvent. Enantiomers and diastereomers can also be obtained from diastereomerically- or enantiomerically-pure intermediates, reagents, and catalysts by well known asymmetric synthetic methods.

When administered to a patient, *e.g*., to a non-human animal for veterinary use or for improvement of livestock, or to a human for clinical use, the compounds of the invention may be administered in isolated form or as the isolated form in a pharmaceutical composition. As used herein, "isolated" means that the compounds of the invention are separated from other components of either (a) a natural source, such as a plant or cell, preferably bacterial culture, or (b) a synthetic organic chemical reaction mixture. Preferably, via conventional techniques, the compounds of the invention are purified. As used herein, "purified" means that when isolated, the isolate contains at least 95%, preferably at least 98%, of a single compound of the invention by weight of the isolate.

Only those choices and combinations of substituents that result in a stable structure are contemplated. Such choices and combinations will be apparent to those of ordinary skill in the art and may be determined without undue experimentation.

The invention can be understood more fully by reference to the following detailed description and illustrative examples, which are intended to exemplify non-limiting embodiments of the invention.

### SPECIFIC EMBODIMENTS

The invention relates to compounds and pharmaceutical compositions that are particularly useful for immunosuppression or to treat or prevent inflammatory conditions and immune disorders.

In one embodiment of the invention compounds of formula (I): 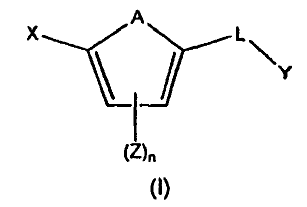 and Pharmaceutical acceptable salts, solvates or clathrates thereof are used in an in vitro method of inhibiting immune all activation, wherein:
X is an optionally substituted phenyl, an optionally substituted triazolyl, an optionally substituted pyridyl, or an optionally substituted indolizinyl;
Y is an optionally substituted aryl or an optionally substituted heteroaryl, which may be optionally substituted with 1-3 substituents selected from halo, C₁₋₄ alkyl, halogenated C₁-C₄ alkyl, or C₁-C₄ alkoxy;
A is -CH=CH-, -CZ=CH- or -CH=CZ-;
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄. -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂- and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted , cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4; and
p is 0, 1, or 2.

In another embodiment, the invention relates to compounds represented by the following structural formula (II): 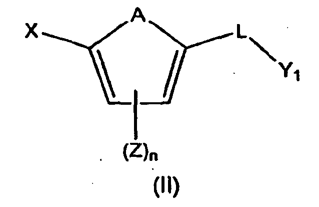 and pharmaceutically acceptable salts, solvates or clathrates thereof wherein:
X, A, Z, L, and n are defined as for formula (I); and
Y, is an optionally substituted aryl or an optionally substituted heteroaryl, which may be optionally substituted with 1-3 substituents independently selected from halo, C₁₋₄ alkyl, halogenated C₁-C₄ alkyl, or C₁-C₄ alkoxy;

In this embodiment, the following conditions are met:
1) Y, is not a heteroaryl that is further substituted with a substituted or unsubstituted aryl or a substituted or unsubstituted heteroaryl;
2) when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y₁ is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydronaphthyl, a substituted pyrazinyl, or a substituted or unsubstituted pyridinyl;
3) when X is m-nitrophenyl or m-(trifluoromethyl)phenyl, Y₁ is not a substituted or unsubstituted phenyl;
4) X is not a phenyl that is substituted in the ortho position with -S(O)₂NH₂; and/or
5) X is not a nitrophenyl when Y₁ is a substituted 1 H-pyrazolyl.

In another embodiment, the invention is related to compounds represented by formula (III): 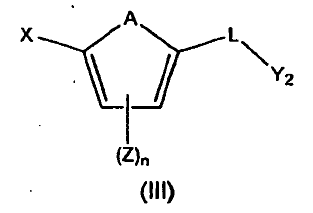 and pharmaceutically acceptable salts, solvates or clathrates thereof wherein:
X, A, Z, L, and n are defined as in formula (I); and
Y₂ is an optionally substituted cycloalkyl, an optionally substituted cycloalkenyl, or an optionally substituted heterocyclyl.

In this embodiment, the following conditions are met:
1) X is not a phenyl that is substituted in the ortho position with -CN or-S(O)₂NH₂;
2) Y₂ is not a 4,5-dihydroisoxazolyl that is further substituted with a substituted or unsubstituted aryl or a substituted or unsubstituted heteroaryl; and/or
3) Y₂ is not a substituted cyclopentenyl.

In another embodiment, the invention relates to compounds represented by formula (IV): 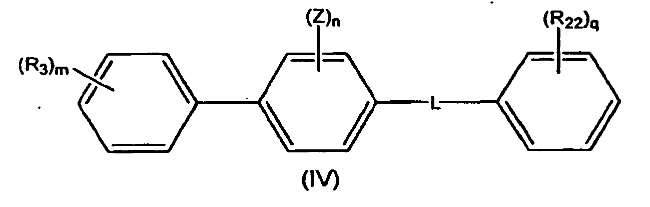 and pharmaceutically acceptable salts, solvates or clathrates thereof, wherein:
L, Z and n are defined as in formula (I);
R₃ and R₂₂, for each occurrence, are, independently, selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
R₁, R₂, R₄, R₅ and p are defined as above;and
m and q are each independently, 0 or an integer from 1 to 5.

In this embodiment, the following conditions are met:
1) when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y or Y₁ is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydronaphthyl, a substituted pyrazinyl, or a substituted or unsubstituted pyridinyl;
2) X is m-nitrophenyl or m-(trifluoromethyl)phenyl, Y or Y₁ is not a substituted or unsubstituted phenyl; and/or
3) X is not a phenyl that is substituted in the ortho position with -S(O)₂NH₂.

In another embodiment, the invention relates to compounds represented by formula (V): 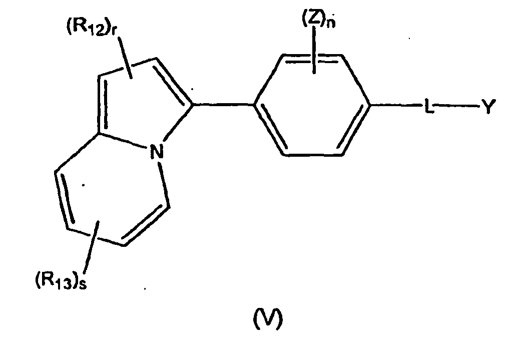 and pharmaceutically acceptable salts, solvates or clathrates thereof, wherein:
Y, L, Z and n are defined as in formula (I);
R₁₂ and R₁₃, for each occurrence, is, independently, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
R₁, R₂, R₄. R₅ and p are defined as above;
r is 0, 1 or 2; and
s is 0 or an integer from 1 to 4.

In another embodiment, the invention relates to compounds represented by formula (VI): 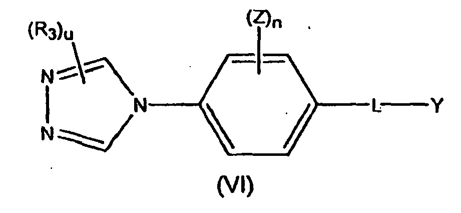 and pharmaceutically acceptable salts, solvates or clathrates thereof, wherein:
Y, Z, L, and n are defined as for formula (I);
R₃, is defined as for formula (IV); and
u is 0, 1, or 2.

In another embodiment, the invention relates to compounds represented by formula (VII): 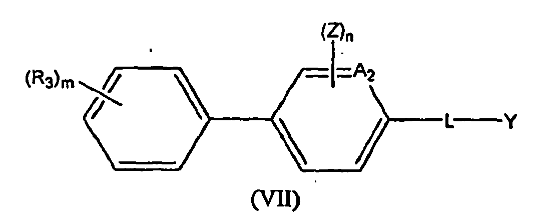 and pharmaceutically acceptable salts, solvates or clathrates thereof, wherein:
Y, Z, L, and n are defined as in formula (I);
A₂ is CH or CZ; and
R₃ and m are defined as in formula (IV).

In another embodiment, the invention relates to compounds represented by formula (VIII): 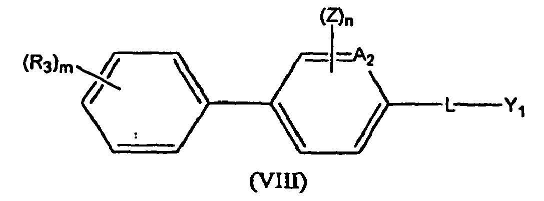 and pharmaceutically acceptable salts, solvates or clathrates thereof, wherein:
L, Z and n are defined as for formula (I);
Y₁ is defined as for formula (II);
R₃ and m are defined as for formula (IV); and
A₂ is defined as for formula (VII).

In another embodiment, the invention relates to compounds represented by formula (IX): 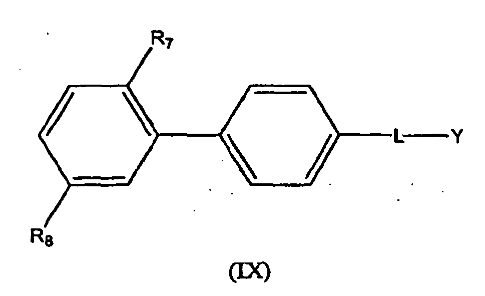 and pharmaceutically acceptable salts, solvates or clathrates thereof, wherein:
Y and L are defined as in formula (I); and
R₇ and R₈ are each, independently, -H, -CF₃, -CN, -C(O)CH₃, -F, -Cl, -OCH₃, -OCH₂CH₃, -C(O)OCH₂CH₃, -SCH₃, -NHCH₃, or C₁-C₄ alkyl, provided that at least one of R₇ or R₈ is not -H.

In another embodiment, the invention relates to compounds represented by formula (X): 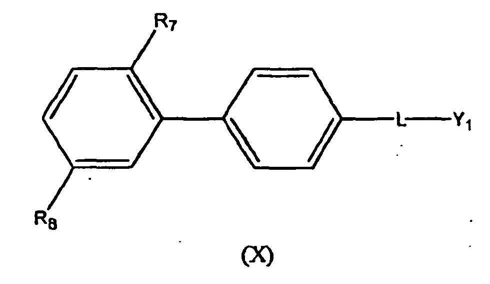 and pharmaceutically acceptable salts, solvates or clathrates thereof, wherein:
L is defined as for formula (I);
Y₁ is defined as for formula (II)
R₇ and R₈ are defined as for formula (IX).

In another embodiment, the invention relates to compounds represented by formula (XI): 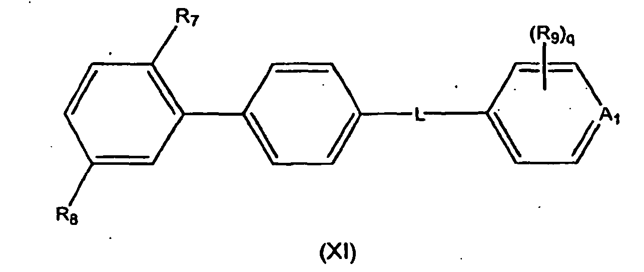 and pharmaceutically acceptable salts, solvates or clathrates thereof, wherein:
L is defined as for formula (I);
R₇ and R₈ are defined as for formula (IX);
A₁ is CH, CR₉, N or N→O;
R₉, for each occurrence, is, independently, halo, C₁-C₄ alkyl, C₁-C₄ haloalkyl, C₁-C₄ alkoxy, C₁-C₄ haloalkoxy, or hydroxyl; and
q is 0 or an integer from 1 to 5.

In another embodiment, the invention relates to compounds represented by formula (XII): 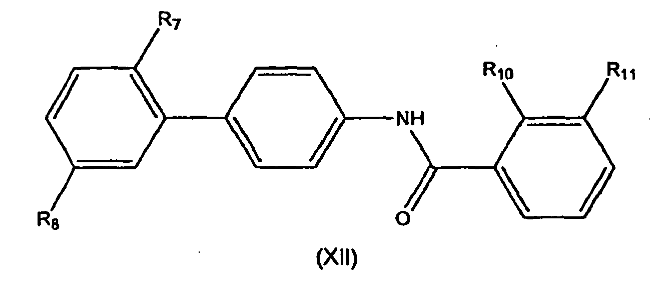 and pharmaceutically acceptable salts, solvates or clathrates thereof, wherein:
R₇ and R₈ are defined as in formula (IX); and
R₁₀ and R₁₁ are each, independently, -F, -Cl, a C₁-C₄ alkyl, a C₁-C₄ haloalkyl, a C₁-C₄ alkoxy or a C₁-C₄ haloalkoxy.

In another embodiment, the invention relates to compounds represented by formula (XIII): 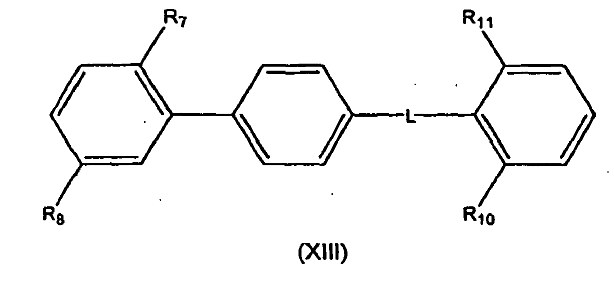 and pharmaceutically acceptable salts, solvates or clathrates thereof, wherein:
L is defined as for formula (I);
R₇ and R₈ are defined as for formula (IX); and
R₁₀ and R₁₁ are defined as in formula (XII).

In one embodiment, the invention relates to compounds represented by formula (XIV): 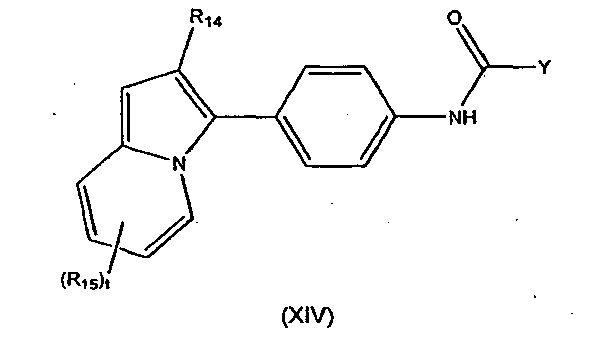 and pharmaceutically acceptable salts, solvates or clathrates thereof, wherein:
Y is defined as for formula (I):
R₁₄ and R₁₅ are each, independently, -CF₃, -OCH₃, -F,-Cl, or -C(O)OCH₃;
   and
t is 0, 1 or 2.

In another embodiment, the invention relates to compounds represented by formula (XV): 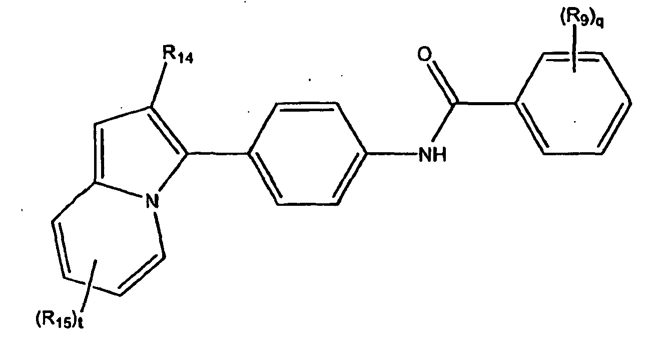 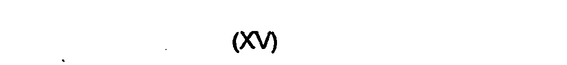 and pharmaceutically acceptable salts, solvates or clathrates thereof, wherein:
R₉ and q are defined as for formula (XI); and
R₁₄, R₁₅ and t are defined as for formula (XIV).

In another embodiment, the invention relates to compounds represented formula (XVI): 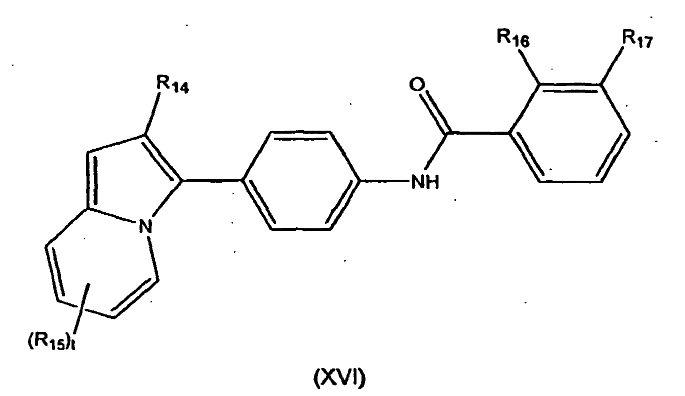 and pharmaceutically acceptable salts, solvates or clathrates thereof, wherein:
R₁₄, R₁₅, and t are defined as for formula (XIV); and
R₁₆ and R₁₇ are each, independently, -F or -OCH₃.

In another embodiment, the invention relates to compounds represented by formula (XVII): 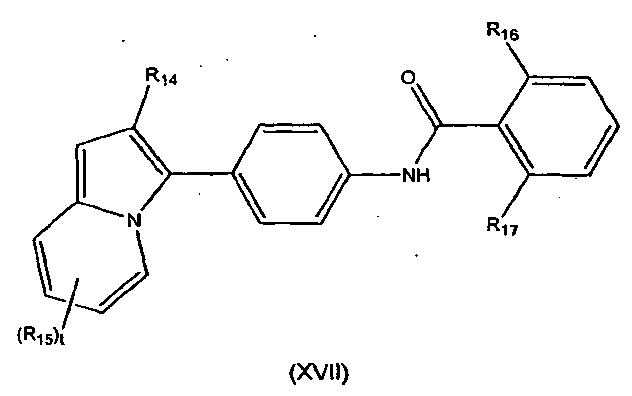 and pharmaceutically acceptable salts, solvates or clathrates thereof, wherein:
R₁₄, R₁₅, and t are defined as for formula (XIV); and
R₁₆ and R₁₇ are defined as for formula (XVI).

In another embodiment, the invention relates to compounds represented by formula (XVIII): 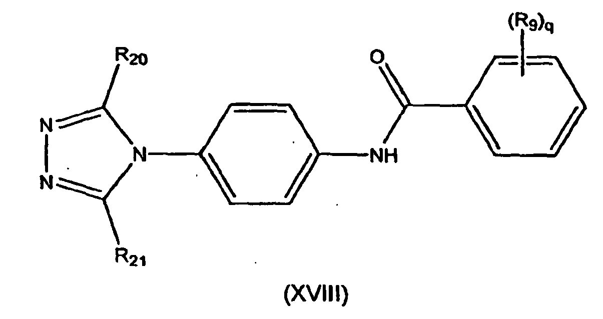 and pharmaceutically acceptable salts, solvates or clathrates thereof, wherein:
R₉ and q are defined as for formula (XI); and
R₂₀ and R₂₁ are each, independently, -H, -F, -Cl, a lower alkyl, thiophenyl, -OCH₃, -CF₃, or -OCF₃;

A different class of compounds is represented by formula (XIX): 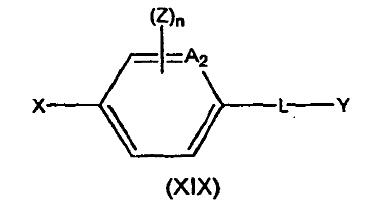 and pharmaceutically acceptable salts, solvates or clathrates thereof, wherein:
X, Y, L, Z, and n are defined as for formula (I); and
A₂ is defined as for formula (VII).

Further different compounds are represented by formula (XX): 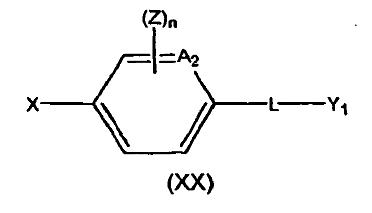 and pharmaceutically acceptable salts, solvates or clathrates thereof, wherein:
X, L, Z, and n are defined as for formula (I);
Y₁ is defined as in formula (II); and
A₂ is defined as for formula (VII).

The above-described compounds are represented by formula (XX), as defined above, wherein one or more (for example, all) of
the following conditions are met:
1) Y₁ is not a heteroaryl that is further substituted with a substituted or unsubstituted aryl or a substituted or unsubstituted heteroaryl;
2) when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydronaphthyl, a substituted pyrazinyl, or a substituted or unsubstituted pyridinyl;
3) when X is m-nitrophenyl or m-(trifluoromethyl)phenyl, Y₁ is not a substituted or unsubstituted phenyl;
4) X is not a phenyl that is substituted in the ortho position with -S(O)₂NH₂; and/or
5) X is not a nitrophenyl when Y₁ is a substituted 1*H*-pyrazolyl.

Other different compounds are represented by formula (XXI): 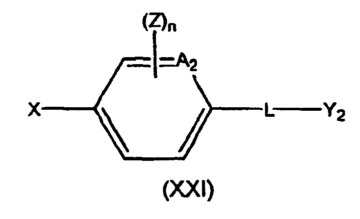 and pharmaceutically acceptable salts, solvates, clathrates, and prodrugs thereof, wherein:
X, L, Z, and n are defined as for formula (I);
Y₂ is defined as in formula (III); and
A₂ is defined as for formula (VII).

The above-referenced compounds are represented by formula (XXI), as defined above, wherein one or more (for example, all) of the following conditions are met:
1) X is not a phenyl that is substituted in the ortho position with -CN or -(S(O)₂NH₂;
2) Y₂ is not a 4,5-dihydroisoxazlyl that is further substituted with a substituted or unsubstituted aryl or a substituted or unsubstituted heteroaryl; and/or
3) Y₂ is not a substituted cyclopentenyl.
   In another embodiment, the invention relates to compounds selected from the following group:
   3-Fluoro-N-(2'-trifluoromethyl- biphenyl-4-yl)-isonicotinamide;
   3-Fluoro-N-(2'-methyl-biphenyl-4-yl)-isonicotinamidei
   3-Fluoro-N-(3'-trifluoromethyl- biphenyl-4-yl)-isonicotinamide;
   N-(2,2'-Bis-trifluoromethyl- biphenyl-4-yl)-2,3-difluoro- benzamide;
   N-[4-(1,2-Dimethyl-but-1-enyl)-3-trifluoromethyl-phenyl]-2,3-difluorobenzamide;
   4'-(2,3-Difluoro-benzoylamino)-biphenyl-2-carboxylic acid dimethylamide;
   N-(2'-Trifluoromethyl-biphenyl-4-yl)-nicofinamide;
   N-(2'-Trifluoromethyl-biphenyl-4-yl)-isonicotinamide;
   Thiophene-2-carboxylic acid (2'-trifluoromethyl-biphenyl-4- yl)-amide;
   4-Fluoro-N-(2'-trifluoromethyl- biphenyl-4-yl)-benzamide;
   2,4-Dimethyl-thiazole-5- carboxylic acid (2'-trifluoromethyl-biphenyl-4- yl)-amide;
   4-Trifluoromethyl-N- (2'-trifluoromethyl-biphenyl-4- yl)-nicotinamide;
   2-Methyl-5-trifluoromethyl- oxazole-4-carboxylic acid (2'-trifluoromethyl-biphenyl- 4-yl)-amide;
   2-Ethyl-5-methyl-2H-pyrazole-3-carboxylic acid (2'-trifluoromethyl-biphenyl-4- yl)-amide;
   2,3-Difluoro-N-(2'-trifluoromethyl-biphenyl-4-yl)- benzamide;
   2,5-Difluoro-N-(2'-trifluoromethyl-biphenyl-4-yl)- benzamide;
   2,3-Difluoro-N-(3-fluoro-2'- trifluoromethyl-biphenyl-4-yl)-benzamide;
   N-(2',5'-Bis-trifluoromethyl- biphenyl-4-yl)-2,3-difluoro-benzamide;
   2,3-Difluoro-N-(2'-fluoro-5'-trifluoromethyl-biphenyl-4-yl)-benzamide;
   2,3-Difluoro-N-(4'-fluoro-2'- trifluoromethyl-biphenyl-4-yl)-benzamide;
   2,3-Difluoro-N-[4-(2- trifluoromethyl-indolizin-3-yl)- phenyl]-benzamide;
   2,3-Difluoro-N-(2'-fluoro-6'- trifluoromethyl-biphenyl-4-yl)-benzamide;
   2,3-Difluoro-N-(2'-chloro-5'- trifluoromethyl-biphenyl-4-yl)-benzamide;
   4-Methyl-[1,2,3]thiadiazole-5- carboxylic acid (2',5'-bis-trifluoromethyl- biphenyl-4-yl)-amide;
   Pyridine-2-carboxylic acid (2'-trifluoromethyl-biphenyl-4- yl)-amide;
   Pyrazine-2-carboxylic acid (2'-trifluoromethyl-biphenyl-4- yl)-amide;
   4-Methyl-[1,2,3]thiadiazole-5- carboxylic acid (2'-chloro-5'-trifluoromethyl-biphenyl-4-yl)-amide;
   N-(2',5'-Bis-trifluoromethyl- biphenyl-4-yl)-2,5-difluoro- benzamide;
   N-(2',5'-Dichloro-biphenyl-4-yl)-2,3-difluoro-benzamide;
   N-(5'-Cyano-2'-methoxy- biphenyl-4-yl)-2,3-difluoro-benzamide;
   N-(2',5'-Dimethoxy-biphenyl-4- yl)-2,3-difluoro-benzamide;
   N-[4-(3,5-Bis-trifluoromethyl- [1,2,4]triazol-4-yl)-phenyl]-2,3-difluoro-benzamide;
   3-Methyl-thiophene-2-carboxylic acid-(4-(3,5-bis-trifluoromethyl-[1,2,4]triazol-4-yl)-phenyl)-amide;
   N-[4-(3-trifluoromethyl-5-(thiophen-4-yl)-[1,2,4]triazol-4-yl)-phenyl]-2,3-difluoro-benzamide;
   N-[4-(3-trifluoromethyl-5-(thiophen-4-yl)-[1,2,4]triazol-4-yl)-phenyl]-2,3-difluoro-benzamide;
   N-[4-(3-trifluoromethyl-indolizin-3-yl)-phenyl]- 2,3-difluoro-benzamide;
   N-[4-(3-cyano-5-trifluoromethyl-pyrid-2-yl)-phenyl]-2,3-difluoro-benzamide;
   N-(2',5'-bis-trifluoromethyl-biphenyl-4-yl)-2-methoxy-benzamide;
   5-Methyl-isoxazol-3-carboxylic acid (2',5'-bis-trifluoromethyl-biphenyl-4-yl)-amide;
   1,3-Dimethyl-1H-pyrazol-5-carboxylic acid (2',5'-bis-trifluoromethyl-biphenyl-4-yl)-amide;
   [1,2,3]-Thiadiazole-4-carboxylic acid (2',5'-bis-trifluoromethyl-biphenyl-4-yl)-amide;
   Isoxazole-5-carboxylic acid (2',5'-bis-trifluoromethyl-biphenyl- 4-yl)-amide;
   3,5-dimethylisoxazole-4-carboxylic acid (2',5'-bis-trifluoromethyl-biphenyl-4-yl)-amide;
   N-(2'-methoxy-5'-chloro -biphenyl-4-yl)-2,3-difluoro-benzamide;
   N-(2',5'-bis-trifluoromethyl-biphenyl-4-yl)-2-methoxybenzamide;
   N-(2'-methoxy-5'-methyl-biphenyl-4-yl)-2,3-difluorobenzamide;
   N-(2',5'-dimethyl-biphenyl-4-yl)-2,3-difluorobenzamide;
   3-methylisoxazole-4-carboxylic acid (2',5'-bis-trifluoromethyl-biphenyl- 4-yl)-amide;
   N-(2',5'-bis-trifluoromethyl-biphenyl-4-yl)-2-hydroxybenzamide;
   N-(2'-methoxy-5'-acetyl- biphenyl-4-yl)-2,3-difluorobenzamide;
   5-methylisoxazole-4-carboxylic acid (2',5'-bis-trifluoromethyl-biphenyl-4- yl)-amide;
   N-(2',4',5'-trimethyl- biphenyl-4-yl)-2,3-difluorobenzamide;
   N-(2',5'-bis-trifluoromethyl- biphenyl-4-yl)-2,3-dimethylbenzamide;
   N-(2',5'-bis-trifluoromethyl- biphenyl-4-yl)-2-methyl-3-chlorobenzamide;
   N-(2',5'-bis-trifluoromethyl- biphenyl-4-yl)-2-methyl-3-fluorobenzamide;
   N-(2',5'-bis-trifluoromethyl-biphenyl-4-yl)-2-methyl-3-methoxybenzamide;
   4-methyl-[1,2,3]-thiadiazole-5-carboxylic acid (2',5'-dimethoxy biphenyl-4-yl)-amide;
   N-(2',5'-dimethoxy- biphenyl-4-yl)-2-methylbenzamide;
   2-methyl-pyridine-3-carboxylic acid (2',5'-bis-trifluoromethyl-biphenyl- 4-yl)-amide;
   2,3-dihydro-benzo[1,4]dioxine-5-carboxylic acid (2',5'-bis-trifluoromethyl-biphenyl-4-yl)-amide;
   1-methyl-1H-imidazole-5-carboxylic acid (2',5'-bis-trifluoromethyl-biphenyl- 4-yl)-amide;
   3-methyl-pyridine-4-carboxylic acid (2',5'-dimethoxy-biphenyl- 4-yl)-amide;
   3-methyl-pyridine-4-carboxylic acid (2',5'-bis-trifluoromethyl-biphenyl- 4-yl)-amide;
   3-methyl-pyridine-carboxylic acid (2'-methoxy-5'-chlorobiphenyl-4-yl)-amide;
   3-fluoro-pyridine-4-carboxylic acid (2',5'-dimethoxybiphenyl- 4-yl)-amide;
   3-fluoro-pyridine-4-carboxylic acid (2'-methoxy-5'-chlorobiphenyl-4-yl)- amide;
   3-fluoro-pyridine-4-carboxylic acid (2',5'-bis-trifluoromethylbiphenyl-4-yl)-amide;
   3-methyl-pyridine-4-carboxylic acid (2'-methoxy-5'-methylbiphenyl-4-yl)-amide;
   3-methyl-pyridine-4-carboxylic acid (2',5'-dimethylbiphenyl-4-yl)-amide;
   4-methyl-[1,2,3]-thiadiazole-5-carboxylic acid (2'-methoxy-5'-acetylbiphenyl- 4-yl)-amide;
   3-fluoro-pyridine-4-carboxylic acid (2'-difluoromethoxy- 5'-chlorobiphenyl- 4-yl)-amide;
   4-methyl-[1,2,3]-thiadiazole-5-carboxylic acid {2'-(N,N-dimethylamino)- 5'-trifluoromethoxybiphenyl-4-yl}-amide;
   3-methyl-pyridine-4-carboxylic acid (2'-chloro-5'-trifluoromethylbiphenyl- 4-yl)-amide;
   3-methyl-pyridine-4-carboxylic acid (2'-methylsulfanyl-biphenyl- 4-yl)-amide;
   3-methyl-pyridine-4-carboxylic acid (2'-ethyl-biphenyl-4-yl)-amide;
   3-methyl-pyridine-4-carboxylic acid (2'-isopropyl-biphenyl-4-yl)-amide;
   N-{5-(2',5'-dimethoxyphenyl)- pyrid-2-yl}-2-methylbenzamide;
   3-methyl-pyridine-4-carboxylic acid (2',5'-diethylbiphenyl-4-yl)-amide;
   3-methyl-pyridine-4-carboxylic acid {2'-(N,N-dimethylamino)-5'-methoxybiphenyl-4-yl}-amide;
   3-methyl-pyridine-4-carboxylic acid {2'-(N-dimethylamino)-5'-carbethoxybiphenyl-4-yl}-amide;
   3-methyl-pyridine-4-carboxylic acid (2'-ethoxy-5'-chlorobiphenyl- 4-yl)-amide;
   N-(2'-dimethoxy-5'-chloro biphenyl-4-yl)-2,6-difluorobenzamide;
   N-(2'-methoxy-5'-chloro- biphenyl-4-yl)-2,4,5-trifluorobenzamide;
   N-(2',5'-bis-trifluoromethyl biphenyl-4-yl)-2,6-difluorobenzamide;
   N-(2'-chloro-5'-trifluoromethyl biphenyl-4-yl)-2,6-difluorobenzamide;
   N-(2',5'-dimethyl biphenyl-4-yl)-2,6-difluorobenzamide;
   N-(2',5'-dichloro biphenyl-4-yl)-2,6-difluorobenzamide;
   2,3-Difluoro-N-[4-(2-trifluoromethyl-indolizin-3-yl)-phenyl]-benzamide;
   2,5-Difluoro-N-[4-(2-trifluoromethyl-indolizin-3-yl)-phenyl]-benzamide;
   3,4-dimethoxy-N-[4-(2-trifluoromethyl-indolizin-3-yl)-phenyl]-benzamide;
   N-[4-(5-chloro-2-trifluoromethyl-indolizin-3-yl)-phenyl]-2,3-difluoro-benzamide;
   5-chloro-3-[4-(2,3-difluoro-benzoylamino)-phenyl]-2-trifluoromethyl-indolizine-6-carboxylic acid methyl ester;
   3-[4-(2,3-difluoro-benzoylamino)-phenyl]-2-trifluoromethyl-indolizine-6-carboxylic acid methyl ester,
   2,3-difluoro-N-[4-(6-methoxy-2-trifluoromethyl-indolizin-3-yl)-phenyl]- benzamide;
   N-[4-(5-fluoro-2-trifluoromethyl-indolizin-3-yl)-phenyl]-2,3-difluoro-benzamide;
   N-[4-(5-methoxy-2-trifluoromethyl-indolizin-3-yl)-phenyl]-2,3-difluoro- benzamide;
   N-[4-(5-Chloro-2,7-bis-trifluoromethyl-indolizin-3-yl)-phenyl]-2,3-difluoro-benzamide;
   5-methoxy-3-[4-(2,3-difluoro-benzoylamino)-phenyl]-2-trifluoromethyl- indolizine-6-carboxylic acid methyl ester;
   2,3-difluoro-N-[4-(8-methoxy-2-trifluoromethyl-indolizin-3-yl)-phenyl]- benzamide;
   5-Chloro-3-[4-(2,3-difluoro-benzoylamino)-phenyl]-2-trifluoromethyl-indolizine-7-carboxylic acid methyl ester;
   5-Chloro-3-[4-(2,6-difluoro-benzoylamino)-phenyl]-2-trifluoromethyl-indolizine-7-carboxylic acid methyl ester;
   2,6-difluoro-N-[4-(8-methoxy-2-trifluoromethyl-indolizin-3-yl)-phenyl]- benzamide;
   5-Methoxy-3-[4-(2,6-difluoro-benzoylamino)phenyl]-2-trifluoromethyl- indolizine-6-carboxylic acid methyl ester;
   N-[4-(5-chloro-2,7-bis-trifluoromethyl-indolizin-3-yl)-phenyl]- 2,6-difluoro-benzamide;
   N-[4-(5-methoxy-2-trifluoromethyl-indolizin-3-yl)-phenyl]-2,6-difluoro- benzamide;
   N-[4-(5-fluoro-2-trifluoromethyl-indolizin-3-yl)-phenyl]-2,6-difluoro-benzamide;
   2,6-difluoro-N-[4-(6-methoxy-2-trifluoromethyl-indolizin-3-yl)-phenyl]- benzamide;
   3-[4-(2,6-difluoro-benzoylamino)-phenyl]-2-trifluoromethyl-indolizine-6- carboxylic acid methyl ester;
   5-Chloro-3-[4-(2,6-difluoro-benzoylamino)-phenyl]-2-trifluoromethyl-indolizine-6-carboxylic acid methyl ester;
   N-[4-(5-Chloro-2-trifluoromethyl-indolizin-3-yl)-phenyl]-2,6-difluoro-benzamide;
   N-[4-(5-Chloro-2-trifluoromethyl-indolizin-3-yl)-phenyl]-2,4,5-trifluoro- benzamide;
   5-Chloro-3-[4-(2,4,5-trifluoro-benzoylamino)-phenyl]-2-trifluoromethyl- indolizine-6-carboxylic acid methyl ester;
   3-[4-(2,4,5-trifluoro-benzoylamino)-phenyl]-2-trifluoromethyl-indolizine-6- carboxylic acid methyl ester,
   2,4,5-trifluoro-N-[4-(5-fluoro-2-trifluoromethyl-indolizin-3-yl)-phenyl]-benzamide;
   2,4,5-trifluoro-N-[4-(6-methoxy-2-trifluoromethyl-indolizin-3-yl)-phenyl]- benzamide;
   2,4,5-trifluoro-N-[4-(5-methoxy-2-trifluoromethyl-indolizin-3-yl)-phenyl]- benzamide;
   N-[4-(5-Chloro-2,7-bis-trifluoromethyl-indolizin-3-yl)-phenyl]- 2,4,5-trifluoro-benzamide;
   5-Methoxy-3-[4-(2,4,5-trifluoro-benzoylamino)-phenyl]-2-trifluoromethyl-indolizine-6-carboxylic acid methyl ester;
   2,4,5-trifluoro-N-[4-(8-methoxy-2-trifluoromethyl-indolizin-3-yl)-phenyl]- benzamide;
   5-Chloro-3-[4-(2,4,5-trifluoro-benzoylamino)-phenyl]-2-trifluoromethyl- indolizine-7-carboxylic acid methyl ester;
   2,4,5-trifluoro-N-[4-(7-methoxy-2-trifluoromethyl-indolizin-3-yl)-phenyl]-benzamide;
   2,6-difluoro-N-[4-(7-methoxy-2-trifluoromethyl-indolizin-3-yl)-phenyl]-benzamide;
   2,3-difluoro-N-[4-(7-methoxy-2-trifluoromethyl-indolizin-3-yl)-phenyl]- benzamide;
   N-(2',5'-dimethoxy-biphenyl-4-yl)-2,6-difluoro-benzamide;
   N-(2'-trifluoromethyl-5'-methyl-biphenyl-4-yl)-2,6-difluoro-benzamide;
   N-(2',5'-bis-trifluoromethyl-biphenyl-4-yl)-2,6-difluoro-benzyl amine;
   N-(2',5'-bis-trifluoromethyl-biphenyl-4-yl)-2,6-difluoro-benzyl amine HCl salt;
   N-(2'-methoxy-5'-chloro-biphenyl-4-yl)-2,6-difluoro-benzyl amine;
   N-(2'-methoxy-5'-chloro-biphenyl-4-yl)-2,6-difluoro-benzyl amine HCl salt;
   N',N'-diethyl-N-(2',5'-bis-trifluoromethyl biphenyl-4-yl) urea;
   2,3-difluoro-N-[4-(2-trifluoro-methyl-indolizin-3-yl)-phenyl]-benzamide;
   4-methyl-N-[4-(2-methyl-indolizin-3-yl)-phenyl]-[1,2,3]thiadiazole 5-carboxylic acid amide; and
      pharmaceutically acceptable salts, solvates or clathrates thereof.

Particular compounds of any one of formulas (II) through (XVIII) include those wherein:
A is -CH=CH-, -CZ=CH- or -CH=CZ-;
L is a linker selected from the group consisting of -NHC(O)- or -NHCH₂-;
Y or Y₁ is an optionally substituted 5 or 6 membered aryl or an optionally substituted 5 or 6 membered heteroaryl; or Y or Y₁ is an optionally substituted phenyl, an optionally substituted pyridyl, an optionally substituted thiophenyl, [1,2,3]thiadiazolyl, an optionally substituted isoxazolyl, pyrazolyl, quinolinyl, imidazolyl, or 2,3-dihydrobenzo[1,4]dioxine; or Y or Y₁ is an optionally substituted phenyl, an optionally substituted pyridyl, or an optionally substituted [1,2,3]thiadiazolyl; or Y or Y₁ is optionally substituted aryl or optionally substituted heteroaryl (e.g., phenyl, thiadiazolyl, pyridyl, thienyl, thiazolyl, oxazolyl, isoxazolyl, quinolinyl, imidazolyl, pyrazolyl or pyrazinyl), any of which may be optionally substituted with 1-3 (e.g., 1-2) substituents independently selected from halo (e.g., F or Cl), C₁-C₄ alkyl (e.g., methyl or ethyl), halogenated C₁-C₄ alkyl (e.g., CF₃), or C₁-C₄ alkoxy; or Y or Y₁ is selected from the group consisting of: 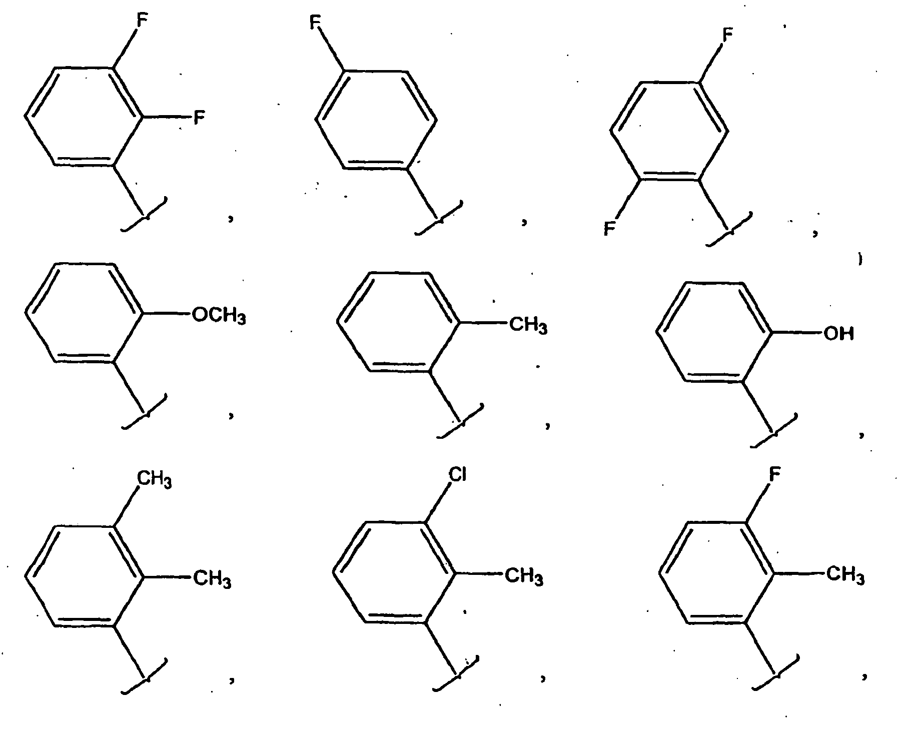 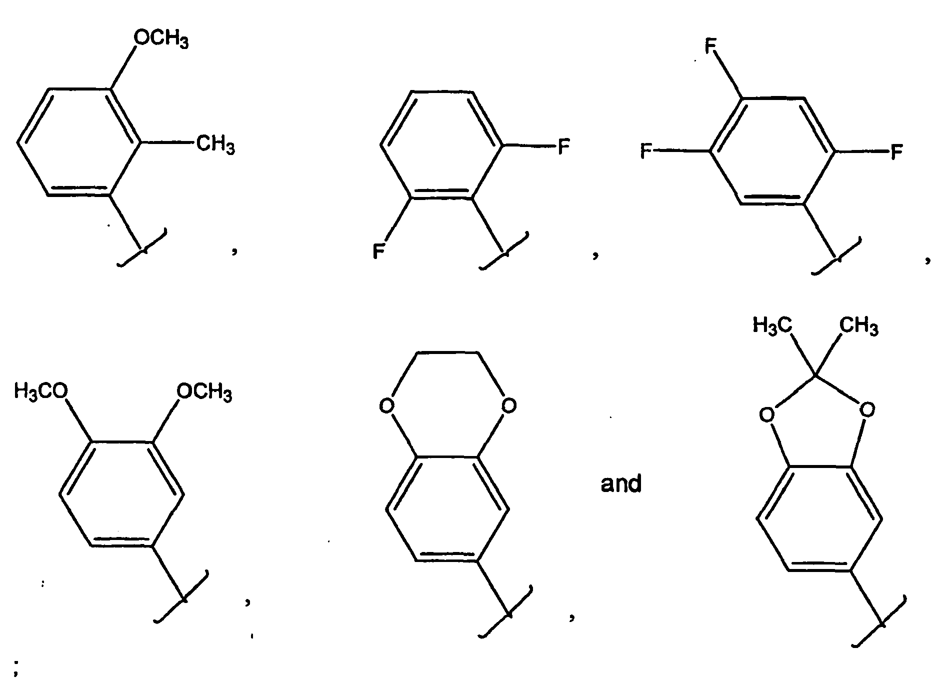
or Y or Y₁ is selected from the group consisting of: 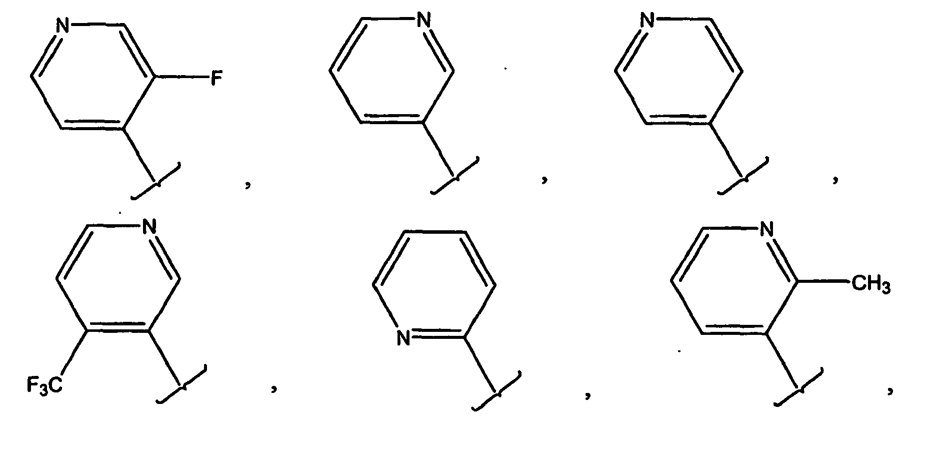 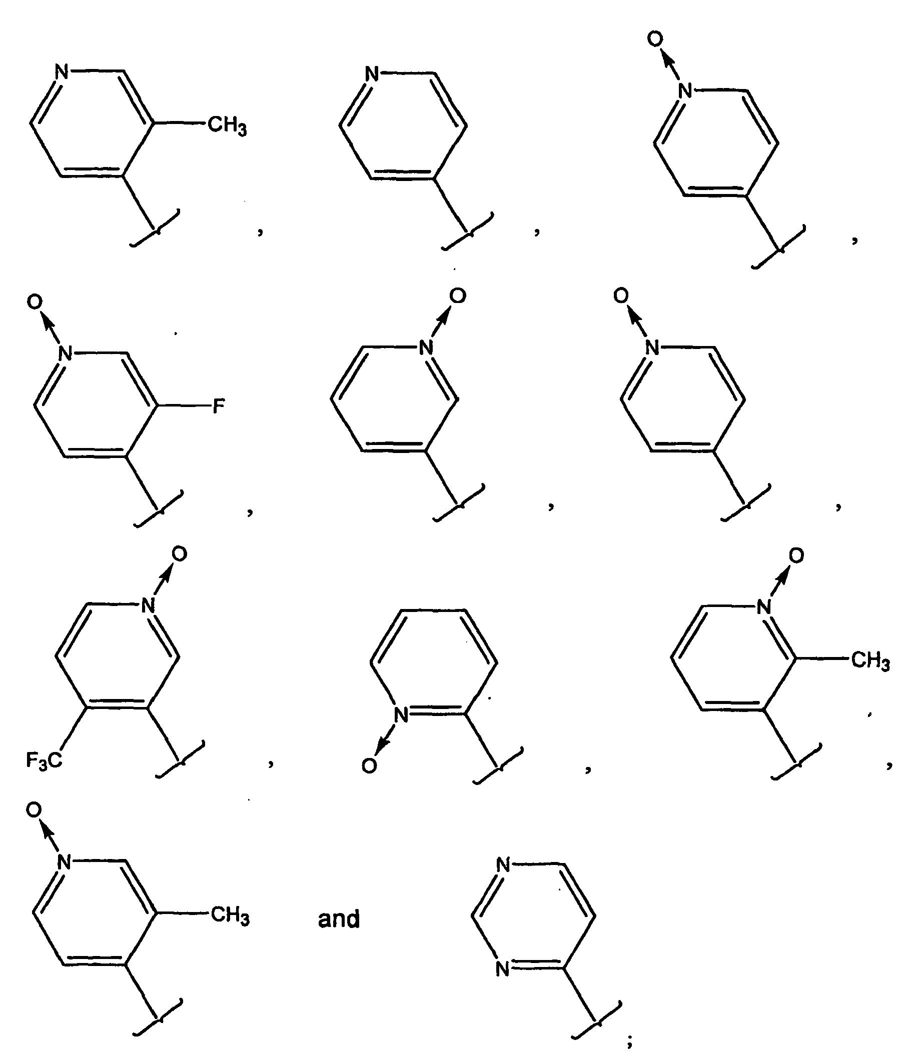
or Y or Y₁ is selected from the group consisting of: 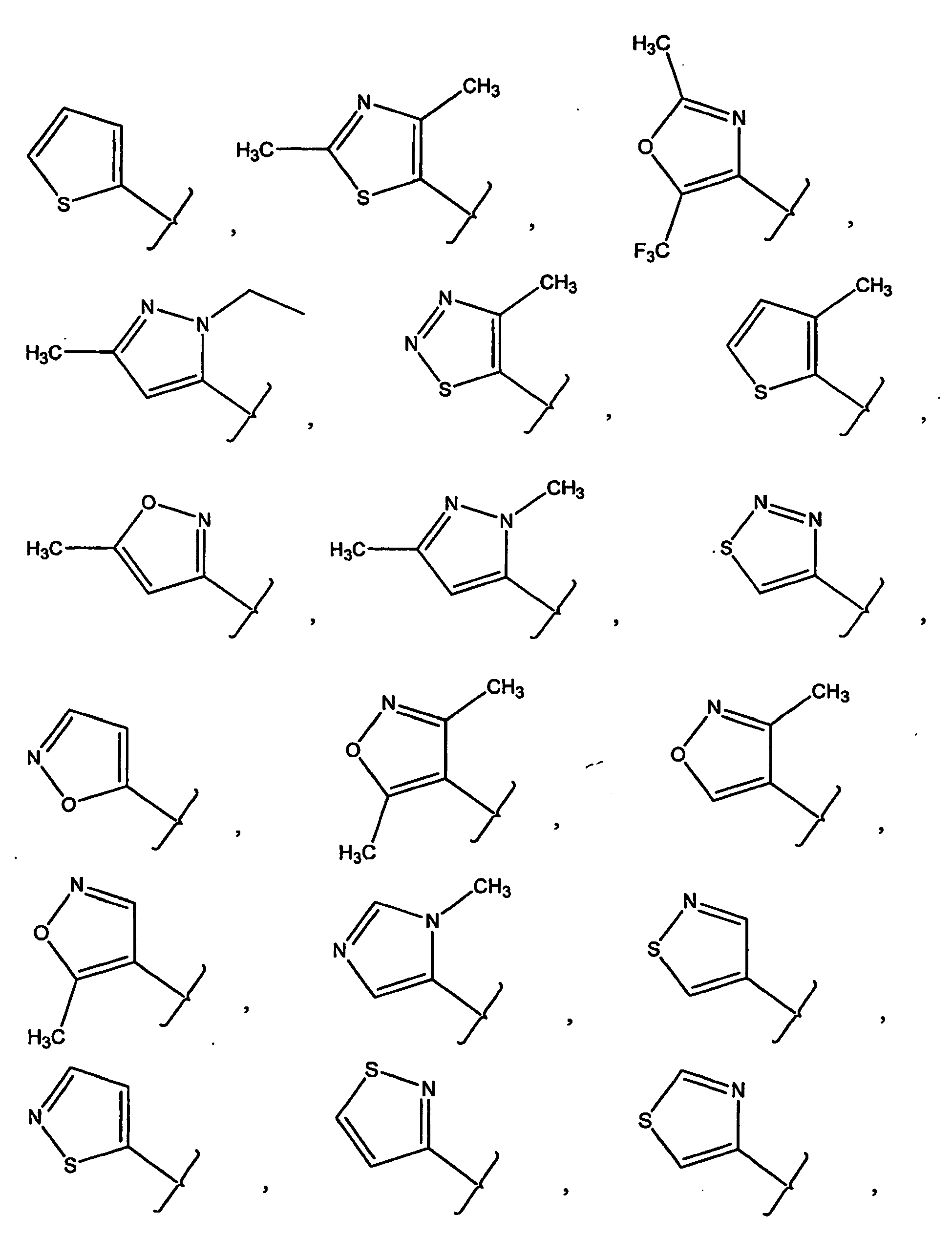 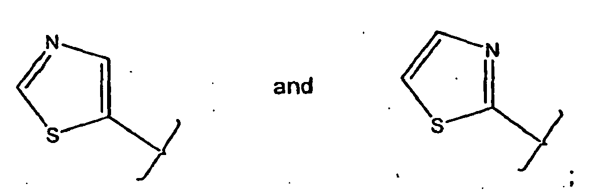
X is phenyl, triazolyl, or indolizinyl, any of which may be optionally substituted with 1-3 substituents independently selected from the group consisting of halo, C₁-C₄ alkyl, halogenated C₁-C₄ alkyl, C₁-C₄ alkoxy, acetyl, a C₁-C₄ mono- or di-alkyl amino, a C₁-C₄ alkyl sulfanyl, and a C₁-C₄ alkyl ester (e.g., -C(O)OCH₂CH₃); or X is a phenyl, triazolyl, or indolizinyl that is optionally substituted with 1-2 substituents independently selected from halo (e.g., F or Cl), C₁-C₄ alkyl (e.g., methyl or ethyl), halogenated C₁-C₄ alkyl (e.g., CF₃), C₁-C₄ alkoxy, acetyl, a C₁-C₄ mono- or di-alkyl amino, a C₁-C₄ alkyl sulfanyl, and a C₁-C₄ alkyl ester (e.g., -C(O)OCH₂CH₃); or X is selected from the group consisting of: 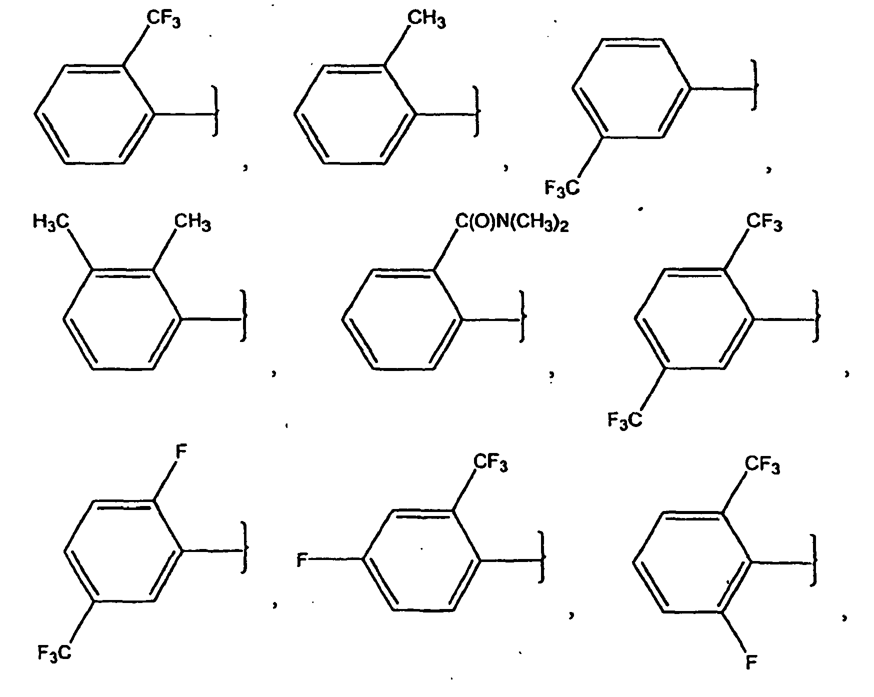 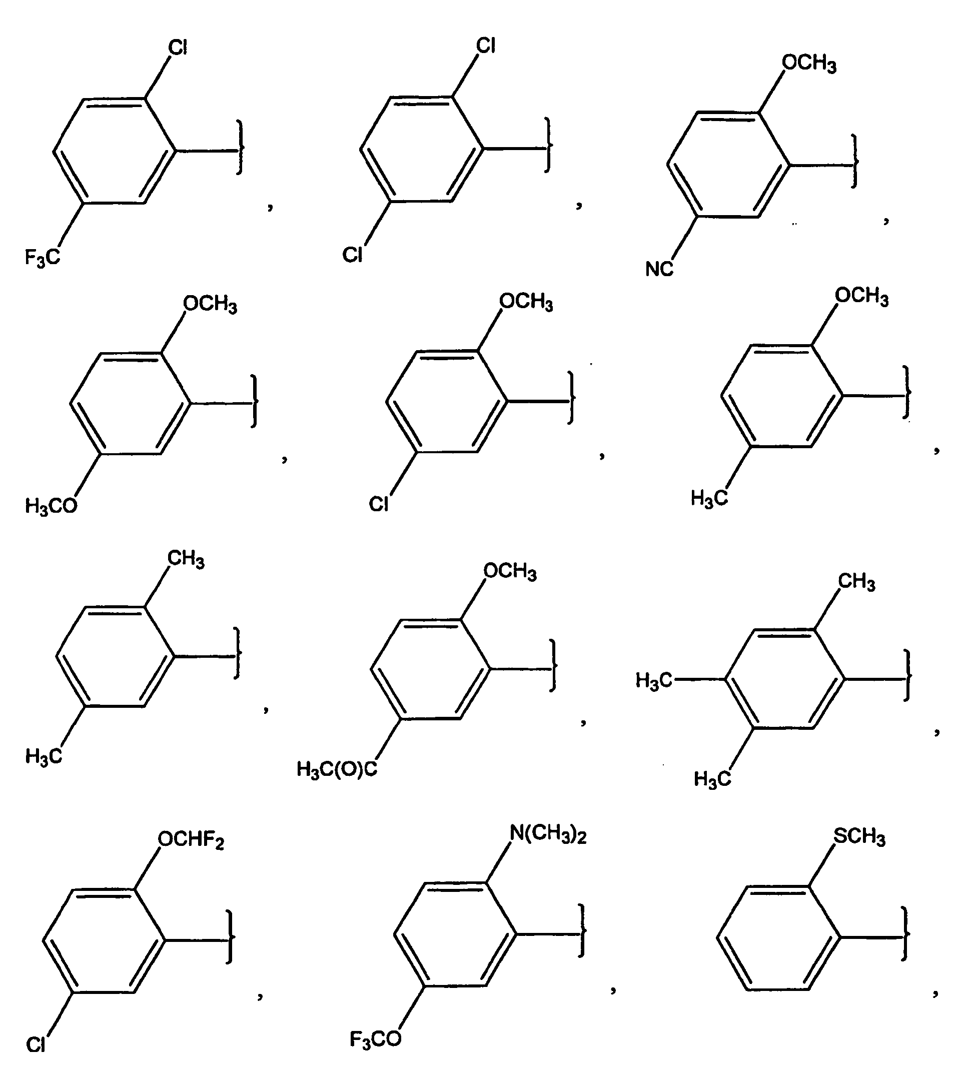 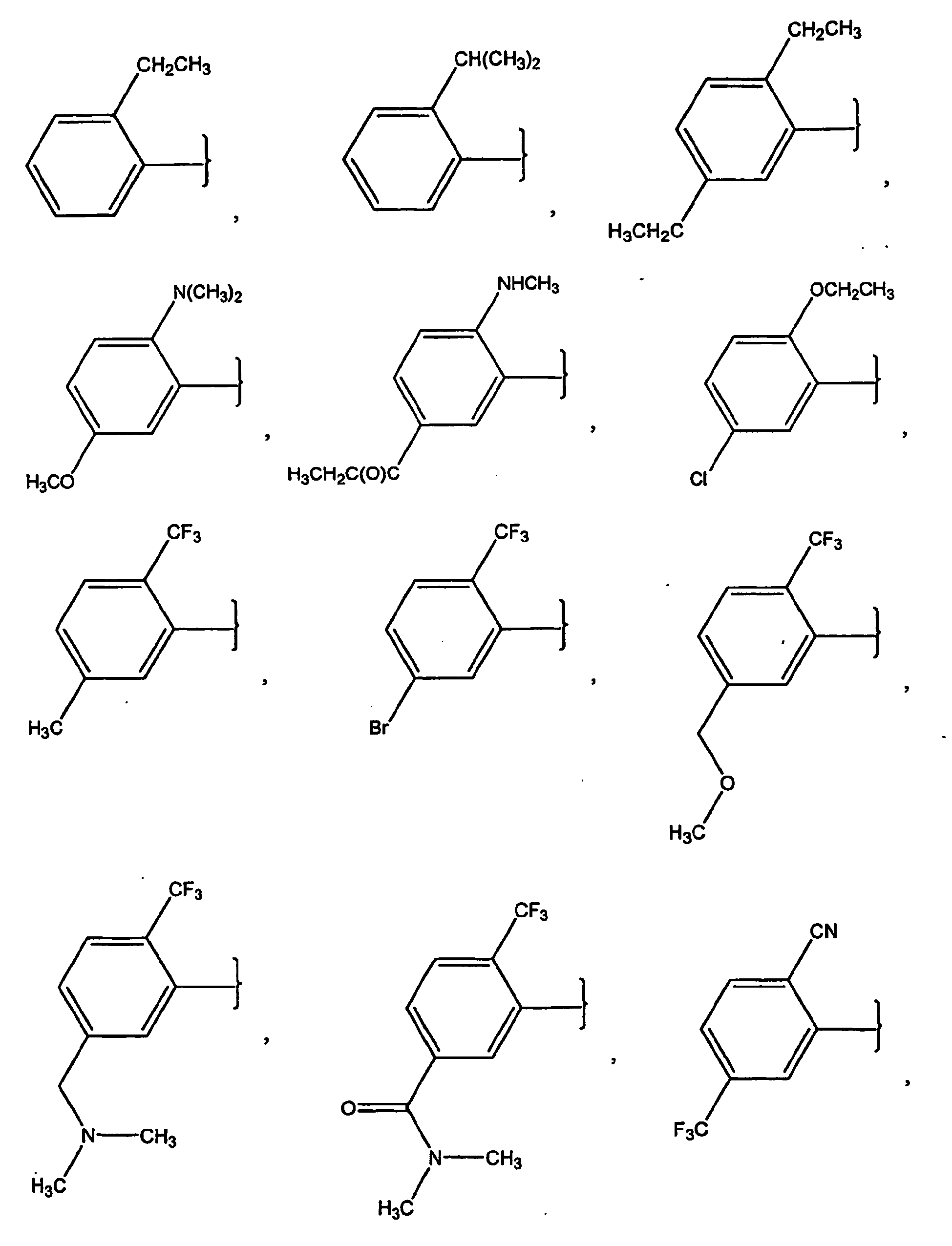 and 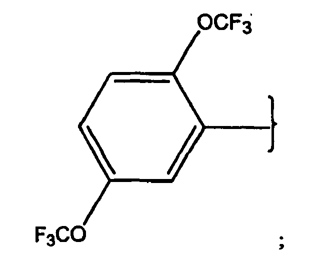
or X is selected from the group consisting of: 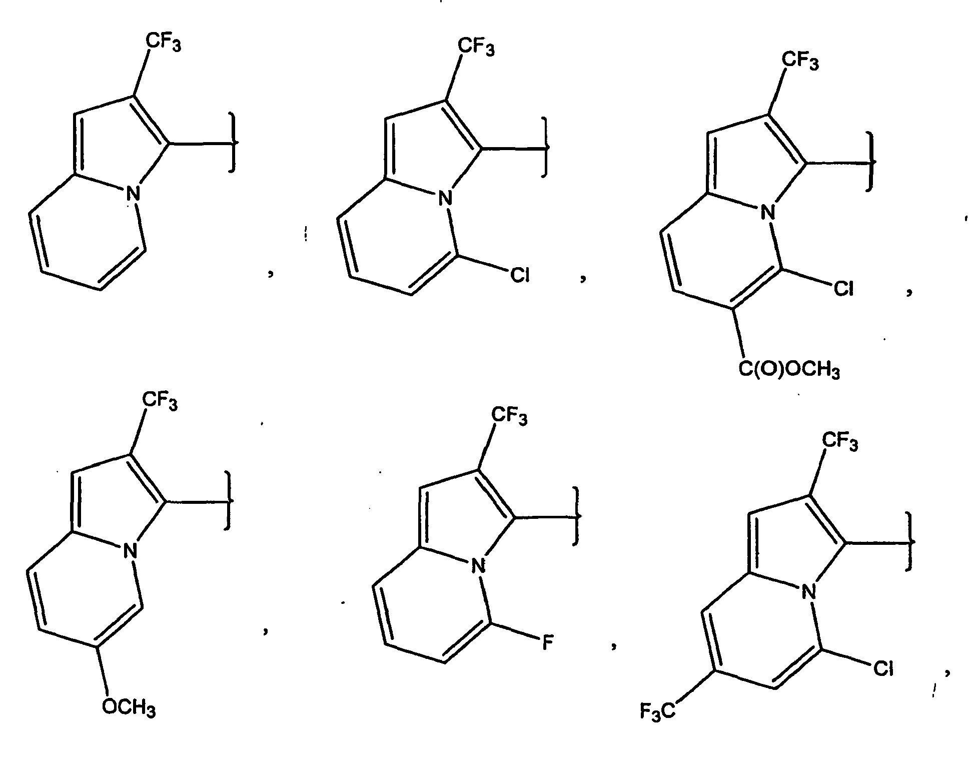 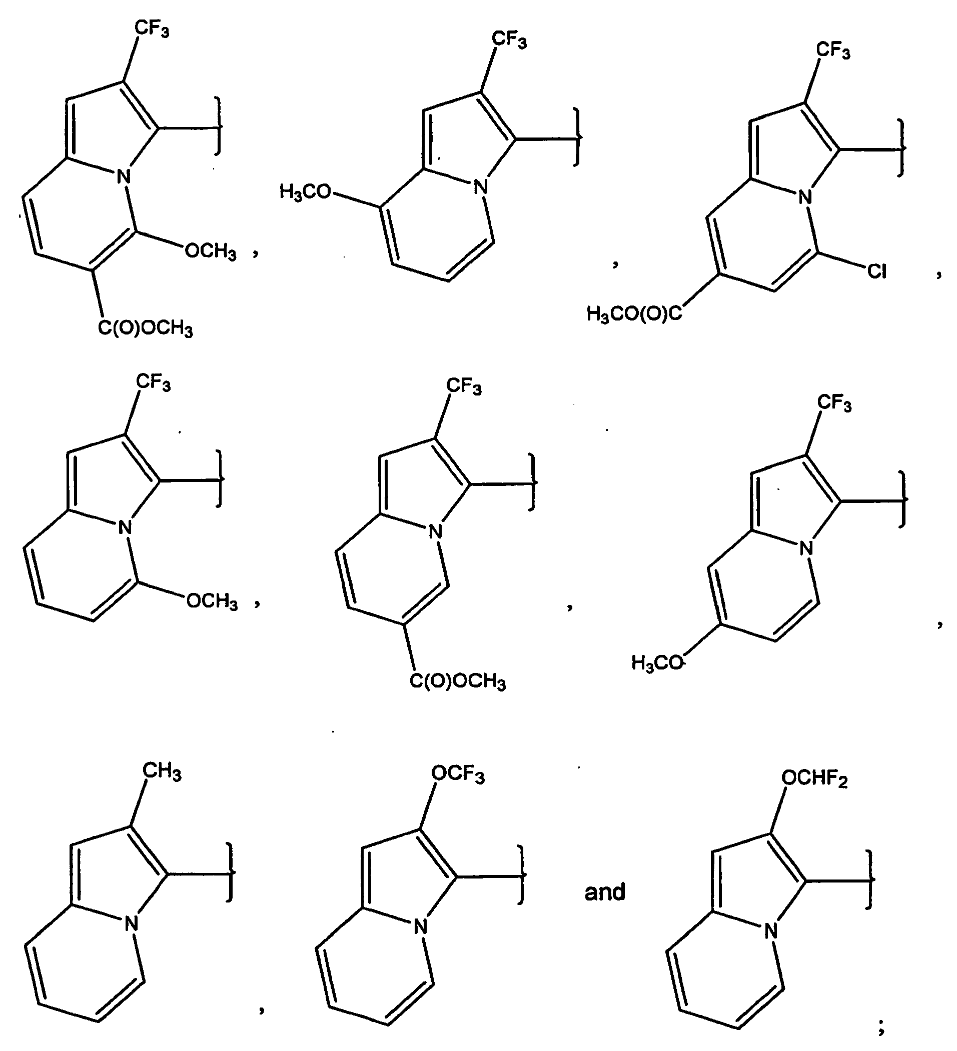
or X is selected from the group consisting of: 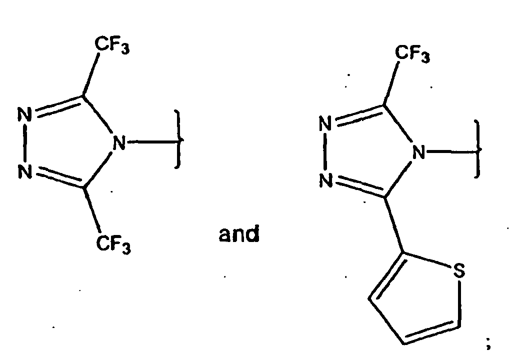
each Z is independently selected from the group consisting of optionally substituted C₁-C₄ alkyl (e.g., CH₃ or CF₃) and halo (e.g., F); in one embodiment, n is 0 and Z is absent;
each R is independently selected from -H, acetyl, tert-butoxycarbonyl, benzyloxycarbonyl (e.g., -H);
n is an integer selected from 0-4 (e.g., 0 or 1); and
   combinations thereof.

The substituents used for compounds of formulas (I) through (XVIII) or any of the specific compound shown below can be used in any combination that results in the formation of a stable compound. All such combinations are expressly encompassed in this invention.

In another embodiment, the invention relates to pharmaceutical compositions that comprise a compound of any one of formulas (I) through (XVIII), or a pharmaceutically acceptable salt, solvate, clathrate, or prodrug thereof, as an active ingredient, and a pharmaceutically acceptable carrier or vehicle. The compositions are useful for immunosuppression or to treat or prevent inflammatory conditions and immune disorders.

In another embodiment, compounds of any one of formulas (I) through (X\/III), or a pharmaceutically acceptable salt, solvate or clathrate thereof, are particularly useful for inhibiting immune cell (e.g., T-cells and/or B-cells) activation (e.g., activation in response to an antigen) and/or T cell and/or B cell proliferation. Indicators of immune cell activation include secretion of IL-2 by T cells, proliferation of T cells and/or B cells, and the like. In one embodiment, a compound of any one of formulas (I) through(XVIII) inhibits immune cell activation and/or T cell and/or B cell proliferation in cells.

In another embodiment, compounds of of any one of formula (I) through (XVIII),or a pharmaceutically acceptable salt, solvate or clathrate thereof, can inhibit the production of certain cytokines that regulate immune cell activation. For example, compounds of any one of formulas (I) through (XVIII), or a pharmaceutically acceptable salt, solvate or clathrate thereof, can inhibit the production of IL-2, IL-4, IL-5, IL-13, GM-CSF, IFN-γ, TNF-α and combinations thereof. In one embodiment, a compound of any one of formulas (I) through (XVIII) inhibits cytokine production in cells.

In another embodiment, compounds of any one of formulas (I) through (XVIII),or a pharmaceutically acceptable salt, solvate or clathrate thereof, can modulate the activity of one or more ion channel involved in activation of immune cells, such as CRAC ion channel, TRPM4 and Kv1.3. In one embodiment, a compound of any one of formulas (I) through (XIII), and particularly compounds of formulas (IV), (VII), (VIII). (IX), (X), (XI), (XII), and (XVIII), can inhibit the influx of calcium ions into an immune cell (e.g., T cells and/or B cells) by inhibiting the action of CRAC ion channels. In general, a decrease in I_{CRAC} current upon contacting a cell with a compound is one indicator that the compound inhibitions CRAC ion channels. I_{CRAC} current can be measured, for example, using a patch clamp technique, which is described in more detail in the examples below. In another embodiment, a compound of any one of formulas (I) through (XVIII) activates TRPM4 ion channels. In another embodiment, a compound of any one of formulas (I) through (XVIII) inhibits, Kv1.3 ion channels. In one embodiment, a compound of any one of formulas (I) through (XVIII) modulates an ion channel in a cell.

### EXEMPLARY COMPOUNDS OF THE INVENTION

Exemplary compounds of the invention are depicted in the table below.

| Compound No. | Structure | Chemical Name |
|---|---|---|
| 1 | 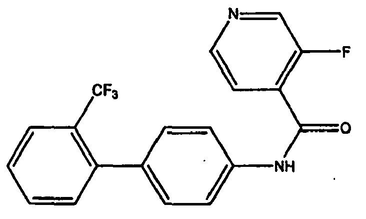 | 3-Fluoro-N-(2'-trifluoromethyl-biphenyl-4-yl)-isonicotinamide |
| 2 | 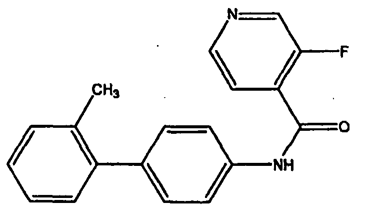 | 3-Fluoro-N-(2'-methyl-biphenyl-4-yl)-isonicotinamide |
| 3 | 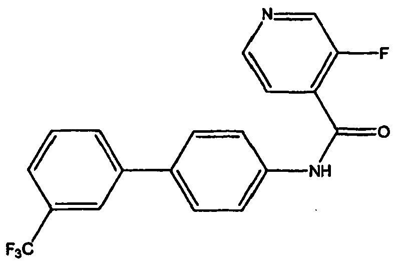 | 3-Fluoro-N-(3'-trifluoromethyl-biphenyl-4-yl)-isonicotinamide |
| 4 | 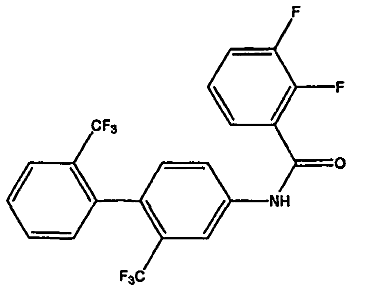 | N-(2,2'-Bis-trifluoromethyl-biphenyl-4-yl)-2,3-difluoro-benzamide |
| 5 | 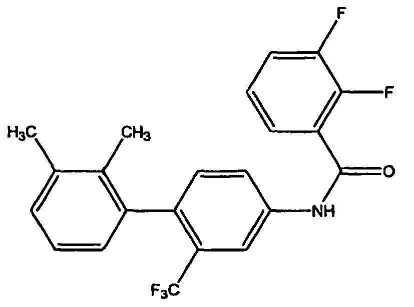 | N-[4-(1,2-Dimethyl-but-1-enyl)-3-trifluoromethyl-phenyl]-2,3-difluoro-benzamide |
| 6 | 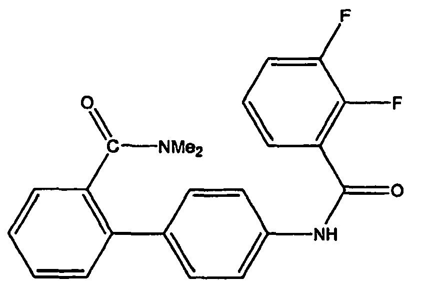 | 4'-(2,3-Difluoro-benzoylamino)-biphenyl-2-carboxylic acid dimethylamide |
| 7 | 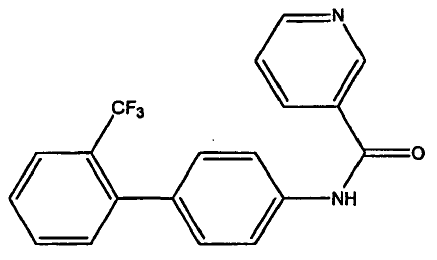 | N-(2'-Trifluoromethyl-biphenyl-4-yl)-nicotinamide |
| 8 | 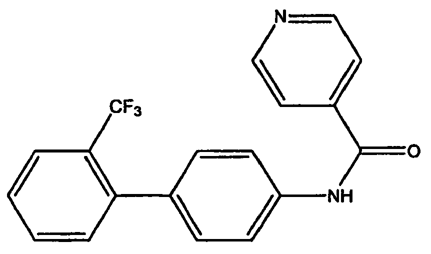 | N-(2'-Trifluoromethyl-biphenyl-4-yl)-isonicotinamide |
| 9 | 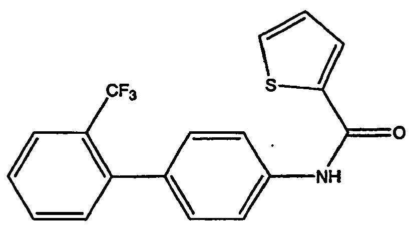 | Thiophene-2-carboxylic acid (2'-trifluoromethyl-biphenyl-4-yl)-amide |
| 10 | 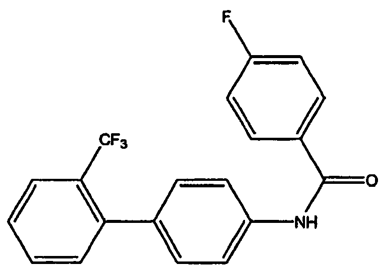 | 4-Fluoro-N-(2'-trifluoromethyl-biphenyl-4-yl)-benzamide |
| 11 | 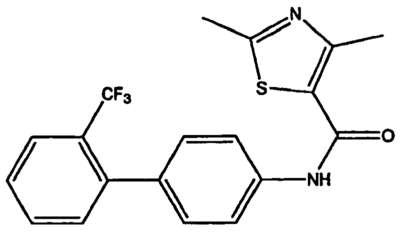 | 2,4-Dimethyl-thiazole-5-carboxylic acid (2'-trifluoromethyl-biphenyl-4- yl)-amide |
| 12 | 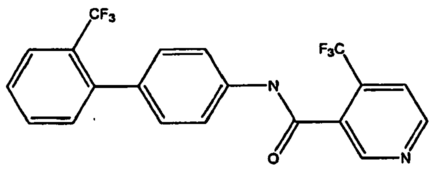 | 4-Trifluoromethyl-N- (2'-trifluoromethyl-biphenyl-4- yl)-nicotinamide |
| 13 | 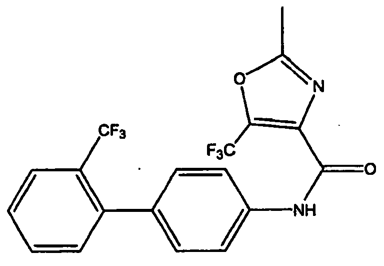 | 2-Methyl-5-trifluoromethyl-oxazole-4-carboxylic acid (2'-trifluoromethyl-biphenyl- 4-yl)-amide |
| 14 | 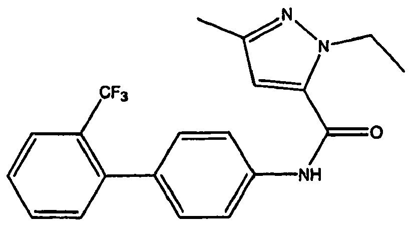 | 2-Ethyl-5-methyl-2H-pyrazole-3-carboxylic acid (2'-trifluoromethyl-biphenyl-4- yl)-amide |
| 15 | 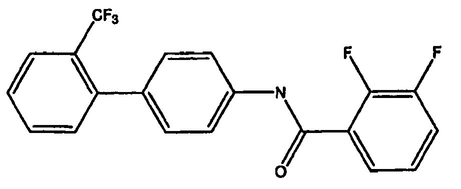 | 2,3-Difluoro-N-(2'-trifluoromethyl-biphenyl-4-yl)-benzamide |
| 16 | 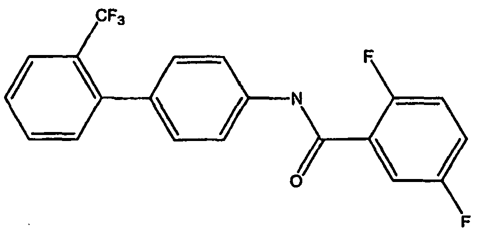 | 2,5-Difluoro-N-(2'-trifluoromethyl-biphenyl-4-yl)-benzamide |
| 17 | 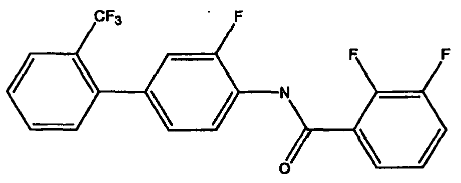 | 2,3-Difluoro-N-(3-fluoro-2'-trifl uoromethyl-biphenyl-4-yl)-benzamide |
| 18 | 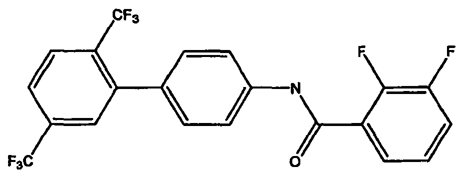 | N-(2',5'-Bis-trifluoromethyl-biphenyl-4-yl)-2,3-difluoro-benzamide |
| 19 | 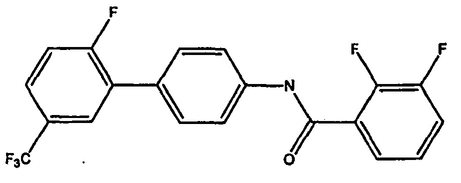 | 2,3-Difluoro-N-(2'-fluoro-5'-trifluoromethyl-biphenyl-4-yl)-benzamide |
| 20 | 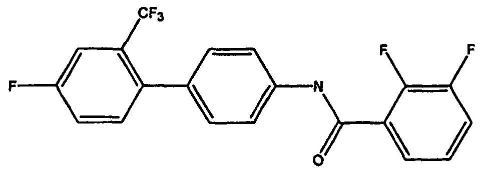 | 2,3-Difluoro-N-(4'-fluoro-2'-trifluoromethyl-biphenyl-4-yl)-benzamide |
| 21 | 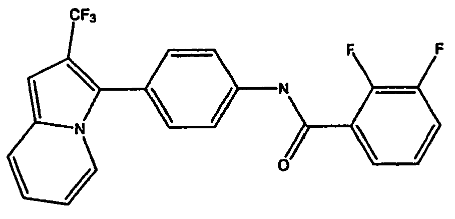 | 2,3-Difluoro-N-[4-(2-trifluoromethyl-indolizin-3-yl)-phenyl]-benzamide |
| 22 | 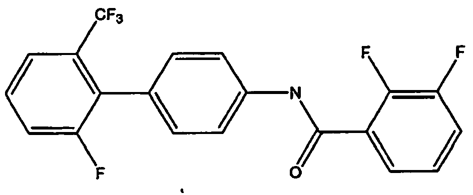 | 2,3-Difluoro-N-(2'-fluoro-6'-trifluoromethyl-bi phenyl-4-yl)-benzamide |
| 23 | 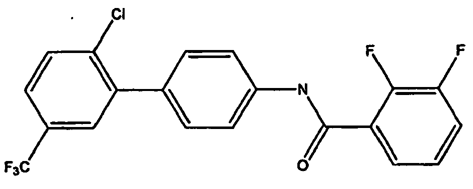 | 2,3-Difluoro-N-(2'-chloro-5'-trifluoromethyl-biphenyl-4-yl)-benzamide |
| 24 | 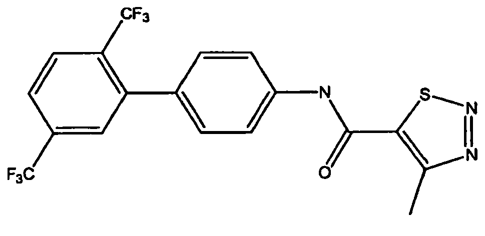 | 4-Methyl-[1,2,3]thiadiazole-5-carboxylic acid (2',5'-bis-trifluoromethyl- biphenyl-4-yl)-amide |
| 25 | 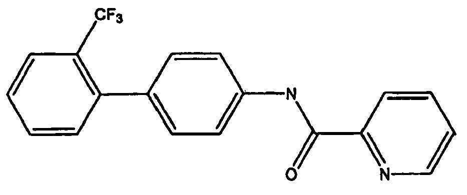 | Pyridine-2-carboxylic acid (2'-trifluoromethyl-biphenyl-4- yl)-amide |
| 26 | 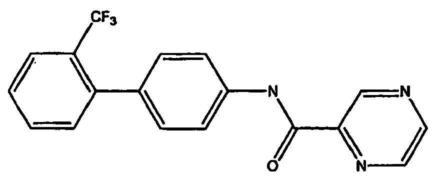 | Pyrazine-2-carboxylic acid (2'-trifluoromethyl-biphenyl-4- yl)-amide |
| 27 | 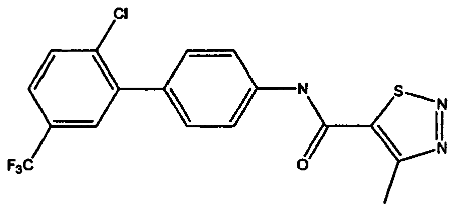 | 4-Methyl-[1,2,3jthiadiazole-5-carboxylic acid (2'-chloro-5'-trifluoromethyl- biphenyl-4-yl)-amide |
| 28 | 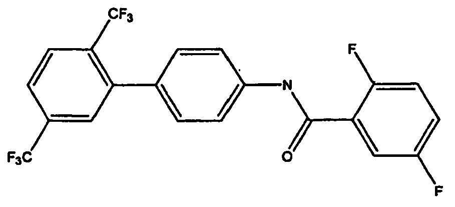 | N-(2',5'-Bis-trifluoromethyl-biphenyl-4-yi)-2,5-difluoro-benzamide |
| 29 | 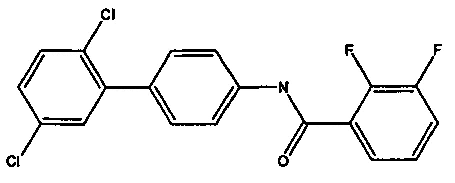 | N-(2',5'-Dichloro-biphenyl-4-yl)-2,3-difluoro-benzamide |
| 30 | 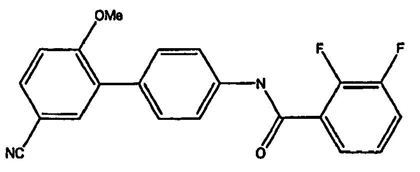 | N-(5'-Cyano-2'-methoxy-biphenyl-4-yl)-2,3-difluoro-benzamide |
| 31 | 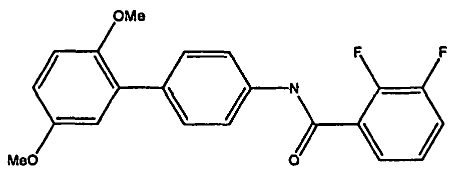 | N-(2',5'-Dimethoxy-biphenyl-4-yl)-2,3-difluoro-benzamide |
| 32 | 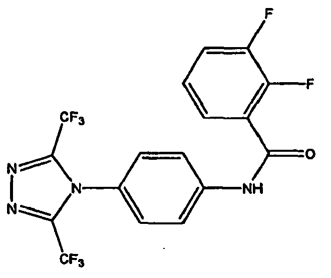 | N-[4-(3,5-Bis-trifluoromethyl-[1,2,4]triazol-4-yl)-phenyl]- 2,3-difluoro-benzamide |
| 33 | 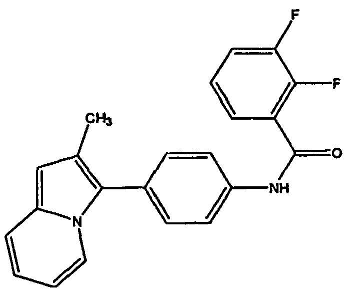 | 2,3-difluoro-N-[4-(2-trifluoro-methyl-indolizin-3-yl)-phenyl]-benzamide |
| 34 | 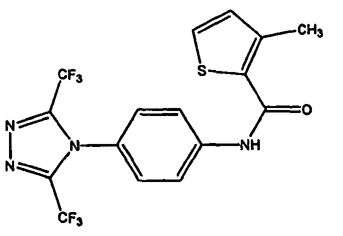 | 3-Methyl-thiophene-2-carboxylic acid-(4-(3,5-bis-trifluoromethyl-[1,2,4]triazol-4-yl)-phenyl)-amide |
| 35 | 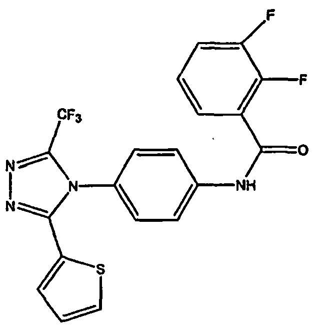 | N-[4-(3-trifluoromethyl-5-(thiophen-4-yl)-[1,2,4]triazol-4-yl)-phenyl]- 2,3-difluoro-benzamide |
| 36 | 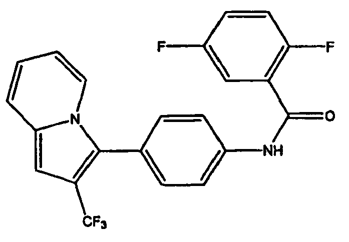 | N-[4-(3-trifluoromethyl-indolizin-3-yl)-phenyl]- 2,3-difluoro-benzamide |
| 37 | 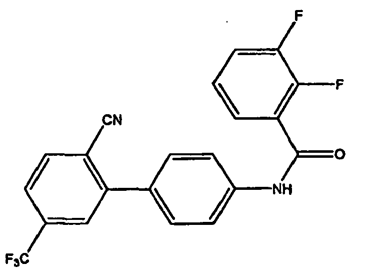 | N-[4-(3-cyano-5-trifluoromethyl-pyrid-2-yl)-phenyl]- 2,3-difluoro-benzamide |
| 38 | 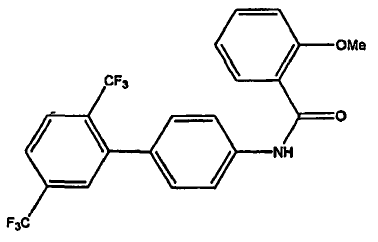 | N-(2',5'-bis-trifluoromethyl-biphenyl-4-yl)-2-methoxy-benzamide |
| 39 | 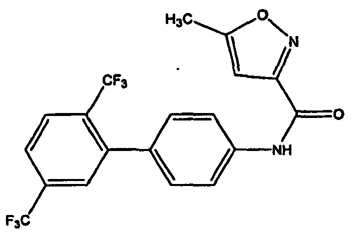 | 5-Methyl-isoxazol-3-carboxylic acid (2',5'-bis-trifluoromethyl-biphenyl-4-yl)-amide |
| 40 | 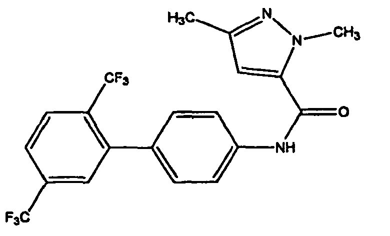 | 1,3-Dimethyl-1H-pyrazol-5-carboxylic acid (2',5'-bis-trifluoromethyl- biphenyl-4-yl)-amide |
| 41 | 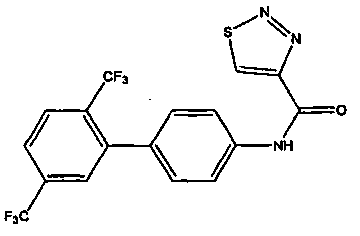 | [1,2,3]-Thiadiazole-4-carboxylic acid (2',5'-bis-trifluoromethyl-biphenyl-4-yl)-amide |
| 42 | 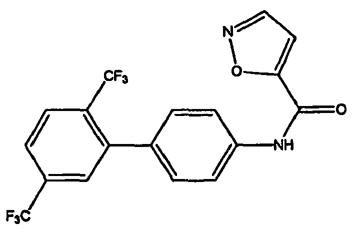 | Isoxazole-5-carboxylic acid (2',5'-bis-trifluoromethyl-biphenyl-4-yl)-amide |
| 43 | 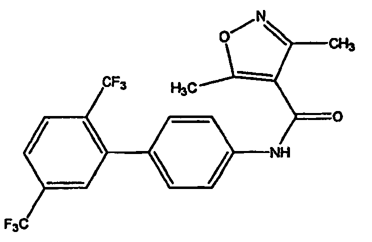 | 3,5-dimethylisoxazole-4-carboxylic acid (2',5'-bis-trifluoromethyl- biphenyl-4-yl)-amide |
| 44 | 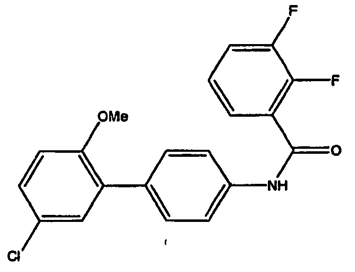 | N-(2'-methoxy-5'-chloro -biphenyl-4-yl)-2,3-difluoro-benzamide |
| 45 | 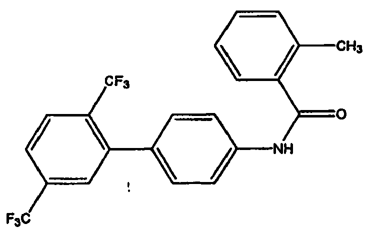 | N-(2',5'-bis-trifluoromethyl-biphenyl-4-yl)-2-methoxybenzamide |
| 46 | 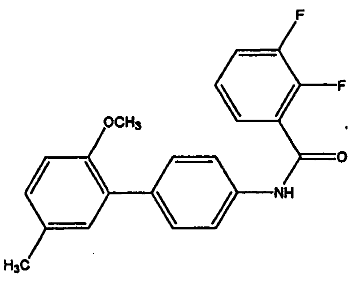 | N-(2'-methoxy-5'-methyl-biphenyl-4-yl)-2,3-difluorobenzamide |
| 47 | 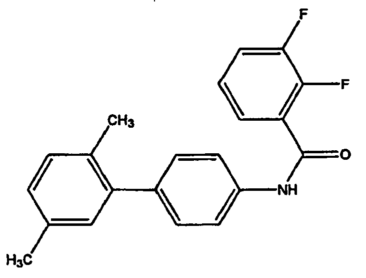 | N-(2',5'-dimethyl-biphenyl-4-yl)-2,3-difluorobenzamide |
| 48 | 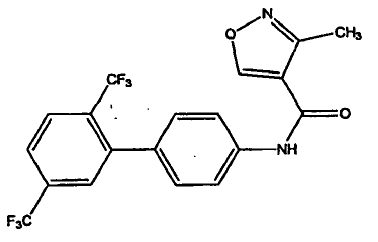 | 3-methylisoxazole-4-carboxylic acid (2',5'-bis-trifluoromethyl-biphenyl-4-yl)-amide |
| 49 | 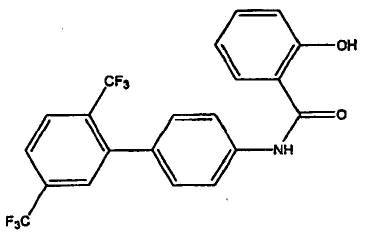 | N-(2',5'-bis-trifluoromethyl-biphenyl-4-yl)-2-hydroxybenzamide |
| 50 | 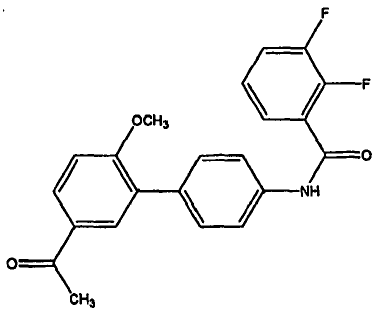 | N-(2'-methoxy-5'-acetyl-biphenyl-4-yl)-2,3-difluorobenzamide |
| 51 | 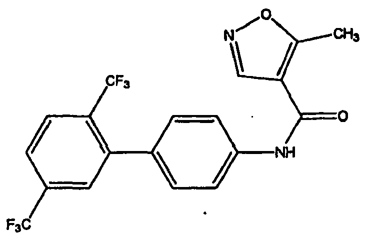 | 5-methylisoxazole-4-carboxylic acid (2',5'-bis-trifluoromethyl-biphenyl-4-yl)-amide |
| 52 | 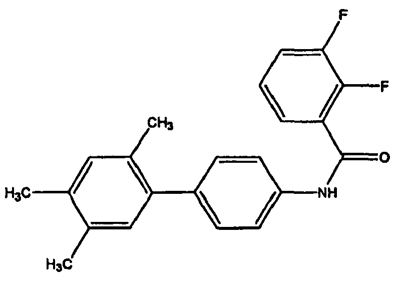 | N-(2',4',5'-trimethyl- biphenyl-4-yl)-2,3-difluorobenzamide |
| 53 | 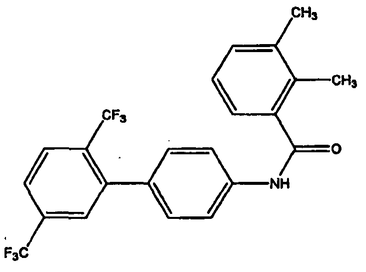 | N-(2',5'-bis-trifluoromethyl-biphenyl-4-yl)-2,3-dimethylbenzamide |
| 54 | 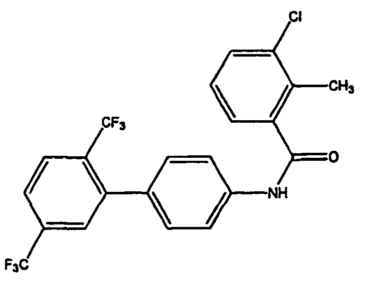 | N-(2',5'-bis-trifluoromethyl-biphenyl-4-yl)-2-methyl-3-chlorobenzamide |
| 55 | 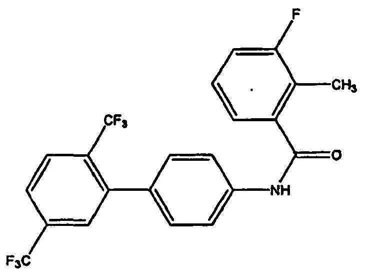 | N-(2',5'-bis-trifluoromethyl-biphenyl-4-yl)-2-methyl-3-fluorobenzamide |
| 56 | 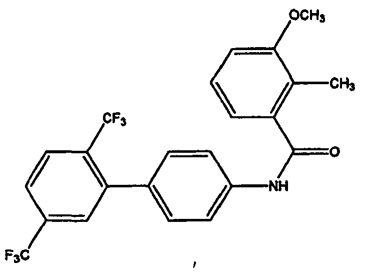 | N-(2',5'-bis-trifluoromethyl-biphenyl-4-yl)-2-methyl-3-methoxybenzamide |
| 57 | 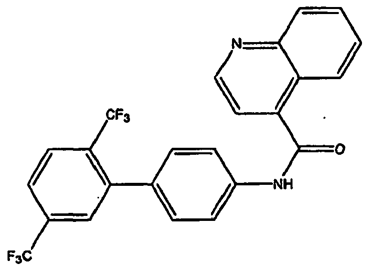 | quinoline-4-carboxylic acid (2',5'-bis-trifluoromethyl-biphenyl-4-yl)-amide |
| 58 | 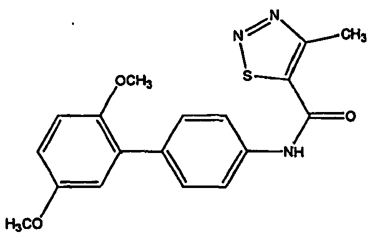 | 4-methyl-[1,2,3]-thiadiazole-5-carboxylic acid (2',5'-dimethoxy-biphenyl-4-yl)-amide |
| 59 | 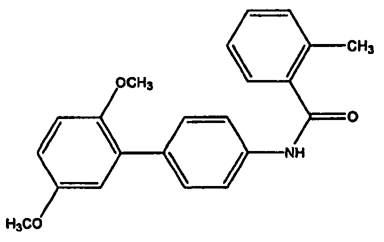 | N-(2',5'-dimethoxy- biphenyl-4-yl)-2-methylbenzamide |
| 60 | 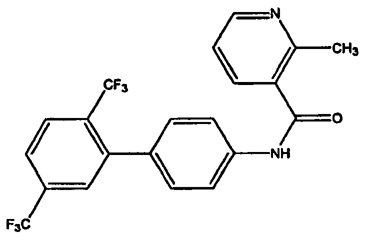 | 2-methyl-pyridine-3-carboxylic acid (2',5'-bis-trifluoromethyl-biphenyl-4-yl)-amide |
| 61 | 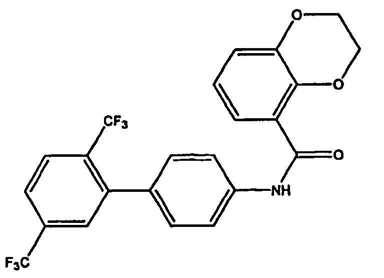 | 2,3-dihydro-benzo[1,4]dioxine-5-carboxylic acid (2',5'-bis-trifluoromethyl-biphenyl-4-yl)-amide |
| 62 | 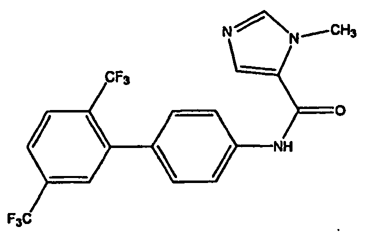 | 1-methyl-1H-imidazole-5-carboxylic acid (2',5'-bis-trifluoromethyl-biphenyl-4-yl)-amide |
| 63 | 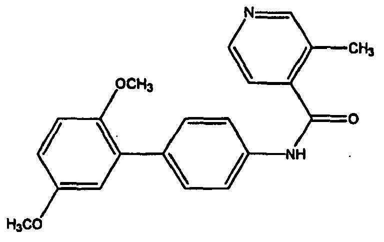 | 3-methyl-pyridine-4-carboxylic acid (2',5'-dimethoxy-biphenyl-4-yl)-amide |
| 64 | 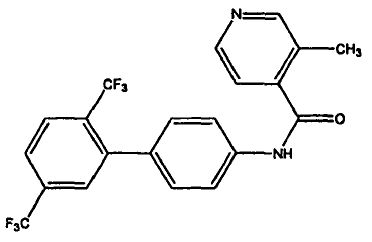 | 3-methyl-pyridine-4-carboxylic acid (2',5'-bis-trifluoromethyl-biphenyl-4-yl)-amide |
| 65 | 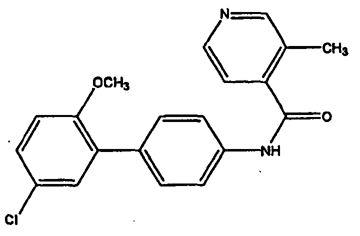 | 3-methyl-pyridine-4-carboxylic acid (2'-methoxy-5'-chlorobiphenyl-4-yl)-amide |
| 66 | 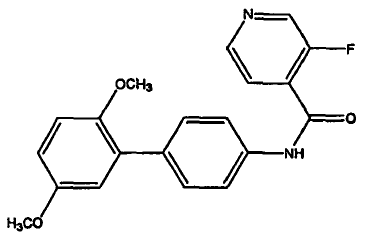 | 3-fluoro-pyridine-4-carboxylic acid (2',5'-dimethoxybiphenyl-4-yl)-amide |
| 67 | 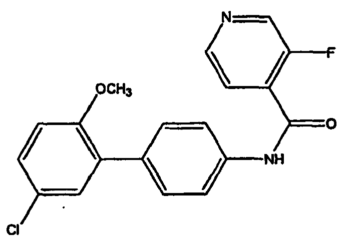 | 3-fluoro-pyridine-4-carboxylic acid (2'-methoxy-5'-chlorobiphenyl-4-yl)-amide |
| 68 | 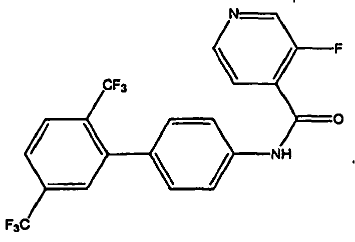 | 3-fluoro-pyridine-4-carboxylic acid (2',5'-bis-trifluoromethylbiphenyl-4-yl)-amide |
| 69 | 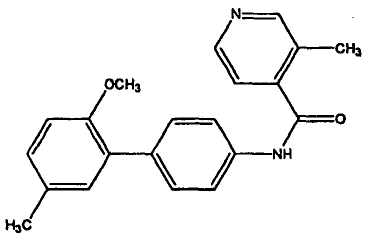 | 3-methyl-pyridine-4-carboxylic acid (2'-methoxy-5'-methylbiphenyl-4-yl)-amide |
| 70 | 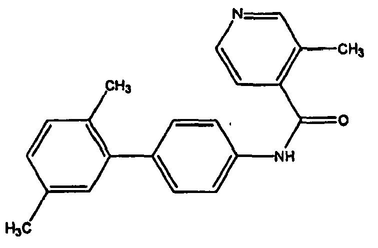 | 3-methyl-pyridine-4-carboxylic acid (2',5'-dimethylbiphenyl-4-yl)-amide |
| 71 | 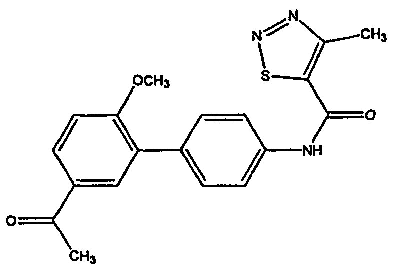 | 4-methyl-[1,2,3]-thiadiazole-5-carboxylic acid (2'-methoxy-5'-acetylbiphenyl-4-yl)-amide |
| 72 | 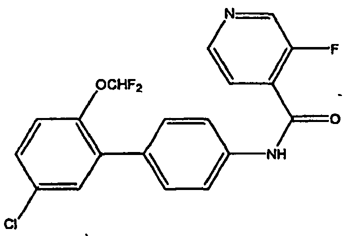 | 3-fluoro-pyridine-4-carboxylic acid (2'-difluoromethoxy-5'-chlorobiphenyl-4-yl)-amide |
| 73 | 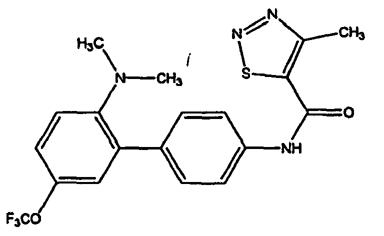 | 4-methyl-[1,2,3]-thiadiazole-5-carboxylic acid {2'-(N,N-dimethylamino)-5'-trifluoromethoxybiphenyl-4-yl}-amide |
| 74 | 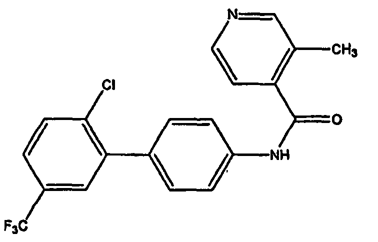 | 3-methyl-pyridine-4-carboxylic acid (2'-chloro-5'-trifluoromethylbiphenyl-4-yl)-amide |
| 75 | 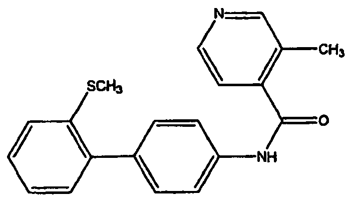 | 3-methyl-pyridine-4-carboxylic acid (2'-methylsulfanyl-biphenyl-4-yl)-amide |
| 76 | 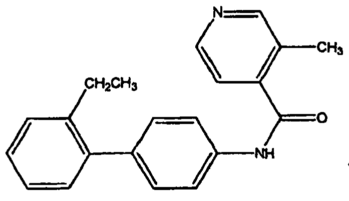 | 3-methyl-pyridine-4-carboxylic acid (2'-ethyl-biphenyl-4-yl)-amide |
| 77 | 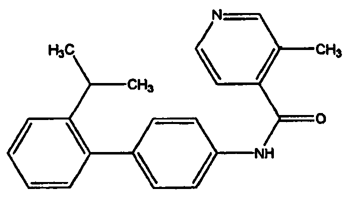 | 3-methyl-pyridine-4-carboxylic acid (2'-isopropyl-biphenyl-4-yl)-amide |
| 78 | 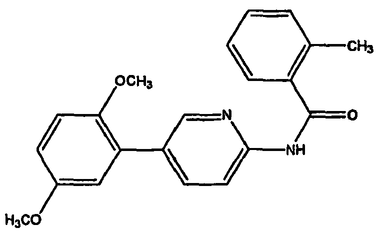 | N-{5-(2',5'-dimethoxyphenyl)-pyrid-2-yl}-2-methylbenzamide |
| 79 | 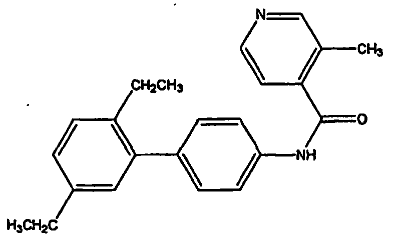 | 3-methyl-pyridine-4-carboxylic acid (2',5'-diethylbiphenyl-4-yl)-amide |
| 80 | 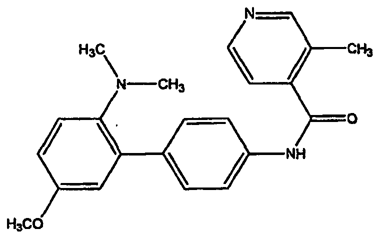 | 3-methyl-pyridine-4-carboxylic acid {2'-(N,N-dimethylamino)-5'-methoxybiphenyl-4-yl}-amide |
| 81 | 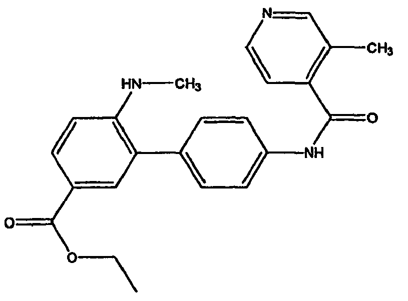 | 3-methyl-pyridine-4-carboxylic acid {2'-(N-dimethylamino)-5'-carbethoxybiphenyl-4-yl}-amide |
| 82 | 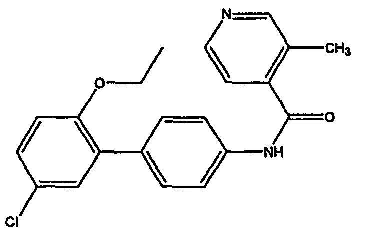 | 3-methyl-pyridine-4-carboxylic acid (2'-ethoxy-5'-chlorobiphenyl-4-yl)-amide |
| 83 | 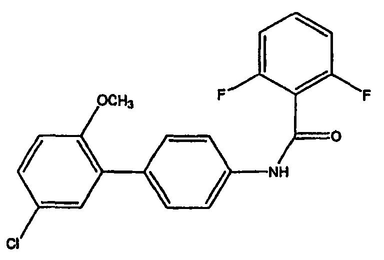 | N-(2'-dimethoxy-5'-chloro biphenyl-4-yl)-2,6-difluorobenzamide |
| 84 | 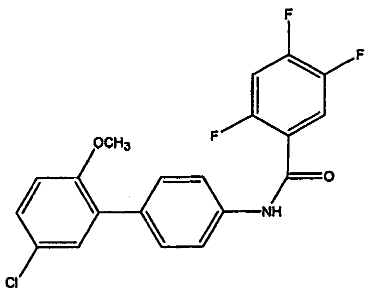 | N-(2'-methoxy-5'-chloro-biphenyl-4-yl)-2,4,5-trifluorobenzamide |
| 85 | 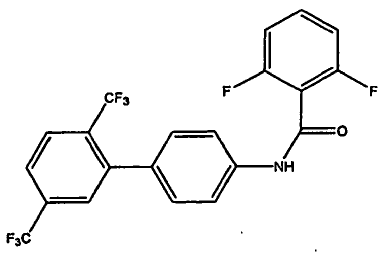 | N-(2',5'-bis-trifluoromethyl biphenyl-4-yl)-2,6-difluorobenzamide |
| 86 | 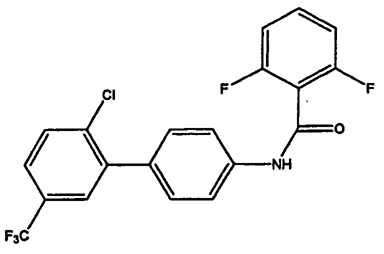 | N-(2'-chloro-5'-trifluoromethyl biphenyl-4-yl)-2,6-difluorobenzamide |
| 87 | 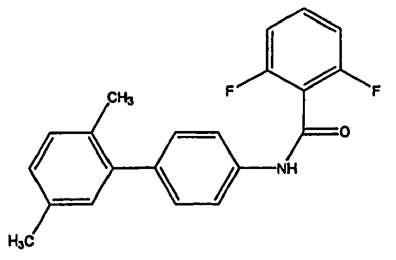 | N-(2',5'-dimethyl biphenyl-4-yl)-2,6-difluorobenzamide |
| 88 | 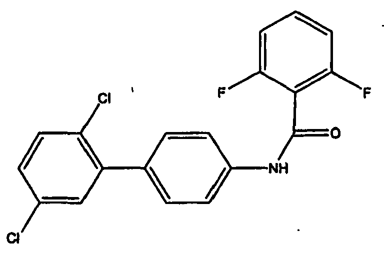 | N-(2',5'-dichloro biphenyl-4-yl)-2,6-difluorobenzamide |
| 89 | 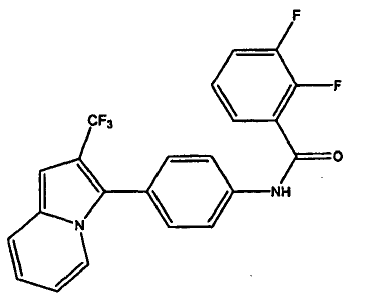 | 2,3-Difluoro-N-[4-(2-trifluoromethyl-indolizin-3-yl)-phenyl]-benzamide |
| 90 | 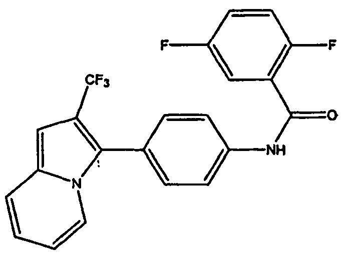 | 2,5-Difluoro-N-[4-(2-trifluoromethyl-indolizin-3-yl)-phenyl]-benzamide |
| 91 | 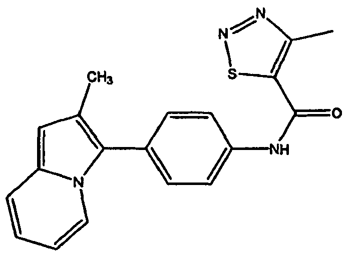 | 4-methyl-N-[4-(2-methyl-indolizin-3-yl)-phenyl]-[1,2,3]thiadiazale 5-carboxylic acid amide |
| 92 | 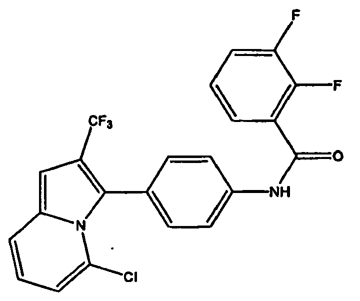 | N-[4-(5-chloro-2-trifluoromethyl-indolizin-3-yl)-phenyl]- 2,3-difluoro-benzamide |
| 93 | 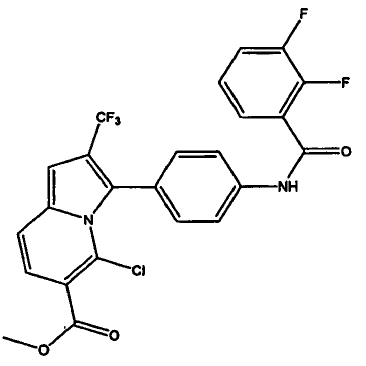 | 5-chloro-3-[4-(2,3-difluoro-benzoylamino)-phenyl]-2-trifluoromethyl-indolizine-6-carboxylic acid methyl ester |
| 94 | 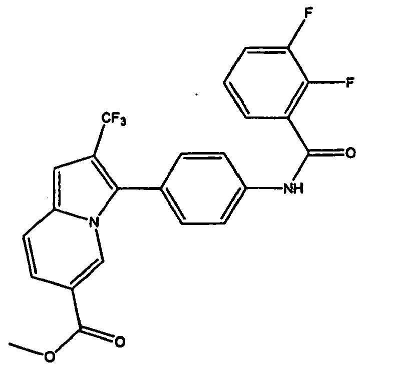 | 3-[4-(2,3-difluoro-benzoylamino)-phenyl]-2-trifluoromethyl-indolizine-6-carboxylic acid methyl ester |
| 95 | 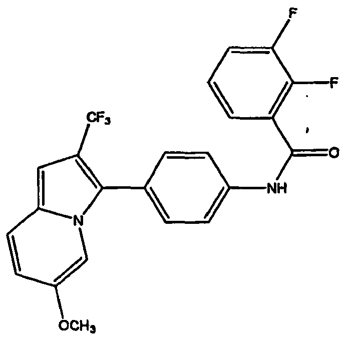 | 2,3-difluoro-N-[4-(6-methoxy-2-trifluoromethyl-indolizin-3-yl)-phenyl]- benzamide |
| 96 | 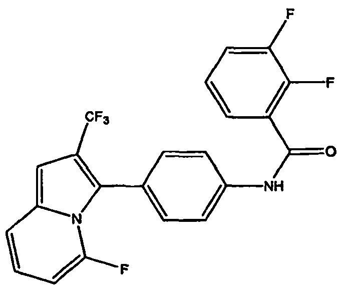 | N-[4-(5-fluoro-2-trifluoromethyl-indolizin-3-yl)-phenyl]- 2,3-difluoro-benzamide |
| 97 | 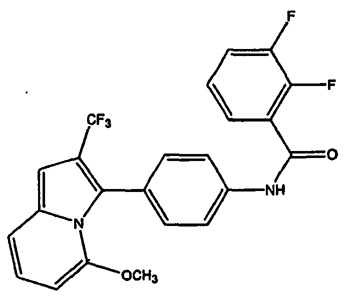 | N-[4-(5-methoxy-2-trifluoromethyl-indolizin-3-yl)-phenyl]- 2,3-difluoro-benzamide |
| 98 | 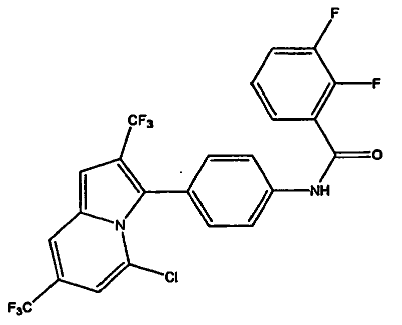 | N-[4-(5-Chloro-2,7-bis-trifluoromethyl-indolizin-3-yl)-phenyl]- 2,3-difluoro-benzamide |
| 99 | 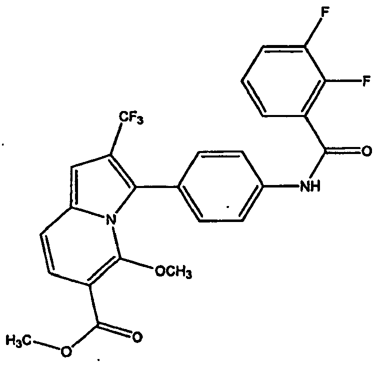 | 5-methoxy-3- [4-(2,3-difluoro-benzoylamino)-phenyl]-2-trifluoromethyl-indolizine-6-carboxylic acid methyl ester |
| 100 | 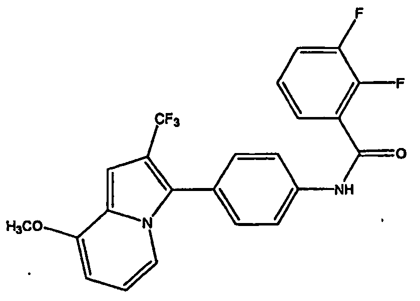 | 2,3-difluoro- N-[4-(8-methoxy-2-trifluoromethyl-indolizin-3-yl)-phenyl]-benzamide |
| 101 | 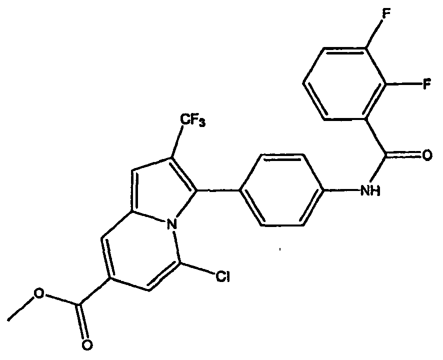 | 5-Chloro-3- [4-(2,3-difluoro-benzoylamino)-phenyl]-2-trifluoromethyl-indolizine-7-carboxylic acid methyl ester |
| 102 | 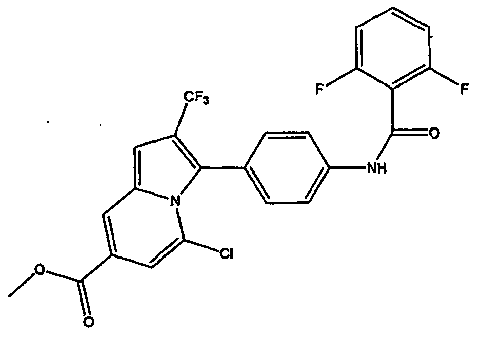 | 5-Chloro-3- [4-(2,6-difluoro-benzoylamino)-phenyl]-2-trifluoromethyl-indolizine-7-carboxylic acid methyl ester |
| 103 | 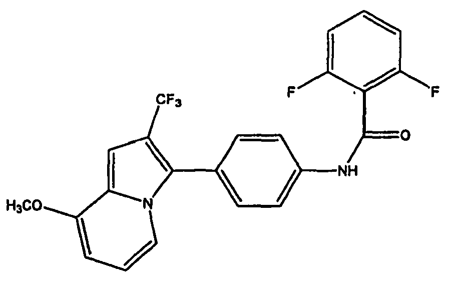 | 2,6-difluoro- N-[4-(8-methoxy-2-trifluoromethyl-indolizin-3-yl)-phenyl]-benzamide |
| 104 | 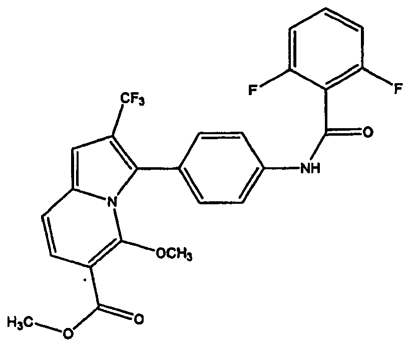 | 5-Methoxy-3- [4-(2,6-difluoro-benzoylamino)-phenyl]-2-trifluoromethyl-indolizine-6-carboxylic acid methyl ester |
| 105 | 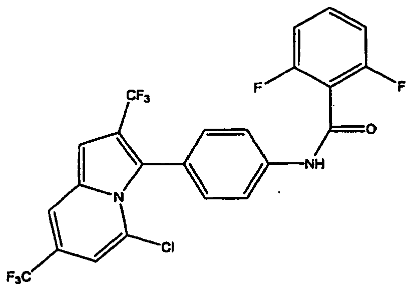 | N-[4-(5-chloro-2,7-bis-trifluoromethyl-indolizin-3-yl)-phenyl]- 2,6-difluoro-benzamide |
| 106 | 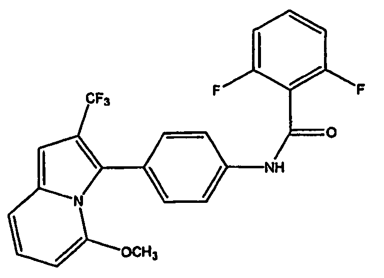 | N-[4-(5-methoxy-2-trifluoromethyl-indolizin-3-yl)-phenyl]- 2,6-difluoro-benzamide |
| 107 | 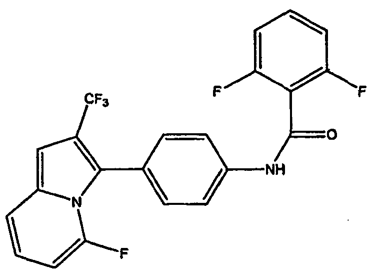 | N-[4-(5-fluoro-2-trifluoromethyl-indolizin-3-yl)-phenyl]- 2,6-difluoro-benzamide |
| 108 | 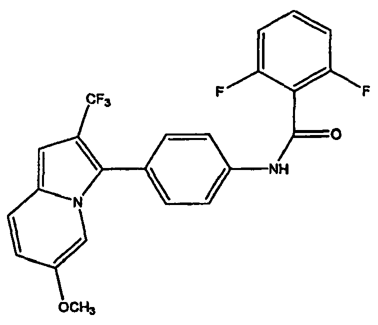 | 2,6-difluoro-N-[4-(6-methoxy-2-trifluoromethyl-indolizin-3-yl)-phenyl]- benzamide |
| 109 | 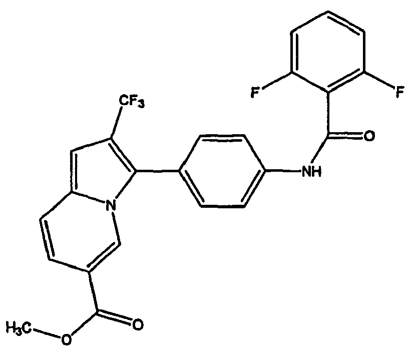 | 3-[4-(2,6-difluoro-benzoylamino)-phenyl]-2-trifluoromethyl-indolizine-6-carboxylic acid methyl ester |
| 110 | 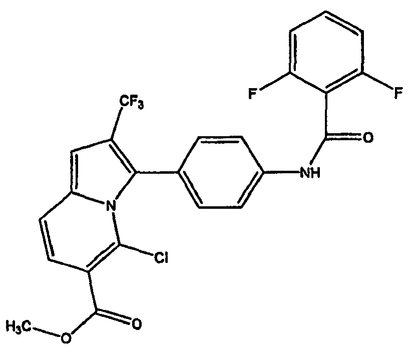 | 5-Chloro-3-[4-(2,6-difluoro-benzoylamino)-phenyl]-2-trifluoromethyl-indolizine-6-carboxylic acid methyl ester |
| 111 | 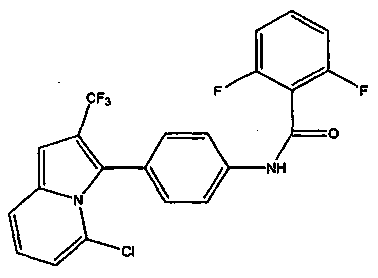 | N-[4-(5-Chloro-2-trifluoromethyl-indolizin-3-yl)-phenyl]- 2,6-difluoro-benzamide |
| 112 | 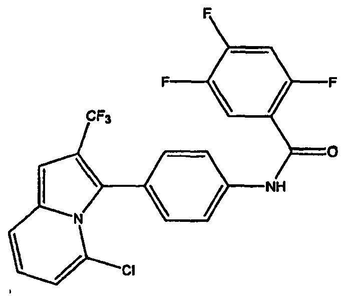 | N-[4-(5-Chloro-2-trifluoromethyl-indolizin-3-yl)-phenyl]- 2,4,5-trifluoro-benzamide |
| 113 | 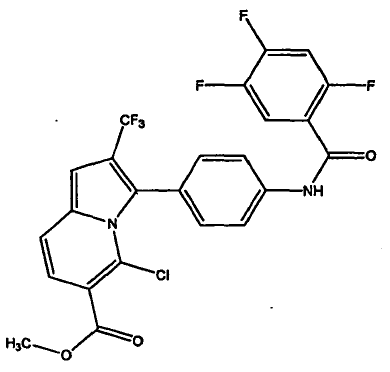 | 5-Chloro-3-[4-(2,4,5-trifluoro-benzoylamino)-phenyl]-2-trifluoromethyl-indolizine-6-carboxylic acid methyl ester |
| 114 | 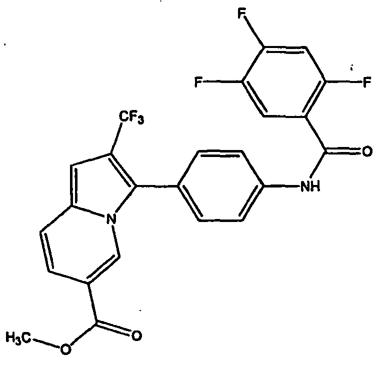 | 3-[4-(2,4,5-trifluoro-benzoylamino)-phenyl]-2-trifluoromethyl-indolizine-6-carboxylic acid methyl ester |
| 115 | 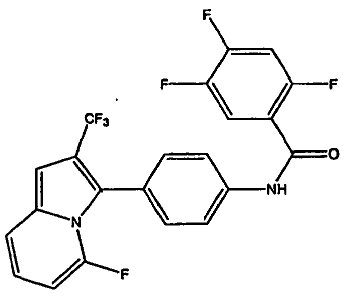 | 2,4,5-trifluoro-N-[4-(5-fluoro-2-trifluoromethyl-indolizin-3-yl)-phenyl]-benzamide |
| 116 | 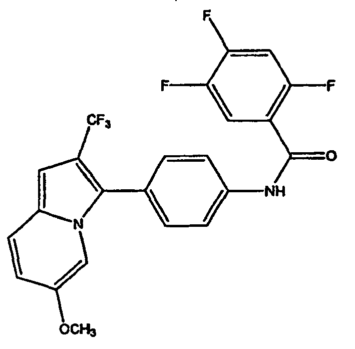 | 2,4,5-trifluoro-N-[4-(6-methoxy-2-trifluoromethyl-indolizin-3-yl)-phenyl]-benzamide |
| 117 | 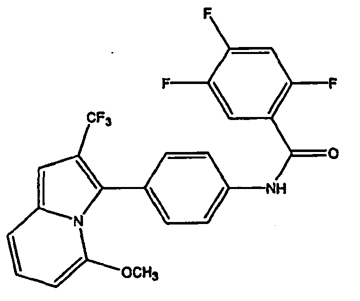 | 2,4,5-trifluoro-N-[4-(5-methoxy-2-trifluoromethyl-indolizin-3-yl)-phenyl]-benzamide |
| 118 | 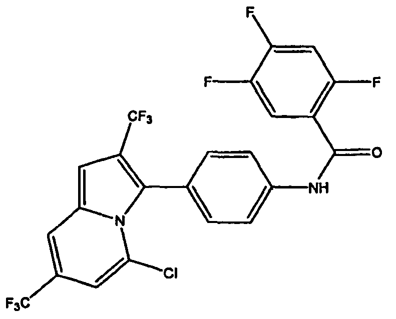 | N-[4-(5-Chloro-2,7-bis-trifluoromethyl-indolizin-3-yl)-phenyl]- 2,4,5-trifluoro-benzamide |
| 119 | 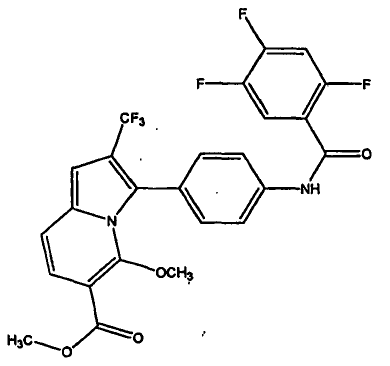 | 5-Methoxy-3-[4-(2,4,5-trifluoro-benzoylamino)-phenyl]-2-trifluoromethyl-indolizine-6-carboxylic acid methyl ester |
| 120 | 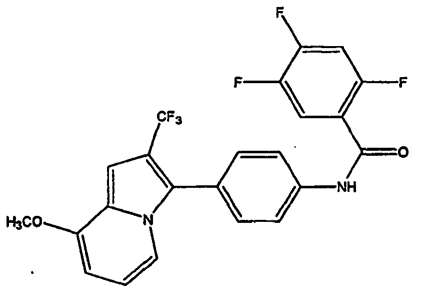 | 2,4,5-trifluoro-N-[4-(8-methoxy-2-trifluoromethyl-indolizin-3-yl)-phenyl]- benzamide |
| 121 | 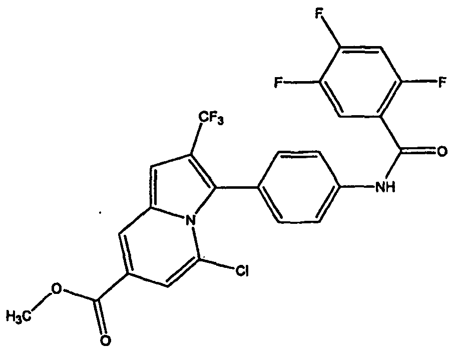 | 5-Chloro-3-[4-(2,4,5-trifluoro-benzoylamino)-phenyl]-2-trifluoromethyl-indolizine-7-carboxylic acid methyl ester |
| 122 | 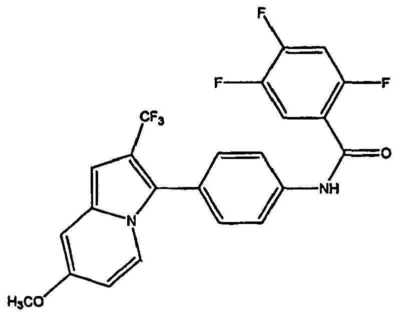 | 2,4,5-trifluoro-N-[4-(7-methoxy-2-trifluoromethyl-indolizin-3-yl)-phenyl]- benzamide |
| 123 | 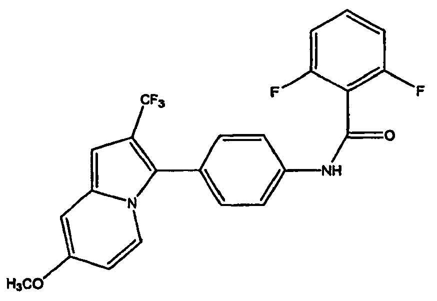 | 2,6-difluoro-N-[4-(7-methoxy-2-trifluoromethyl-indolizin-3-yl)-phenyl]- benzamide |
| 124 | 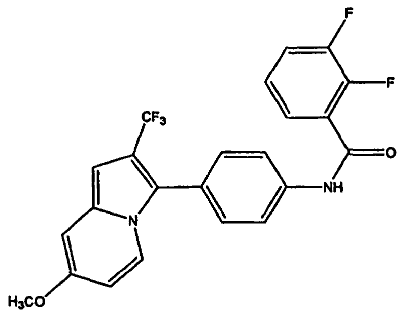 | 2,3-difluoro-N-[4-(7-methoxy-2-trifluoromethyl-indolizin-3-yl)-phenyl]- benzamide |
| 125 | 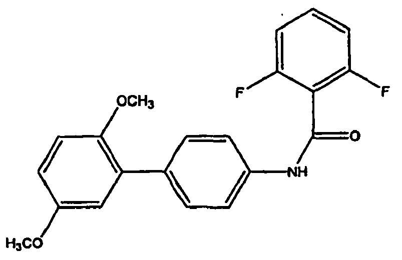 | N-(2',5'-dimethoxy-biphenyl-4-yl)-2,6-difluoro-benzamide |
| 126 | 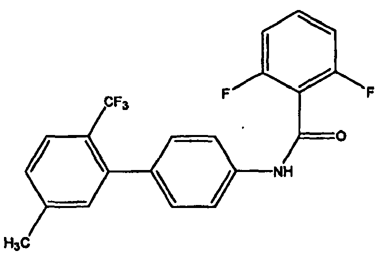 | N-(2'-trifluoromethyl-5'-methyl-biphenyl-4-yl)-2,6-difluoro-benzamide |
| 127 | 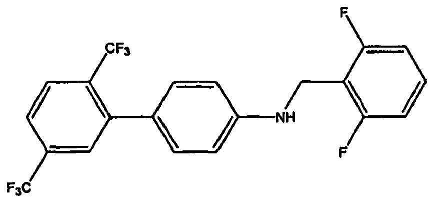 | N-(2',5'-bis-trifluoromethyl-biphenyl-4-yl)-2,6-difluoro-benzyl amine |
| 128 | 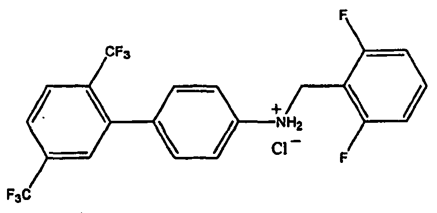 | N-(2',5'-bis-trifluoromethyl-biphenyl-4-yl)-2,6-difluoro-benzyl amine HCl salt |
| 129 | 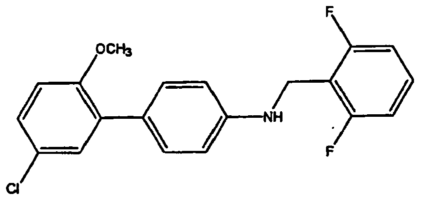 | N-(2'-methoxy-5'-chloro-biphenyl-4-yl)-2,6-difluoro-benzyl amine |
| 130 | 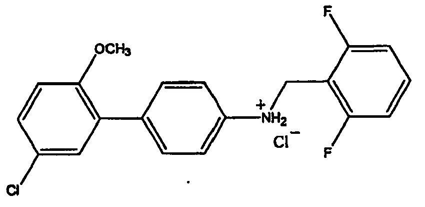 | N-(2'-methoxy-5'-chloro-biphenyl-4-yl)-2,6-difluoro-benzyl amine HCl salt |
| 131 | 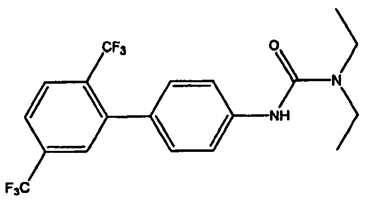 | N',N'-diethyl-N-(2',5'-bis-trifluoromethyl biphenyl-4-yl) urea |
| 132 | 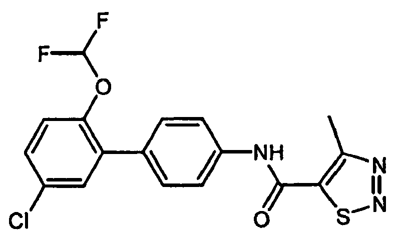 | 4-Methyl-[1,2,3]thiadiazole-5-carboxylic acid (5'-chloro-2'-difluoromethoxy-biphenyl- 4-yl)-amide |
| 133 | 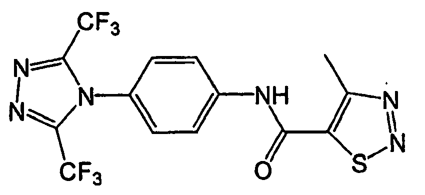 | 4-Methyl-[1,2,3]thiadiazole-5-carboxylic acid 4-(2,5-bis-trifluoromethyl-[1,2,4]triazol-1-yl)-phenyl- 4-yl)-amide |

### MECHANISM OF ACTION

Activation of T-lymphocytes in response to an antigen are dependent on calcium ion oscillations. Calcium ion oscillations in T-lymphocytes are triggered through stimulation of the T-cell antigen receptor, and involve calcium ion influx through the stored-operated Ca²⁺-release-activated Ca²⁺ (CRAC) channel. Although the molecular structure of the CRAC ion channel has not been identified, a detailed electrophysiological profile of the channel exist. Thus, inhibition of CRAC ion channels can be measured by measuring inhibition of the I_{CRAC} current. Calcium ion oscillations in T-cells have been implicated in the activation of several transcription factors (e.g., NFAT, Oct/Oap and NFκB) which are critical for T-cell activation (Lewis, Biochemical Society Transactions (2003), 31:925-929,

Compounds of any one of formulas (I) through (XVIII), and particularly compounds of formulas (IV), (VII), (VIII), (IX), (X), (XII), and (XIII), inhibit the activity of CRAC ion channels and, therefore, inhibit immune cell activation.

Compounds of any one of formulas (I) through (XVIII) activate transient receptor potential melastatin 4 (TRPM4) ion channels. TRPM4 ion channels have been shown to modulate the membrane potential of the cell and, when activated, depolarize the cell membrane, thereby inhibiting calcium entry through other calcium permeable pathways (see Launay et a/., Cell (2002), 109:397-407.

Therefore, it has been suggested that activation of the TRPM4 channels inhibits T-cell activation by inhibiting the activation of transcription factors that are dependent on calcium ion signalling.

Compounds of any one of formulas (I) through (XVIII) have been shown to inhibit the activity of Kv1.3 potassium ion channels. Kv1.3 is another ion channel which is involved in control of membrane potential and calcium influx. Blockade of Kv1.3 has been shown to prevent T-cell activation and attenuate immune responses *in vivo* (Koo et al., Cellular Immunology (1999), 197:99-107, the entire teachings of which are incorporated herein by reference).

### USE FOR TREATMENT AND PREVENTION

An effective amount of a compound of any one of formulas (I) through (XVIII) or a pharmaceutically acceptable salt, solvate, clathrate can be used for the manufacture of a pharmaceutical composition to treat a
patient in need of immunosuppression or in need of treatment or prevention of an inflammatory condition or immune disorder. Such patients may be treatment naïve or may experience partial or no response to conventional therapies.

Responsiveness of a particular inflammatory condition or immune disorder in a subject can be measured directly (e.g., measuring blood levels of inflammatory cytokines (such as IL-2, IL-4, IL-5, IL-13, GM-CSF, TNF-α, IFN-γ and the like) after administration of a compound or formulation of this invention), or can be inferred based on an understanding of disease etiology and progression. The compounds of any one of formulas (I) through (XVIII) or pharmaceutically acceptable salts, solvates or clathrates thereof can be assayed in *vitro* or *in vivo,* for the desired therapeutic or prophylactic activity, prior to use *in* humans. For example, known animal models of inflammatory conditions or immune disorders can be used to demonstrate the safety and efficacy of compounds of this invention.

### PHARMACEUTICAL COMPOSITIONS AND DOSAGE FORMS

Pharmaceutical compositions and dosage forms comprise one or more active ingredients in relative amounts and formulated in such a way that a given pharmaceutical composition or dosage form can be used for immunosuppression or to treat or prevent inflammatory conditions and immune disorders. Preferred pharmaceutical compositions and dosage forms comprise a compound of any one of formulas (I) through (XVIII), or a pharmaceutically acceptable salt, solvate, or clathrate thereof, optionally in combination with one or more additional active agents.

Single unit dosage forms are suitable for oral, mucosal (*e.g*., nasal, sublingual, vaginal, buccal, or rectal), parenteral (*e.g*., subcutaneous, intravenous, bolus injection, intramuscular, or intraarterial), or transdermal administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (*e.g*., nasal sprays or inhalers); gels; liquid dosage forms suitable for oral or mucosal administration to a patient, including suspensions (*e.g*., aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g*., crystalline or amorphous solids) that can be reconstituted to provide liquid dosage forms suitable for parenteral administration to a patient.

The composition, shape, and type of dosage forms of the invention will typically vary depending on their use. For example, a dosage form suitable for mucosal administration may contain a smaller amount of active ingredient(s) than an oral dosage form used to treat the same indication. This aspect of the invention will be readily apparent to those skilled in the art. See, *e.g*., Remington's Pharmaceutical Sciences (1990) 18th ed., Mack Publishing, Easton PA.

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms.

The suitability of a particular excipient may also depend on the specific active ingredients in the dosage form. For example, the decomposition of some active ingredients can be accelerated by some excipients such as lactose, or when exposed to water. Active ingredients that comprise primary or secondary amines (*e.g*., N-desmethylvenlafaxine and N,N-didesmethylvenlafaxine) are particularly susceptible to such accelerated decomposition. Consequently, this invention encompasses pharmaceutical compositions and dosage forms that contain little, if any, lactose. As used herein, the term "lactose-free" means that the amount of lactose present, if any, is insufficient to substantially increase the degradation rate of an active ingredient. Lactose-free compositions of the invention can comprise excipients that are well known in the art and are listed, for example, in the U.S. Pharmocopia (USP) SP (XXI)/NF (XVI). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

This invention further encompasses anhydrous pharmaceutical compositions and dosage forms comprising active ingredients, since water can facilitate the degradation of some compounds. For example, the addition of water (*e.g*., 5%) is widely accepted in the pharmaceutical arts as a means of simulating long-term storage in order to determine characteristics such as shelf-life or the stability of formulations over time. *See, e.g.,* Jens T. Carstensen (1995) Drug Stability: Principles & Practice, 2d. Ed., Marcel Dekker, NY, NY, 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (*e.g*., vials), blister packs, and strip packs.

The invention further encompasses pharmaceutical compositions
that comprise one or more compounds that reduce the rate by which an active ingredient will decompose. Such compounds, which are referred to herein as "stabilizer" include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. However, typical dosage forms of the invention comprise a compound of any one of formulas (I) through (XVIII), or a pharmaceutically acceptable salt, solvate,

or clathrate thereof in an amount of from about 1 mg to about 1000 mg, preferably in an amount of from about 50 mg to about 500 mg, and most preferably in an amount of from about 75 mg to about 350 mg. A typical total daily dosage of a compound of any one of formulas (I) through (XVIII), or a pharmaceutically acceptable salt, solvate, clathrate, or prodrug thereof may range from about 1 mg to about 5000 mg per day, preferably in an amount from about 50 mg to about 1500 mg per day, more preferably from about 75 mg to about 1000 mg per day. It is within the skill of the art to determine the appropriate dose and dosage form for a given patient.

### ORAL DOSAGE FORMS

Pharmaceutical compositions of the invention that are suitable for oral administration can be presented as discrete dosage forms, such as, but are not limited to, tablets (*e.g*., chewable tablets), caplets, capsules, and liquids (*e.g*., flavored syrups). Such dosage forms contain predetermined amounts of active ingredients, and may be prepared by methods of pharmacy well known to those skilled in the art. See *generally,* Remington's Pharmaceutical Sciences (1990) 18th ed., Mack Publishing, Easton PA.

Typical oral dosage forms of the invention are prepared by combining the active ingredient(s) in an admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g*., powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g*., Nos. 2208, 2906, 2910), microcrystalline cellulose, and mixtures thereof.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. One specific binder is a mixture of microcrystalline cellulose and sodium carboxymethyl cellulose sold as AVICEL RC-581. Suitable anhydrous or low moisture excipients or additives include AVICEL-PH-103J and Starch 1500 LM.

Examples of fillers suitable for use in the pharmaceutical compositions and dosage forms disclosed herein include, but are not limited to, talc, calcium carbonate (*e.g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Disintegrants are used in the compositions of the invention to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms of the invention include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g*., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

### CONTROLLED RELEASE DOSAGE FORMS

Active ingredients of the invention can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients of the invention. The invention thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g*., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

A particular extended release formulation comprises a therapeutically or prophylactically effective amount of a compound of formula (I), or a pharmaceutically acceptable salt, solvate, hydrate, clathrate thereof, in spheroids which further comprise microcrystalline cellulose and, optionally, hydroxypropylmethyl-cellulose coated with a mixture of ethyl cellulose and hydroxypropylmethylcellulose. Such extended release formulations can be prepared according to U.S. Patent No. 6,274,171,

A specific controlled-release formulation may comprise from about 6% to about 40% a compound of any one of formulas (I) through (XXI) by weight, about 50% to about 94% microcrystalline cellulose, NF, by weight, and optionally from about 0.25% to about 1% by weight of hydroxypropyl-methylcellulose, USP. wherein the spheroids are coated with a film coating composition comprised of ethyl cellulose and hydroxypropylmethylcellulose.

### PARENTERAL DOSAGE FORMS

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a phamarceutically acceptable vehicle for injection, suspensions ready for injection, and emulsions.

Suitable vehicles that can be used to provide parenteral dosage forms
are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms of the invention.

### TRANSDERMAL, TOPICAL, AND MUCOSAL DOSAGE FORMS

Transdermal, topical, and mucosal dosage forms include, but are not limited to, ophthalmic solutions, sprays, aerosols, creams, lotions, ointments, gels, solutions, emulsions, suspensions, or other forms known to one of skill in the art. See, *e.g*., Remington's Pharmaceutical Sciences (1980 & 1990) 16th and 18th eds., Mack Publishing, Easton PA and Introduction to Pharmaceutical Dosage Forms (1985) 4th ed., Lea & Febiger, Philadelphia. Dosage forms suitable for treating mucosal tissues within the oral cavity can be formulated as mouthwashes or as oral gels. Further, transdermal dosage forms include "reservoir type" or "matrix type" patches, which can be applied to the skin and worn for a specific period of time to permit the penetration of a desired amount of active ingredients.

Suitable excipients (*e.g*., carriers and diluents) and other materials that can be used to provide transdermal, topical, and mucosal dosage forms encompassed by this invention are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof to form lotions, tinctures, creams, emulsions, gels or ointments, which are non-toxic and pharmaceutically acceptable. Moisturizers or humectants can also be added to pharmaceutical compositions and dosage forms if desired. Examples of such additional ingredients are well known in the art. See, *e.g*., Remington's Pharmaceutical Sciences (1980 & 1990) 16th and 18th eds., Mack Publishing, Easton PA.

Depending on the specific tissue to be treated, additional components may be used prior to, in conjunction with, or subsequent to treatment with active ingredients of the invention. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyn-olidone; Kollidon grades (Povidone, Polyvidone); urea; and various water-soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

### COMBINATION THERAPY

Methods for immunosuppression or for treating or preventing inflammatory conditions and immune disorders in a patient in need thereof may further comprise administering to the patient being administered a compound of this invention, an effective amount of one or more other active agents. Such active agents may include those used conventionally for immunosuppression or for inflammatory conditions or immune disorders. These other active agents may also be those that provide other benefits when administered in combination with the compounds of this invention. For example, other therapeutic agents may include, without limitation, steroids, non-steroidal anti-inflammatory agents, antihistamines, analgesics, immunosuppressive agents and suitable mixtures thereof. In such combination therapy treatment, both the compounds of this invention and the other drug agent(s) are administered to a subject (e.g., humans, male or female) by conventional methods. The agents may be administered in a single dosage form or in separate dosage forms. Effective amounts of the other therapeutic agents and dosage forms are well known to those skilled in the art. It is well within the skilled artisan's purview to determine the other therapeutic agent's optimal effective-amount range.

Where another therapeutic agent is administered to a subject, the effective amount of the compound of this invention is less than its effective amount when the other therapeutic agent is not administered. In another embodiment, the effective amount of the conventional agent is less than its effective amount when the compound of this invention is not administered. In this way, undesired side effects associated with high doses of either agent may be minimized. Other potential advantages (including without limitation improved dosing regimens and/or reduced drug cost) will be apparent to those of skill in the art.

Relating to autoimmune and inflammatory conditions, the other therapeutic agent may be a steroid or a non-steroidal anti-inflammatory agent. Particularly useful non-steroidal anti-inflammatory agents, include, but are not limited to, aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal; flufenisal, piroxicam, sudoxicam, isoxicam; salicylic acid derivatives, including aspirin, sodium salicylate, choline magnesium trisalicylate, salsalate, diflunisal, salicylsalicylic acid, sulfasalazine, and olsalazin; para-aminophennol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid, and meclofenamic acid; enolic acids, including oxicams (piroxicam, tenoxicam), and pyrazolidinediones (phenylbutazone, oxyphenthartazone); and alkanones, including nabumetone and pharmaceutically acceptable salts thereof and mixtures thereof. For a more detailed description of the NSAIDs, *see* Paul A. Insel, Analgesic-Antipyretic and Antiinflammatory Agents and Drugs Employed in the Treatment of Gout, in Goodman & Gilman's The Pharmacological Basis of Therapeutics 617-57 (Perry B. Molinhoff and Raymond W. Ruddon eds., 9th ed 1996) and Glen R. Hanson, Analgesic, Antipyretic and Anti-Inflammatory Drugs in Remington: The Science and Practice of Pharmacy Vol 111196-1221 (A.R. Gennaro ed. 19th ed. 1995),

Of particular relevance to allergic disorders, the other therapeutic agent may be an anthihistamine. Useful antihistamines include, but are not limited to, loratadine, cetirizine, fexofenadine, desloratadine, diphenhydramine, chlorpheniramine, chlorcyclizine, pyrilamine, promethazine, terfenadine, doxepin, carbinoxamine, clemastine, tripelennamine, brompheniramine, hydroxyzine, cyclizine, meclizine, cyproheptadine, phenindamine, acrivastine, azelastine, levocabastine, and mixtures thereof. For a more detailed description of anthihistamines, see Goodman & Gilman's The Pharmacological Basis of Therapeutics (2001) 651-57, 10th ed).

Immunosuppressive agents include glucocorticoids, corticosteroids (such as Prednisone or Solumedrol), T cell blockers (such as cyclosporin A and FK506), purine analogs (such as azathioprine (Imuran)), pyrimidine analogs (such as cytosine arabinoside), alkylating agents (such as nitrogen mustard, phenylalanine mustard, buslfan, and cyclophosphamide), folic acid antagonsists (such as aminopterin and methotrexate), antibiotics (such as rapamycin, actinomycin D, mitomycin C, puramycin, and chloramphenicol), human IgG, antilymphocyte globulin (ALG), and antibodies (such as anti-CD3 (OKT3), anti-CD4 (OKT4), anti-CD5, anti-CD7, anti-IL-2 receptor, anti-alpha/beta TCR, anti-ICAM-1, anti-CD20 (Rituxan), anti-IL-12 and antibodies to immunotoxins).

The foregoing and other useful combination therapies will be understood and appreciated by those of skill in the art. Potential advantages of such combination therapies include a different efficacy profile, the ability to use less of each of the individual active ingredients to minimize toxic side effects, synergistic improvements in efficacy, improved ease of administration or use and/or reduced overall expense of compound preparation or formulation.

### OTHER EMBODIMENTS

The compounds of this invention may be used as research tools (for example, as a positive control for evaluating other potential TRPM4 activators, CRAC or Kv1.3 inhibitors, or IL-2, IL-4, IL-5, IL-13, GM-CSF, TNF-α, and/or INF-γ inhibitors). These and other uses and embodiments of the compounds and compositions of this invention will be apparent to those of ordinary skill in the art.

The invention is further defined by reference to the following examples describing in detail the preparation of compounds of the invention. It will be apparent to those skilled in the art that many modifications, both to materials and methods, may be practiced without departing from the purpose and interest of this invention. The following examples are set forth to assist in understanding the invention and should not be construed as specifically limiting the invention described and claimed herein. Such variations of the invention, including the substitution of all equivalents now known or later developed, which would be within the purview of those skilled in the art, and changes in formulation or minor changes in experimental design, are to be considered to fall within the scope of the invention incorporated herein.

### EXAMPLES

### EXPERIMENTAL RATIONALE

Without wishing to be bound by theory, it is believed that the compounds of this invention activate TRPM4, thereby inhibiting production of IL-2 and other key cytokines involved with inflammatory and immune responses. The examples that follow demonstrate these properties.

### MATERIALS AND GENERAL METHODS

Reagents and solvents used below can be obtained from commercial sources such as Aldrich Chemical Co. (Milwaukee, Wisconsin, USA). 1H-NMR and 13C-NMR spectra were recorded on a Varian 300MHz NMR spectrometer. Significant peaks are tabulated in the order: δ (ppm): chemical shift, multiplicity (s, singlet; d, doublet; t, triplet; q, quartet; m, multiplet; br s, broad singlet),coupling constant(s) in Hertz (Hz) and number of protons.

Human leukemic T cells (Jurkat cells) and HEK 293 cells transfected with the FLAG-humanTRPM4/pCDNA4/TO construct were grown on glass coverslips with DMEM medium supplemented with 10% FBS, blasticidin (5 µg/mL) and zeocin (0.4 g/mL). TRPM4 expression was induced one day before use by adding 1 µg/mL tetracycline to the culture medium and patch clamp experiments were performed 16-24 hours post-induction (for additional details see Launay et al. (2000)).

Patch clamp experiments were performed in the tight-seal whole-cell configuration at 21-25°C. High resolution current recordings were acquired by a computer-based patch clamp amplifier system (EPC-9, HEKA, Lambrecht, Germany). Patch pipettes had resistances between 2-4 MΩ after filling with the standard intracellular solution. Immediately following establishment of the whole-cell configuration, voltage ramps of 50-200 ms duration spanning the voltage range of -100 to +100 mV were delivered at a rate of 0.5 Hz over a period of 300-400 seconds. All voltages were corrected fro a liquid junction potential of 10 mV between external and internal solutions when using glutamate as the intracellular anion. Currents were filtered at 2.9 kHz and digitized at 10 µs intervals. Capacitive currents and series resistance were determined and corrected before each voltage ramp using the automatic capacitance compensation of the EPC-9. The low resolution temporal development of membrane currents was assessed by extracting the current amplitude at -80 mV or +80 mV from individual ramp current records.

### EXAMPLE 1: SYNTHESIS OF REPRESENTATIVE EXEMPLARY COMPOUNDS OF THIS INVENTION

### Compound 18:

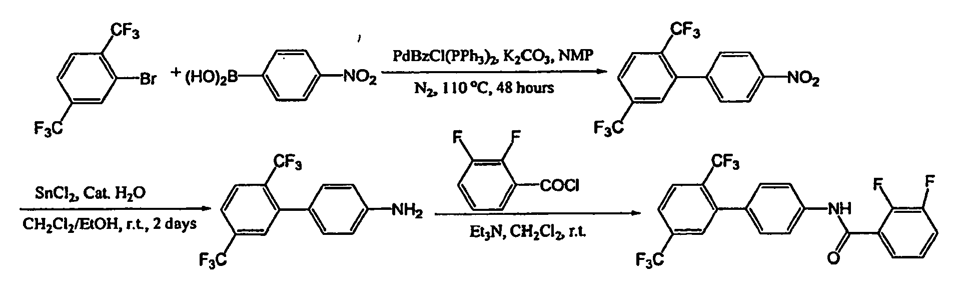

2,5-Bis(trifluoromethyl)bromobenzene (0.59 g, 2.00 mmol, 1.00 equiv.), 4-nitrophenylbronic acid (0.334 g, 2.00 mmol, 1.00 equiv.), trans-benzyl(chloro)bis(triphenylphosphine)palladium(II) (0.076 g, 0.10 mmol, 0.05 equiv.), K₂CO₃ 1.38 g, 10.00 mmol, 5.00 equiv.) and 10 mL dry NMP were charged to a 25 mL round bottom flask. The mixture was thoroughly deoxygenated by subjecting to vacuum/nitrogen cycle three times, and heated at 110 °C for 2 days under nitrogen protection. Usual workup yielded crude product 4'-nitro-2,5-bis-trifluoromethyl-biphenyl as brown viscous oil (0.66 g, 1.97 mmol, 99%). ¹H NMR (300 MHz, CDCl₃), δ (ppm): 8.32 (d, *J* = 8.7 Hz, 2H); 7.82-8.00 (m, 2H); 7.52-7.61 (m, 3H).
The crude product made in the last step was dissolved in 10 mL methylene chloride and 10 mL ethanol. Tin (II) chloride (2.28 g, 12.00 mmol, 6.00 equiv) was added, followed by addition of 1 mL water. After stirring at room temperature for 2 days, the mixture was neutralized with 2 N NaOH solution, and subjected to usual workup to yield crude product 4'-amino-2,5-bis-trifluoromethyl-biphenyl (0.52.g, 1.70 mmol, 85% crude yield) as brown viscous oil.
4'-Amino-2,5-bis-trifluoromethyl-biphenyl (0.16 g, 0.50 mmol, 1.00 equiv) was dissolved in 10 mL methylene chloride. To the solution was added 2,3-difluorobenzoyl chloride (0.088 g, 0.50 mmol, 1.00 equiv), and triethylamine (0.061 g, 0.60 mmol, 1.20 equiv). The mixture was stirred at room temperature for one hour, and loaded to column for flash chromatography. The title compound was isolated as light yellow solid (0.12 g, 0.27 mmol, 54%). ¹H NMR (300 MHz, CDCl₃), δ (ppm): 8.39 (d, *J* = 10.5 Hz, 1H); 7.75-7.95 (m, 6H); 7.63 (s, 1H); 7.26-7.443 (m, 3H); ESMS calcd. for C₂₁H₁₂F₈NO (M + H)⁺: 446.0; Found: 446.0.

### Compound 24:

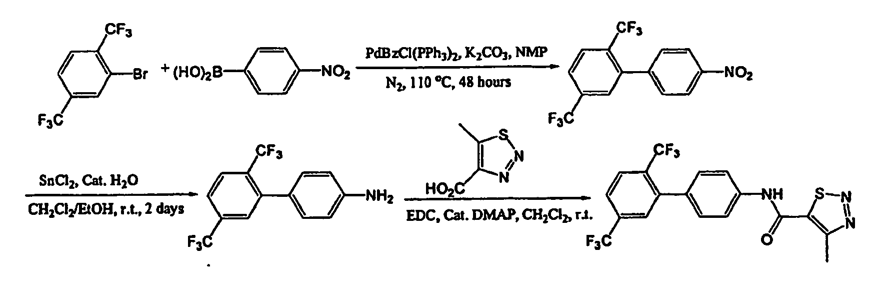

2,5-Bis(trifluoromethyl)bromobenzene (0.59 g, 2.00 mmol, 1.00 equiv.), 4-nitrophenylbronic acid (0.334 g, 2.00 mmol, 1.00 equiv.), trans-benzyl(chloro)bis(triphenylphosphine)palladium(II) (0.076 g, 0.10 mmol, 0.05 equiv.), K₂CO₃ 1.38 g, 10.00 mmol, 5.00 equiv.) and 10 mL dry NMP were charged to a 25 mL round bottom flask. The mixture was thoroughly deoxygenated by subjecting to vacuum/nitrogen cycle three times, and heated at 110°C for 2 days under nitrogen protection. Usual workup yielded crude product 4'-nitro-2,5-bis-trifluoromethyl-biphenyl as brown viscous oil (0.66 g, 1.97 mmol, 99%). ¹H NMR (300 MHz, CDCl₃), δ (ppm): 8.32 (d, *J* = 8.7 Hz, 2H); 7.82-8.00 (m, 2H); 7.52-7.61 (m, 3H).

The crude product made in the last step was dissolved in 10 mL methylene chloride and 10 mL ethanol. Tin (II) chloride (2.28 g, 12.00 mmol, 6.00 equiv) was added, followed by addition of 1 mL water. After stirring at room temperature for 2 days, the mixture was neutralized with 2 N NaOH solution, and subjected to usual workup to yield crude product 4'-amino-2,5-bis-trifluoromethyl-biphenyl (0.52 g, 1.70 mmol, 85% crude yield) as brown viscous oil.

A solution of 4'-amino-2,5-bis-trifluoromethyl-biphenyl (0.20 g, 0.60 mmol, 1.00 equiv), 4-methyl-1,2,3-thiadiazole-5-carboxylic acid (0.080 g, 0.60 mmol, 1.0 equiv), EDC (0.191 g, 1.00 mmol, 1.70 equiv) and DMAP (0.012 g, 0.10 mmol, 0.17 equiv) in10 mL methylene chloride was stirred at room temperature for 24 hours. The mixture was then loaded to column for flash chromatography, yielding the title compound as off-white solid (0.22 g, 0.51 mmol, 85%).
¹H NMR (300 MHz, CDCl₃), δ (ppm): 7.61-7.92 (m, 6H); 7.37 (d, *J* = 8.7 Hz, 2H); 3.00 (s, 3H); ESMS calcd. for C₁₈H₁₂F₆N₃OS (M + H)⁺: 432.0; Found: 432.0.

### Compound 32:

To a solution of 1,4-phenylenediame (1.95 g, 18.00 mmol, 6.00 equiv.) and Et₃N (0.364 g, 3.6 mmol, 1.20 equiv) in 20 mL DMF/CH₂Cl₂ (1:1) at 0 °C was added 2,3-difluorobenzoyl chloride (0.53 g, 3.00 mmol, 1.00 equiv.). The mixture was stirred at 0°C for 2 hours. Solid precipitated out during reaction was removed by filtration, and the solution was subjected to EtOAc/aqueous wash workup to remove DMF. The residue was purified by flash chromatography to yield (N-4-aminophenyl)-2,3-difluorobenzamide as a light yellow solid (0.31 g, 1.25 mmol, 42%). ¹H NMR (300 MHz, CDCl₃), δ ppm: 7.87-8.14 (m, 2H); 7.19-7.43 (m, 4H); 6.70 (d, *J* = 7.8 Hz, 2H); 3.66 (br s, 2H).
(N-4-Aminophenyl)-2,3-difluorobenzamide (0.20 g, 0.81 mmol, 1.10 equiv), 2,5-bistrifluoromethyl-[1,3,4]oxadiazole (0.15 g, 0.73 mmol, 1.00 equiv), HOAc (0.010 g, 0.25 equiv) and 5 mL NMP were mixed in a sealed-tube, and heated at 140 oC for 3 days. Usual workup and flash chromatography yielded N-[4-(3,5-bistrifluoromethyl-[1,2,4]triazol-4-yl-phenyl]-2,3-difluorobenzamide as a white solid (0.267 g, 0.61 mmol, 84 %). ¹H NMR (300 MHz, CDCl₃), δ (ppm): 8.52 (d, *J* = 13.8 Hz, 1H); 7.91-7.95 (m, 3H); 7.26-7.48 (m, 4H); ESMS calcd. for C₁₇H₉F₈N₄O (M + H)⁺: 437.0; Found: 437.0.

### GENERAL SYNTHESIS OF COMPOUNDS 33, 90, 91, 92, 93, AND 125

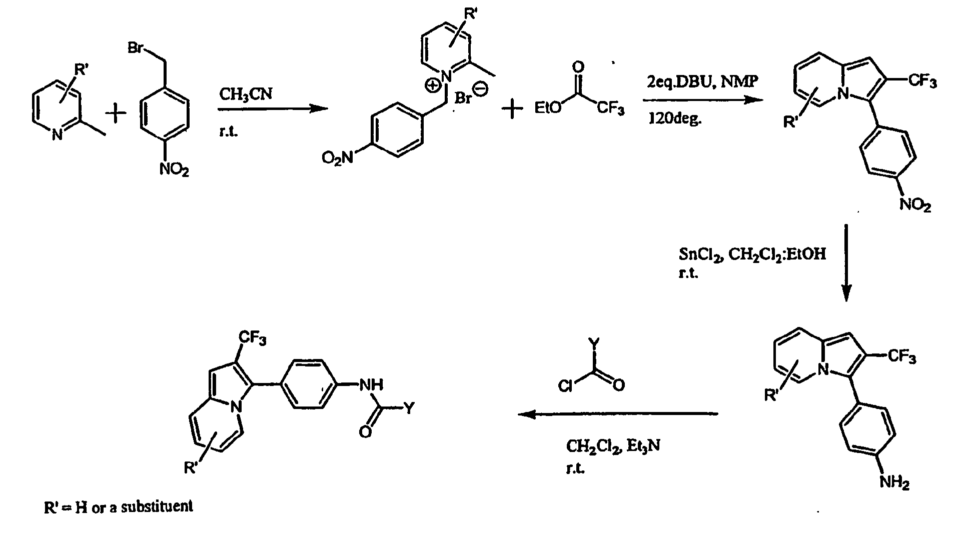

A mixture of (optionally substituted)-2-Picoline (10.73 mmols) and p-nitrobenzyl-bromide (10.73 mmols) was stirred overnight in 10 mL of acetonitrile at room temperature. The white solid obtained was filtered, washed with acetonitrile and dried to afford optionally substituted 2-methyl-1-(4-nitrobenzyl)-pyridinium bromide in 50%-95% yields.

A mixture of optionally substituted 2-methyl-1-(4-nitrobenzyl)-pyridinium bromide (3.23 mmols), trifluoroacetic acid ethyl ester (3.23mmols)and DBU (6.47 mmols) in 5mL of anhydrous NMP was heated in a pressure tube at 130-140 °C for 0.5-8h. The tube was cooled, the contents were poured into 100 mL of water and the product was extracted with ethylacetate (15 mL x 3). The combined extracts were washed with brine and dried over anhydrous Na₂SO₄. The product was concentrated, followed by column chromatography on silica gel using a mixture of hexane/EtOAc to afford the cyclized product, optionally substituted 3-(4-nitrophenyl)-2-trifluoromethylphenyl- indolizine in 10%-80% yields.

To a stirred solution of optionally substituted 3-(4-nitro-phenyl)-2-trifluoromethylphenyl-indolizine (1.31 mmols) in 20mL of 1:1 CH₂Cl₂:EtOH, was added of SnCl₂ (13.06 mmols) followed by a few drops of water. The mixture was stirred overnight and concentrated. To the residue, was added 20 mL of water and the solution the brought to a pH of approximately 8-9 using 2N NaOH. The resulting mixture was successively extracted with ethylacetate (20mL x 4), washed with brine (20mL) and dried over Na₂SO₄. The organic layer was concentrated *in vacuo* to afforded optionally substituted 4-(2-trifluoromethyl-indolyzin-3-yl)-phenylamine in 70-97% yields.

To a solution of optionally substituted 4-(2-trifluoromethyl-indolyzin-3-yl)-phenylamine (0.30 mmols) in 5 mL of CH₂Cl₂, was added the an acyl chloride (0.30mmols), followed by diisopropyl-ethylamine (0.60 mmols). The resultant mixture was stirred at room temperature for 30 min and eluted through a short pad of silica gel using mixture of hexane:ethylacetate to afford the product, compounds 33, 90, 91, 92, 93, or 125, in 80-96% yields.

### SYNTHESIS OF COMPOUNDS 127 AND 128

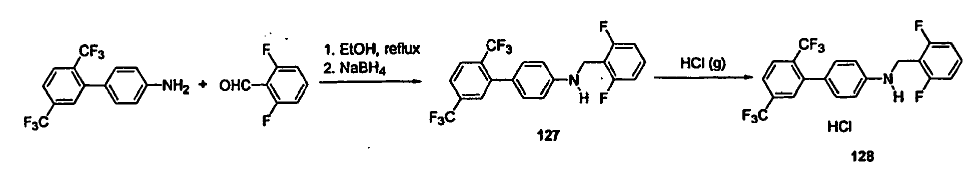

**General Procedure:** A stirred mixture of 2,5-bis-trifluoromethyl biphenyl-4-yl amine (1.0 mmol) and 2,6-difluoro-benzaldehyde (1.2 mmol) in ethanol (20 mL) was refluxed for 4 h. The mixture was cooled to room temperature, NaBH₄ (2 mmol) was added and the mixture was stirred for 4 h. The mixture was poured into water and extracted with CH₂Cl₂. The organic extract was washed with water and dried (Na₂SO₄). The oil obtained on concentration of the organic layer was flash chromatographed on silica gel to give compound 127 as yellowish oil (305 mg).
Gaseous HCl was bubbled through a stirred solution of compound 127 (294 mg) in EtOH for 5 min. Removal of solvent *in vacuo* gave salt 128 as white solid (323mg).

The other exemplary compounds of this invention were synthesized using analogous methods to those described above.

### REPRESENTATIVE ANALYTICAL DATA FOR OTHER EXEMPLARY COMPOUNDS OF THIS INVENTION:

### Compound 1:

¹H NMR (300 MHz, CDCl₃), δ(ppm): 8.48-8.67 (m, 3H); 8.02 (dd, *J* = 6.6 Hz, 5.1 Hz, 1H); 7.69-7.77 (m, 3H); 7.57 (t, *J* = 7.2 Hz, 1H); 7.48 (t, *J* = 7.2 Hz, 1H); 7.18-7.38 (m, 3H); ESMS calcd. for C₁₉H₁₁F₄N₂O (M - H)⁻: 359.0; Found: 359.0.

### Compound 2:

¹H NMR (300 MHz, CDCl₃), δ (ppm): 8.51-8.65 (m, 3H); 8.01 (d, *J* = 4.8 Hz, 1H); 7.69-7.72 (m, 2H); 7.57 (t, *J* = 7.2 Hz, 1H); 7.20-7.58 (m, 6H); 2.29 (s, 3H); ESMS calcd. for C₁₉H₁₆FN₂O (M + H)⁺: 307.1; Found: 307.1.

### Compound 3:

¹H NMR (300 MHz, CDCl₃), δ (ppm): 8.49-8.68 (m, 3H); 8.03 (t, *J* = 5.7 Hz, 1H); 7.54-7.83 (m, 7H); 7.18-7.30 (m, 1H); ESMS calcd. for C₁₉H₁₃F₄N₂O (M + H)⁺: 361.0; Found: 361.1.

### Compound 4:

¹H NMR (300 MHz, CDCl₃), δ (ppm): 8.36-8.48 (m, 1H); 7.74-8.06 (m, 4H); 7.26-7.59 (m, 6H); ESMS calcd. forC₂₁H₁₂F₈NO (M + H)⁺: 446.1; Found: 446.1.

### Compound 7:

¹H NMR (300 MHz, CDCl₃), δ (ppm): 9.11 (d, *J* = 2.1 Hz, 1H); 8.73 (dd, *J* = 4.8 Hz, 1.8 Hz, 1H); 8.60 (s, 1H); 8.22 (dt, *J* = 5.1 Hz, 2.1 Hz, 1H); 7.70-7.76 (m, 3H); 7.26-7.58 (m, 6H); ESMS calcd. for C₁₉H₁₄F₃N₂O (M + H)⁺: 343.1; Found: 343.1.

### Compound 8:

¹H NMR (300 MHz, CDCl₃), δ (ppm): 8.79 (dd, *J* = 7.5 Hz, 1.5 Hz, 2H); 8.29 (s, 1H); 7.70-7.76 (m, 5H); 7.57 (d, *J* = 7.5 Hz, 1H); 7.48 (d, *J* = 7.5 Hz, 1H); 7.26-7.37 (m, 3H); ESMS calcd. for C₁₉H₁₄F₃N₂O (M + H)⁺: 343.1; Found: 343.1.

### Compound 12:

¹H NMR (300 MHz, CDCl₃), δ (ppm): 9.00 (s, 1H); 8.92 (d, *J*= 5.4Hz, 2H); 7.26-7.77 (m, 9H); ESMS calcd. for C₂₀H₁₃F₆N₂O (M + H)⁺: 411.0; Found: 411.0.

### Compound 15:

¹H NMR (300 MHz, CDCl₃), δ (ppm): 8.39 (d, *J* = 12.9 Hz, 1H); 7.86-7.91 (m, 1H); 7.74 (d, *J* = 7.8 Hz, 1H); 7.70 (d, *J* = 8.4 Hz, 2H); 7.56 (t, *J* = 7.5 Hz, 1H); 7.46 (t, *J* = 7.5 Hz, 1H); 7.21-7.40 (m, 5H); ESMS calcd. for C₂₀H₁₃F₅NO (M + H)⁺: 378.0; Found: 378.0.

### Compound 16:

¹H NMR (300 MHz, CDCl₃), δ (pom): 8.55 (d, *J* = 12.9 Hz, 1H); 7.83-7.89 (m, 1H); 7.68-7.76 (m, 3H); 7.56 (t, *J* = 7.5 Hz, 1H); 7.46 (t, *J* = 7.5 Hz, 1H); 7.32-7.36 (m, 3H); 7.15-7.24 (m, 2H); ESMS calcd. for C₂₀H₁₃F₅NO (M + H)⁺: 378.0; Found: 378.0.

### Compound 19:

¹H NMR (300 MHz, CDCl₃), δ (ppm): 8.47 (d, *J* = 12.6 Hz, 1H); 7.69-7.86 (m, 4H); 7.54-7.60 (m, 3H); 7.180-7.35 (m, 3H); ESMS calcd. for C₂₀H₁₂F₆NO (M + H)⁺: 396.1; Found: 396.0.

### Compound 20:

¹H NMR (300 MHz, CDCl₃), δ (ppm): 8.37 (d, *J* = 13.2 Hz, 1H); 7.87-7.92 (m, 1H); 7.70 (d, *J* = 8.7 Hz, 2H); 7.45 (dd, *J* = 9.0 Hz, 2.4 Hz, 1H); 7.19-7.39 (m, 6H); ESMS calcd. for C₂₀H₁₂F₆NO (M + H)⁺: 396.1; Found: 396.0.

### Compound 21

¹H NMR (300 MHz, CDCl₃), δ (ppm): 8.42 (d, *J* = 12.9Hz, 1H), 7.94-7.89 (m, 1H), 7.85-7.80 (m, 3H), 7.52-7.49 (m, 2H), 7.45-7.34 (m, 2H), 7.31-7.24 (m, 1H), 6.77 (td, *J* = 1.2, 6.3Hz, 1H), 6.74 (s, 1H), 6.53 (td, *J* = 1.2, 7.5Hz, 1H); ESMS calcd. for C₂₂H₁₃F₅N₂O (M + H)⁺: 416.09; Found: 417.1

### Compound 22:

¹H NMR (300 MHz, CDCl₃), δ (ppm): 8.38 (d, *J* = 12.9 Hz, 1H); 7.89 (t, *J* = 7.2 Hz, 1H); 7.75 (d, *J* = 8.7 Hz, 2H); 7.19-7.58 (m, 7H); ESMS calcd. for C₂₀H₁₂F₆NO (M + H)⁺: 396.1; Found: 396.0.

### Compound 23:

¹H NMR (300 MHz, CDCl₃), δ (ppm): 8.43 (d, *J* = 12.9 Hz, 1H); 7.89 (t, *J* = 4.2 Hz, 1H); 7.77 (d, *J* = 8.7 Hz, 2H); 7.20-7.62 (m, 7H); ESMS calcd. for C₂₀H₁₂ClF₅NO (M + H)⁺: 412.0; Found: 412.0,

### Compound 27:

¹H NMR (300 MHz, CDCl₃), δ (ppm): 8.03 (s, 1H); 7.70 (d, *J* = 8.7 Hz, 2H); 725-7.63 (m, 5H); 2.97 (s, 3H); ESMS calcd. for C₁₇H₁₂ClF₃N₃OS (M + H)⁺: 398.0; Found: 398.0.

### Compound 29:

¹H NMR (300 MHz, CDCl₃), δ (ppm): 8.41 (d, *J* = 12.9 Hz, 1H); 7.87-7.92 (m, 1H); 7.75 (d, *J* = 8.7 Hz, 2H); 7.22-7.48 (m, 7H); ESMS calcd. for C₁₉H₁₂Cl₂F₂NO (M + H)⁺: 378.0; Found: 378.0.

### Compound 30

¹H NMR (300 MHz, CDCl₃), δ (ppm): 8.41 (d, *J* = 12.6 Hz, 1H); 7.9 (d, *J* = 7.2 Hz, 1H); 7.74 (d, *J* = 8.4 Hz, 2H); 7.59-7.65 (m, 2H); 7.51 (d, *J* = 8.4 Hz, 2H); 7.23-7.42 (m, 2H); 7.04 (d, *J* = 8.1 Hz, 1H); 3.89 (s, 3H); ESMS calcd. for C₂₁H₁₅F₂N₂O₂ (M + H)⁺: 365.0; Found: 365.0.

### Compound 31

¹H NMR (300 MHz, CDCl₃), δ (ppm): 8.38 (d, *J* = 12.6 Hz, 1H); 7.83-7.89 (m, 1H); 7.69 (dd, *J* = 8.4 Hz, 1.5 Hz, 2H); 7.54-7.57 (m, 2H); 7.19-7.35 (m, 2H); 6.82-6.93 (m, 3H); 3.80 (s, 3H); 3.75 (s, 3H); ESMS calcd. for C₂₁H₁₈F₂NO₃ (M + H)⁺: 370.1; Found: 370.1.

### Compound 34

¹H NMR (300 MHz, CDCl₃), δ (ppm): 7.85 (d, *J* = 8.7 Hz, 2H); 7.72 (br s, 1H); 7.41 (d, *J* = 5.1 Hz, 1H); 7.36 (d, *J* = 8.7 Hz, 2H); 7.01 (d, *J* = 5.1 Hz, 1H); 2.63 (s, 3H); ESMS calcd. for C₁₆H₁₁F₆N₄OS (M + H)⁺: 421.0; Found: 421.0.

### Compound 35

¹H NMR (300 MHz, CDCl₃), δ (ppm): 8.63 (d, *J* = 13.2 Hz, 1H); 7.88-7.97 (m, 3H); 7.27-7.44 (m, 5H); 7.06-7.08 (m, 1H); 6.97 (dd, *J* = 5.4 Hz, 3.9 Hz, 1H); ESMS calcd. for C₂₀H₁₂F₅N₄OS (M + H)⁺: 451.0; Found: 451.0.

### Compound 37

¹H NMR (300 MHz, CDCl₃), δ (ppm): 8.51 (d, *J* = 12.9 Hz, 1H); 8.27 (d, *J* = 8.1 Hz, 1H); 8.09 (dd, *J* = 6.9 Hz, 2.1 Hz, 2H); 7.86-7.92 (m, 3H); 7.72 (d, *J* = 7.8 Hz, 1H); 7.24-7.43 (m, 2H); ESMS calcd. for C₂₀H₁₁F₅N₃O (M + H)⁺: 404.0; Found: 404.0.

### Compound 38

¹H NMR (300 MHz, CDCl₃), δ (ppm): 9.95 (s, 1H); 8.29 (dd, *J* = 7.8 Hz, 1.8 Hz, 1H); 7.88 (d, *J* = 8.1 Hz, 1H); 7.78 (d, *J* = 8.1 Hz, 2H); 7.72 (d, *J* = 8.4 Hz, 1H); 7.64 (s, 1H); 7.48 (t, *J* = 7.8 Hz, 1H); 7.34 (d, *J* = 8.1 Hz, 2H); 7.11 (t, *J* = 7.8 Hz, 1H); 7.03 (d, *J* = 8.4 Hz, 1H); 4.04 (s, 3H); ESMS calcd. for C₂₂H₁₆F₆NO₂ (M + H)⁺: 440.1; Found: 440.2.

### Compound 39

¹H NMR (300 MHz, CDCl₃), δ (ppm): 8.64 (s, 1H); 7.89 (d, *J* = 8.4 Hz, 1H); 7.73-7.77 (m, 3H); 7.62 (s, 1H); 7.35 (d, *J* = 8.7 Hz, 2H); 6.56 (s, 1H); 2.53 (s, 3H); ESMS calcd. for C₁₉H₁₃F₆N₂O₂ (M + H)⁺: 415.1; Found: 415.1.

### Compound 40

¹H NMR (300 MHz, CDCl₃), δ (ppm): 8.02 (s, 1H); 7.87 (d, *J* = 8.4 Hz, 1H); 7.73 (d, *J* = 8.1 Hz, 1H); 7.66 (d, *J* = 8.4 Hz, 2H); 7.60 (s, 1H); 7.32 (d, *J* = 8.4 Hz, 1H); 4.13 (s, 3H); 2.27 (s, 3H); ESMS calcd. for C₂₀H₁₆F₆N₃O (M + H)⁺: 428.1; Found: 428.2.

### Compound 41

¹H NMR (300 MHz, CDCl₃), δ (ppm): 9.49 (s, 1H); 9.38 (s, 1H); 7.74-7.92 (m, 4H); 7.64 (s, 1H); 7.40 (d, *J* = 8.7 Hz, 2H); ESMS calcd. for C₁₇H₁₀F₆N₃OS (M + H)⁺: 418.0; Found: 418.1.

### Compound 42

¹H NMR (300 MHz, CDCl₃), δ (ppm): 8.43 (d, *J* = 2.1 Hz, 1H); 8.35 (s, 1H); 7.90 (d, *J* = 8.4 Hz, 1H); 7.76 (d, *J* = 8.4 Hz, 2H); 7.62 (s, 1H); 7.38 (d, *J* = 8.4 Hz, 2H); 7.08 (d, *J* = 2.1 Hz, 1H); ESMS calcd. for C₁₈H₁₁F₆N₂O₂ (M + H)⁺: 401.0; Found: 401.1.

### Compound 43

¹H NMR (300 MHz, CDCl₃), δ (ppm): 7.90 (d, *J* = 8.1 Hz, 1H); 7.75 (d, *J* = 8.1 Hz, 1H); 7.61-7.66 (m, 3H); 7.44 (s, 1H); 7.35 (d, *J* = 8.4 Hz, 2H); 2.69 (s, 3H); 2.52 (s, 3H); ESMS calcd. for C₂₀H₁₅F₆N₂O₂ (M + H)⁺: 429.1; Found: 429.2.

### Compound 44

¹H NMR (300 MHz, CDCl₃), δ (ppm): 8.41 (d, *J* = 12.3 Hz, 1H); 7.81-7.86 (m 1H); 7.70 (d, *J* = 8.4 Hz, 2H); 7.51 (d, *J* = 8.4 Hz, 2H); 7.16-7.34 (m, 4H); 6.88 (d, *J* = 8.4 Hz, 1H); 3.78 (s, 3H); ESMS calcd. for C₂₀H₁₅ClF₂NO₂ (M + H)⁺: 374.1; Found: 374.1.

### Compound 45

¹H NMR (300 MHz, CDCl₃), δ (ppm): 7.90 (d, *J* = 8.1 Hz, 1H); 7.50-7.75 (m, 6H); 7.28-7.40 (m, 5H); 2.54 (s, 3H); ESMS calcd. for C₂₂H₁₆F₆NO (M + H)⁺: 424.1; Found: 424.2.

### Compound 46

¹H NMR (300 MHz, CDCl₃), δ (ppm): 8.33 (d, *J* = 12.9 Hz, 1H); 7.91 (t, *J* = 7.2 Hz, 1H); 7.11-7.71 (m, 8H); 6.88 (d, *J* = 8.1 Hz, 1H); 3.79 (s, 3H); 2.34 (s, 3H); ESMS calcd. for C₂₁H₁₈F₂NO₂ (M + H)⁺: 354.1; Found: 354.1.

### Compound 47

¹H NMR (300 MHz, CDCl₃), δ (ppm): 8.34 (d, *J* = 13.2 Hz, 1H); 7.89-7.94 (m, 1H); 7.69 (d, *J* = 8.4 Hz, 2H); 7.22-7.42 (m, 3H); 7.16 (d, *J* = 7.2 Hz, 1H); 7.07 (d, *J* = 7.8 Hz, 2H); 2.35 (s, 3H); 2.25 (s, 3H); ESMS calcd. for C₂₁H₁₈F₂NO (M + H)⁺: 338.1; Found: 338.1.

### Compound 48

¹H NMR (300 MHz, CDCl₃), δ (ppm): 8.65 (s, 1H); 7.89 (d, *J* = 8.4 Hz, 1H); 7.73-7.76 (m, 3H); 7.62 (s, 1H); 7.36 (d, *J* = 8.7 Hz, 2H); 6.56 (s, 1H); 2.53 (s, 3H); ESMS calcd. for C₁₉H₁₃F₆N₂O₂ (M + H)⁺: 415.1; Found: 415.1.

### Compound 49

¹H NMR (300 MHz, CDCl₃), δ (ppm): 11.92 (s, 1H); 10.78 (s, 1H); 7.37-8.02 (m, 8H); 6.86-7.08 (m, 3H); ESMS calcd. for C₂₁H₁₄F₆NO₂ (M + H)⁺: 426.1; Found: 426.1.

### Compound 50

¹H NMR (300 MHz, CDCl₃), δ (ppm): 8.36 (d, *J* = 13.8Hz, 1H), 8.00-7.90 (m, 3H) 7.74-7.72 (m, 2H), 7.59-7.54 (m, 2H), 7.42-7.33 (m, 1H), 7.30-7.23 (m, 1H), 7.03 (d, *J* = 8.4Hz, 1H), 3.90 (s, 3H), 2.60 (s, 3H); ESMS calcd. for C₂₂H₁₇F₂NO₃ (M + H)⁺: 381.12; Found: 382.1

### Compound 51

¹H NMR (300 MHz, CDCl₃), δ (ppm): 8.48 (s, 1H); 7.90 (d, *J* = 8.1 Hz, 1H); 7.75 (d, *J* = 8.4 Hz, 1H); 7.61-7.67 (m, 3H); 7.42 (s, 1H); 7.35 (d, *J* = 8.7 Hz, 2H); 2.80 (s, 3H); ESMS calcd. for C₁₉H₁₃F₆N₂O₂ (M + H)⁺: 415.1; Found: 415.1.

### Compound 52

¹H NMR (300 MHz, CDCl₃), δ (ppm): 8.35 (d, *J* = 13.2 Hz, 1H); 7.87-7.93 (m, 1H); 7.68 (d, *J* = 8.4 Hz, 2H); 7.21-7.38 (m, 4H); 7.04 (d, *J* = 9.6 Hz, 2H); 2.28 (s, 3H); 2.26 (s, 3H); 2.23 (s, 3H); ESMS calcd. for C₂₂H₂₀F₂NO (M + H)⁺: 352.1; Found: 352.1.

### Compound 53

¹H NMR (300 MHz, CDCl₃), δ (ppm): 7.86 (d, *J* = 8.1 Hz, 1H); 7.70 (d, *J* = 8.4 Hz, 2H); 7.60 (s, 1H); 7.49 (d, *J* = 8.4 Hz, 2H); 7. 25 (d, *J* = 8.4 Hz, 2H); 6.38 (s, 1H); 3.40 (q, *J* = 7.2 Hz, 4H); 1.26 (t, *J* = 7.2 Hz, 6H); ESMS calcd. for C₁₉H₁₉F₆N₂O (M + H)⁺: 405.1; Found: 405.1.

### Compound 54

¹H NMR (300 MHz, CDCl₃), δ (ppm): 7.90 (d, *J* = 8.4 Hz, 1H); 7.70-7.76 (m, 3H); 7.62 (s, 1H); 7.52 (s, 1H); 7.16-7.36 (m, 5H); 2.41 (s, 3H); 2.34 (s, 3H); ESMS calcd. for C₂₃H₁₈F₆NO (M + H)⁺: 438.1; Found: 438.1.

### Compound 55

¹H NMR (300 MHz, CDCl₃), δ (ppm): 7.90 (d, *J*= 8.1 Hz, 1H); 7.69-7.76 (m, 3H); 7.61 (s, 2H); 7.48 (d, *J* = 8.1 Hz, 1H); 7.34-7.40 (m, 3H); 7.22 (t, *J* = 8.1 Hz, 1H); 2.52 (s, 3H); ESMS calcd. for C₂₂H₁₅ClF₆NO (M + H)⁺: 458.1; Found: 458.1.

### Compound 56

¹H NMR (300 MHz, CDCl₃), δ (ppm): 7.90 (d, *J* = 8.1 Hz, 1H); 7.61-7.76 (m, 5H); 7.12-7.37 (m, 5H); 5.42 (d, *J* = 2.1 Hz, 3H); ESMS calcd. for C₂₂H₁₅F₇NO (M + H)⁺: 458.1; Found: 458.1.

### Compound 57

¹H NMR (300 MHz, CDCl₃), δ (ppm): 7.90 (d, *J* = 8.4 Hz, 1H); 7.54-7.75 (m, 4H); 7.34 (d, *J* = 8.4 Hz, 2H); 7.23-7.29 (m, 1H); 7.06-7.11 (m, 2H); 6.96 (d, *J* = 8.1 Hz, 1H); 3.88 (s, 3H); 2.37 (s, 3H); ESMS calcd. for C₂₃H₁₈F₆NO₂ (M + H)⁺: 454.1; Found: 454.1.

### Compound 58

¹H NMR (300 MHz, CDCl₃), δ (ppm): 8.92 (d, *J* = 4.5 Hz, 1H); 8.27 (d, *J* = 8.4 Hz, 1H); 8.17 (d, *J* = 8.4 Hz, 2H); 7.62-7.93 (m, 7H); 7.50 (d, *J* = 4.5 Hz, 1H); 7.41 (d, *J*= 8.4 Hz, 2H); ESMS calcd. for C₂₄H₁₅F₆N₂O (M + H)⁺: 461.1; Found: 461.1.

### Compound 59

¹H NMR (300 MHz, CDCl₃), δ (ppm): 7.86 (s, 1H); 7.54-7.62 (m, 4H); 6.83-6.93 (m, 3H); 3.81 (s, 3H); 3.76 (s, 3H); 2.95 (s, 3H); ESMS calcd. for C₁₈H₁₈N₃O₃S (M + H)⁺: 356.1; Found: 356.1.

### Compound 60

¹H NMR (300 MHz, CDCl₃), δ (ppm): 6.83-7.69 (m, 12H); 3.81 (s, 3H); 3.76 (s, 3H); 2.51 (s, 3H); ESMS calcd. for C₂₂H₂₂NO₃ (M + H)⁺: 348.1; Found: 348.1.

### Compound 61

¹H NMR (300 MHz, CDCl₃), δ (ppm): 8.61 (dd, *J* = 4.8 Hz, 1.8 Hz, 1H); 7.89 (d, *J* = 8.4 Hz, 1H); 7.68-7.81 (m, 5H); 7.61 (s, 1H); 7.35 (d, *J* = 8.1 Hz, 2H); 7.21-7.25 (m, 1H); 2.74 (s, 3H); ESMS calcd. for C₂₁H₁₅F₆N₂O (M + H)⁺: 425.1; Found: 425.1.

### Compound 62

¹H NMR (300 MHz, CDCl₃), δ (ppm): 9.62 (s, 1H); 7.70-7.90 (m, 5H); 7.63 (s, 1H); 7.33 (d, *J* = 8.4 Hz, 2H); 6.95-7.07 (m, 2H); 4.50-4.52 (m, 2H); 4.34-4.37 (m, 2H); ESMS calcd. for C₂₃H₁₆F₆NO₃ (M + H)⁺: 468.1; Found: 468.1.

### Compound 63

¹H NMR (300 MHz, CDCl₃), δ (ppm): 8.43 (s, 1H); 7.89 (d, *J* = 8.1 Hz, 1H); 7.67-7.76 (m, 4H); 7.59 (d, *J* = 8.7 Hz, 2H); 7.33 (d, *J* = 8.4 Hz, 2H); 3.99 (s, 3H); ESMS calcd. for C₁₉H₁₄F₆N₃O (M + H)⁺: 414.1; Found: 414.1.

### Compound 64

¹H NMR (300 MHz, CDCl₃), δ (ppm): 8.40-8.48 (m, 3H); 7.68 (d, *J* = 8.7 Hz, 2H); 7.55 (d, *J* = 8.7 Hz, 2H); 7.28 (d, *J* = 4.8 Hz, 1H); 6.84-6.93 (m, 2H); 3.81 (s, 3H); 3.75 (s, 3H); 2.44 (s, 3H); ESMS calcd. for C₂₁H₂₁N₂O₃ (M + H)⁺: 349.1; Found: 349.1.

### Compound 65

¹H NMR (300 MHz, CDCl₃), δ (ppm): 8.60 (s, 1H); 8.38-8.48 (m, 2H); 7.90 (d, *J* = 8.1 Hz, 1H); 7.73-7.75 (m, 3H); 7.61 (s, 1H); 7.27-7.37 (m, 3H); 2.48 (s, 3H); ESMS calcd. for C₂₁H₁₅F₆N₂O (M + H)⁺: 425.1; Found: 425.1.

### Compound 66

¹H NMR (300 MHz, CDCl₃), δ (ppm): 8.67 (s, 1H); 8.44 (s, 1H); 8.37 (d, *J* = 5.1 Hz, 1H); 7.70 (d, *J* = 8.7 Hz, 2H); 7.51 (d, *J* = 8.7 Hz, 2H); 7.24-7.29 (m, 3H); 6.90 (d, *J* = 8.4 Hz, 1H); 3.79 (s, 3H); 2.44 (s, 3H); ESMS calcd. for C₂₀H₁₈ClN₂O₂ (M + H)⁺: 453.1; Found: 453.1.

### Compound 67

¹H NMR (300 MHz, CDCl₃), δ (ppm): 8.64-8.68 (m, 2H); 8.43 (d, *J* = 13.2 Hz, 1H); 8.05 (dd, *J* = 6.6 Hz, 4.8 Hz, 1H); 7.69-7.72 (m, 2H); 7.57-7.61 (m, 2H); 6.84-6.95 (m, 3H); 3.82 (s, 3H); 3.77 (s, 3H); ESMS calcd. for C₂₀H₁₈FN₂O₃ (M + H)⁺: 353.1; Found: 353.1.

### Compound 68

¹H NMR (300 MHz, CDCl₃), δ (ppm): 8.62-8.66 (m, 2H); 8.47 (d, *J* = 13.5 Hz, 1H); 8.01 (dd, *J* = 6.6 Hz, 4.8 Hz, 1H); 7.69-7.73 (m, 2H); 7.52-7.57 (m, 2H); 7.25-7.30 (m, 2H); 6.90 (d, *J* = 8.4 Hz, 1H); 3.80 (s, 3H); ESMS calcd. for C₁₉H₁₅ClFN₂O₂ (M + H)⁺: 357.1; Found: 357.1.

### Compound 69

¹H NMR (300 MHz, CDCl₃), δ (ppm): 8.66-8.70 (m, 2H); 8.49 (d, *J* = 13.8 Hz, 1H); 8.05 (dd, *J* = 6.6 Hz, 4.8 Hz, 1H); 7.90 (d, *J* = 8.7 Hz, 1H); 7.76 (d, *J* = 8.4 Hz, 2H); 7.63 (s, 1H); 7.38 (d, *J* = 8.4 Hz, 2H); ESMS calcd. for C₂₀H₁₂F₇N₂O (M + H)⁺: 429.1; Found: 429.1.

### Compound 70

¹H NMR (300 MHz, CDCl₃), δ (ppm): 8.73 (s, 1H); 8.41 (s, 1H); 8.35 (d, *J* = 4.5 Hz, 1H); 7.68 (d, *J* = 8.7 Hz, 2H); 7.53 (d, *J* = 8.7 Hz, 2H); 7.25 (d, *J* = 4.2 Hz, 1H); 7.10-7.13 (m, 2H); 6.87 (d, *J* = 9.3 Hz, 1H); 3.77 (s, 3H); 2.42 (s, 3H); 2.33 (s, 3H); ESMS calcd. for C₂₁H₂₁N₂O₂ (M + H)⁺: 333.1; Found: 333.1.

### Compound 72

¹H NMR (300 MHz, CDCl₃), δ (ppm): 8.00-7.94 (m, 2H), 7.80 (s, 1H), 7.67-7.65 (m, 2H), 7.59-7.55 (m, 2H), 7.03 (d, *J* = 8.7Hz, 1H), 3.90 (s, 3H), 3.00 (s, 3H), 2.59 (s, 3H); ESMS calcd. for C₁₉H₁₇N₃O₃S (M + H)⁺: 367.1; Found: 368.1

### Compound 73

¹H NMR (300 MHz, CDCl₃), δ (ppm): 8.68 (d, *J* = 3.0Hz, 1H), 8.66 (dd, *J* = 1.5, 4.8Hz,1H), 8.48 (d, *J* = 13.8Hz, 1H), 8.05 (dd, *J* = 4.8, 6.6Hz, 1H), 7.77-7.73 (m, 2H), 7.54-7.50 (m, 2H), 7.41 (d, *J* = 2.7Hz, 1H), 7.33 (dd, *J* = 2.7, 9.0Hz, 1H), 7.20 (d, *J* = 9.0Hz, 1H), 6.32 (t, *J* = 73.2Hz, 1H); ESMS calcd. for C₁₉H₁₂ClF₃N₂O₂ (M + H)⁺: 392.05; Found: 393.0

### Compound 74

¹H NMR (300 MHz, CDCl₃), δ (ppm): 7.63 (s, 5H), 7.16-7.08 (m, 2H), 7.00 (d, *J* = 8.7Hz, 1H), 3.00 (s, 3H), 2.54 (s, 6H); ESMS calcd. for C₁₉H₁₇F₃N₄O₂S (M + H)⁺: 422.1; Found: 423.3

### Compound 75

¹H NMR (300 MHz, CDCl₃), δ (ppm): 8.39-8.50 (m, 3H); 7.76 (d, *J* = 8.7 Hz, 2H); 7.46-7.62 (m, 5H); 7.33 (d, *J* = 4.8 Hz, 1H); 2.48 (s, 3H); ESMS calcd. for C₂₀H₁₅ClF₃N₂O (M + H)⁺: 391.1; Found: 391.3.

### Compound 76

¹H NMR (300 MHz, CDCl₃), δ (ppm): 8.50-8.55 (m, 2H); 7.90 (s, 1H); 7.70 (d, *J* = 8.4 Hz, 2H); 7.45 (d, *J* = 8.4 Hz, 2H); 7.21-7.38 (m, 5H); 2.49 (s, 3H); 2.39 (s 3H); ESMS calcd. for C₂₀H₁₉N₂OS (M + H)⁺: 335.1; Found: 335.1.

### Compound 77

¹H NMP (300 MHz, CDCl₃), δ (ppm): 8.50 (s, 1H); 8.46 (d, *J* = 4.8 Hz, 1H); 8.27 (s, 1H); 7.69 (d, *J* = 8.7 Hz, 2H); 7.18-7.36 (m, 7H); 2.62 (q, *J* = 7.8 Hz, 2H); 2.49 (s, 3H); 1.11 (t, *J* = 7.2 Hz, 3H); ESMS calcd. for C₂₁H₂₁N₂O (M + H)⁺: 317.2; Found: 317.3.

### Compound 78

¹H NMR (300 MHz, CDCl₃), δ (ppm): 8.54 (s, 1H); 8.51 (d, *J* = 4.8 Hz, 1H); 7.95 (s, 1H); 7.68 (d, *J* = 8.7 Hz, 2H); 7.16-7.41 (m, 7H); 3.08 (hept, *J* = 6.9 Hz, 1H); 2.50 (s, 3H); 1.17 (d, *J* = 6.9 Hz, 6H); ESMS calcd. for C₂₂H₂₃N₂O (M + H)⁺: 331.2; Found: 331.3.

### Compound 79

¹H NMR (300 MHz, CDCl₃), δ (ppm): 9.79 (s, 1H); 8.44 (d, *J* = 8.7 Hz, 1H); 7.92 (dd, *J* = 8.4 Hz, 2.7 Hz, 1H); 7.75 (d, *J* = 2.1 Hz, 1H); 7.51 (d, *J* = 7.2 Hz, 1H); 7.19-7.27 (m, 3H); 6.88-6.90 (m, 2H); 7.76 (d, *J* = 2.7 Hz, 1H); 3.83 (s, 3H); 3.75 (s, 3H); 2.51 (s, 3H); ESMS calcd. for C₂₁H₂₁N₂O₃ (M + H)⁺: 349.2; Found: 349.3.

### Compound 80

¹H NMR (300 MHz, CDCl₃), δ (ppm): 8.54-8.57 (m, 2H); 7.62-7.70 (m, 3H); 7.32-7.37 (m, 3H); 7.04-7.26 (m, 3H); 2.55-2.70 (m, 4H); 2.51 (s, 3H); 1.25 (t, *J* = 7.2 Hz, 3H); 1.10 (t, *J* = 7.2 Hz, 3H); ESMS calcd. for C₂₃H₂₅N₂O (M + H)⁺: 345.2; Found: 345.2.

### Compound 81

¹H NMR (300 MHz, CDCl₃), δ (ppm): 8.51 (s, 1H); 8.47 (d, *J* = 4.5 Hz, 1H); 8.12 (S, 1H); 7.68 (d, *J* = 8.4 Hz, 2H); 7.59 (d, *J* = 8.4 Hz, 2H); 7.18-7.34 (m, 3H); 6.95 (d, *J* = 8.4 Hz, 1H); 2.53 (s, 6H); 2.49 (s, 3H); ESMS calcd. for C₂₁H₂₁ClN₃O (M +H)⁺: 366.1; Found: 366.1.

### Compound 82

¹H NMR (300 MHz, CDCl₃), δ (ppm): 8.98 (s, 1H); 8.44 (s, 1H); 8.39 (d, *J* = 4.8 Hz, 1H); 7.89-7.92 (m, 1H); 7.68-7.76 (m, 3H); 7.26-7.38 (m, 3H); 6.92 (d, *J* = 8.4 Hz, 1H); 4.45-4.51 (m, 1H); 4.17 (q, *J* = 7.2 Hz, 2H); 2.85 (d, *J* = 4.8 Hz, 3H); 2.43 (s, 3H); 1.30 (t, *J* = 7.2 Hz, 3H); ESMS calcd. for C₂₃H₂₄N₃O₃ (M + H)⁺: 390.2; Found: 390.1

### Compound 83

¹H NMR (300 MHz, CDCl₃), δ (ppm): 8.48 (s, 1H); 8.43 (d, *J* = 5.1 Hz, 1H); 8.34 (s, 1H); 7.69 (d, *J* = 8.7 Hz, 2H); 7.55 (d, *J* = 8.7 Hz, 2H); 7.21-7.31 (m, 3H); 6.88 (d, *J* = 8.4 Hz, 1H); 4.01 (q, *J* = 7.2 Hz, 2H); 2.46 (s, 3H); 1.35 (t, *J* = 7.2 Hz, 3H); ESMS calcd. for C₂₁H₂₀ClN₂O₂(M + H)⁺: 367.1; Found: 367.1

### Compound 87

¹H-NMR (CDCl₃) δ (ppm) 7.8 (m, 3H), 7.6 (m, 2H), 7.4 (m, 4H), 7.0 (m, 2H); ESMS clcd for C₂₀H₁₁ClF₅NO: 412.0; Found: 412.0 (M+H)⁺.

### Compound 88

¹H-NMR (CDCl₃) δ (ppm) 7.9 (br, 1H), 7.6 (d, 2H, J = 8), 7.4 (m, 2H), 7.2 (d, 2H, J = 8), 7.1 (m, 1H), 7.0 (m, 1H), 6.9 (m, 2H), 2.30 (s, 3H), 2.18 (s, 3H); ESMS clcd for C₂₁H₁₇F₂NO: 338.1; Found: 338.0 (M+H)⁺.

### Compound 89

¹H-NMR (CDCl₃) δ (ppm) 8.0 (br, 1H), 7.6 (d, 2H, J = 8), 7.4 (m, 3H), 7.2 (m, 3H), 6.9 (m, 2H); ESMS clcd for C₁₉H₁₁Cl₂F₂NO: 378.0; Found: 378.0 (M+H)⁺.

### Compound 91

¹H NMR (300 MHz, CDCl₃), δ (ppm): 8.58 (d, *J* = 16.8Hz, 1H), 7.94-7.88 (m, 1H), 7.86-7.80 (m, 3H), 7.51 (d, *J* = 8.4Hz, 2H), 7.43 (d, *J* = 9.0Hz, 1H), 7.26-7.19 (m, 2H), 6.80-6.77 (m, 1H), 6.75 (s, 1H), 6.54 (td, *J* = 1.5, 7.8Hz, 1H); ESMS calcd, for C₂₂H₁₃F₅N₂O (M + H)⁺: 416.09; Found: 417.0

### Compound 92

¹H NMR (300 MHz, CDCl₃), δ (ppm): 7.87-7.83 (m, 2H), 7.80 (d, *J* = 8.7Hz, 2H), 7.53-7.41 (m, 5H), 6.95 (d, *J* = 8.4Hz, 1H), 6.80-6.78 (m, 1H), 6.74 (s, 1H), 6.56-6.51 (m, 1H), 3.98 (s, 3H), 3.97 (s, 3H); ESMS calcd, for C24H19F3N2O3 (M + H)⁺: 440.13; Found: 441.1

### Compound 126

¹H-NMR (CDCl₃) δ (ppm) 7.9 (br, 1H), 7.7 (d, 2H, J = 8), 7.5 (d, 2H, J = 8), 7.4 (m, 1H), 7.0 (m, 2H), 6.9 (m, 3H), 3.80 (s, 3H), 3.70 (s, 3H); ESMS clcd for C₂₁H₁₇F₂NO₃: 370.1; Found: 370.0 (M+H)⁺.

### Compound 127

¹H-NMR (CDCl₃) δ (ppm) 7.9 (m, 1H), 7.7 (m, 3H), 7.61 (s, 1H), 7.5 (m, 1H), 7.4 (d, 2H, J = 8), 7.0 (m, 3H); ESMS clcd for C₂₁H₁₁F₈NO: 446.5; Found: 446.0 (M+H)⁺.

### Compound 127

¹H-NMR (DMSO-d₆) δ (ppm) 10.9 (br, 1H), 7.8 (m, 3H), 7.6 (d, 2H, J = 8), 7.4 (m, 1H), 7.2 (m, 4H), 2.40 (s, 3H); ESMS clcd for C₂₁H₁₄F₅NO: 392.1; Found: 392.0 (M+H)⁺.

### Compound 128

¹H-NMR (DMSO-d₆) δ (ppm) 8.0 (m, 2H), 7.65 (s, 1H), 7.4 (m, 1H), 7.1 (m, 4H), 6.7 (d, 2H, J = 8), 6.3 (t, 1H, J = 6), 4.3 (d, 2H, J = 6); ESMS clcd for C₂₁H₁₃F₈N: 432.1; Found: 432.0 (M+H)⁺.

### Compound 129

¹H-NMR (DMSO-d₆) δ (ppm) 8.0 (d, 1H, J = 8), 7.9 (d, 1H, J = 8), 7.65 (s, 1H), 7.4 (m, 1H), 7.1 (m, 4H), 6.7 (d, 2H, J = 8), 4.3 (s, 2H); ESMS clcd for C₂₁H₁₃F₈N: 432.1; Found: 432.0 (M+H)⁺.

### Compound 130

¹H-NMR (CDCl₃) δ (ppm) 7.3 (m, 3H), 7.2 (m, 2H), 6.9 (m, 3H), 6.7 (d, 2H, J = 8), 4.4 (d, 2H, J = 6), 4.2 (t, 1H, J = 6), 3.78 (s, 3H); ESMS clcd for C₂₀H₁₆ClF₂NO: 360.1; Found: 360.0 (M+H)⁺.

### Compound 131

¹H-NMR (DMSO-d₆) δ (ppm) 7.4 (m, 1H), 7.3 (m, 3H), 7.2 (d, 1H, J = 3), 7.1 (m, 3H), 6.7 (d, 2H, J = 8), 4.3 (s, 2H), 3.74 (s, 3H); ESMS clcd for C₂₀H₁₆ClF₂NO: 360.1; Found: 360.0 (M+H)⁺.

### Compound 132

¹H NMR (300 MHz, CDCl₃), δ (ppm): 7.68-7.62 (m, 3H), 7.53-7.50 (m, 2H), 7.41 (d, *J* = 2.7Hz, 1H), 7.33 (dd, *J* = 2.7, 8.7Hz, 1H), 7.20 (d, *J* = 8.7Hz, 1H), 6.31 (t, *J* = 73.8Hz, 1H), 3.00 (s, 3H); ESMS calcd. for C₁₇H₁₂ClF₂N₃O₂S (M + H)⁺: 395.03; Found: 396.0

### EXAMPLE 2: INHIBITION OF IL-2 PRODUCTION

Jurkat cells were placed in a 96 well plate (0.5 million cells per well in 1% FBS medium) then test compounds of this invention were added at different concentrations. After 10 minutes, the cells were activated with PHA (final concentration 2.5 µg/mL) and incubated for 20 hours at 37°C under CO₂. The final volume was 200 µL. Following incubation, the cells were centrifuged and the supernatants collected and stored at -70°C prior to assaying for IL-2 production. A commercial ELISA kit (IL-2 Eli-pair, Diaclone Research, Besancon, France) was used to detect production of IL-2, from which dose response curves were obtained. The IC₅₀ value was calculated as the concentration at which 50% of maximum IL-2 production after stimulation was inhibited versus a non-stimulation control.

| IC₅₀ | Compounds |
|---|---|
| <100 nM | 18, 20, 21, 23*, 24, 27, 28, 43, 44, 45, 46, 47, 50, 52, 53, 54, 55, 56, 58, 59, 63, 64, 65, 66, 67, 68, 69, 72, 73, 74, 75, 76, 79, 81, 82, 83, 84, 85, 86, 87, 88, 89, 125, 126, 127, 128, 129, 130, and 131 |
| 100-500 nM | 1, 2, 8, 12, 15, 16, 19, 22, 32, 33, 34, 36, 37, 38, 40, 41, 42, 48, 51, 60, 62, 70, 71, 77, 78, 80, 90, and 91 |
| 500 nM -1 µM | 3, 4, 7, 35, 39, 49, 57, 61, and 131 |
| >1 µM | 5, 6, 9, 10, 11, 13, 14, 17, 25, and 26 |
| Not tested | 29, 30, 31 |

| | |
|---|---|
| * Lowest IC₅₀ value | |

### EXAMPLE 3: ACTIVATION OF TRPM4 CHANNEL

TRPM4 currents were measured in Jurkat cells and HEK-293 cells overexpressing TRPM4. The external solution contained the following (mM): NaCl 140, KCl 2.8, MgCl₂ 2, CaCl₂ 1, glucose 10, and HEPES-NaOH 10 (ph 7.2). The internal solution contained the following (mM): K-glutamate 120, NaCl 8, MgCl₂ 1, K-BAPTA 10, HEPES·CsOH 10 (ph 7.2). Ramps were given every 2 s (-100 to +100 mV in 50 ms) and cells were held at -80 mV between ramps. Free intracellular calcium was adusted to 300 nM.

Representative compounds of this invention (including Compounds 23 and 24) were tested for the ability to activate TRPM4 using this method. EC₅₀ values ranged from 20-50 nM.

### EXAMPLE 4: PATCH CLAMP STUDIES OF INHIBITION OF I_{CRAC} CURRENT IN RBL CELLS, JURKAT CELLS, AND PRIMARY T CELLS

In general, a whole cell patch clamp method was used to examine the effects of a compound of the invention on a channel that mediates I_{crac}. In such experiments, a baseline measurement was established for a patched cell. Then a compound to be tested was perfused (or puffed) to cells in the external solution and the effect of the compound on I_{crac} was measured. A compound that modulates I_{crac} (e.g., inhibits) is a compound that is useful in the invention for modulating CRAC ion channel activity.

### 1) RBL cells

### Cells

Rat basophilic leukemia cells (RBL-2H3) were grown in DMEM media supplemented with 10% fetal bovine serum in an atmosphere of 95% air/5% CO₂. Cells were seeded on glass coverslips 1-3 days before use.

### Recording Conditions

Membrane currents of individual cells were recorded using the whole-cell configuration of the patch clamp technique with an EPC10 (HEKA Electronik, Lambrecht, Germany). Electrodes (2-5 MΩ in resistance) were fashioned from borosilicate glass capillary tubes (Sutter Instruments, Novato, Ca). The recordings were done at room temperature.

### Intracellular pipette solution

Cs-Glutamate 120mM; CsCl 20mM; CsBAPTA 10mM; CsHEPES 10mM; NaCl 8mM; MgCl₂ 1mM; IP3 0.02mM; pH=7.4 adjusted with CsOH. (Shielded from light and kept on ice before experiment)

### Extracellular solution

NaCl 138mM; NaHEPES, 10mM; CsCl 10mM; CaCl₂ 10mM; Glucose 5.5mM; KCl 5.4mM; KH₂PO₄ 0.4mM; Na₂HPO₄H₂ O.3mM at pH=7.4 adjusted with NaOH.

### Compound treatment

Each compound was diluted from a 10 mM stock in series using DMSO (10µM, 3.2µM, 1µM, 316 nM, 100 nM 32 nM). The final DMSO concentration was always kept at 0.1 %.

### Experimental procedure

I_{CRAC} currents were monitored every 2 seconds using a 50 msec protocol, where the voltage was ramped from -100 mV to +100 mV. The membrane potential was held at 0 mV between the test ramps. In a typical experiment the peak inward currents would develop within 50-100 seconds. Once the I_{CRAC} currents were stabilized, the cells were perfused with compounds in the extracellular solution. At the end of an experiment the remaining I_{CRAC} currents were then challenged with a control compound (SKF96365, 10 µM) to ensure that the current could still be inhibited.

### Data analysis

The I_{CRAC} current level was determined by measuring the inward current amplitude at -80 mV of the voltage ramp in an off-line analysis using MATLAB. The I_{CRAC} current inhibition for each concentration was calculated using peak amplitude in the beginning of the experiment from the same cell. The IC₅₀ value and Hill coefficient for each compound was estimated by fitting all the individual data points to a single Hill equation.

### Results

Table 1 shows the concentration of compounds of the invention which inhibits 50 % of the I_{CRAC} current in RBL cells. As can be seen from the data in Table 1, several compounds of the invention inhibit I_{CRAC} current at concentration of less than 300 nM.

**Table 1: Concentration of compounds of the invention which inhibit 50 % of the I_{CRAC} current in RBL cells.**

| **Compound Number** | **IC₅₀** |
|---|---|
| 31 | 0.1 µM |
| 75 | 0.2 µM |
| 66 | 0.3 µM |
| 84 | 0.3 µM |
| 27 | 0.4µM |
| 79 | 0.8 µM |
| SKF96365 | 4 µM |

### 2) Jurkat cells

### Cells

Jurkat T cells were grown on glass coverslips, transferred to the recording chamber and kept in a standard modified Ringer's solution of the following composition: NaCl 145mM, KCl 2.8mM, CsCl 10mM, CaCl₂ 10mM, MgCl₂ 2mM, glucose 10mM, HEPES·NaOH 10mM, pH 7.2.

### Extracellular Solution

The external solution contained 10 mM CaNaR, 11.5 mM glucose and the test compound at the concentrations described below.

### Intracellular Pipette Solution

The standard intracellular pipette solution contained: Cs-glutamate 145 mM, NaCl 8 mM, MgCl₂ 1 mM, ATP 0.5 mM, GTP 0.3 mM, pH 7.2 adjusted with CsOH. The solution was supplemented with a mixture of 10 mM Cs-BAPTA and 4.3-5.3 mM CaCl₂ to buffer [Ca²⁺]i to resting levels of 100-150 nM.

### Patch-clamp recordings

Patch-clamp experiments were performed in the tight-seal whole-cell configuration at 21-25 °C. High-resolution current recordings were acquired by a computer-based patch-clamp amplifier system (EPC-9, HEKA, Lambrecht, Germany). Sylgard®- coated patch pipettes had resistances between 2-4 MΩ after filling with the standard intracellular solution. Immediately following establishment of the whole-cell configuration, voltage ramps of 50 ms duration spanning the voltage range of -100 to +100 mV were delivered from a holding potential of 0 mV at a rate of 0.5 Hz over a period of 300 to 400 seconds. All voltages were corrected for a liquid junction potential of 10 mV between external and internal solutions. Currents were filtered at 2.3 kHz and digitized at 100 µs intervals. Capacitive currents and series resistance were determined and corrected before each voltage ramp using the automatic capacitance compensation of the EPC-9.

### Data analysis

The very first ramps before activation of I_{CRAC} (usually 1 to 3) were digitally filtered at 2 kHz, pooled and used for leak-subtraction of all subsequent current records. The low-resolution temporal development of inward currents was extracted from the leak-corrected individual ramp current records by measuring the current amplitude at -80 mV or a voltage of choice.

### Results

1 µM Compound 66 resulted in >90% inhibition of I_{crac} in Jurkat cells (n = 3).
1 µM Compound 31 resulted in >43% inhibition of I_{crac} in Jurkat cells (n = 3).
10 µM Compound 66 had no effect on TRPM4 currents in Jurkat cells.

### 3) Primary T Cells

### Preparation of Primary T Cells

Primary T cells were obtained from human whole blood samples by adding 100µL of RosetteSep® human T cell enrichment cocktail to 2 mL of whole blood. The mixture was incubated for 20 minutes at room temperature, then diluted with an equal volume of PBS containing 2% FBS. The mixture was layered on top of RosetteSep® DM-L density medium and then centrifuged for 20 minutes at 1200 g at room temperature. The enriched T cells were recovered from the plasma/density medium interface, then washed with PBS containing 2% FBS twice, and used in patch clamp experiments following the procedure described for RBL cells.

### Results:

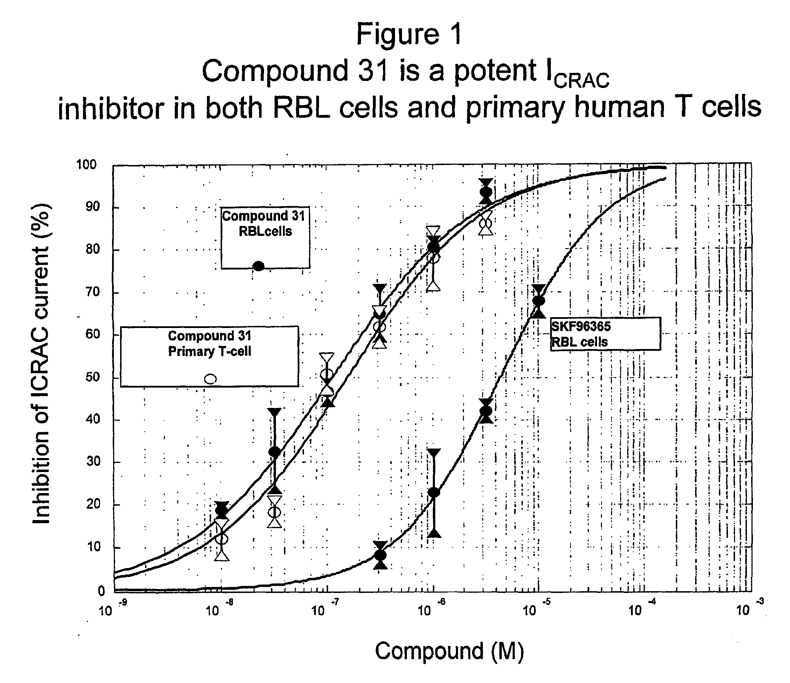

Figure 1 is a graph of percent inhibition of I_{CRAC} current in RBL cells and primary T cells in the presence of varying concentrations of compound 31. Inhibition of I_{CRAC} current by SKF96365, a known inhibitor of I_{CRAC} was used as a control. As can be seen from Figure 1 compound 31 inhibits I_{CRAC} current equally well in primary T cells as in RBL cells.

### EXAMPLE 5: INHIBITION OF MULTIPLE CYTOKINES IN PRIMARY HUMAN PBMCs

Peripheral blood mononuclear cells (PBMCs) were stimulated with phytohemagglutinin (PHA) in the presence of varying concentrations of compounds of the invention or cyclosporine A (CsA), a known inhibitor of cytokine production. Cytokine production was measured using commercially available human ELISA assay kits (from Cell Science, Inc.) following the manufacturers instructions.

Table 2 shows the concentration of CsA and compounds 31, 66, and 75 which inhibit 50% of a cytokine production. As can be seen from the data, compounds 31, 66, and 75 are potent inhibitors of IL-2, IL-4, IL-5, IL-13, GM-CSF, INF-γ and TNF-α. In addition, compounds of the invention do not inhibit the anti-inflammatory cytokine, IL-10.

**Table 2: IC₅₀ values for cytokine inhibition.**

| **Compound Number** | **IL-2 (nM)** | **IL-4 (nM)** | **IL-5 (nM)** | **IL-10 (nM)** | **IL-13 (nM)** | **GM-CSF (nM)** | **INF-γ (nM)** | **TNF-α (nM)** |
|---|---|---|---|---|---|---|---|---|
| CsA | 3 | 25 | 7 | 948 | 67 | 109 | 18 | 26 |
| 75 | 4 | 103 | 7 | >1000 | 15 | 152 | 23 | 81 |
| 31 | 6 | 71 | 11 | >1000 | 51 | 97 | 42 | 68 |
| 66 | 20 | 445 | 21 | >1000 | 75 | 448 | 88 | 271 |

### EXAMPLE 6: COMPOUNDS OF THE INVENTION ARE POTENT INHIBITORS OF DEGRANULATION IN RBL CELLS

### Procedure:

The day before the assay was performed, RBL cells, that had been grown to confluence in a 96 well plate, were incubated at 37°C for at least 2 hours. The medium was replaced in each well with 100 µL of fresh medium containing 2µLg/mL of anti-DNP IgE.

On the following day, the cells were washed once with PRS (2.6 mM glucose and 0.1% BSA) and 160µL of PRS was added to each well. The test compound was added to a well in a 20µL solution at 10X of the desired concentration and incubated for 20 to 40 minutes at 37°C. 20µL of 10X mouse anti-IgE (10 µL/mL). Maximum degranulation occurred between 15 to 40 minutes after addition of anti-IgE.

### Results:

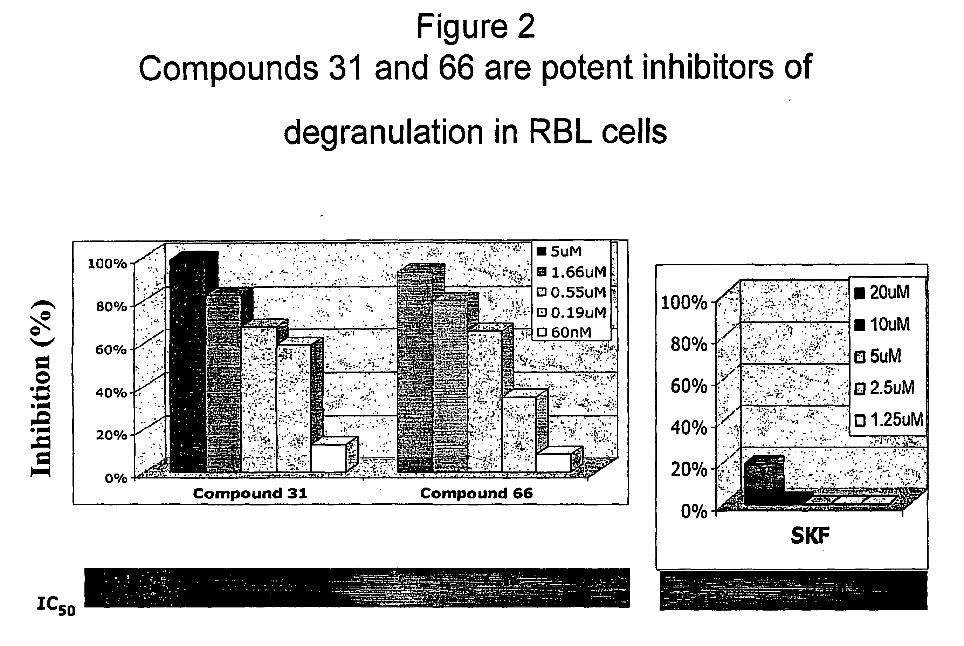

Figure 2 is a graph of the percent inhibition of degranulation in RBL cell at various concentrations of compound 31 and compound 66. The concentration at which 50% of degranulation is inhibited is 0.38µM for compound 31 and 0.43µM for compound 66. The known inhibitor of stored-operated channels, SKF96365, required greater than 20 µM to inhibit 50% of degranulation.

### EXAMPLE 7: CYTOKINE INHIBITION IN WHOLE BLOOD COLLECTED FROM CYNOMOLGUS MONKEYS AFTER ADMINISTRATION OF COMPOUNDS OF THE INVENTION

### Procedure for IV and Oral Dosing:

3 male non-naïve cynomolgus monkeys were feasted overnight prior to and for four (4) hours following each dose. Each monkey was given a single intravenous dose of the appropriate test compound followed by a 1 mL rinse of saline to flush the catheter. The dose was infused over one hour using calibrated syringe pumps. Following a one week washout, the same three monkeys were given a single oral gavage dose of the appropriate test compound dose formulation followed by a 10 mL flush with tap water. Following a second one week washout, the same three monkeys received a second oral dose of the appropriate test compound dose formulation followed by a 10 mL flush with tap water. Following a third one week washout, the same three monkeys received a third oral dose of the appropriate test compound dose formulation followed by a 10 mL flush with tap water. Prior to and at 1, 2, and 4 hours following each dose (measured from the start of dosing for IV doses), blood samples (3 mL/sample for Group 1 and 2 mL/sample for Groups 2 and 3) were drawn into tubes containing sodium heparin and stored at room temperature, and the concentration of the test compounds in each sample was measured. Table 3 summarizes the dose level, dose route and vehicle receive by each monkey. CsA was included as a positive control.

| **Dose** | **Group** | **Monkey** | **Test compound Formulation** | **Dose Route** | **Vehicle** | **Dose Level (mg/kg)** | **Dose Volume (mL/kg)** | **Matrix Collected** |
|---|---|---|---|---|---|---|---|---|
| 1 | 1 | 1 | CsA | IV | 5% PEG 200 / 5% Cremophor EL / 4.5% Dextrose | 5 | 2 | Blood^{a} |
| | 2 | 2 | Compound 31 | IV | 5% PEG 200 / 5% Cremophor EL / 4.5% Dextrose | 5 | 2 | Blood^{b,c} |
| | 3 | 3 | Compound 75 | IV | 5% PEG 200 / 5% Cremophor EL / 4.5% Dextrose | 5 | 2 | Blood^{b,c} |
| 2 | 1 | 1 | CsA | PO | 0.5% methylcellulose | 30 | 1 | Blood^{a} |
| | 2 | 2 | Compound 31 | PO | 0.5% methylcellulose | 30 | 1 | Blood^{b,c} |
| | 3 | 3 | Compound 75 | PO | 0.5% methylcellulose | 30 | 1 | Blood^{b,c} |
| 3 | 1 | 1 | CsA | PO | 0.5% methylcellulose | 30 | 1 | Blood^{a} |
| | 2 | 2 | Compound 31 | PO | 0.5% methylcellulose | 30 | 1 | Blood^{b,c} |
| | 3 | 3 | Compound 75 | PO | 0.5% methylcellulose | 30 | 1 | Blood^{b,c} |
| 4 | 1 | 1 | CsA | PO | 0.5% methylcellulose | 30 | 1 | Blood^{a} |
| | 2 | 2 | Compound 31 | PO | 0.5% methylcellulose | 30 | 1 | Blood^{b,c} |
| | 3 | 3 | Compound 75 | PO | 0.5% methylcellulose | 30 | 1 | Blood^{b,c} |

| | | | | | | | | |
|---|---|---|---|---|---|---|---|---|
| ^{a} 3 mL of blood collected prior to dosing and at 1, 2, and 4 hours postdose into tubes containing sodium heparin. ^{b}2 mL of blood collected prior to dosing and at 1, 2, and 4 hours postdose into tubes containing sodium heparin. ^{c} 0.5 mL of blood collected prior to dosing and at 1, 2, 4, 8, and 24 hours postdose into tubes containing sodium heparin | | | | | | | | |

### Results:

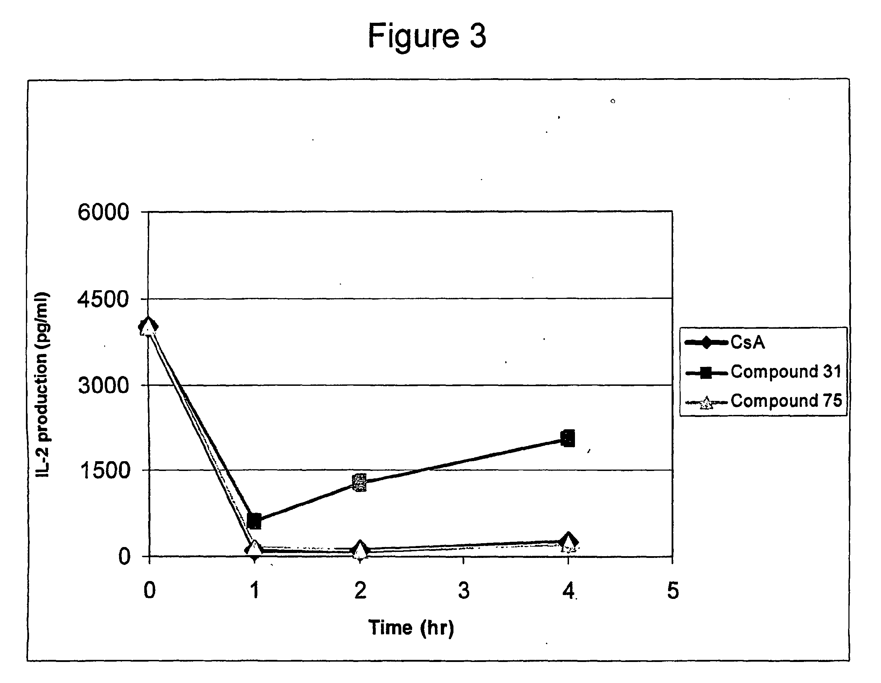

IL-2 production after stimulation with PMA/ionomycin in whole blood samples taken before dosing and at 1, 2, and 4 hours IV infusion is shown in Figure 3. The data indicates that 4 hours after dosing compounds 31 and 75 significantly inhibit IL-2 production.

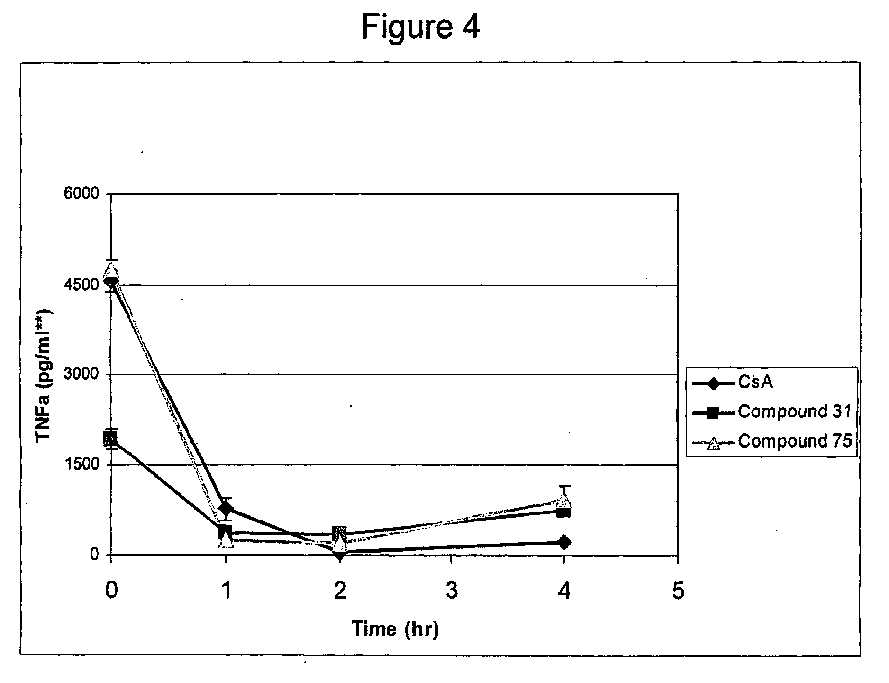

TNF-α production after stimulation with PMA/ionomycin in whole blood samples taken before dosing and at 1, 2, and 4 hours after IV infusion is shown in Figure 4. The data indicates that 4 hours after dosing both compounds 31 and 75 significantly inhibit TNF-α production.

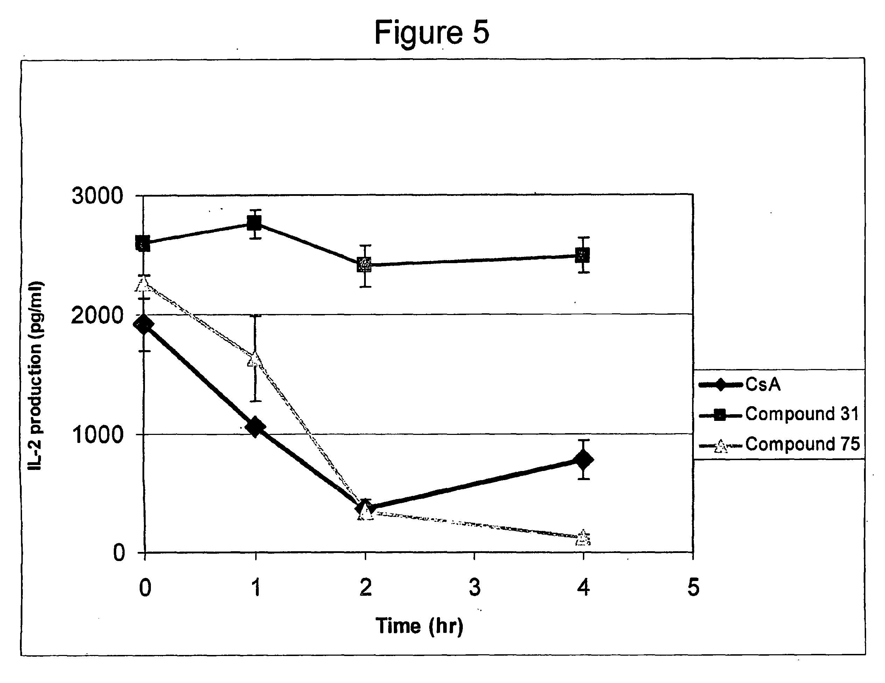

IL-2 production after stimulation with PMA/ionomycin in whole blood samples taken before dosing and at 1, 2, and 4 hours after oral dosing is shown in Figure 5. The data indicates that 4 hours after dosing compound 75 significantly inhibit IL-2.

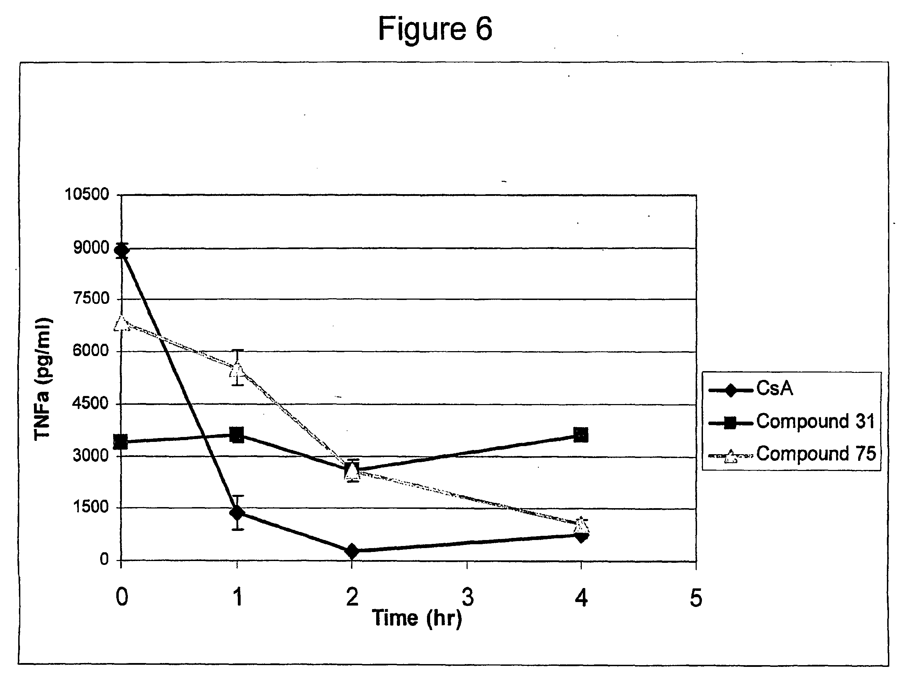

TNF-α production after stimulation with PMA/ionomycin in whole blood samples taken before dosing and at 1, 2, and 4 hours after oral dosing is shown in Figure 6. The data indicates that 4 hours after dosing compound 75 significantly inhibit TNF-α production.

## Claims

1. An in vitro method of inhibiting immune cell activation comprising administering to the cell a compound of formula (I): 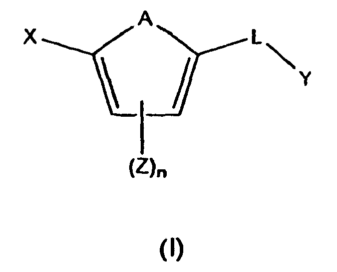 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
A is -CH=CH-, -CZ=CH-, or -CH=CZ-;
each Z is independently selected from the group consisting of an optionally substituted alkyl, an optionally substituted alkenyl, an optionally substituted-alkynyl, an-optionally substituted-cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4; and
p is 0, 1, or 2.

2. An in vitro method of inhibiting cytokine production in a cell, comprising administering to the cell a compound represented by the following structural formula: 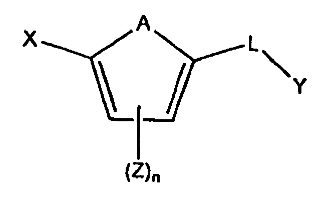 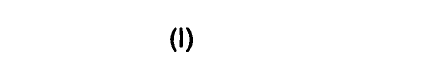 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
A is -CH=CH-, -CZ=CH-, or -CH=CZ-.
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4; and
p is 0, 1, or 2.

3. The method of Claim 2, wherein the cytokine which is inhibited is selected from the group consisting of IL-2, IL-4, IL-5, IL-13, GM-CSF, TNF-α, IFN-γ and combinations thereof.

4. An in vitro method of modulating an ion channel in a cell, wherein the ion channel is involved in immune-cell activation, comprising administering to the cell a compound represented by the following structural formula: 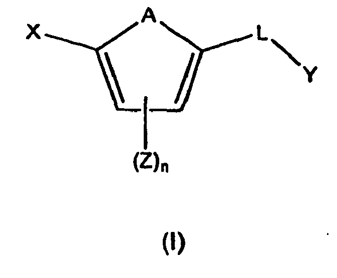 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
A is -CH=CH-, -CZ=CH-, or -CH=CZ-.
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or
R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is an integer selected from 0-4; and
p is 0, 1, or 2.

5. The method of Claim 4, wherein the ion channel is TRPM4.

6. An in vitro method of inhibiting T-cell and/or B-cell proliferation in response to an antigen, comprising administering to the cell a compound represented by the following structural formula: 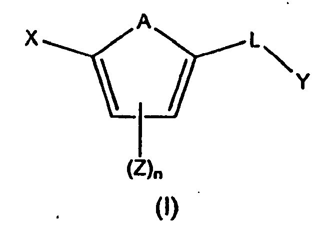 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
A is -CH=CH-, -CZ=CH-, or -CH=CZ-.
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)pNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally-substituted-heterocyclyl, an optionally substituted aryl, an optionally substituted heteroaryl, an optionally substituted aralkyl, or an optionally substituted heteraralkyl; or
R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4; and
p is 0, 1, or 2.

7. The method of Claim 1, 2, 4 or 6 wherein the compound is represented by the following structural formula: 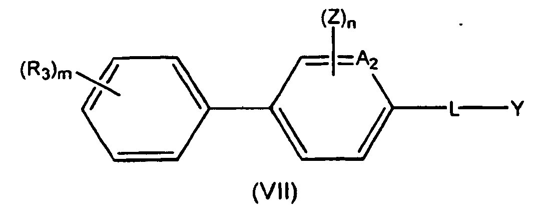 or a pharmaceutically acceptable salt, solvate, or clathrate thereof, wherein:
A₂ is CH or CZ;
R₃ is an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂; and
m is 0 or an integer from 1 to 5.

8. The method of Claim 7, wherein A₂ is CH.

9. The method of Claim 7, wherein L is -NHC(O)- or -NHCH₂-.

10. The method of Claim 9, wherein Y is an optionally substituted phenyl, an optionally substituted pyridyl, an optionally substituted thiophenyl, [1,2,3]thiadiazolyl, an optionally substituted isoxazolyl, 1H-pyrazolyl, quinolinyl, imidazolyl, or 2,3-dihydrobenzo[1,4]dioxine.

11. The method of Claim 10, wherein Y is an optionally substituted phenyl, an optionally substituted pyridyl, or an optionally substituted [1,2,3]thiadiazolyl.

12. The method of Claim 11, wherein the compound is represented by the following structural formula: 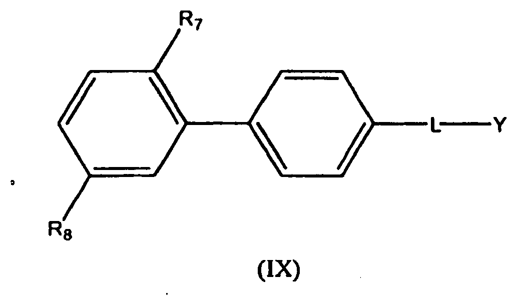 or a pharmaceutically acceptable salt, solvate, or clathrate thereof, wherein:
R₇ and R₈ are each, independently, -H, -CF₃, -CN, -C(O)CH₃, -F, -Cl, -OCH₃, -OCH₂CH₃, -C(O)OCH₂CH₃, -SCH₃, -NHCH₃, or C₁-C₄ alkyl, provided that at least one of R₇ or R₈ is not -H.

13. The method of Claim 12, wherein the compound is represented by the following structural formula: 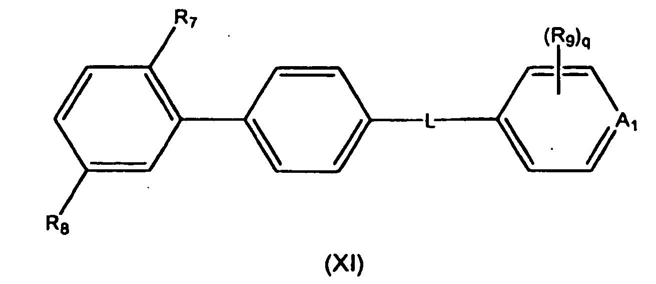 or a pharmaceutically acceptable salt, solvate, or clathrate thereof, wherein:
A₁ is CH, CR₉, N or N→O;
R₉, for each occurrence, is, independently, halo, C₁-C₄ alkyl, C₁-C₄ haloalkyl, C₁-C₄ alkoxy, C₁-C₄ haloalkoxy, or hydroxyl; and
q is 0 or an integer from 1 to 5.

14. The method of Claim 13, wherein the compound is represented by the following structural formula: 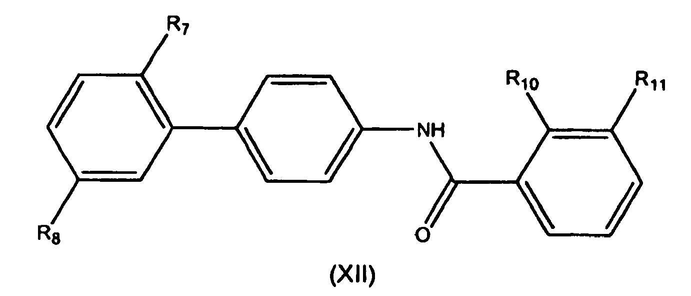 or a pharmaceutically acceptable salt, solvate, or clathrate thereof, wherein R₁₀ and R₁₁ are each, independently, -F, -Cl, a C₁-C₄ alkyl, a C₁-C₄ haloalkyl, a C₁-C₄ alkoxy or a C₁-C₄ haloalkoxy.

15. The method of Claim 13, wherein the compound is represented by the following structural formula: 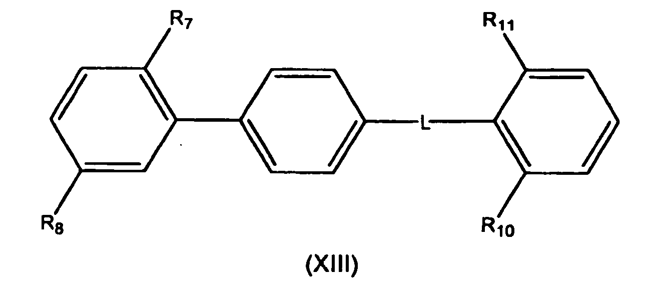 or a pharmaceutically acceptable salt, solvate, or clathrate thereof, wherein R₁₀ and R₁₁ are each, independently, -F, -Cl, a C₁-C₄ alkyl, a C₁-C₄ haloalkyl, a C₁-C₄ alkoxy and a C₁-C₄ haloalkoxy.

16. The method of Claim 1, 2, 4 or 6, wherein the compound is represented by the following structural formula: 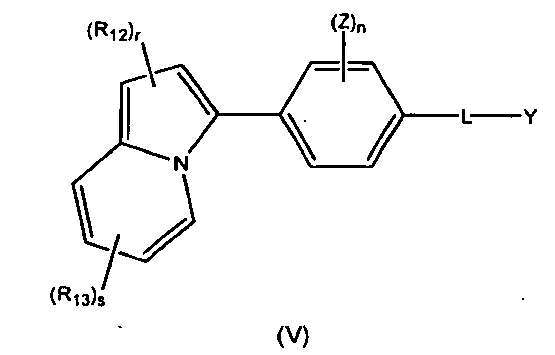 or a pharmaceutically acceptable salt, solvate, or clathrate thereof, wherein:
R₁₂ and R₁₃, for each occurrence, is, independently, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
r is 0, 1 or 2; and
s is 0 or an integer from 1 to 4.

17. The method of Claim 16, wherein L is -NHC(O)- and Y is an optionally substituted phenyl.

18. The method of Claim 17, wherein the compound is represented by the following structural formula: 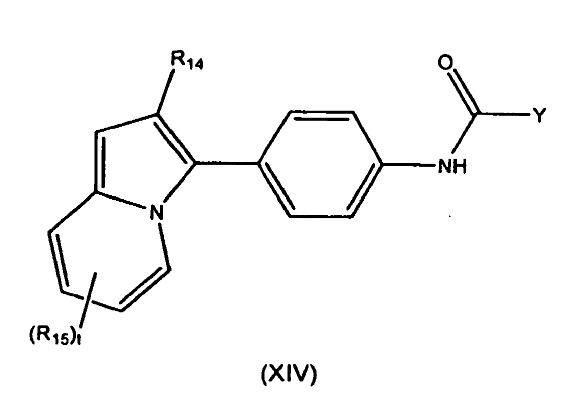 or a pharmaceutically acceptable salt, solvate, or clathrate thereof, wherein:
R₁₄ and R₁₅ are each, independently, -CF₃, -OCH₃, -F, -Cl, or -C(O)OCH₃;
and
t is 0, 1 or 2.

19. The method of Claim 18, wherein the compound is represented by the following structural formula: 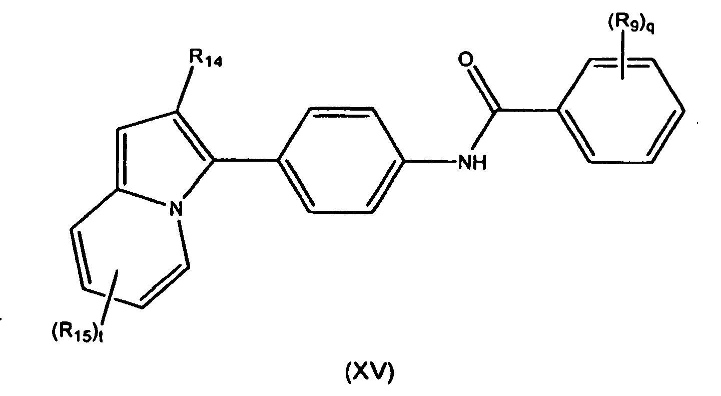 or a pharmaceutically acceptable salt, solvate, or clathrate thereof, wherein:
R₉, for each occurrence, is, independently, halo, C₁-C₄ alkyl, C₁-C₄ haloalkyl, C₁-C₄ alkoxy, C₁-C₄ haloalkoxy, or hydroxyl; and
q is 0 or an integer from 1 to 5.

20. The method of Claim 19, wherein the compound is represented by the following structural formula: 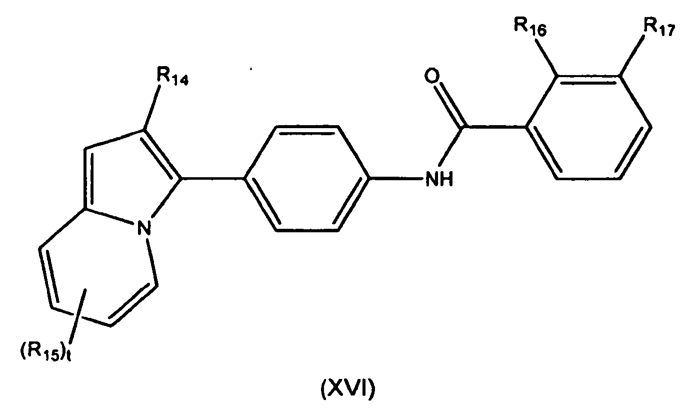 or a pharmaceutically acceptable salt, solvate, or clathrate thereof, wherein R₁₆ and R₁₇ are each, independently, -F, or -OCH₃.

21. The method of Claim 19, wherein the compound is represented by the following structural formula: 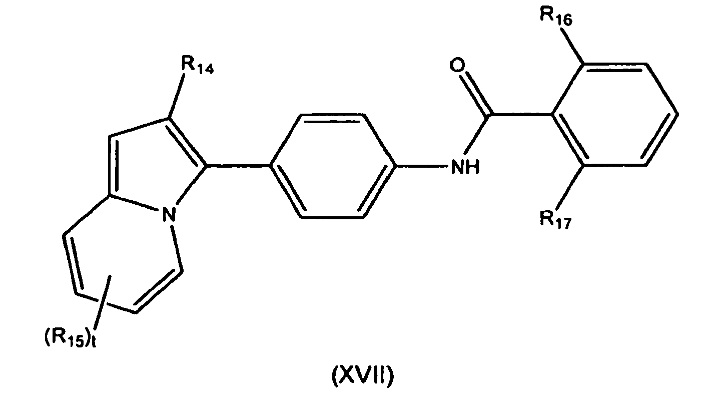 or a pharmaceutically acceptable salt, solvate, or clathrate thereof, wherein R₁₆ and R₁₇ are each, independently, -F, or -OCH₃.

22. The method of Claim 1, 2, 4 or 6, wherein the compound is represented by the following structural formula: 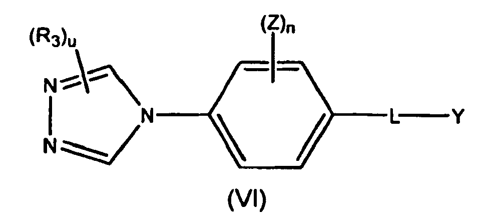 or a pharmaceutically acceptable salt, solvate, or clathrate thereof, wherein:
R₃ is an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂; and
u is 0, 1, or 2.

23. The method of Claim 22, wherein the compound is represented by the following structural formula: 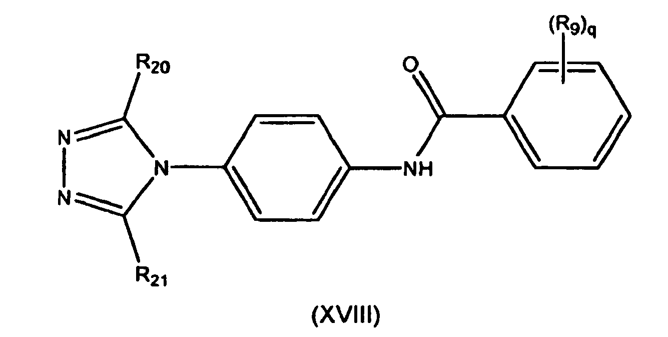 or a pharmaceutically acceptable salt, solvate, or clathrate thereof, wherein:
R₂₀ and R₂₁ are each, independently, -H, -F, -Cl, a C₁-C₄ alkyl, thiophenyl, -OCH₃, -CF₃, or -OCF₃;
R₉, for each occurrence, is, independently, halo, C₁-C₄ alkyl, C₁-C₄ haloalkyl, C₁-C₄ alkoxy, C₁-C₄ haloalkoxy, or hydroxyl; and
q is 0 or an integer from 1 to 5.

24. The method of Claim 1, 2, 4 or 6, wherein the cell is administered one or more compounds selected from the group consisting of:
3-Fluoro-N-(2'-trifluoromethyl- biphenyl-4-yl)-isonicotinamide;
3-Fluoro-N-(2'-methyl-biphenyl-4-yl)-isonicotinamide;
3-Fluoro-N-(3'-trifluoromethyl- biphenyl-4-yl)-isonicotinamide:
N-(2,2'-Bis-trifluoromethyl- biphenyl-4-yl)-2,3-difluoro- benzamide;
N-[4-(1,2-Dimethyl-but-1-enyl)-3-triftuoromethyl-phenyl]-2,3-difluoro-benzamide;
4'-(2,3-Difluoro-benzoylamino)-biphenyl-2-carboxylic acid dimethylamide;
N-(2'-Trifluoromethyl-biphenyl-4-yl)-nicotinamide;
N-(2'-Trifluoromethyl-biphenyl-4-yl)-isonicotinamide;
Thiophene-2-carboxylic acid (2'-trifluoromethyl-biphenyl-4- yl)-amide;
4-Fluoro-N-(2'-trifluoromethyl- biphenyl-4-yl)-benzamide;
2,4-Dimethyl-thiazole-5- carboxylic acid (2'-trifluoromethyl-biphenyl-4- yl)-amide; -
4-Trifluoromethyl-N- (2'-trifluoromethyl-biphenyl-4- yl)-nicotinamide;
2-Methyl-5-trifluoromethyl- oxazole-4-carboxylic acid (2'-trifluoromethyl-biphenyl- 4-yl)-amide;
2-Ethyl-5-methyl-2H-pyrazole-3-carboxylic acid (2'-trifluoromethyl-biphenyl-4- yl)-amide;
2,3-Difluoro-N-(2'-trifluoromethyl-biphenyl-4-yl)- benzamide;
2,5-Difluoro-N-(2'-trifluoromethyl-biphenyl-4-yl)- benzamide;
2,3-Difluoro-N-(3-fluoro-2'- trifluoromethyl-biphenyl-4-yl)-benzamide ;
N-(2',5'-Bis-tritluoromethyl- biphenyl-4-yl)-2,3-difluoro-benzamide;
2,3-Difluoro-N-(2'-fluoro-5'- trifluoromethyl-biphenyl-4-yl)-benzamide;
2,3-Difluoro-N-(4'-fluoro-2'- trifluoromethyl-biphenyl-4-yl)-benzamide;
2,3-Difluoro-N-[4-(2- trifluoromethyl-indolizin-3-yl)- phenyl]-benzamide;
2,3-Difluoro-N-(2'-fluoro-6'- trifluoromethyl-biphenyl-4-yl)-benzamide;
2,3-Difluoro-N-(2'-chloro-5'- trifluoromethyl-biphenyl-4-yl)-benzam ide;
4-Methyl-[1,2,3]thiadiazole-5- carboxylic acid (2',5'-bis-trifluoromethyl-biphenyl-4-yl)-amide;
Pyridine-2-carboxylic acid (2'-trifluoromethyl-biphenyl-4- yl)-amide;
Pyrazine-2-carboxylic acid (2'-trifluoromethyl-biphenyl-4- yl)-amide;
4-Methyl-[1,2,3]thiadiazole-5- carboxylic acid (2'-chloro-5'- trifluoromethyl-biphenyl-4-yl)-amide;
N-(2',5'-Bis-trifluoromethyl- biphenyl-4-yl)-2,5-difluoro- benzamide;
N-(2',5'-Dichloro-biphenyl-4-yl)-2,3-difluoro-benzamide;
N-(5'-Cyano-2'-methoxy- biphenyl-4-yl)-2,3-difluoro-benzamide;
N-(2',5'-Dimethoxy-biphenyl-4- yl)-2,3-difluoro-benzamide;
N-[4-(3,5-Bis-trifluoromethyl- [1,2,4]triazol-4-yl)-phenyl]-2,3-difluoro-benzamide;
3-Methyl-thiophene-2-carboxylic acid-(4-(3,5-bis-trifluoromethyl-[1,2,4]triazol-4-yl)-phenyl)-amide;
N-[4-(3-trifluoromethyl-5-(thiophen-4-yl)-[1,2,4]triazol-4-yl)-phenyl]-2,3-difluoro-benzamide;
N-[4-(3-trifluoromethyl-5-(thiophen-4-yl)-[1,2,4]triazol-4-yl)-phenyl]-2,3-difluoro-benzamide;
N-[4-(3-trifluoromethyl-indolizin-3-yl)-phenyl]-2,3-difluoro-benzamide;
N-[4-(3-cyano-5-trifluoromethyl-pyrid-2-yl)-phenyl]-2,3-difluoro-benzamide;
N-(2',5'-bis-trifluoromethyl-biphenyl-4-yl)-2-methoxy-benzamide; 5-Methyl-isoxazol-3-carboxylic acid (2',5'-bis-trifluoromethyl- biphenyl-4-yl)-amide;
1,3-Dimethyl-1H-pyrazol-5-carboxylic acid (2',5'-bis-trifluoromethyl-biphenyl-4-yl)-amide;
[1,2,3]-Thiadiazole-4-carboxylic acid (2',5'-bis-trifluoromethyl- biphenyl-4-yl)-amide;
Isoxazole-5-carboxylic acid (2',5'-bis-trifluoromethyl-biphenyl-4-yl)-amide;
3,5-dimethylisoxazole-4-carboxylic acid (2',5'-bis-trifluoromethyl- biphenyl-4-yl)-amide;
N-(2'-methoxy-5'-chloro -biphenyl-4-yl)-2,3-difluoro-benzamide;
N-(2',5'-bis-trifluoromethyl-biphenyl-yl)-2-methoxybenzamide;
N-(2'-methoxy-5'-methyl-biphenyl-4-yl)-2,3-difluorobenzamide;
N-(2',5'-dimethyl-biphenyl-4-yl)-2,3-difluorobenzamide;
3-methylisoxazole-4-carboxylic acid (2',5'-bis-trifluoromethyl-biphenyl- 4-yl)-amide;
N-(2',5'-bis-trifluoromethyl-biphenyl-4-yl)-2-hydroxybenzamide;
N-(2'-methoxy-5'-acetyl- biphenyl-4-yl)-2,3-difluorobenzamide;
5-methylisoxazole-4-carboxylic acid (2',5'-bis-trifluoromethyl- biphenyl-4-yl)-amide;
N-(2',4',5'-trimethyl-biphenyl-4-yl)-2,3-difluorobenzamide;
N-(2',5'-bis-trifluoromethyl- biphenyl-4-yl)-2,3-dimethylbenzamide;
N-(2',5'-bis-trifluoromethyl- biphenyl-4-yl)-2-methyl-3- chlorobenzamide;
N-(2',5'-bis-trifluoromethyl- biphenyl-4-yl)-2-methyl-3-fluorobenzamide;
N-(2',5'-bis-trifluoromethyl-biphenyl-4-yl)-2-methyl-3- methoxybenzamide;
4-methyl-[1,2,3]-thiadiazole-5-carboxylic acid (2',5'-dimethoxy biphenyl-4-yl)-amide;
N-(2',5'-dimethoxy- biphenyl-4-yl)-2-methylbenzamide;
2-methyl-pyridine-3-carboxylic acid (2',5'-bis-trifluoromethyl-biphenyl- 4-yl)-amide;
2,3-dihydro-benzo[1,4]dioxine-5-carboxylic acid (2',5'-bis- trifluoromethyl-biphenyl-4-yl)-amide;
1-methyl-1H-imidazole-5-carboxylic acid (2',5'-bis-trifluoromethyl-biphenyl- 4-yl)-amide;
3-methyl-pyridine-4-carboxylic acid (2',5'-dimethoxy-biphenyl- 4-yl)-amide;
3-methyl-pyridine-4-carboxylic acid (2',5'-bis-trifluoromethyl-biphenyl- 4-yl)-amide;
3-methyl-pyridine-4-carboxylic acid (2'-methoxy-5'-chlorobiphenyl- 4-yl)-amide;
3-fluoro-pyridine-4-carboxylic acid (2',5'-dimethoxybiphenyl- 4-yl)-amide;
3-fluoro-pyridine-4-carboxylic acid (2'-methoxy-5'-chlorobiphenyl-4-yl)-amide;
3-fluoro-pyridine-4-carboxylic acid (2',5'-bis-trifluoromethylbiphenyl- 4-yl)-amide;
3-methyl-pyridine-4-carboxylic acid (2'-methoxy-5'-methylbiphenyl- 4-yl)-amide;
3-methyl-pyridine-4-carboxylic acid (2',5'-dimethylbiphenyl-4-yl)-amide;
4-methyl-[1,2,3]-thiadiazole-5-carboxylic acid (2'-methoxy-5'-acetylbiphenyl- 4-yl)-amide;
3-fluoro-pyridine-4-carboxylic acid (2'-difluoromethoxy- 5'-chlorobiphenyl-4-yl)-amide;
4-methyl-[1,2,3]-thiadiazole-5-carboxylic acid {2'-(N,N-dimethylamino)- 5'-trifluoromethoxybiphenyl-4-yl}-amide;
3-methyl-pyridine-4-carboxylic acid (2'-chloro-5'- trifluoromethylbiphenyl-4-yl)-amide;
3-methyl-pyridine-4-carboxylic acid (2'-methylsulfanyl-biphenyl- 4-yl)-amide;
3-methyl-pyridine-4-carboxylic acid (2'-ethyl-biphenyl-4-yl)-amide;
3-methyl-pyridine-4-carboxylic acid (2'-isopropyl-biphenyl-4-yl)-amide;
N-{5-(2',5'-dimethoxyphenyl)- pyrid-2-yl}-2-methylbenzamide;
3-methyl-pyridine-4-carboxylic acid (2',5'-diethylbiphenyl-4-yl)-amide;
3-methyl-pyridine-4-carboxylic acid {2'-(N,N-dimethylamino)-5'-methoxybiphenyl-4-yl}-amide;
3-methyl-pyridine-4-carboxylic acid {2'-(N-dimethylamino)-5'-carbethoxybiphenyl-4-yl}-amide;
3-methyl-pyridine-4-carboxylic acid (2'-ethoxy-5'-chlorobiphenyl- 4-yl)-amide;
N-(2'-dimethoxy-5'-chloro biphenyl-4-yl)-2,6-difluorobenzamide; N-(2'-methoxy-5'-chloro- biphenyl-4-yl)-2,4,5-trifluorobenzamide; N-(2',5'-bis-trifluoromethyl biphenyl-4-yl)-2,6-difluorobenzamide; N-(2'-chloro-5'-trifluoromethyl biphenyl-4-yl)-2,6-difluorobenzamide; N-(2',5'-dimethyl biphenyl-4-yl)-2,6-difluorobenzamide; N-(2',5'-dichloro biphenyl-4-yl)-2,6-difluorobenzamide; 2,3-Difluoro-N-[4-(2-trifluoromethyl-indolizin-3-yl)-phenyl]-benzamide; 2,5-Difluoro-N-[4-(2-trifluoromethyl-indolizin-3-yl)-phenyl]-benzamide; 3,4-dimethoxy-N-[4-(2-trifluoromethyl-indolizin-3-yl)-phenyl]- benzamide; N-[4-(5-chloro-2-trifluoromethyl-indolizin-3-yl)-phenyl]-2,3-difluoro-benzamide;
5-chloro-3-[4-(2,3-difluoro-benzoylamino)-phenyl]-2-trifluoromethyl-indolizine-6-carboxylic acid methyl ester;
3-[4-(2,3-difluoro-benzoylamino)-phenyl]-2-trifluoromethyl-indolizine-6-carboxylic acid methyl ester;
2,3-difluoro-N-[4-(6-methoxy-2-trifluoromethyl-indolizin-3-yl)-phenyl]-benzamide;
N-[4-(5-fluoro-2-trifluoromethyl-indolizin-3-yl)-phenyl]-2,3-difluoro-benzamide;
N-[4-(5-methoxy-2-trifluoromethyl-indolizin-3-yl)-phenyl]-2,3-difluoro-benzamide;
N-[4-(5-Chloro-2,7-bis-trifluoromethyl-indolizin-3-yl)-phenyl]-2,3- difluoro-benzamide;
5-methoxy-3-[4-(2,3-difluoro-benzoylamino)-phenyl]-2-trifluoromethyl-indolizine-6-carboxylic acid methyl ester;
2,3-difluoro-N-[4-(8-methoxy-2-trifluoromethyl-indolizin-3-yl)-phenyl]-benzamide; .
5-Chloro-3-[4-(2,3-difluoro-benzoylamino)-phenyl]-2-trifluoromethyl-indolizine-7-carboxylic acid methyl ester;
5-Chloro-3-[4-(2,6-difluoro-benzoylamino)-phenyl]-2-trifluoromethyl-indolizine-7-carboxylic acid methyl ester;
2,6-difluoro-N-[4-(8-methoxy-2-trifluoromethyl-indolizin-3-yl)-phenyl]-benzamide;
5-Methoxy-3-[4-(2,6-difluoro-benzoylamino)-phenyl]-2-trifluoromethyl-indolizine-6-carboxylic acid methyl ester;
N-[4-(5-chloro-2,7-bis-trifluoromethyl-indolizin-3-yl)-phenyl]- 2,6-difluoro-benzamide;
N-[4-(5-methoxy-2-trifluoromethyl-indolizin-3-yl)-phenyl]-2,6-difluoro-benzamide;
N-[4-(5-fluoro-2-trifluoromethyl-indolizin-3-yl)-phenyl]-2,6-difluoro-benzamide;
2,6-difluoro-N-[4-(6-methoxy-2-trifluoromethyl-indolizin-3-yl)-phenyl]-benzamide;
3-[4-(2,6-difluoro-benzoylamino)-phenyl]-2-trifluoromethyl-indolizine-6-carboxylic acid methyl ester;
5-Chloro-3-[4-(2,6-difluoro-benzoylamino)-phenyl]-2-trifluoromethyl-indolizine-6-carboxylic acid methyl ester;
N-[4-(5-Chloro-2-trifluoromethyl-indolizin-3-yl)-phenyl]-2,6-difluoro-benzamide;
N-[4-(5-Chloro-2-trifluoromethyl-indolizin-3-yl)-phenyl]-2,4,5-trifluorobenzamide;
5-Chloro-3-[4-(2,4,5-trifluoro-benzoylamino)-phenyl]-2-trifluoromethyl-indolizine-6-carboxylic acid methyl ester;
3-[4-(2,4,5-trifluoro-benzoylamino)-phenyl]-2-trifluoromethyl-indolizine-6-carboxylic acid methyl ester;
2,4,5-trifluoro-N-[4-(5-fluoro-2-trifluoromethyl-indolizin-3-yl)-phenyl]-benzamide;
2,4,5-trifluoro-N-[4-(6-methoxy-2-trifluoromethyl-indolizin-3-yl)-phenyl]-benzamide;
2,4,5-trifluoro-N-[4-(5-methoxy-2-trifluoromethyl-indolizin-3-yl)-phenyl]-benzamide;
N-[4-(5-Chloro-2,7-bis-trifluoromethyl-indolizin-3-yl)-phenyl]- 2,4,5-trifluorobenzamide;
5-Methoxy-3-[4-(2,4,5-trifluoro-benzoylamino)-phenyl]-2-trifluoromethyl-indolizine-6-carboxylic acid methyl ester;
2,4,5-trifluoro-N-[4-(8-methoxy-2-trifluoromethyl-indolizin-3-yl)-phenyl]-benzamide;
5-Chloro-3-[4-(2,4,5-trifluoro-benzoylamino)-phenyl]-2-trifluoromethyl-indolizine-7-carboxylic acid methyl ester;
2,4,5-trifluoro-N-[4-(7-methoxy-2-trifluoromethyl-indolizin-3-yl)-phenyl]-benzamide;
2,6-difluoro-N-[4-(7-methoxy-2-trifluoromethyl-indolizin-3-yl)-phenyl]-benzamide;
2,3-difluoro-N-[4-(7-methoxy-2-trifluoromethyl-indolizin-3-yl)-phenyl]-benzamide;
N-(2',5'-dimethoxy-biphenyl-4-yl)-2,6-difluoro-benzamide;
N-(2'-trifluoromethyl-5'-methyl-biphenyl-4-yl)-2,6-difluoro-benzamide;
N-(2',5'-bis-trifluoromethyl-biphenyl-4-yl)-2,6-difluoro-benzyl amine;
N-(2',5'-bis-trifluoromethyl-biphenyl-4-yl)-2,6-difluoro-benzyl amine HCl salt;
N-(2'-methoxy-5'-chloro-biphenyl-4-yl)-2,6-difluoro-benzyl amine;
N-(2'-methoxy-5'-chloro-biphenyl-4-yl)-2,6-difluoro-benzyl amine HCl salt;
N',N'-diethyl-N-(2',5'-bis-trifluoromethyl biphenyl-4-yl) urea;
2,3-difluoro-N-[4-(2-trifluoro- methyl-indolizin-3-yl)-phenyl]- benzamide; .
4-methyl-N-[4-(2-methyl- indolizin-3-yl)-phenyl]- [1,2,3]thiadiazole 5-carboxylic acid amide; and
pharmaceutically acceptable salts, solvates, or clathrates thereof.

25. A compound represented by the following structural formula (II): 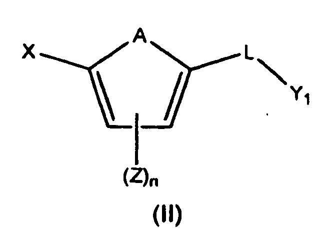 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y₁ is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
A is -CH=CH-, -CZ=CH-, -CH=CZ-;
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is an integer selected from 0-4; and
p is 0, 1, or 2,
provided that Y₁ is not a heteroaryl that is further substituted with a substituted or unsubstituted aryl or a substituted or unsubstituted heteroaryl;
provided that when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y₁ is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydronaphthyl, a substituted pyrazinyl, or a substituted or unsubstituted pyridinyl;
provided that when X is m-nitrophenyl or m-(trifluoromethyl)phenyl, Y₁ is not a substituted or unsubstituted phenyl;
provided that X is not a phenyl that is substituted in the ortho position with -S(O)₂NH₂; and
provided that X is not a nitrophenyl when Y₁ is a substituted 1*H*-pyrazolyl.

26. The compound of Claim 25, wherein the compound is represented by the following structural formula: 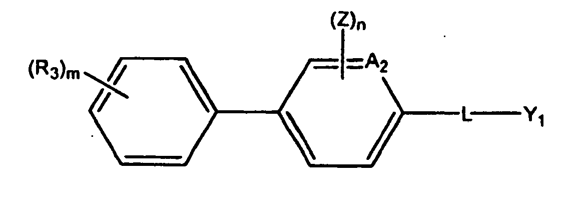 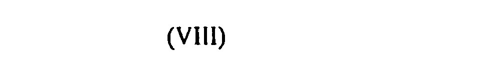 or a pharmaceutically acceptable salt, solvate, or clathrate thereof, wherein:
A₂ is CH or CZ; - .
R₃ is an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂; and
m is 0 or an integer from 1 to 5.

27. The compound of Claim 26, wherein A₂ is CH.

28. The compound of Claim 26, wherein L is -NHC(O)- or -NHCH₂-.

29. The compound of Claim 28, wherein Y₁ is an optionally substituted phenyl, an optionally substituted pyridyl, an optionally substituted thiophenyl, [1,2,3]thiadiazolyl, an optionally substituted isoxazolyl, 1H-pyrazolyl, quinolinyl, imidazolyl, or 2,3-dihydrobenzo[1,4]dioxine.

30. The compound of Claim 29, wherein Y₁ is an optionally substituted phenyl, an optionally substituted pyridyl, or an optionally substituted [1,2,3]thiadiazolyl.

31. The compound of Claim 30, wherein the compound is represented by the following structural formula: 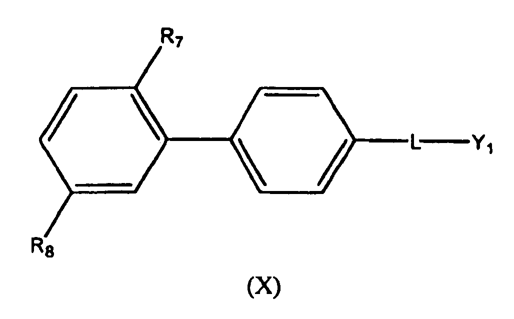 or a pharmaceutically acceptable salt, solvate, or clathrate thereof, wherein:
R₇ and R₈ are each, independently, -H, -CF₃, -CN, -C(O)CH₃, -F, -Cl, -OCH₃, -OCH₂CH₃, -C(O)OCH₂CH₃, -SCH₃, -NHCH₃, or C₁-C₄ alkyl, provided that at least one of R₇ or R₈ is not -H.

32. A compound represented by the following structural formula: 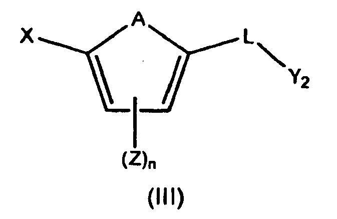 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y₂ is an optionally substituted cycloalkyl, an optionally substituted cycloalkenyl, or an optionally substituted heterocyclyl;
A is -CH=CH-, -CZ=CH-, -CH=CZ-;
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4; and
p is 0, 1, or 2,
provided that X is not a phenyl that is substituted in the ortho position with -CN or -S(O)₂NH₂;
provided that Y₂ is not a 4,5-dihydroisoxazlyl that is further substituted with a substituted or unsubstituted aryl or a substituted or unsubstituted heteroaryl; and
provided that Y₂ is not a substituted cyclopentenyl.

33. A compound selected from the group consisting of:
3-Fluoro-N-(2'-trifluoromethyl- biphenyl-4-yl)-isonicotinamide;
3-Fluoro-N-(2'-methyl-biphenyl-4-yl)-isonicotinamide;
3-Fluoro-N-(3'-trifluoromethyl- biphenyl-4-yl)-isonicotinamide;
N-(2,2'-Bis-trifluoromethyl- biphenyl-4-yl)-2,3-difluoro- benzamide;
N-[4-(1,2-Dimethyl-but-1-enyl)-3-trifluoromethyl-phenyl]-2,3-difluoro-benzamide;
4'-(2,3-Difluoro-benzoylamino)-biphenyl-2-carboxylic acid dimethylamide;
N-(2'-Trifluoromethyl-biphenyl-4-yl)-nicotinamide;
N-(2'-Trifluoromethyl-biphenyl-4-yl)-isonicotinamide;
Thiophene-2-carboxylic acid (2'-trifluoromethyl-biphenyl-4- yl)-amide;
4-Fluoro-N-(2'-trifluoromethyl- biphenyl-4-yl)-benzamide;
2,4-Dimethyl-thiazole-5- carboxylic acid (2'-trifluoromethyl-biphenyl-4- yl)-amide;
4-Trifluoromethyl-N- (2'-trifluoromethyl-biphenyl-4- yl)-nicotinamide;
2-Methyl-5-trifluoromethyl- oxazole-4-carboxylic acid (2'-trifluoromethyl-biphenyl- 4-yl)-amide;
2-Ethyl-5-methyl-2H-pyrazole-3-carboxylic acid (2'-trifluoromethyl-biphenyl-4- yl)-amide;
2,3-Difluoro-N-(2'- trifluoromethyl-biphenyl-4-yl)- benzamide;
2,5-Difluoro-N-(2'- trifluoromethyl-biphenyl-4-yl)- benzamide;
2,3-Difluoro-N-(3-fluoro-2'-trifluoromethyl-biphenyl-4-yl)-benzamide;
N-(2',5'-Bis-trifluoromethyl-biphenyl-4-yl)-2,3-difluoro-benzamide;
2,3-Difluoro-N-(2'-fluoro-5'- trifluoromethyl-biphenyl-4-yl)- benzamide;
2,3-Difluoro-N-(4'-fluoro-2'- trifluoromethyl-biphenyl-4-yl)- benzamide;
2,3-Difluoro-N-[4-(2- trifluoromethyl-indolizin-3-yl)- phenyl]-benzamide;
2,3-Difluoro-N-(2'-fluoro-6'- trifluoromethyl-biphenyl-4-yl)- benzamide;
2,3-Difluoro-N-(2'-chloro-5'- trifluoromethyl-biphenyl-4-yl)- benzamide;
4-Methyl-[1,2,3]thiadiazole-5- carboxylic acid (2',5'-bis-trifluoromethyl-biphenyl-4-yl)-amide;
Pyridine-2-carboxylic acid (2'-trifluoromethyl-biphenyl-4- yl)-amide;
Pyrazine-2-carboxylic acid (2'-trifluoromethyl-biphenyl-4- yl)-amide;
4-Methyl-[1,2,3]thiadiazole-5-carboxylic acid (2'-chloro-5'- trifluoromethyl-biphenyl-4-yl)-amide;
N-(2',5'-Bis-trifluoromethyl- biphenyl-4-yl)-2,5-difluoro- benzamide;
N-(2',5'-Dichloro-biphenyl-4-yl)-2,3-difluoro-benzamide;
N-(5'-Cyano-2'-methoxy- biphenyl-4-yl)-2,3-difluoro-benzamide;
N-(2',5'-Dimethoxy-biphenyl-4-yl)-2,3-difluoro-benzamide;
N-[4-(3,5-Bis-trifluoromethyl-[1,2,4]triazol-4-yl)-phenyl]-2,3-difluoro-benzamide;
3-Methyl-thiophene-2-carboxylic acid-(4-(3,5-bis-trifluoromethyl-[1,2,4]triazol-4-yl)-phenyl)-amide;
N-[4-(3-trifluoromethyl-5-(thiophen-4-yl)-[1,2,4]triazol-4-yl)-phenyl]-2,3-difluoro-benzamide;
N-[4-(3-trifluoromethyl-5-(thiophen-4-yl)-[1,2,4]triazol-4-yl)-phenyl]- 2,3-difluoro-benzamide;
N-[4-(3-trifluoromethyl-indolizin-3-yl)-phenyl]-2,3-difluoro-benzamide;
N-[4-(3-cyano-5-trifluoromethyl-pyrid-2-yl)-phenyl]-2,3-difluoro-benzamide;
N-(2',5'-bis-trifluoromethyl-biphenyl-4-yl)-2-methoxy-benzamide;
5-Methyl-isoxazol-3-carboxylic acid (2',5'-bis-trifluoromethyl-biphenyl- 4-yl)-amide;
1,3-Dimethyl-1H-pyrazol-5-carboxylic acid (2',5'-bis-trifluoromethyl-biphenyl-4-yl)-amide;
[1,2,3]-Thiadiazole-4-carboxylic acid (2',5'-bis-trifluoromethyl- biphenyl-4-yl)-amide;
Isoxazole-5-carboxylic acid (2',5'-bis-trifluoromethyl-biphenyl-4-yl)- amide;
3,5-dimethylisoxazole-4-carboxylic acid (2',5'-bis-trifluoromethyl- biphenyl-4-yl)-amide;
N-(2'-methoxy-5'-chloro-biphenyl-4-yl)-2,3-difluoro-benzamide;
N-(2',5'-bis-trifluoromethyl-biphenyl-4-yl)-2-methoxybenzamide;
N-(2'-methoxy-5'-methyl-biphenyl-4-yl)-2,3-difluorobenzamide;
N-(2',5'-dimethyl-biphenyl-4-yl)-2,3-difluorobenzamide;
3-methylisoxazole-4-carboxylic acid (2',5'-bis-trifluoromethyl- biphenyl-4-yl)-amide;
N-(2',5'-bis-trifluoromethyl-biphenyl-4-yl)-2-hydroxybenzamide;
N-(2'-methoxy-5'-acetyl- biphenyl-4-yl)-2,3-difluorobenzamide;
5-methylisoxazole-4-carboxylic acid (2',5'-bis-trifluoromethyl- biphenyl-4-yl)-amide;
N-(2',4',5'-trimethyl- biphenyl-4-yl)-2,3-difluorobenzamide;
N-(2',5'-bis-trifluoromethyl- biphenyl-4-yl)-2,3-dimethylbenzamide;
N-(2',5'-bis-trifluoromethyl-biphenyl-4-yl)-2-methyl-3-chlorobenzamide;
N-(2',5'-bis-trifluoromethyl- biphenyl-4-yl)-2-methyl-3-fluorobenzamide;
N-(2',5'-bis-trifluoromethyl-biphenyl-4-yl)-2-methyl-3- methoxybenzamide;
4-methyl-[1,2,3]-thiadiazole-5-carboxylic acid (2',5'-dimethoxy- biphenyl-4-yl)-amide;
N-(2',5'-dimethoxy-biphenyl-4-yl)-2-methylbenzamide;
2-methyl-pyridine-3-carboxylic acid (2',5'-bis-trifluoromethyl-biphenyl- 4-yl)-amide;
2,3-dihydro-benzo[1,4]dioxine-5-carboxylic acid (2',5'-bis- trifluoromethyl-biphenyl-4-yl)-amide;
1-methyl-1*H*-imidazole-5-carboxylic acid (2',5'-bis-trifluoromethyl-biphenyl- 4-yl)-amide;
3-methyl-pyridine-4-carboxylic acid (2',5'-dimethoxy-biphenyl-4-yl)- amide;
3-methyl-pyridine-4-carboxylic acid (2',5'-bis-trifluoromethyl-biphenyl- 4-yl)-amide;
3-methyl-pyridine-4-carboxylic acid (2'-methoxy-5'-chlorobiphenyl- 4-yl)-amide;
3-fluoro-pyridine-4-carboxylic acid (2',5'-dimethoxybiphenyl-4-yl)- amide;
3-fluoro-pyridine-4-carboxylic acid (2'-methoxy-5'-chlorobiphenyl-4-yl)-amide;
3-fluoro-pyridine-4-carboxylic acid (2',5'-bis-trifluoromethylbiphenyl- 4-yl)-amide;
3-methyl-pyridine-4-carboxylic acid (2'-methoxy-5'-methylbiphenyl- 4-yl)-amide;
3-methyl-pyridine-4-carboxylic acid (2',5'-dimethylbiphenyl-4-yl)-amide;
4-methyl-[1,2,3]-thiadiazole-5-carboxylic acid (2'-methoxy-5'-acetylbiphenyl-4-yl)-amide;
3-fluoro-pyridine-4-carboxylic acid (2'-difluoromethoxy-5'- chlorobiphenyl-4-yl)-amide;
4-methyl-[1,2,3]-thiadiazole-5-carboxylic acid {2'-(N,N-dimethylamino)- 5'-trifluoromethoxybiphenyl-4-yl}-amide;
3-methyl-pyridine-4-carboxylic acid (2'-chloro-5'- trifluoromethylbiphenyl-4-yl)-amide;
3-methyl-pyridine-4-carboxylic acid (2'-methylsulfanyl-biphenyl-4-yl)-amide;
3-methyl-pyridine-4-carboxylic acid (2'-ethyl-biphenyl-4-yl)-amide;
3-methyl-pyridine-4-carboxylic acid (2'-isopropyl-biphenyl-4-yl)-amide;
N-{5-(2',5'-dimethoxyphenyl)-pyrid-2-yl}-2-methylbenzamide;
3-methyl-pyridine-4-carboxylic acid (2',5'-diethylbiphenyl-4-yl)-amide;
3-methyl-pyridine-4-carboxylic acid {2'-(N,N-dimethylamino)-5'-methoxybiphenyl-4-yl}-amide;
3-methyl-pyridine-4-carboxylic acid {2'-(N-dimethylamino)- 5'-carbethoxybiphenyl-4-yl}-amide;
3-methyl-pyridine-4-carboxylic acid (2'-ethoxy-5'-chlorobiphenyl- 4-yl)-amide;
N-(2'-dimethoxy-5'-chloro biphenyl-4-yl)-2,6-difluorobenzamide;
N-(2'-methoxy-5'-chloro- biphenyl-4-yl)-2,4,5-trifluorobenzamide;
N-(2',5'-bis-trifluoromethyl biphenyl-4-yl)-2,6-difluorobenzamide;
N-(2'-chloro-5'-trifluoromethyl biphenyl-4-yl)-2,6-difluorobenzamide;
N-(2',5'-dimethyl biphenyl-4-yl)-2,6-difluorobenzamide;
N-(2',5'-dichloro biphenyl-4-yl)-2,6-difluorobenzamide;
2,3-Difluoro-N-[4-(2-trifluoromethyl-indolizin-3-yl)-phenyl]-benzamide;
2,5-Difluoro-N-[4-(2-trifluoromethyl-indolizin-3-yl)-phenyl]-benzamide;
3,4-dimethoxy-N-[4-(2-trifluoromethyl-indolizin-3-yl)-phenyl]- benzamide;
N-[4-(5-chloro-2-trifluoromethyl-indolizin-3-yl)-phenyl]-2,3-difluoro-benzamide;
5-chloro-3-[4-(2,3-difluoro-benzoylamino)-phenyl]-2-trifluoromethyl-indolizine-6-carboxylic acid methyl ester;
3-[4-(2,3-difluoro-benzoylamino)-phenyl]-2-trifluoromethyl-indolizine-6-carboxylic acid methyl ester;
2,3-difluoro-N-[4-(6-methoxy-2-trifluoromethyl-indolizin-3-yl)-phenyl]-benzamide;
N-[4-(5-fluoro-2-trifluoromethyl-indolizin-3-yl)-phenyl]-2,3-difluoro-benzamide;
N-[4-(5-methoxy-2-trifluoromethyl-indolizin-3-yl)-phenyl]-2,3-difluoro-benzamide;
N-[4-(5-Chloro-2,7-bis-trifluoromethyl-indolizin-3-yl)-phenyl]-2,3-difluoro-benzamide;
5-methoxy-3-[4-(2,3-difluoro-benzoylamino)-phenyl]-2-trifluoromethyl-indolizine-6-carboxylic acid methyl ester;
2,3-difluoro-N-[4-(8-methoxy-2-trifluoromethyl-indolizin-3-yl)-phenyl]-benzamide;
5-Chloro-3-[4-(2,3-difluoro-benzoylamino)-phenyl]-2-trifluoromethyl-indolizine-7-carboxylic acid methyl ester;
5-Chloro-3-[4-(2,6-difluoro-benzoylamino)-phenyl]-2-trifluoromethyl-ndolizine-7-carboxylic acid methyl ester;
2,6-difluoro-N-[4-(8-methoxy-2-trifluoromethyl-indolizin-3-yl)-phenyl]-benzamide;
5-Methoxy-3-[4-(2,6-difluoro-benzoylamino)-phenyl]-2-trifluoromethyl-indolizine-6-carboxylic acid methyl ester;
N-[4-(5-chloro-2,7-bis-trifluoromethyl-indolizin-3-yl)-phenyl]-2,6-difluoro-benzamide;
N-[4-(5-methoxy-2-trifluoromethyl-indolizin-3-yl)-phenyl]-2,6-difluoro-benzamide;
N-[4-(5-fluoro-2-trifluoromethyl-indolizin-3-yl)-phenyl]-2,6-difluoro benzamide;
2,6-difluoro-N-[4-(6-methoxy-2-trifluoromethyl-indolizin-3-yl)-phenyl]-benzamide;
3-[4-(2,6-difluoro-benzoylamino)-phenyl]-2-trifluoromethyl-indolizine-6-carboxylic acid methyl ester;
5-Chloro-3-[4-(2,6-difluoro-benzoylamino)-phenyl]-2-trifluoromethyl-indolizine-6-carboxylic acid methyl ester;
N-[4-(5-Chloro-2-trifluoromethyl-indolizin-3-yl)-phenyl]-2,6-difluoro-benzamide;
N-[4-(5-Chloro-2-trifluoromethyl-indolizin-3-yl)-phenyl]-2,4,5-trifluorobenzamide;
5-Chloro-3-[4-(2,4,5-trifluoro-benzoylamino)-phenyl]-2-trifluoromethyl-indolizine-6-carboxylic acid methyl ester;
3-[4-(2,4,5-trifluoro-benzoylamino)-phenyl]-2-trifluoromethyl-indolizine-6-carboxylic acid methyl ester;
2,4,5-trifluoro-N-[4-(5-fluoro-2-trifluoromethyl-indolizin-3-yl)-phenyl]-benzamide;
2,4,5-trifluoro-N-[4-(6-methoxy-2-trifluoromethyl-indolizin-3-yl)-phenyl]-benzamide;
2,4,5-trifluoro-N-[4-(5-methoxy-2-trifluoromethyl-indolizin-3-yl)-phenyl]-benzamide;
N-[4-(5-Chloro-2,7-bis-trifluoromethyl-indolizin-3-yl)-phenyl]-2,4,5- trifluorobenzamide;
5-Methoxy-3-[4-(2,4,5-trifluoro-benzoylamino)-phenyl]-2-trifluoromethyl-indolizine-6-carboxylic acid methyl ester;
2,4,5-trifluoro-N-[4-(8-methoxy-2-trifluoromethyl-indolizin-3-yl)-phenyl]-benzamide;
5-Chloro-3-[4-(2,4,5-trifluoro-benzoylamino)-phenyl]-2-trifluoromethyl-indolizine-7-carboxylic acid methyl ester;
2,4,5-trifluoro-N-[4-(7-methoxy-2-trifluoromethyl-indolizin-3-yl)-phenyl]-benzamide;
2,6-difluoro-N-[4-(7-methoxy-2-trifluoromethyl-indolizin-3-yl)-phenyl]-benzamide;
2,3-difluoro-N-[4-(7-methoxy-2-trifluoromethyl-indolizin-3-yl)-phenyl]-benzamide;
N-(2',5'-dimethoxy-biphenyl-4-yl)-2,6-difluoro-benzamide;
N-(2'-trifluoromethyl-5'-methyl-biphenyl-4-yl)-2,6-difluoro-benzamide;
N-(2',5'-bis-trifluoromethyl-biphenyl-4-yl)-2,6-difluoro-benzyl amine;
N-(2',5'-bis-trifluoromethyl-biphenyl-4-yl)-2,6-difluoro-benzyl amine HCl salt;
N-(2'-methoxy-5'-chloro-biphenyl-4-yl)-2,6-difluoro-benzyl amine;
N-(2'-methoxy-5'-chloro-biphenyl-4-yl)-2,6-difluoro-benzyl amine HCl salt;
N',N'-diethyl-N-(2',5'-bis-trifluoromethyl biphenyl-4-yl) urea;
2,3-difluoro-N-[4-(2-trifluoro- methyl-indolizin-3-yl)-phenyl]- benzamide;
4-methyl-N-[4-(2-methyl- indolizin-3-yl)-phenyl]- [1,2,3]thiadiazole 5-carboxylic acid amide; and
pharmaceutically acceptable salts, solvates, or clathrates thereof.

34. A compound represented by the following structural formula: 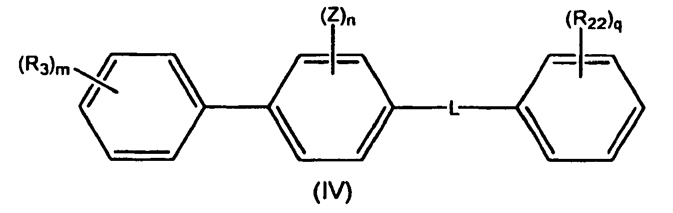 or a pharmaceutically acceptable salt, solvate, or clathrate thereof, wherein:
Z, R₃ and R₂₂, for each occurrence, are, independently, selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)pR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4;
m and q are each independently, 0 or an integer from 1 to 5; and
p is 0.1, or 2,
provided that when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydronaphthyl, a substituted pyrazinyl, or a substituted or unsubstituted pyridinyl;
provided that when X is m-nitrophenyl or m-(trifluoromethyl)phenyl, Y₁ is not a substituted or unsubstituted phenyl; and
provided that X is not a phenyl that is substituted in the ortho position with -S(O)₂NH₂.

35. A compound represented by the following structural formula: 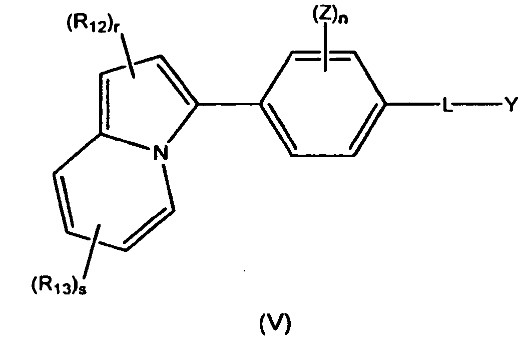 or a pharmaceutically acceptable salt, solvate, or clathrate thereof, wherein:
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
L is a linker selected-from the group consisting of -NRCH₂-, and -NR-C(O)-;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
Z, R₁₂ and R₁₃, for each occurrence, is, independently, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
r is 0, 1 or 2;
n and s are each, independently, 0 or an integer from 1 to 4; and
p is 0, 1 or 2.

36. The compound of Claim 35, wherein L is -NHC(O)- and Y is an optionally substituted phenyl.

37. The compound of Claim 36, wherein the compound is represented by the following structural formula: 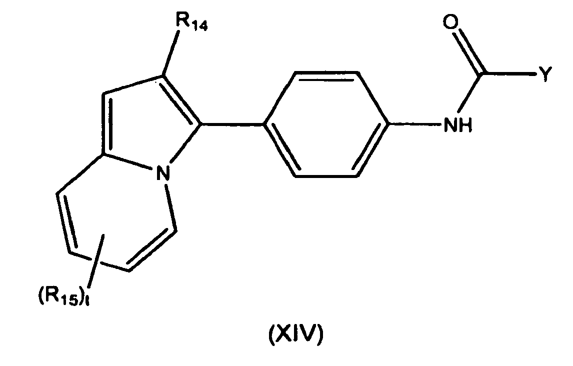 or a pharmaceutically acceptable salt, solvate, or clathrate thereof, wherein R₁₄ and R₁₅ are each, independently, -CF₃, -OCH₃, -F, -Cl, or-C(O)OCH₃; and
t is 0, 1 or 2.

38. The compound of Claim 37, wherein the compound is represented by the following structural formula: 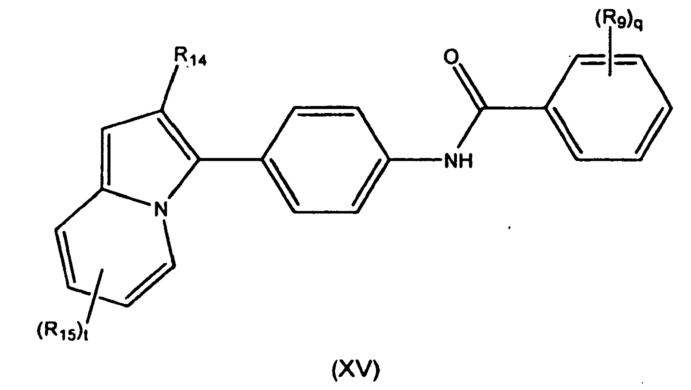 or a pharmaceutically acceptable salt, solvate, or clathrate thereof, wherein:
R₉, for each occurrence, is, independently, halo, C₁-C₄ alkyl, C₁-C₄ haloalkyl, C₁-C₄ alkoxy, C₁-C₄ haloalkoxy, or hydroxyl; and
q is 0 or an integer from 1 to 5.

39. The compound of Claim 38, wherein the compound is represented by the following structural formula: 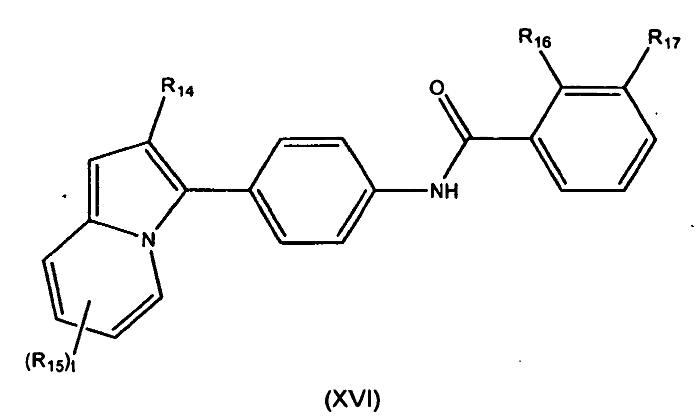 or a pharmaceutically acceptable salt, solvate, or clathrate thereof, wherein R₁₆ and R₁₇ are each, independently, -F, or -OCH₃.

40. The compound of Claim 38, wherein the compound is represented by the following structural formula: 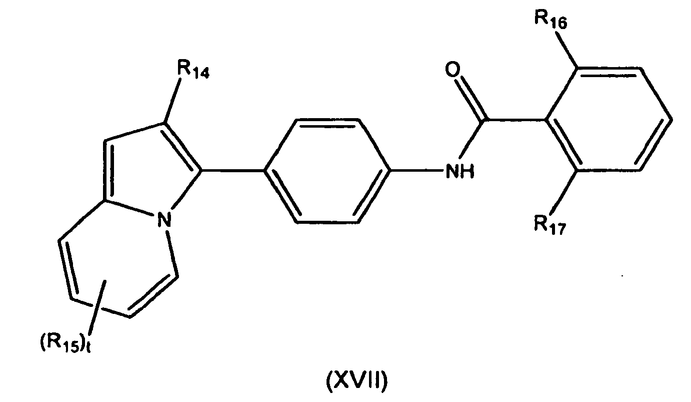 or a pharmaceutically acceptable salt, solvate, or clathrate thereof, wherein R₁₆ and R₁₇ are each, independently, -F, or -OCH₃.

41. A compound represented by the following structural formula: 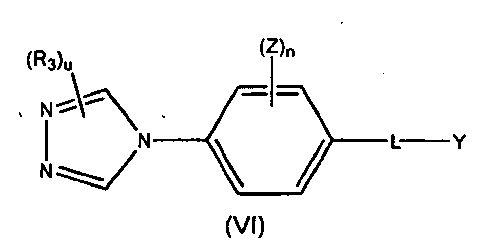 or a pharmaceutically acceptable salt, solvate, or clathrate thereof, wherein:
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
Z and R₃, for each occurrence, are, independently, selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R_{2,} -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each, R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is an integer selected from 0-4;
p is 0, 1, or 2; and
u is 0, 1, or 2.

42. A pharmaceutical composition, comprising a pharmaceutically acceptable carrier and one or more compounds represented by the following structural formula: 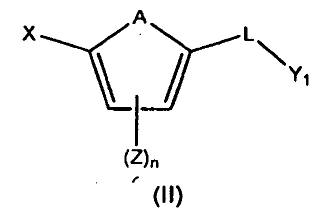 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y₁, is an optionally substituted aryl or an optionally substituted heteroaryl;
A is-CH=CH-, -CZ=CH-, -CH=CZ-;
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, a haloalkyl, -C(O)NR₁R₂. -NR₄C(O)R₅, halo, -OR₄, cyano, nitro, haloalkoxy, -C(O)R₄, -NR₁R₂, -SR₄, -C(O)OR₄, -OC(O)R₄. -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is an integer selected from 0-4; and
p is 0, 1, or 2,
provided that Y₁ is not a heteroaryl that is further substituted with a substituted or unsubstituted aryl or a substituted or unsubstituted heteroaryl;
provided that when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y₁ is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydronaphthyl, a substituted pyrazinyl, or a substituted or unsubstituted pyridinyl;
provided that when X is m-nitrophenyl or m-(trifluoromethyl)phenyl, Y₁ is not a substituted or unsubstituted phenyl;
provided that X is not a phenyl that is substituted in the ortho position with -S(O)₂NH₂; and
provided that X is not a nitrophenyl when Y₁ is a substituted 1*H*-pyrazolyl.

43. The pharmaceutical composition of Claim 42, wherein the compound is represented by the following structural formula: 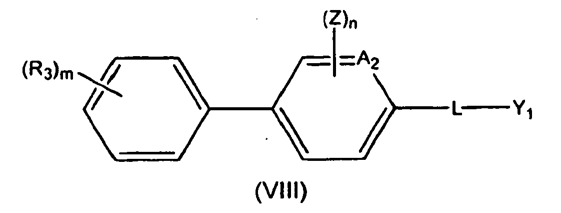 or a pharmaceutically acceptable salt, solvate, or clathrate thereof, wherein:
A₂ is CH or CZ;
R₃ is an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂; and
m is 0 or an integer from 1 to 5.

44. The pharmaceutical composition of Claim 43, wherein A₂ is CH.

45. The pharmaceutical composition of Claim 43, wherein L is -NHC(O)- or -NHCH₂-.

46. The pharmaceutical composition of Claim 45, wherein Y₁ is an optionally substituted phenyl, an optionally substituted pyridyl, an optionally substituted thiophenyl, [1,2,3]thiadiazolyl, an optionally substituted isoxazolyl, 1*H*-pyrazolyl, quinolinyl, imidazolyl, or 2,3-dihydrobenzo[1,4]dioxine.

47. The pharmaceutical composition of Claim 46, wherein Y₁ is an optionally substituted phenyl, an optionally substituted pyridyl, or an optionally substituted [1,2,3]thiadiazolyl.

48. The pharmaceutical composition of Claim 47, wherein the compound is represented by the following structural formula: 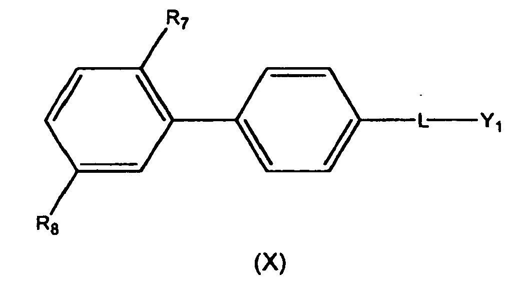 or a pharmaceutically acceptable salt, solvate, or clathrate thereof, wherein:
R₇ and R₈ are each, independently, -H, -CF₃, -CN, -C(O)CH₃, -F, -Cl, -OCH₃, -OCH₂CH₃, -C(O)OCH₂CH₃, -SCH₃, -NHCH₃, or C₁-C₄ alkyl, provided that at least one of R₇ or R₈ is not -H.

49. A pharmaceutical composition, comprising a pharmaceutically acceptable carrier and one or more compounds represented by the following structural formula: 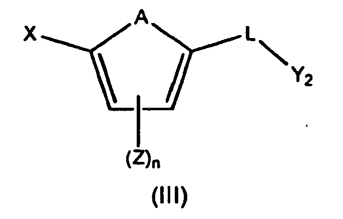 or a pharmaceutically acceptable salt, solvate, or clathrate thereof wherein:
X is an optionally substituted phenyl, an optionally substituted triazolyl, an optionally substituted pyridyl, or an optionally substituted indolizinyl;
Y₂ is an optionally substituted cycloalkyl, an optionally substituted cycloalkenyl, or an optionally substituted heterocyclyl;
A is -CH=CH-, -CZ=CH-, -CH=CZ-;
each Z is independently selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4; and
p is 0, 1, or 2,
provided that X is not a phenyl that is substituted in the ortho position with -CN or -S(O)₂NH₂;
provided that Y₂ is not a 4,5-dihydroisoxazlyl that is further substituted with a substituted or unsubstituted aryl or a substituted or unsubstituted heteroaryl; and
provided that Y₂ is not a substituted cyclopentenyl.

50. A pharmaceutical composition, comprising a pharmaceutically acceptable carrier and one or more compounds selected from the group consisting of:
3-Fluoro-N-(2'-trifluoromethyl- biphenyl-4-yl)-isonicotinamide;
3-Fluoro-N-(2'-methyl-biphenyl-4-yl)-isonicotinamide;
3-Fluoro-N-(3'-trifluoromethyl- biphenyl-4-yl)-isonicotinamide;
N-(2,2'-Bis-trifluoromethyl- biphenyl-4-yl)-2,3-difluoro- benzamide;
N-[4-(1,2-Dimethyl-but-1-enyl)-3-trifluoromethyl-phenyl]-2,3-difluoro-benzamide;
4'-(2,3-Difluoro-benzoylamino)-biphenyl-2-carboxylic acid dimethylamide;
N-(2'-Trifluoromethyl-biphenyl-4-yl)-nicotinamide;
N-(2'-Trifluoromethyl-biphenyl-4-yl)-isonicotinamide;
Thiophene-2-carboxylic acid (2'-trifluoromethyl-biphenyl-4- yl)-amide;
4-Fluoro-N-(2'-trifluoromethyl- biphenyl-4-yl)-benzamide;
2,4-Dimethyl-thiazole-5- carboxylic acid (2'-trifluoromethyl-biphenyl-4- yl)-amide;
4-Trifluoromethyl-N- (2'-trifluoromethyl-biphenyl-4- yl)-nicotinamide;
2-Methyl-5-trifluoromethyl- oxazole-4-carboxylic acid (2'-trifluoromethyl-biphenyl- 4-yl)-amide;
2-Ethyl-5-methyl-2H-pyrazole-3-carboxylic acid (2'-trifluoromethyl-biphenyl-4- yl)-amide;
2,3-Difluoro-N-(2'- trifluoromethyl-biphenyl-4-yl)- benzamide;
2,5-Difluoro-N-(2'- trifluoromethyl-biphenyl-4-yl)- benzamide;
2,3-Difluoro-N-(3-fluoro-2'- trifluoromethyl-biphenyl-4-yl)- benzamide;
N-(2',5'-Bis-trifluoromethyl- biphenyl-4-yl)-2,3-difluoro- benzamide;
2,3-Difluoro-N-(2'-fluoro-5'- trifluoromethyl-biphenyl-4-yl)- benzamide;
2,3-Difluoro-N-(4'-fluoro-2'- trifluoromethyl-biphenyl-4-yl)- benzamide;
2,3-Difluoro-N-[4-(2- trifluoromethyl-indolizin-3-yl)- phenyl]-benzamide;
2,3-Difluoro-N-(2'-fluoro-6'- trifluoromethyl-biphenyl-4-yl)- benzamide;
2,3-Difluoro-N-(2'-chloro-5'- trifluoromethyl-biphenyl-4-yl)- benzamide;
4-Methyl-[1,2,3]thiadiazole-5- carboxylic acid (2',5'-bis-trifluoromethyl-biphenyl-4-yl)-amide;
Pyridine-2-carboxylic acid (2'-trifluoromethyl-biphenyl-4- yl)-amide;
Pyrazine-2-carboxylic acid (2'-trifluoromethyl-biphenyl-4- yl)-amide;
4-Methyl-[1,2,3]thiadiazole-5-carboxylic acid (2'-chloro-5'-trifluoromethyl-biphenyl-4-yl)-amide;
N-(2',5'-Bis-trifluoromethyl- biphenyl-4-yl)-2,5-difluoro- benzamide;
N-(2',5'-Dichloro-biphenyl-4-yl)-2,3-difluoro-benzamide;
N-(5'-Cyano-2'-methoxy- biphenyl-4-yl)-2,3-difluoro-benzamide;
N-(2',5'-Dimethoxy-biphenyl-4- yl)-2,3-difluoro-benzamide;
N-[4-(3,5-Bis-trifluoromethyl-[1,2,4]triazol-4-yl)-phenyl]-2,3-difluoro-benzamide;
3-Methyl-thiophene-2-carboxylic acid-(4-(3,5-bis-trifluoromethyl-[1,2,4]triazol-4-yl)-phenyl)-amide;
N-[4-(3-trifluoromethyl-5-(thiophen-4-yl)-[1,2,4]triazol-4-yl)-phenyl]- 2,3-difluoro-benzamide;
N-[4-(3-trifluoromethyl-5-(thiophen-4-yl)-[1,2,4]triazol-4-yl)-phenyl]- 2,3-difluoro-benzamide;
N-[4-(3-trifluoromethyl-indolizin-3-yl)-phenyl]- 2,3-difluoro-benzamide;
N-[4-(3-cyano-5-trifluoromethyl-pyrid-2-yl)-phenyl]-2,3-difluoro-benzamide;
N-(2',5'-bis-trifluoromethyl-biphenyl-4-yl)-2-methoxy-benzamide;
5-Methyl-isoxazol-3-carboxylic acid (2',5'-bis-trifluoromethyl-biphenyl- 4-yl)-amide;
1,3-Dimethyl-1H-pyrazol-5-carboxylic acid (2',5'-bis-trifluoromethyl-biphenyl-4-yl)-amide;
[1,2,3]-Thiadiazole-4-carboxylic acid (2',5'-bis-trifluoromethyl- biphenyl-4-yl)-amide;
Isoxazole-5-carboxylic acid (2',5'-bis-trifluoromethyl-biphenyl-4-yl)- amide;
3,5-dimethylisoxazole-4-carboxylic acid (2',5'-bis-trifluoromethyl- biphenyl-4-yl)-amide;
N-(2'-methoxy-5'-chloro -biphenyl-4-yl)-2,3-difluoro-benzamide;
N-(2',5'-bis-trifluoromethyl-biphenyl-4-yl)-2-methoxybenzamide;
N-(2'-methoxy-5'-methyl-biphenyl-4-yl)-2,3-difluorobenzamide;
N-(2',5'-dimethyl-biphenyl-4-yl)-2,3-difluorobenzamide;
3-methylisoxazole-4-carboxylic acid (2',5'-bis-trifluoromethyl- biphenyl-4-yl)-amide;
N-(2',5'-bis-trifluoromethyl-biphenyl-4-yl)-2-hydroxybenzamide;
N-(2'-methoxy-5'-acetyl- biphenyl-4-yl)-2,3-difluorobenzamide;
5-methylisoxazole-4-carboxylic acid (2',5'-bis-trifluoromethyl- biphenyl-4-yl)-amide;
N-(2',4',5'-trimethyl- biphenyl-4-yl)-2,3-difluorobenzamide;
N-(2',5'-bis-trifluoromethyl- biphenyl-4-yl)-2,3-dimethylbenzamide;
N-(2',5'-bis-trifluoromethyl-biphenyl-4-yl)-2-methyl-3-chlorobenzamide;
N-(2',5'-bis-trifluoromethyl- biphenyl-4-yl)-2-methyl-3-fluorobenzamide;
N-(2',5'-bis-trifluoromethyl-biphenyl-4-yl)-2-methyl-3- methoxybenzamide;
4-methyl-[1,2,3]-thiadiazole-5-carboxylic acid (2',5'-dimethoxy- biphenyl-4-yl)-amide;
N-(2',5'-dimethoxy- biphenyl-4-yl)-2-methylbenzamide;
2-methyl-pyridine-3-carboxylic acid (2',5'-bis-trifluoromethyl-biphenyl- 4-yl)-amide;
2,3-dihydro-benzo[1,4]dioxine-5-carboxylic acid (2',5'-bis-trifluoromethyl-biphenyl-4-yl)-amide;
1-methyl-1*H*-imidazole-5-carboxylic acid (2',5'-bis-trifluoromethyl-biphenyl- 4-yl)-amide;
3-methyl-pyridine-4-carboxylic acid (2',5'-dimethoxy-biphenyl-4-yl)- amide;
3-methyl-pyridine-4-carboxylic acid (2',5'-bis-trifluoromethyl-biphenyl- 4-yl)-amide;
3-methyl-pyridine-4-carboxylic acid (2'-methoxy-5'-chlorobiphenyl- 4-yl)-amide;
3-fluoro-pyridine-4-carboxylic acid (2',5'-dimethoxybiphenyl-4-yl)- amide;
3-fluoro-pyridine-4-carboxylic acid (2'-methoxy-5'-chlorobiphenyl-4-yl)-amide;
3-fluoro-pyridine-4-carboxylic acid (2',5'-bis-trifluoromethylbiphenyl-4-yl)-amide;
3-methyl-pyridine-4-carboxylic acid (2'-methoxy-5'-methylbiphenyl- 4-yl)-amide;
3-methyl-pyridine-4-carboxylic acid (2',5'-dimethylbiphenyl-4-yl)-amide;
4-methyl-[1,2,3]-thiadiazole-5-carboxylic acid (2'-methoxy-5'-acetylbiphenyl-4-yl)-amide;
3-fluoro-pyridine-4-carboxylic acid (2'-difluoromethoxy-5'- chlorobiphenyl-4-yl)-amide;
4-methyl-[1,2,3]-thiadiazole-5-carboxylic acid {2'-(N,N-dimethylamino)- 5'-trifluoromethoxybiphenyl-4-yl}-amide;
3-methyl-pyridine-4-carboxylic acid (2'-chloro-5'- trifluoromethylbiphenyl-4-yl)-amide;
3-methyl-pyridine-4-carboxylic acid (2'-methylsulfanyl-biphenyl-4-yl)-amide;
3-methyl-pyridine-4-carboxylic acid (2'-ethyl-biphenyl-4-yl)-amide;
3-methyl-pyridine-4-carboxylic acid (2'-isopropyl-biphenyl-4-yl)-amide;
N-{5-(2',5'-dimethoxyphenyl)-pyrid-2-yl}-2-methylbenzamide;
3-methyl-pyridine-4-carboxylic acid (2',5'-diethylbiphenyl-4-yl)-amide;
3-methyl-pyridine-4-carboxylic acid {2'-(N,N-dimethylamino)-5'-methoxybiphenyl-4-yl}-amide;
3-methyl-pyridine-4-carboxylic acid {2'-(N-dimethylamino)- 5'-carbethoxybiphenyl-4-yl}-amide;
3-methyl-pyridine-4-carboxylic acid (2'-ethoxy-5'-chlorobiphenyl- 4-yl)-amide;
N-(2'-dimethoxy-5'-chloro biphenyl-4-yl)-2,6-difluorobenzamide;
N-(2'-methoxy-5'-chloro- biphenyl-4-yl)-2,4,5-trifluorobenzamide;
N-(2',5'-bis-trifluoromethyl biphenyl-4-yl)-2,6-difluorobenzamide;
N-(2'-chloro-5'-trifluoromethyl biphenyl-4-yl)-2,6-difluorobenzamide;
N-(2',5'-dimethyl biphenyl-4-yl)-2,6-difluorobenzamide;
N-(2',5'-dichloro biphenyl-4-yl)-2,6-difluorobenzamide;
2,3-Difluoro-N-[4-(2-trifluoromethyl-indolizin-3-yl)-phenyl]-benzamide;
2,5-Difluoro-N-[4-(2-trifluoromethyl-indolizin-3-yl)-phenyl]-benzamide;
3,4-dimethoxy-N-[4-(2-trifluoromethyl-indolizin-3-yl)-phenyl]- benzamide;
N-[4-(5-chloro-2-trifluoromethyl-indolizin-3-yl)-phenyl]-2,3-difluoro-benzamide;
5-chloro-3-[4-(2,3-difluoro-benzoylamino)-phenyl]-2-trifluoromethyl-indolizine-6-carboxylic acid methyl ester;
3-[4-(2,3-difluoro-benzoylamino)-phenyl]-2-trifluoromethyl-indolizine-6-carboxylic acid methyl ester;
2,3-difluoro-N-[4-(6-methoxy-2-trifluoromethyl-indolizin-3-yl)-phenyl]-benzamide;
N-[4-(5-fluoro-2-trifluoromethyl-indolizin-3-yl)-phenyl]-2,3-difluoro-benzamide;
N-[4-(5-methoxy-2-trifluoromethyl-indolizin-3-yl)-phenyl]-2,3-difluoro-benzamide;
N-[4-(5-Chloro-2,7-bis-trifluoromethyl-indolizin-3-yl)-phenyl]-2,3-difluoro-benzamide;
5-methoxy-3-[4-(2,3-difluoro-benzoylamino)-phenyl]-2-trifluoromethyl-indolizine-6-carboxylic acid methyl ester;
2,3-difluoro-N-[4-(8-methoxy-2-trifluoromethyl-indolizin-3-yl)-phenyl]-benzamide;
5-Chloro-3-[4-(2,3-difluoro-benzoylamino)-phenyl]-2-trifluoromethyl-indolizine-7-carboxylic acid methyl ester;
5-Chloro-3-[4-(2,6-difluoro-benzoylamino)-phenyl]-2-trifluoromethyl-ndolizine-7-carboxylic acid methyl ester;
2,6-difluoro-N-[4-(8-methoxy-2-trifluoromethyl-indolizin-3-yl)-phenyl]-benzamide;
5-Methoxy-3-[4-(2,6-difluoro-benzoylamino)-phenyl]-2-trifluoromethyl-indolizine-6-carboxylic acid methyl ester;
N-[4-(5-chloro-2,7-bis-trifluoromethyl-indolizin-3-yl)-phenyl]- 2,6-difluoro-benzamide;
N-[4-(5-methoxy-2-trifluoromethyl-indolizin-3-yl)-phenyl]-2,6-difluoro-benzamide;
N-[4-(5-fluoro-2-trifluoromethyl-indolizin-3-yl)-phenyl]-2,6-difluoro benzamide;
2,6-difluoro-N-[4-(6-methoxy-2-trifluoromethyl-indolizin-3-yl)-phenyl]-benzamide;
3-[4-(2,6-difluoro-benzoylamino)-phenyl]-2-trifluoromethyl-indolizine-6-carboxylic acid methyl ester;
5-Chloro-3-[4-(2,6-difluoro-benzoylamino)-phenyl]-2-trifluoromethyl-indolizine-6-carboxylic acid methyl ester;
N-[4-(5-Chloro-2-trifluoromethyl-indolizin-3-yl)-phenyl]-2,6-difluoro-benzamide;
N-[4-(5-Chloro-2-trifluoromethyl-indolizin-3-yl)-phenyl]-2,4,5-trifluorobenzamide;
5-Chloro-3-[4-(2,4,5-trifluoro-benzoylamino)-phenyl]-2-trifluoromethyl-indolizine-6-carboxylic acid methyl ester;
3-[4-(2,4,5-trifluoro-benzoylamino)-phenyl]-2-trifluoromethyl-indolizine-6-carboxylic acid methyl ester;
2,4,5-trifluoro-N-[4-(5-fluoro-2-trifluoromethyl-indolizin-3-yl)-phenyl]-benzamide;
2,4,5-trifluoro-N-[4-(6-methoxy-2-trifluoromethyl-indolizin-3-yl)-phenyl]-benzamide;
2,4,5-trifluoro-N-[4-(5-methoxy-2-trifluoromethyl-indolizin-3-yl)-phenyl]-benzamide;
N-[4-(5-Chloro-2,7-bis-trifluoromethyl-indolizin-3-yl)-phenyl]-2,4,5-trifluorobenzamide;
5-Methoxy-3-[4-(2,4,5-trifluoro-benzoylamino)-phenyl]-2-trifluoromethyl-indolizine-6-carboxylic acid methyl ester;
2,4,5-trifluoro-N-[4-(8-methoxy-2-trifluoromethyl-indolizin-3-yl)-phenyl]-benzamide;
5-Chloro-3-[4-(2,4,5-trifluoro-benzoylamino)-phenyl]-2-trifluoromethyl-indolizine-7-carboxylic acid methyl ester;
2,4,5-trifluoro-N-[4-(7-methoxy-2-trifluoromethyl-indolizin-3-yl)-phenyl]-benzamide;
2,6-difluoro-N-[4-(7-methoxy-2-trifluoromethyl-indolizin-3-yl)-phenyl]-benzamide;
2,3-difluoro-N-[4-(7-methoxy-2-trifluoromethyl-indolizin-3-yl)-phenyl]-benzamide;
N-(2',5'-dimethoxy-biphenyl-4-yl)-2,6-difluoro-benzamide;
N-(2'-trifluoromethyl-5'-methyl-biphenyl-4-yl)-2,6-difluoro-benzamide;
N-(2',5'-bis-trifluoromethyl-biphenyl-4-yl)-2,6-difluoro-benzyl amine;
N-(2',5'-bis-trifluoromethyl-biphenyl-4-yl)-2,6-difluoro-benzyl amine HCl salt;
N-(2'-methoxy-5'-chloro-biphenyl-4-yl)-2,6-difluoro-benzyl amine;
N-(2'-methoxy-5'-chloro-biphenyl-4-yl)-2,6-difluoro-benzyl amine HCl salt;
N',N'-diethyl-N-(2',5'-bis-trifluoromethyl biphenyl-4-yl) urea;
2,3-difluoro-N-[4-(2-trifluoro- methyl-indolizin-3-yl)-phenyl]- benzamide;
4-methyl-N-[4-(2-methyl- indolizin-3-yl)-phenyl]- [1,2,3]thiadiazole 5-carboxylic acid amide; and
pharmaceutically acceptable salts, solvates, or clathrates thereof.

51. A pharmaceutical composition, comprising a pharmaceutically acceptable carrier and one or more compounds represented by the following structural formula: 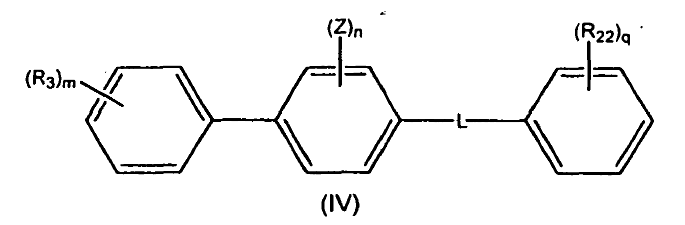 or a pharmaceutically acceptable salt, solvate, or clathrate thereof, wherein:
Z, R₃ and R₂₂, for each occurrence, are, independently, selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 0 or an integer from 1 to 4;
m and q are each independently, 0 or an integer from 1 to 5; and
p is 0, 1, or 2,
provided that when X is p-halophenyl, p-nitrophenyl, p-cyanophenyl, p-(methoxymethyl)phenyl, p-(benzamido)phenyl, a substituted p-(benzamido)phenyl, or p-carboxyphenyl, Y is not a substituted or unsubstituted phenyl, an unsubstituted furyl, a substituted or an unsubstituted thiophenyl, a substituted benzo[b]thiophenyl, an unsubstituted thiazolyl, a substituted 7,8-dihydronaphthyl, a substituted pyrazinyl, or a substituted or unsubstituted pyridinyl;
provided that when X is m-nitrophenyl or m-(trifluoromethyl)phenyl, Y₁ is not a substituted or unsubstituted phenyl; and
provided that X is not a phenyl that is substituted in the ortho position with -S(O)₂NH₂.

52. A pharmaceutical composition, comprising a pharmaceutically acceptable carrier and one or more compound represented by the following structural formula: 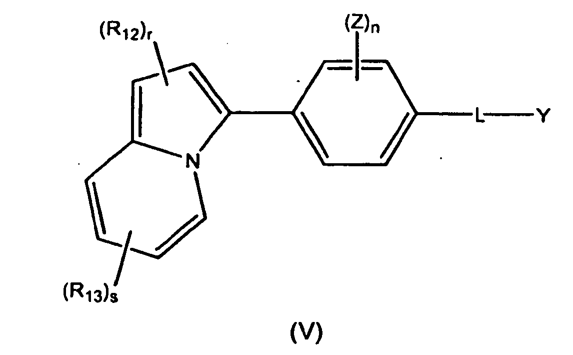 or a pharmaceutically acceptable salt, solvate, or clathrate thereof, wherein:
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
L is a linker selected from the group consisting of -NRCH₂-, and -NR-C(O)-;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
Z, R₁₂ and R₁₃, for each occurrence, is, independently, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
r is 0, 1 or 2;
n and s are each, independently, 0 or an integer from 1 to 4; and
p is 0, 1 or 2.

53. The pharmaceutical composition of Claim 52, wherein L is -NHC(O)- and Y is an optionally substituted phenyl.

54. The pharmaceutical composition of Claim 53, wherein the compound is represented by the following structural formula: 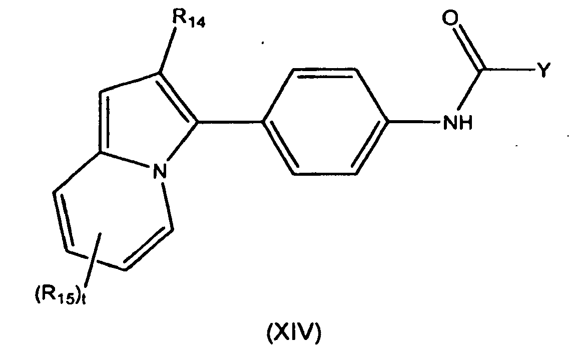 or a pharmaceutically acceptable salt, solvate, or clathrate thereof, wherein R₁₄ and R₁₅ are each; independently, -CF₃, -OCH₃, -F, -Cl, or -C(O)OCH₃; and
t is 0, 1 or 2.

55. The pharmaceutical composition of Claim 54, wherein the compound is represented by the following structural formula: 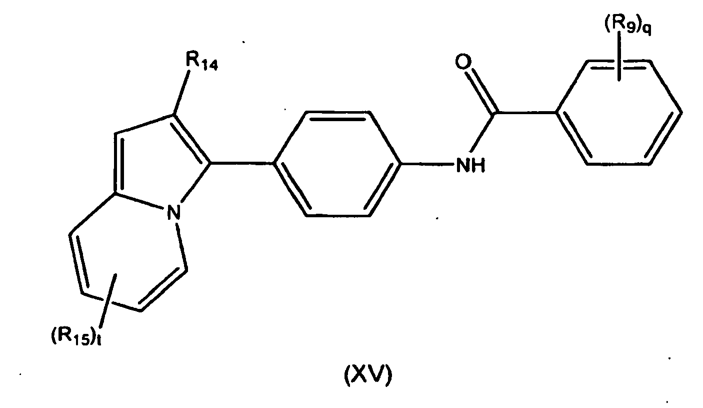 or a pharmaceutically acceptable salt, solvate, or clathrate thereof, wherein:
R₉, for each occurrence, is, independently, halo, C₁-C₄ alkyl, C₁-C₄ haloalkyl, C₁-C₄ alkoxy, C₁-C₄ haloalkoxy, or hydroxyl; and
q is 0 or an integer from 1 to 5.

56. The pharmaceutical composition of Claim 55, wherein the compound is represented by the following structural formula: 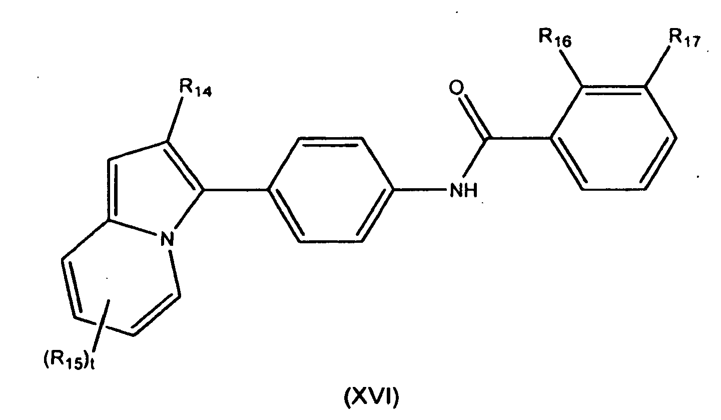 or a pharmaceutically acceptable salt, solvate, or clathrate thereof, wherein R₁₆ and R₁₇ are each, independently, -F, or -OCH₃.

57. The pharmaceutical composition of Claim 55, wherein the compound is represented by the following structural formula: 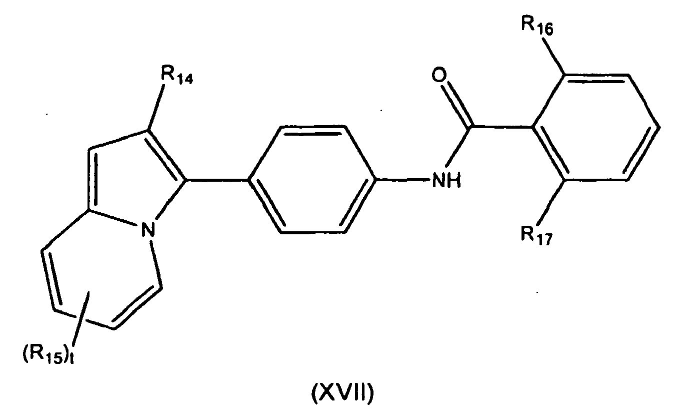 or a pharmaceutically acceptable salt, solvate, or clathrate thereof, wherein R₁₆ and R₁₇ are each, independently, -F, or -OCH₃.

58. A pharmaceutical composition, comprising a pharmaceutically acceptable carrier and one or more compounds represented by the following structural formula: 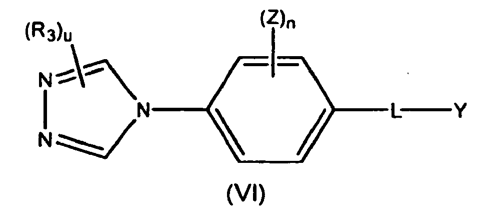 or a pharmaceutically acceptable salt, solvate, or clathrate thereof, wherein:
Y is an optionally substituted aryl or an optionally substituted heteroaryl which may be optionally substituted with 1-3 substituents independently selected from halo, C₁-C₄ alkyl, halogenated C₁-C₄ alkyl, or C₁-C₄ alkoxy;
Z and R₃, for each occurrence, are, independently, selected from the group consisting of an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl,a haloalkyl, -C(O)NR₁R₂, -NR₄C(O)R₅, halo, -OR₄, cyano, nitro, haloalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄, or -S(O)ₚNR₁R₂;
L is a linker selected from the group consisting of -NRCH₂-, and -NR-C(O)-;
each R is independently selected from -H, an alkyl, acetyl, tert-butoxycarbonyl, benzyloxycarbonyl;
R₁ and R₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁ and R₂ taken together with the nitrogen to which they are attached is optionally substituted heterocyclyl or optionally substituted heteroaryl;
R₄ and R₅ for each occurrence is, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is an integer selected from 0-4;
p is 0, 1, or 2; and
u is 0, 1, or 2.

59. The pharmaceutical composition of Claim 40, 49, 50, 51, 52 or 58, further comprising one or more additional therapeutic agents.

60. The pharmaceutical composition according to Claim 59, wherein the additional therapeutic agent is selected from the group consisting of immunosuppressive agents and anti-inflammatory agents and suitable mixtures thereof,

61. The pharmaceutical composition of Claim 60, wherein the additional therapeutic agent is selected from the group consisting of steroids, non-steroidal anti-inflammatory agents, antihistamines, analgesics, and suitable mixtures thereof.

## Patentansprüche

1. In-vitro-Verfahren zur Hemmung der Immunzellaktivierung, das das Verabreichen einer Verbindung der Formel (I): 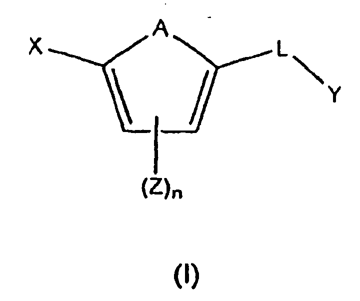 oder eines pharmazeutisch akzeptablen Salzes, Solvats oder Clathrats derselben an die Zelle umfasst, wobei in Formel (I):
X für ein optional substituiertes Phenyl, ein optional substituiertes Triazolyl, ein optional substituiertes Pyridyl oder ein optional substituiertes Indolizinyl steht;
Y für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
A für -CH=CH-, -CZ=CH- oder -CH=CZ- steht;
jedes Z unabhängig voneinander aus der Gruppe von einem optional substituierten Alkyl, einem optional substituierten Alkenyl, einem optional substituierten Alkinyl, einem optional substituierten Cycloalkyl, einem optional substituierten Cycloalkenyl, einem optional substituierten Heterocyclyl, einem optional substituierten Aryl, einem optional substituierten Heteroaryl, einem optional substituierten Aralkyl,
einem optional substituierten Heteroaralkyl, einem Halogenalkyl, -C(O)NR¹R², -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)pNR₁R₂ ausgewählt ist;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für 0 oder eine ganze Zahl von 1 bis 4 steht; und
p für 0, 1 oder 2 steht.

2. In-vitro-Verfahren zur Hemmung der Cyctokinproduktion in einer Zelle, das das Verabreichen einer Verbindung der folgenden Strukturformel: 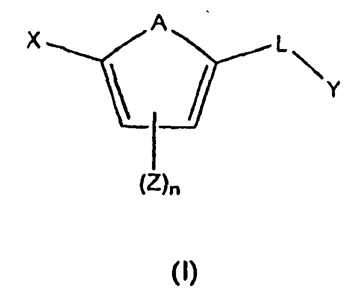 oder eines pharmazeutisch akzeptablen Salzes, Solvats oder Clathrats derselben an die Zelle umfasst, wobei in der Formel (I):
X für ein optional substituiertes Phenyl, ein optional substituiertes Triazolyl, ein optional substituiertes Pyridyl oder ein optional substituiertes Indolizinyl steht;
Y für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
A für -CH=CH-, -CZ=CH- oder -CH=CZ- steht;
jedes Z unabhängig voneinander aus der Gruppe von einem optional substituierten Alkyl, einem optional substituierten Alkenyl, einem optional substituierten Alkinyl, einem optional substituierten Cycloalkyl,
einem optional substituierten Cycloalkenyl, einem optional substituierten Heterocyclyl, einem optional substituierten Aryl, einem optional substituierten Heteroaryl, einem optional substituierten Aralkyl,
einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ ausgewählt ist;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für 0 oder eine ganze Zahl von 1 bis 4 steht; und
p für 0, 1 oder 2 steht.

3. Verfahren nach Anspruch 2, wobei das Cytokin, das gehemmt wird, aus der Gruppe von IL-2, IL-4, IL-5, IL-13, GM-CSF, TNF-α, IFN-γ und Kombinationen derselben ausgewählt ist.

4. In-vitro-Verfahren zur Modulierung eines Ionenkanals in einer Zelle, wobei der Ionenkanal an der Immunzellaktivierung beteiligt ist, wobei das Verfahren das Verabreichen einer Verbindung der folgenden Strukturformel: 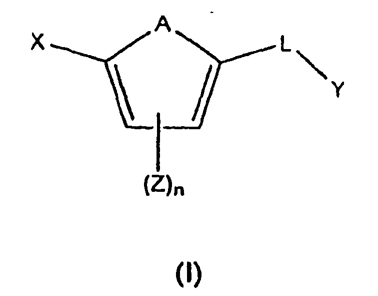 oder eines pharmazeutisch akzeptablen Salzes, Solvats oder Clathrats derselben an die Zelle umfasst, wobei in der Formel (I):
X für ein optional substituiertes Phenyl, ein optional substituiertes Triazolyl, ein optional substituiertes Pyridyl oder ein optional substituiertes Indolizinyl steht;
Y für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
A für -CH=CH-, -CZ=CH- oder -CH=CZ- steht;
jedes Z unabhängig voneinander aus der Gruppe von einem optional substituierten Alkyl, einem optional substituierten Alkenyl, einem optional substituierten Alkinyl, einem optional substituierten Cycloalkyl,
einem optional substituierten Cycloalkenyl, einem optional substituierten Heterocyclyl, einem optional substituierten Aryl, einem optional substituierten Heteroaryl, einem optional substituierten Aralkyl,
einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ ausgewählt ist;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für eine ganze Zahl steht, die aus 0-4 ausgewählt ist; und
p für 0, 1 oder 2 steht.

5. Verfahren nach Anspruch 4, wobei der Ionenkanal TRPM4 ist.

6. In-vitro-Verfahren zur Hemmung der T-Zell- und/oder B-Zellproliferation als Antwort auf ein Antigen, wobei das Verfahren das Verabreichen einer Verbindung der folgenden Strukturformel: 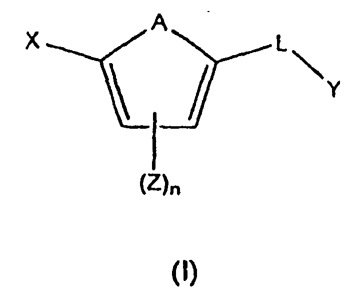 oder eines pharmazeutisch akzeptablen Salzes, Solvats oder Clathrats derselben an die Zelle umfasst, wobei in Formel (I):
X für ein optional substituiertes Phenyl, ein optional substituiertes Triazolyl, ein optional substituiertes Pyridyl oder ein optional substituiertes Indolizinyl steht;
Y für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
A für -CH=CH-, -CZ=CH- oder -CH=CZ- steht;
jedes Z unabhängig voneinander aus der Gruppe von einem optional substituierten Alkyl, einem optional substituierten Alkenyl, einem optional substituierten Alkinyl, einem optional substituierten Cycloalkyl,
einem optional substituierten Cycloalkenyl, einem optional substituierten Heterocyclyl, einem optional substituierten Aryl, einem optional substituierten Heteroaryl, einem optional substituierten Aralkyl,
einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ ausgewählt ist;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für 0 oder eine ganze Zahl von 1 bis 4 steht; und
p für 0, 1 oder 2 steht.

7. Verfahren nach Anspruch 1, 2, 4 oder 6, wobei die Verbindung eine Verbindung der folgenden Strukturformel: 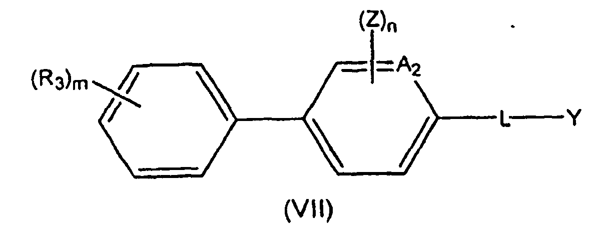 oder ein pharmazeutisch akzeptables Salz, Solvat oder Clathrat derselben ist, wobei:
A₂ für CH oder CZ steht;
R₃ für ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteroaralkyl, ein Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ steht; und
m für 0 oder eine ganze Zahl von 1 bis 5 steht.

8. Verfahren nach Anspruch 7, wobei A₂ CH ist.

9. Verfahren nach Anspruch 7, wobei L für -NHC(O)- oder -NHCH₂- steht.

10. Verfahren nach Anspruch 9, wobei Y für ein optional substituiertes Phenyl, ein optional substituiertes Pyridyl, ein optional substituiertes Thiophenyl, [1,2,3]Thiadiazolyl, ein optional substituiertes Isoxazolyl, 1H-Pyrazolyl, Chinolinyl, Imidazolyl oder 2,3-Dihydrobenzo[1,4]dioxin steht.

11. Verfahren nach Anspruch 10, wobei Y für ein optional substituiertes Phenyl, ein optional substituiertes Pyridyl oder ein optional substituiertes [1,2,3]Thiadiazolyl steht.

12. Verfahren nach Anspruch 11, wobei die Verbindung eine Verbindung der folgenden Strukturformel: 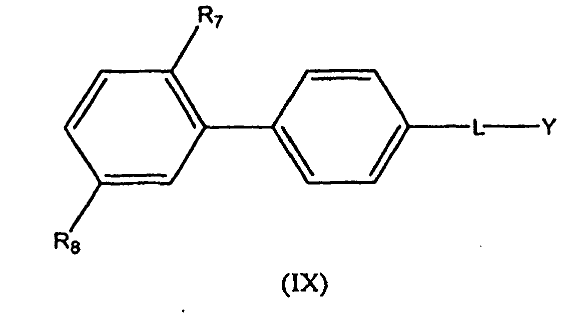 oder ein pharmazeutisch akzeptables Salz, Solvat oder Clathrat derselben ist,
wobei:
R₇ und R₈ jeweils unabhängig voneinander für -H, -CF₃, -CN, -C(O)CH₃, -F, -Cl, -OCH₃, -OCH₂CH₃, -C(O)OCH₂CH₃, -SCH₃, -NHCH₃ oder C₁-C₄-Alkyl stehen, mit der Maßgabe, dass mindestens ein Rest von R₇ oder R₈ nicht -H ist.

13. Verfahren nach Anspruch 12, wobei die Verbindung eine Verbindung der folgenden Strukturformel: 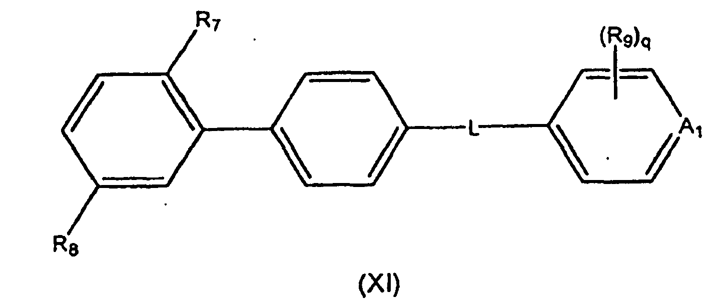 oder ein pharmazeutisch akzeptables Salz, Solvat oder Clathrat derselben ist,
wobei:
A₁ für CH, CR₉, N oder N→O steht;
R₉ bei jedem Vorkommen unabhängig voneinander für Halogen, C₁-C₄-Alkyl, C₁-C₄-Halogenalkyl, C₁-C₄-Alkoxy, C₁-C₄-Halogenalkoxy oder Hydroxyl steht; und
q für 0 oder eine ganze Zahl von 1 bis 5 steht.

14. Verfahren nach Anspruch 13, wobei die Verbindung eine Verbindung der folgenden Strukturformel: 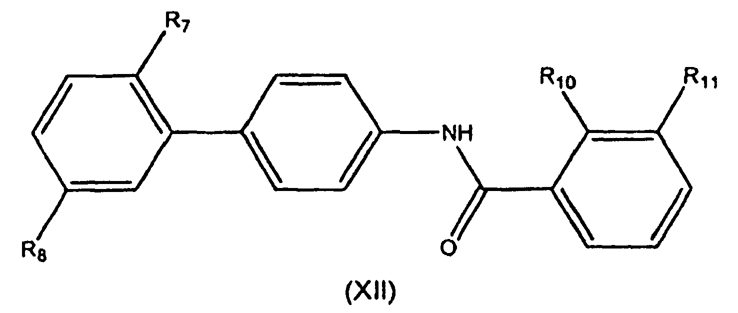 oder ein pharmazeutisch akzeptables Salz, Solvat oder Clathrat derselben ist,
wobei R₁₀ und R₁₁ jeweils unabhängig voneinander für -F, -Cl, ein C₁-C₄-Alkyl, ein C₁-C₄-Halogenalkyl, ein C₁-C₄-Alkoxy oder ein C₁-C₄-Halogenalkoxy stehen.

15. Verfahren nach Anspruch 13, wobei die Verbindung eine Verbindung der folgenden Strukturformel: 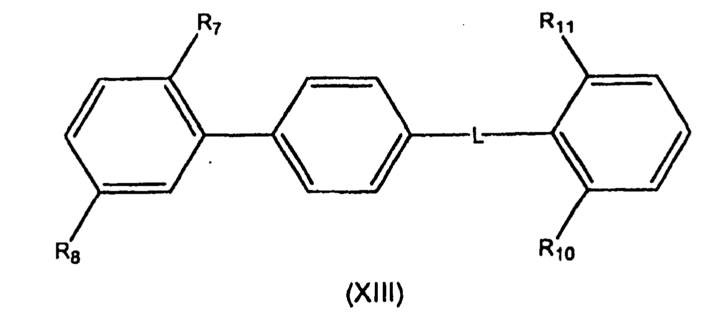 oder ein pharmazeutisch akzeptables Salz, Solvat oder Clathrat derselben ist,
wobei R₁₀ und R₁₁ jeweils unabhängig voneinander für -F, -Cl, ein C₁-C₄-Alkyl, ein C₁-C₄-Halogenalkyl, ein C₁-C₄-Alkoxy oder ein C₁-C₄-Halogenalkoxy stehen.

16. Verfahren nach Anspruch 1, 2, 4 oder 6, wobei die Verbindung eine Verbindung der folgenden Strukturformel: 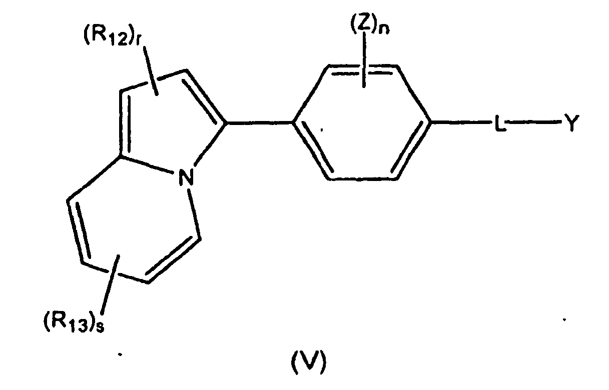 oder ein pharmazeutisch akzeptables Salz, Solvat oder Clathrat derselben ist, wobei:
R₁₂ und R₁₃ bei jedem Vorkommen unabhängig voneinander für ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteroaralkyl, ein Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ stehen;
r für 0, 1 oder 2 steht; und
s für 0 oder eine ganze Zahl von 1 bis 4 steht.

17. Verfahren nach Anspruch 16, wobei L -NHC(O)- ist und Y für ein optional substituiertes Phenyl steht.

18. Verfahren nach Anspruch 17, wobei die Verbindung eine Verbindung der folgenden Strukturformel: 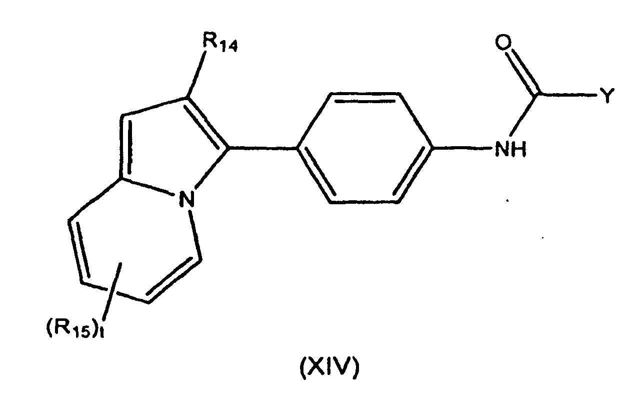 oder ein pharmazeutisch akzeptables Salz, Solvat oder Clathrat derselben ist, wobei:
R₁₄ und R₁₅ jeweils unabhängig voneinander für -CF₃, -OCH₃, -F, -Cl oder -C(O)OCH₃ stehen; und
t für 0, 1 oder 2 steht.

19. Verfahren nach Anspruch 18, wobei die Verbindung eine Verbindung der folgenden Strukturformel: 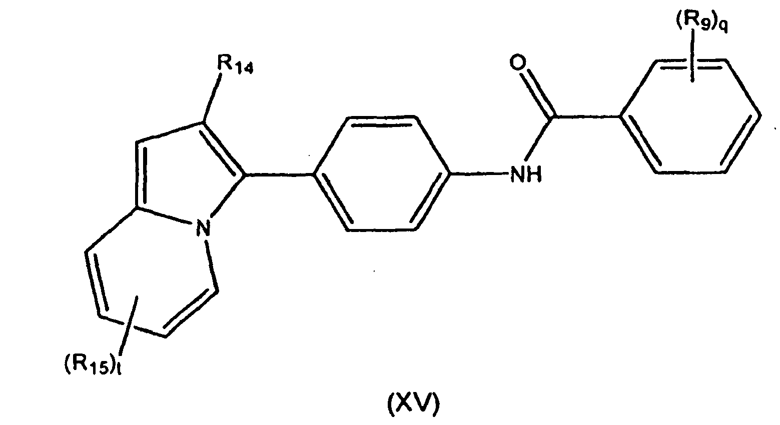 oder ein pharmazeutisch akzeptables Salz, Solvat oder Clathrat derselben ist, wobei:
R₉ bei jedem Vorkommen unabhängig voneinander für Halogen, C₁-C₄-Alkyl, C₁-C₄-Halogenalkyl, C₁-C₄-Alkoxy, C₁-C₄-Halogenalkoxy oder Hydroxyl steht; und
q für 0 oder eine ganze Zahl von 1 bis 5 steht.

20. Verfahren nach Anspruch 19, wobei die Verbindung eine Verbindung der folgenden Strukturformel: 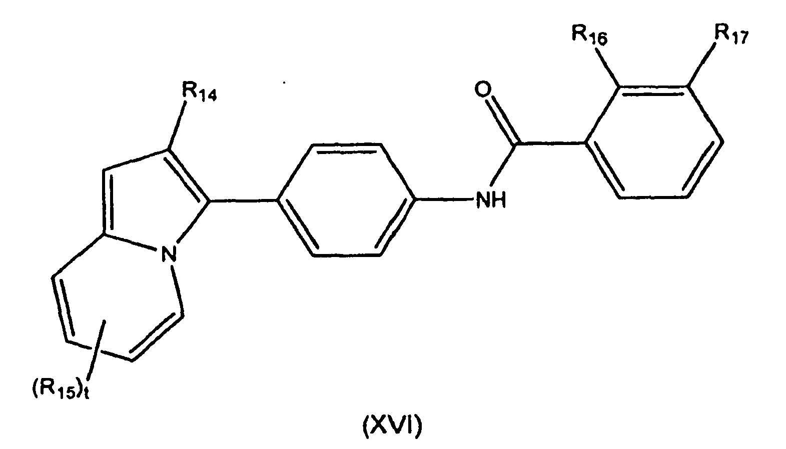 oder ein pharmazeutisch akzeptables Salz, Solvat oder Clathrat derselben ist, wobei R₁₆ und R₁₇ jeweils unabhängig voneinander für -F oder -OCH₃ stehen.

21. Verfahren nach Anspruch 19, wobei die Verbindung eine Verbindung der folgenden Strukturformel: 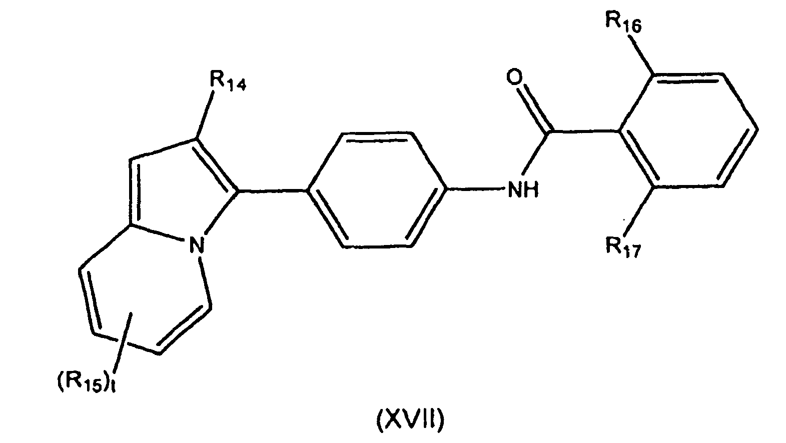 oder ein pharmazeutisch akzeptables Salz, Solvat oder Clathrat derselben ist, wobei R₁₆ und R₁₇ jeweils unabhängig voneinander für -F oder -OCH₃ stehen.

22. Verfahren nach Anspruch 1, 2, 4 oder 6, wobei die Verbindung eine Verbindung der folgenden Strukturformel: 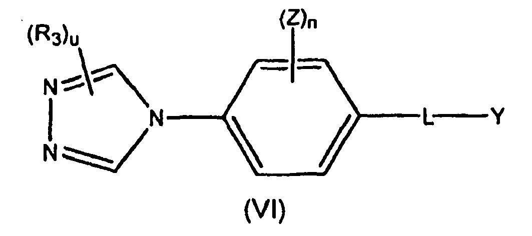 oder ein pharmazeutisch akzeptables Salz, Solvat oder Clathrat derselben ist, wobei:
R₃ für ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteroaralkyl, ein Halogenalkyl, -C(O)NR₁R₂, -NR₄C(O)R₅, Halogen, -OR₄, Cyano, Nitro, Halogenalkoxy, -C(O)R₄, -NR,R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ steht; und
u für 0, 1 oder 2 steht.

23. Verfahren nach Anspruch 22, wobei die Verbindung eine Verbindung der folgenden Strukturformel: 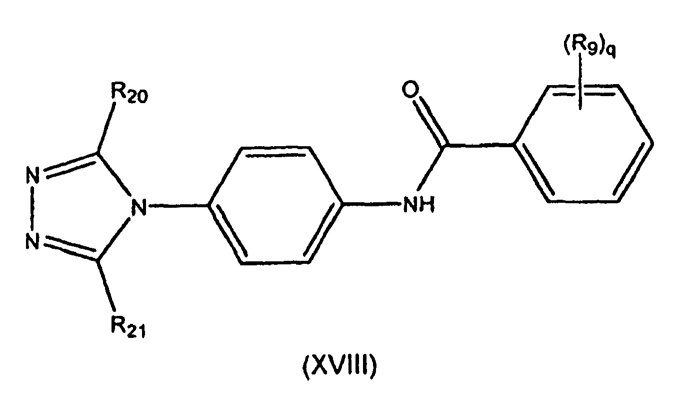 oder ein pharmazeutisch akzeptables Salz, Solvat oder Clathrat derselben ist, wobei:
R₂₀ und R₂₁ jeweils unabhängig voneinander für -H, -F, -Cl, ein C₁-C₄-Alkyl, Thiophenyl, -OCH₃, -CF₃ oder -OCF₃ stehen;
R₉ bei jedem Vorkommen unabhängig voneinander für Halogen, C₁-C₄-Alkyl, C₁-C₄-Halogenalkyl, C₁-C₄-Alkoxy,
C₁-C₄-Halogenalkoxy oder Hydroxyl steht; und
q für 0 oder eine ganze Zahl von 1 bis 5 steht.

24. Verfahren nach Anspruch 1, 2, 4 oder 6, wobei der Zelle eine oder mehrere Verbindungen verabreicht werden, die ausgewählt sind aus der Gruppe von:
3-Fluor-N-(2'-trifluormethyl-biphenyl-4-yl)-isonicotinamid;
3-Fluor-N-(2'-methyl-biphenyl-4-yl)-isonicotinamid;
3-Fluor-N-(3'-trifluormethyl-biphenyl-4-yl)-isonicotinamid;
N-(2,2'-Bis-trifluormethyl-biphenyl-4-yl)-2,3-difluorbenzamid;
N-[4-(1,2-Dimethyl-but-1-enyl)-3-trifluormethylphenyl]-2,3-difluorbenzamid;
4'-(2,3-Difluor-benzoylamino)-biphenyl-2-carbonsäuredimethylamid;
N-(2'-Trifluormethyl-biphenyl-4-yl)-nicotinamid;
N-(2'-Trifluormethyl-biphenyl-4-yl)-isonicotinamid;
Thiophen-2-carbonsäure-(2'-trifluormethyl-biphenyl-4-yl)-amid;
4-Fluor-N-(2'-trifluormethyl-biphenyl-4-yl)-benzamid;
2,4-Dimethyl-thiazol-5-carbonsäure-(2'-trifluormethyl-biphenyl-4-yl)-amid;
4-Trifluormethyl-N-(2'-trifluormethyl-biphenyl-4-yl)-nicotinamid;
2-Methyl-5-trifluormethyl-oxazol-4-carbonsäure-(2'-trifluormethyl-biphenyl-4-yl)-amid;
2-Ethyl-5-methyl-2H-pyrazol-3-carbonsäure-(2'-trifluormethyl-biphenyl-4-yl)-amid;
2,3-Difluor-N-(2'-trifluormethyl-biphenyl-4-yl)-benzamid;
2,5-Difluor-N-(2'-trifluormethyl-biphenyl-4-yl)-benzamid;
2,3-Difluor-N-(3-fluor-2'-trifluormethyl-biphenyl-4-yl)-benzamid;
N-(2',5'-Bis-trifluormethyl-biphenyl-4-yl)-2,3-difluorbenzamid;
2,3-Difluor-N-(2'-fluor-5'-trifluormethyl-biphenyl-4-yl)-benzamid;
2,3-Difluor-N-(4'-fluor-2'-trifluormethyl-biphenyl-4-yl)-benzamid;
2,3-Difluor-N-[4-(2-trifluormethyl-indolizin-3-yl)-phenyl]-benzamid;
2,3-Difluor-N-(2'-fluor-6'-trifluormethyl-biphenyl-4-yl)-benzamid;
2,3-Difluor-N-(2'-chlor-5'-trifluormethyl-biphenyl-4-yl)-benzamid;
4-Methyl-[1,2,3]thiadiazol-5-carbonsäure-(2',5'-bis-trifluormethyl-biphenyl-4-yl)-amid;
Pyridin-2-carbonsäure-(2'-trifluormethyl-biphenyl-4-yl)-amid;
Pyrazin-2-carbonsäure-(2'-trifluormethyl-biphenyl-4-yl)-amid;
4-Methyl-[1,2,3]thiadiazol-5-Carbonsäure-(2'-chlor-5'-trifluormethyl-biphenyl-4-yl)-amid;
N-(2',5'-Bis-trifluormethyl-biphenyl-4-yl)-2,5-difluorbenzamid;
N-(2',5'-Dichlor-biphenyl-4-yl)-2,3-difluorbenzamid;
N-(5'-Cyano-2'-methoxy-biphenyl-4-yl)-2,3-difluorbenzamid;
N-(2',5'-Dimethoxy-biphenyl-4-yl)-2,3-difluorbenzamid;
N-[4-(3,5-Bis-trifluormethyl-[1,2,4]triazol-4-yl)-phenyl]-2,3-difluorbenzamid;
3-Methyl-thiophen-2-carbonsäure-(4-(3,5-bis-trifluormethyl-[1,2,4]triazol-4-yl)-phenyl)-amid;
N-[4-(3-Trifluormethyl-5-(thiophen-4-yl)-[1,2,4]triazol-4-yl)-phenyl]-2,3-difluorbenzamid;
N-[4-(3-Trifluormethyl-5-(thiophen-4-yl)-[1,2,4]triazol-4-yl)-phenyl]-2,3-difluorbenzamid;
N-[4-(3-Trifluormethyl-indolizin-3-yl)-phenyl]-2,3-difluorbenzamid;
N-[4-(3-Cyano-5-trifluormethyl-pyrid-2-yl)-phenyl]-2,3-difluorbenzamid;
N-(2',5'-Bis-trifluormethyl-biphenyl-4-yl)-2-methoxybenzamid;
5-Methyl-isoxazol-3-carbonsäure-(2',5'-bis-trifluormethyl-biphenyl-4-yl)-amid;
1,3-Dimethyl-1H-pyrazol-5-carbonsäure-(2',5'-bis-trifluormethyl-biphenyl-4-yl)-amid;
[1,2,3]-Thiadiazol-4-carbonsäure-(2',5'-bis-trifluormethyl-biphenyl-4-yl)-amid;
Isoxazol-5-carbonsäure-(2',5'-bis-trifluormethyl-biphenyl-4-yl)-amid;
3,5-Dimethylisoxazol-4-carbonsäure-(2',5'-bis-trifluormethyl-biphenyl-4-yl)-amid;
N-(2'-Methoxy-5'-chlor-biphenyl-4-yl)-2,3-difluorbenzamid;
N-(2',5'-Bis-trifluormethyl-biphenyl-4-yl)-2-methoxybenzamid;
N-(2'-Methoxy-5'-methyl-biphenyl-4-yl)-2,3-difluorbenzamid;
N-(2',5'-Dimethyl-biphenyl-4-yl)-2,3-difluorbenzamid;
3-Methylisoxazol-4-carbonsäure-(2',5'-bis-trifluormethyl-biphenyl-4-yl)-amid;
N-(2',5'-Bis-trifluormethyl-biphenyl-4-yl)-2-hydroxybenzamid;
N-(2'-Methoxy-5'-acetyl-biphenyl-4-yl)-2,3-difluorbenzamid;
5-Methylisoxazol-4-carbonsäure-(2',5'-bis-trifluormethyl-biphenyl-4-yl)-amid;
N-(2',4',5'-Trimethyl-biphenyl-4-yl)-2,3-difluorbenzamid;
N-(2',5'-Bis-trifluormethyl-biphenyl-4-yl)-2,3-dimethylbenzamid;
N-(2',5'-Bis-trifluormethyl-biphenyl-4-yl)-2-methyl-3-chlorbenzamid;
N-(2',5'-Bis-trifluormethyl-biphenyl-4-yl)-2-methyl-3-fluorbenzamid;
N-(2',5'-Bis-trifluormethyl-biphenyl-4-yl)-2-methyl-3-methoxybenzamid;
4-Methyl-[1,2,3]-thiadiazol-5-carbonsäure-(2',5'-dimethoxy-biphenyl-4-yl)-amid;
N-(2',5'-Dimethoxy-biphenyl-4-yl)-2-methylbenzamid;
2-Methyl-pyridin-3-carbonsäure-N-(2',5,'-bis-trifluormethyl-biphenyl-4-yl)-amid;
2,3-Dihydro-benzo[1,4]dioxin-5-carbonsäure-(2',5'-bis-trifluormethyl-biphenyl-4-yl)-amid;
1-Methyl-1H-imidazol-5-carbonsäure-(2',5'-bis-trifluormethyl-biphenyl-4-yl)-amid;
3-Methyl-pyridin-4-carbonsäure-(2',5'-dimethoxy-biphenyl-4-yl)-amid;
3-Methyl-pyridin-4-carbonsäure-(2',5'-bis-trifluormethyl-biphenyl-4-yl)-amid;
3-Methyl-pyridin-4-carbonsäure-(2'-methoxy-5'-chlorbiphenyl-4-yl)amid;
3-Fluor-pyridin-4-carbonsäure-(2',5'-dimethoxybiphenyl-4-yl)-amid;
3-Fluor-pyridin-4-carbonsäure-(2'-methoxy-5'-chlorbiphenyl-4-yl)-amid;
3-Fluor-pyridin-4-carbonsäure-(2',5'-bis-trifluormethylbiphenyl-4-yl)-amid;
3-Methyl-pyridin-4-carbonsäure-(2'-methoxy-5'-methylbiphenyl-4-yl)-amid;
3-Methyl-pyridin-4-carbonsäure-(2',5'-dimethylbiphenyl-4-yl)-amid;
4-Methyl-[1,2,3]-thiadiazol-5-carbonsäure-(2'-methoxy-5'-acetylbiphenyl-4-yl)-amid;
3-Fluor-pyridin-4-carbonsäure-(2'-difluormethoxy-5'-chlorbiphenyl-4-yl)-amid;
4-Methyl-[1,2,3]-thiadiazol-5-carbonsäure-{2'-(N,N-dimethylamino)-5'-trifluormethoxybiphenyl-4-yl}-amid;
3-Methyl-pyridin-4-carbonsäure-(2'-chlor-5'-trifluormethylbiphenyl-4-yl)-amid;
3-Methyl-pyridin-4-carbonsäure-(2'-methylsulfanyl-biphenyl-4-yl)-amid;
3-Methyl-pyridin-4-carbonsäure-(2'-ethyl-biphenyl-4-yl)-amid;
3-Methyl-pyridin-4-carbonsäure-(2'-isopropyl-biphenyl-4-yl)-amid;
N-{5-(2',5'-Dimethoxyphenyl)-pyrid-2-yl}-2-methylbenzamid;
3-Methyl-pyridin-4-carbonsäure-(2',5'-diethylbiphenyl-4-yl)-amid;
3-Methyl-pyridin-4-carbonsäure-{2'-(N,N-dimethylamino)-5'-methoxybiphenyl-4-yl)-amid;
3-Methyl-pyridin-4-carbonsäure-{2'-(N-dimethylamino)-5'-carbethoxybiphenyl-4-yl}-amid;
3-Methyl-pyridin-4-carbonsäure-(2'-ethoxy-5'-chlorbiphenyl-4-yl)-amid;
N-(2'-Dimethoxy-5'-chlor-biphenyl-4-yl)-2,6-difluorbenzamid;
N-(2'-Methoxy-5'-chlor-biphenyl-4-yl)-2,4,5-trifluorbenzamid;
N-(2',5'-Bis-trifluormethyl-biphenyl-4-y1)-2,6-difluorbenzamid;
N-(2'-Chlor-5'-trifluormethyl-biphenyl-4-yl)-2,6-difluorbenzamid;
N-(2',5'-Dimethyl-biphenyl-4-yl)-2,6-difluorbenzamid;
N-(2',5'-Dichlor-biphenyl-4-yl)-2,6-difluorbenzamid;
2,3-Difluor-N-[4-(2-trifluormethyl-indolizin-3-yl)-phenyl]-benzamid;
2,5-Difluor-N-[4-(2-trifluormethyl-indolizin-3-yl)-phenyl]-benzamid;
3,4-Dimethoxy-N-[4-(2-trifluormethyl-indolizin-3-yl)-phenyl]-benzamid;
N-[4-(5-Chlor-2-trifluormethyl-indolizin-3-yl)-phenyl]-2,3-difluor-benzamid;
5-Chlor-3-[4-(2,3-difluor-benzoylamino)-phenyl]-2-trifluormethyl-indolizin-6-carbonsäuremethylester;
3-[4-(2,3-Difluor-benzoylamino)-phenyl]-2-trifluormethyl-indolizin-6-carbonsäuremethylester;
2,3-Difluor-N-[4-(6-methoxy-2-trifluormethyl-indolizin-3-yl)-phenyl]-benzamid;
N-[4-(5-Fluor-2-trifluormethyl-indolizin-3-yl)-phenyl]-2,3-difluor-benzamid;
N-[4-(5-Methoxy-2-trifluormethyl-indolizin-3-yl)-phenyl]-2,3-difluor-benzamid;
N-[4-(5-Chlor-2,7-bis-trifluormethyl-indolizin-3-yl)-phenyl]-2,3-difluor-benzamid;
5-Methoxy-3-[4-(2,3-difluor-benzoylamino)-phenyl)-2-trifluormethyl-indolizin-6-carbonsäuremethylester;
2,3-Difluor-N-[4-(8-methoxy-2-trifluormethyl-indolizin-3-yl)-phenyl]-benzamid; 5-Chlor-3-[4-(2,3-difluor-benzoylamino)-phenyl]-2-trifluormethyl-indolizin-7-carbonsäuremethylester; 5-Chlor-3-[4-(2,6-difluor-benzoylamino)-phenyl]-2-trifluormethyl-indolizin-7-carbonsäuremethylester; 2,6-Difluor-N-[4-(8-methoxy-2-trifluormethyl-indolizin-3-yl)-phenyl]-benzamid; 5-Methoxy-3-[4-(2,6-difluor-benzoylamino)-phenyl]-2-trifluormethyl-indolizin-6-carbonsäuremethylester; N-[4-(5-Chlor-2,7-bis-trifluormethyl-indolizin-3-yl)-phenyl]-2,6-difluor-benzamid; N-[4-(5-Methoxy-2-trifluormethyl-indolizin-3-yl)-phenyl]-2,6-difluor-benzamid; N-[4-(5-Fluor-2-trifluormethyl-indolizin-3-yl)-phenyl]-2,6-difluor-benzamid; 2,6-Difluor-N-[4-(6-methoxy-2-trifluormethyl-indolizin-3-yl)-phenyl]-benzamid; 3-[4-(2,6-Difluor-benzoylamino)-phenyl]-2-trifluormethyl-indolizin-6-carbonsäuremethylester; 5-Chlor-3-[4-(2,6-difluor-benzoylamino)-phenyl]-2-trifluormethyl-indolizin-6-carbonsäuremethylester; N-[4-(5-Chlor-2-trifluormethyl-indolizin-3-yl)-phenyl]-2,6-difluor-benzamid; N-[4-(5-Chlor-2-trifluormethyl-indolizin-3-yl)-phenyl]-2,4,5-trifluor-benzamid; 5-Chlor-3-[4-(2,4,5-trifluor-benzoylamino)-phenyl]-2-trifluormethyl-indolizin-6-carbonsäuremethylester; 3-[4-(2,4,5-Trifluor-benzoylamino)-phenyl]-2-trifluormethyl-indolizin-6-carbonsäuremethylester; 2,4,5-Trifluor-N-[4-(5-fluor-2-trifluormethyl-indolizin-3-yl)-phenyl]-benzamid; 2,4,5-Trifluor-N-[4-(6-methoxy-2-trifluormethyl-indolizin-3-yl)-phenyl]-benzamid; 2,4,5-Trifluor-N-[4-(5-methoxy-2-trifluormethyl-indolizin-3-yl)-phenyl]-benzamid;
N-[4-(5-Chlor-2,7-bis-trifluormethyl-indolizin-3-yl)-phenyl]-2,4,5-trifluor-benzamid; 5-Methoxy-3-[4-(2,4,5-trifluor-benzoylamino)-phenyl]-2-trifluormethyl-indolizin-6-carbonsäuremethylester; 2,4,5-Trifluor-N-[4-(8-methoxy-2-trifluormethyl-indolizin-3-yl)-phenyl]-benzamid; 5-Chlor-3-[4-(2,4,5-trifluor-benzoylamino)-phenyl]-2-trifluormethyl-indolizin-7-carbonsäuremethylester; 2,4,5-Trifluor-N-[4-(7-methoxy-2-trifluormethyl-indolizin-3-yl)-phenyl]-benzamid; 2,6-Difluor-N-[4-(7-methoxy-2-trifluormethyl-indolizin-3-yl)-phenyl]-benzamid; 2,3-Difluor-N-[4-(7-methoxy-2-trifluormethyl-indolizin-3-yl)-phenyl]-benzamid; N-(2',5'-Dimethoxy-biphenyl-4-yl)-2,6-difluor-benzamid; N-(2'-Trifluormethyl-5'-methyl-biphenyl-4-yl)-2,6-difluor-benzamid; N-(2',5'-Bis-trifluormethyl-biphenyl-4-yl)-2,6-difluor-benzylamin; N-(2',5'-Bis-trifluormethyl-biphenyl-4-yl)-2,6-difluor-benzylamin-HCl-Salz; N-(2'-Methoxy-5'-chlor-biphenyl-4-yl)-2,6-difluor-benzylamin; N-(2'-Methoxy-5'-chlor-biphenyl-4-yl)-2,6-difluor-benzylamin-HCl-Salz; N',N'-Diethyl-N-(2',5'-bis-trifluormethyl-biphenyl-4-yl)-harnstoff; 2,3-Difluor-N-[4-(2-trifluor-methyl-indolizin-3-yl)-phenyl]-benzamid; 4-Methyl-N-[4-(2-methyl-indolizin-3-yl)-phenyl]-[1,2,3]thiadiazol-5-carbonsäureamid;
und pharmazeutisch akzeptablen Salzen, Solvaten oder Clathraten derselben.

25. Verbindung der folgenden Strukturformel (II): 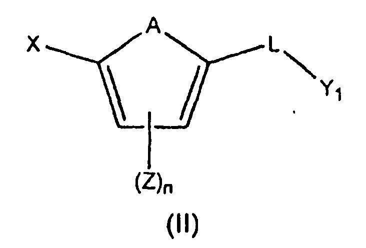 oder ein pharmazeutisch akzeptables Salz, Solvat oder Clathrat derselben, wobei:
X für ein optional substituiertes Phenyl, ein optional substituiertes Triazolyl, ein optional substituiertes Pyridyl oder ein optional substituiertes Indolizinyl steht;
Y₁ für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
A für -CH=CH-, -CZ=CH-, -CH=CZ- steht;
jedes Z unabhängig voneinander aus der Gruppe von einem optional substituierten Alkyl, einem optional substituierten Alkenyl, einem optional substituierten Alkinyl, einem optional substituierten Cycloalkyl,
einem optional substituierten Cycloalkenyl, einem optional substituierten Heterocyclyl, einem optional substituierten Aryl, einem optional substituierten Heteroaryl, einem optional substituierten Aralkyl,
einem optional substituierten Heteroaralkyl, einem Halogenalkyl, -C(O)NR₁R₂, -NR₄C(O)R₅, Halogen, -OR₄, Cyano, Nitro, Halogenalkoxy, -C (O) R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ ausgewählt ist;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für eine ganze Zahl steht, die aus 0-4 ausgewählt ist; und
p für 0, 1 oder 2 steht,
mit der Maßgabe, dass Y₁ nicht für ein Heteroaryl, das ferner mit einem substituierten oder unsubstituierten Aryl oder einem substituierten oder unsubstituierten Heteroaryl substituiert ist, steht;
mit der Maßgabe, dass, wenn X für ein p-Halogenphenyl, p-Nitrophenyl, p-Cyanophenyl, p-(Methoxymethyl)phenyl, p-(Benzamido)phenyl, ein substituiertes p-(Benzamido)phenyl oder p-Carboxyphenyl steht, Y₁ nicht für ein substituiertes oder unsubstituiertes Phenyl,
ein unsubstituiertes Furyl, ein substituiertes oder unsubstituiertes Thiophenyl, ein substituiertes Benzo[b]thiophenyl, ein unsubstituiertes Thiazolyl,
ein substituiertes 7,8-Dihydronaphthyl, ein substituiertes Pyrazinyl oder ein substituiertes oder unsubstituiertes Pyridinyl steht;
mit der Maßgabe, dass, wenn X m-Nitrophenyl oder m-(Trifluormethyl)phenyl ist, Y₁ nicht für ein substituiertes oder unsubstituiertes Phenyl steht;
mit der Maßgabe, dass X nicht für ein Phenyl, das in der ortho-Position mit -S(O)₂NH₂ substituiert ist, steht; und
mit der Maßgabe, dass X nicht für ein Nitrophenyl steht, wenn Y₁ für ein substituiertes 1H-Pyrazolyl steht.

26. Verbindung nach Anspruch 25, wobei die Verbindung eine Verbindung der folgenden Strukturformel: 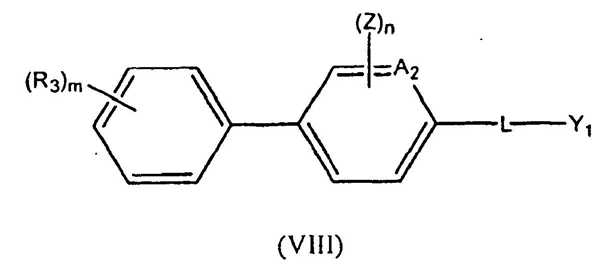 oder ein pharmazeutisch akzeptables Salz, Solvat oder Clathrat derselben ist,
wobei:
A₂ für CH oder CZ steht;
R₃ für ein optional substituiertes Alkyl, ein optional substituiertes Alkenyl, ein optional substituiertes Alkinyl, einem optional substituierten Cycloalkyl, ein optional substituiertes Cycloalkenyl, ein optional substituiertes Heterocyclyl, ein optional substituiertes Aryl, ein optional substituiertes Heteroaryl,
ein optional substituiertes Aralkyl, ein optional substituiertes Heteroaralkyl, ein Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ steht; und
m für 0 oder eine ganze Zahl von 1 bis 5 steht.

27. Verbindung nach Anspruch 26, wobei A₂ CH ist.

28. Verbindung nach Anspruch 26, wobei L -NHC(O)- oder -NHCH₂- ist.

29. Verbindung nach Anspruch 28, wobei Y₁ für ein optional substituiertes Phenyl, ein optional substituiertes Pyridyl, ein optional substituiertes Thiophenyl, [1,2,3]Thiadiazolyl, ein optional substituiertes Isoxazolyl, 1H-Pyrazolyl, Chinolinyl, Imidazolyl oder 2,3-Dihydrobenzo[1,4]dioxin steht.

30. Verbindung nach Anspruch 29, wobei Y₁ für ein optional substituiertes Phenyl, ein optional substituiertes Pyridyl oder ein optional substituiertes [1,2,3]Thiadiazolyl steht.

31. Verbindung nach Anspruch 30, wobei die Verbindung eine Verbindung der folgenden Strukturformel: 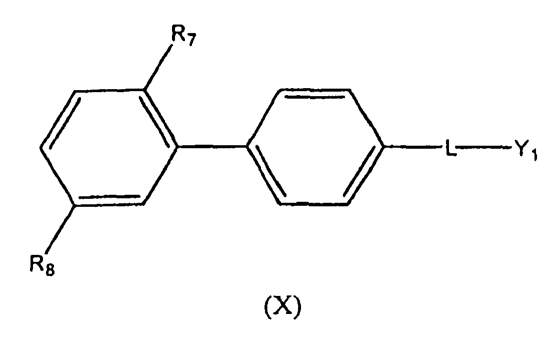 oder ein pharmazeutisch akzeptables Salz, Solvat oder Clathrat derselben ist,
wobei:
R₇ und R₈ jeweils unabhängig voneinander für -H, -CF₃, -CN, -C(O)CH₃, -F, -Cl, -OCH₃, -OCH₂CH₃, -C(O)OCH₂CH₃, -SCH₃, -NHCH₃ oder C₁-C₄-Alkyl stehen, mit der Maßgabe, dass mindestens ein Rest von R₇ oder R₈ nicht -H ist.

32. Verbindung der folgenden Strukturformel: 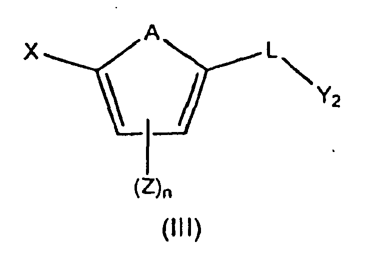 oder ein pharmazeutisch akzeptables Salz, Solvat oder Clathrat derselben,
wobei:
X für ein optional substituiertes Phenyl, ein optional substituiertes Triazolyl, ein optional substituiertes Pyridyl oder ein optional substituiertes Indolizinyl steht;
Y₂ für ein optional substituiertes Cycloalkyl, ein optional substituiertes Cycloalkenyl oder ein optional substituiertes Heterocyclyl steht;
A für -CH=CH-, -CZ=CH-, -CH=CZ- steht;
jedes Z unabhängig voneinander aus der Gruppe von einem optional substituierten Alkyl, einem optional substituierten Alkenyl, einem optional substituierten Alkinyl, einem optional substituierten Cycloalkyl,
einem optional substituierten Cycloalkenyl, einem optional substituierten Heterocyclyl, einem optional substituierten Aryl, einem optional substituierten Heteroaryl, einem optional substituierten Aralkyl,
einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)pR₄ oder -S(O)ₚNR₁R₂ ausgewählt ist;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für 0 oder eine ganze Zahl von 1 bis 4 steht; und
p für 0, 1 oder 2 steht,
mit der Maßgabe, dass X nicht für ein Phenyl, das in der ortho-Position mit -CN oder -S(O)₂NH₂ substituiert ist, steht; und
mit der Maßgabe, dass Y₂ nicht für ein 4,5-Dihydroisoxazolyl, das ferner mit einem substituierten oder unsubstituierten Aryl oder einem substituierten oder unsubstituierten Heteroaryl substituiert ist, steht;
und mit der Maßgabe, dass Y₂ nicht für ein substituiertes Cyclopentenyl steht.

33. Verbindung, die ausgewählt ist aus der Gruppe von:
3-Fluor-N-(2'-trifluormethyl-biphenyl-4-yl)-isonicotinamid;
3-Fluor-N-(2'-methyl-biphenyl-4-yl)-isonicotinamid;
3-Fluor-N-(3'-trifluormethyl-biphenyl-4-yl)-isonicotinamid;
N-(2,2'-Bis-trifluormethyl-biphenyl-4-yl)-2,3-difluorbenzamid;
N-[4-(1,2-Dimethyl-but-1-enyl)-3-trifluormethylphenyl]-2,3-difluorbenzamid;
4'-(2,3-Difluor-benzoylamino)-biphenyl-2-carbonsäuredimethylamid;
N-(2'-Trifluormethyl-biphenyl-4-yl)-nicotinamid;
N-(2'-Trifluormethyl-biphenyl-4-yl)-isonicotinamid;
Thiophen-2-carbonsäure-(2'-trifluormethyl-biphenyl-4-yl)-amid;
4-Fluor-N-(2'-trifluormethyl-biphenyl-4-yl)-benzamid;
2,4-Dimethyl-thiazol-5-carbonsäure-(2'-trifluormethyl-biphenyl-4-yl)-amid;
4-Trifluormethyl-N-(2'-trifluormethyl-biphenyl-4-yl)-nicotinamid;
2-Methyl-5-trifluormethyl-oxazol-4-carbonsäure-(2'-trifluormethyl-biphenyl-4-yl)-amid;
2-Ethyl-5-methyl-2H-pyrazol-3-carbonsäure-(2'-trifluormethyl-biphenyl-4-yl)-amid;
2,3-Difluor-N-(2'-trifluormethyl-biphenyl-4-yl)-benzamid;
2,5-Difluor-N-(2'-trifluormethyl-biphenyl-4-yl)-benzamid;
2,3-Difluor-N-(3-fluor-2'-trifluormethyl-biphenyl-4-yl)-benzamid;
N-(2',5'-Bis-trifluormethyl-biphenyl-4-yl)-2,3-difluorbenzamid;
2,3-Difluor-N-(2'-fluor-5'-trifluormethyl-biphenyl-4-yl)-benzamid;
2,3-Difluor-N-(4'-fluor-2'-trifluormethyl-biphenyl-4-yl)-benzamid;
2,3-Difluor-N-[4-(2-trifluormethyl-indolizin-3-yl)-phenyl]-benzamid;
2,3-Difluor-N-(2'-fluor-6'-trifluormethyl-biphenyl-4-yl)-benzamid;
2,3-Difluor-N-(2'-chlor-5'-trifluormethyl-biphenyl-4-yl)-benzamid;
4-Methyl-[1,2,3]thiadiazol-5-carbonsäure-(2',5'-bis-trifluormethyl-biphenyl-4-yl)-amid;
Pyridin-2-carbonsäure-(2'-trifluormethyl-biphenyl-4-yl)-amid;
Pyrazin-2-carbonsäure-(2'-trifluormethyl-biphenyl-4-yl)-amid;
4-Methyl-[1,2,3]thiadiazol-5-carbonsäure-(2'-chlor-5'-trifluormethyl-biphenyl-4-yl)-amid;
N-(2',5'-Bis-trifluormethyl-biphenyl-4-yl)-2,5-difluorbenzamid;
N-(2',5'-Dichlor-biphenyl-4-yl)-2,3-difluorbenzamid;
N-(5'-Cyano-2'-methoxy-biphenyl-4-yl)-2,3-difluorbenzamid;
N-(2',5'-Dimethoxy-biphenyl-4-yl)-2,3-difluorbenzamid;
N-[4-(3,5-Bis-trifluormethyl-[1,2,4]triazol-4-yl)-phenyl]-2,3-difluorbenzamid;
3-Methyl-thiophen-2-carbonsäure-(4-(3,5-bis-trifluormethyl-[1,2,4]triazol-4-yl)-phenyl)-amid;
N-[4-(3-Trifluormethyl-5-(thiophen-4-yl)-[1,2,4]triazol-4-yl)-phenyl]-2,3-difluorbenzamid;
N-[4-(3-Trifluormethyl-5-(thiophen-4-yl)-[1,2,4]triazol-4-yl)-phenyl]-2,3-difluorbenzamid;
N-[4-(3-Trifluormethyl-indolizin-3-yl)-phenyl]-2,3-difluorbenzamid;
N-[4-(3-Cyano-5-trifluormethyl-pyrid-2-yl)-phenyl]-2,3-difluorbenzamid;
N-(2',5'-Bis-trifluormethyl-biphenyl-4-yl)-2-methoxybenzamid;
5-Methyl-isoxazol-3-carbonsäure-(2',5'-bis-trifluormethyl-biphenyl-4-yl)-amid;
1,3-Dimethyl-1H-pyrazol-5-carbonsäure-(2',5'-bis-trifluormethyl-biphenyl-4-yl)-amid;
[1,2,3]-Thiadiazol-4-carbonsäure-(2',5'-bis-trifluormethyl-biphenyl-4-yl)-amid;
Isoxazol-5-carbonsäure-(2',5'-bis-trifluormethyl-biphenyl-4-yl)-amid;
3,5-Dimethylisoxazol-4-carbonsäure-(2',5'-bis-trifluormethyl-biphenyl-4-yl)-amid;
N-(2_{'}-Methoxy-5'-chlor-biphenyl-4-yl)-2,3-difluorbenzamid;
N-(2',5'-Bis-trifluormethyl-biphenyl-4-yl)-2-methoxybenzamid;
N-(2'-Methoxy-5'-methyl-biphenyl-4-yl)-2,3-difluorbenzamid;
N-(2',5'-Dimethyl-biphenyl-4-yl)-2,3-difluorbenzamid;
3-Methylisoxazol-4-carbonsäure-(2',5'-bis-trifluormethyl-biphenyl-4-yl)-amid;
N-(2',5'-Bis-trifluormethyl-biphenyl-4-yl)-2-hydroxybenzamid;
N-(2'-Methoxy-5'-acetyl-biphenyl-4-yl)-2,3-difluorbenzamid;
5-Methylisoxazol-4-carbonsäure-(2',5'-bis-trifluormethyl-biphenyl-4-yl)-amid;
N-(2',4',5'-Trimethyl-biphenyl-4-yl)-2,3-difluorbenzamid;
N-(2',5'-Bis-trifluormethyl-biphenyl-4-yl)-2,3-dimethylbenzamid;
N-(2',5'-Bis-trifluormethyl-biphenyl-4-yl)-2-methyl-3-chlorbenzamid;
N-(2',5'-Bis-trifluormethyl-biphenyl-4-yl)-2-methyl-3-fluorbenzamid;
N-(2',5'-Bis-trifluormethyl-biphenyl-4-yl)-2-methyl-3-methoxybenzamid;
4-Methyl-[1,2,3]-thiadiazol-5-carbonsäure-(2',5'-dimethoxy-biphenyl-4-yl)-amid;
N-(2',5'-Dimethoxy-biphenyl-4-yl)-2-methylbenzamid;
2-Methyl-pyridin-3-carbonsäure-(2',5'-bis-trifluormethyl-biphenyl-4-yl)-amid;
2,3-Dihydro-benzo[1,4]dioxin-5-carbonsäure-(2',5'-bis-trifluormethyl-biphenyl-4-yl)-amid;
1-Methyl-1H-imidazol-5-carbonsäure-(2',5'-bis-trifluormethyl-biphenyl-4-yl)-amid;
3-Methyl-pyridin-4-carbonsäure-(2',5'-dimethoxy-biphenyl-4-yl)-amid;
3-Methyl-pyridin-4-carbonsäure-(2',5'-bis-trifluormethyl-biphenyl-4-yl)-amid;
3-Methyl-pyridin-4-carbonsäure-(2'-methoxy-5'-chlorbiphenyl-4-yl)-amid;
3-Fluor-pyridin-4-carbonsäure-(2',5'-dimethoxybiphenyl-4-yl)-amid;
3-Fluor-pyridin-4-carbonsäure-(2'-methoxy-5'-chlorbiphenyl-4-yl)-amid;
3-Fluor-pyridin-4-carbonsäure-(2',5'-bis-trifluormethylbiphenyl-4-yl)-amid;
3-Methyl-pyridin-4-carbonsäure-(2'-methoxy-5'-methylbiphenyl-4-yl)-amid;
3-Methyl-pyridin-4-carbonsäure-(2',5'-dimethylbiphenyl-4-yl)-amid;
4-Methyl-[1,2,3]-thiadiazol-5-carbonsäure-(2'-methoxy-5'-acetylbiphenyl-4-yl)-amid;
3-Fluor-pyridin-4-carbonsäure-(2'-difluormethoxy-5'-chlorbiphenyl-4-yl)-amid;
4-Methyl-[1,2,3]-thiadiazol-5-carbonsäure-{2'-(N,N-dimethylamino)-5'-trifluormethoxybiphenyl-4-yl}-amid;
3-Methyl-pyridin-4-carbonsäure-(2'-chlor-5'-trifluormethylbiphenyl-4-yl)-amid;
3-Methyl-pyridin-4-carbonsäure-(2'-methylsulfanyl-biphenyl-4-yl)-amid;
3-Methyl-pyridin-4-carbonsäure-(2'-ethyl-biphenyl-4-yl)-amid;
3-Methyl-pyridin-4-carbonsäure-(2'-isopropyl-biphenyl-4-yl)-amid;
N-{5-(2',5'-Dimethoxyphenyl)-pyrid-2-yl}-2-methylbenzamid;
3-Methyl-pyridin-4-carbonsäure-(2',5'-diethylbiphenyl-4-yl)-amid;
3-Methyl-pyridin-4-carbonsäure-{2'-(N,N-dimethylamino)-5'-methoxybiphenyl-4-yl}-amid;
3-Methyl-pyridin-4-carbonsäure-{2'-(N-dimethylamino)-5'-carbethoxybiphenyl-4-yl}-amid;
3-Methyl-pyridin-4-carbonsäure-(2'-ethoxy-5'-chlorbiphenyl-4-yl)-amid;
N-(2'-Dimethoxy-5'-chlor-biphenyl-4-yl)-2,6-difluorbenzamid;
N-(2'-Methoxy-5'-chlor-biphenyl-4-yl)-2,4,5-trifluorbenzamid;
N-(2',5'-Bis-trifluormethyl-biphenyl-4-yl)-2,6-difluorbenzamid;
N-(2'-Chlor-5'-trifluormethyl-biphenyl-4-yl)-2,6-difluorbenzamid;
N-(2',5'-Dimethyl-biphenyl-4-yl)-2,6-difluorbenzamid;
N-(2',5'-Dichlor-biphenyl-4-yl)-2,6-difluorbenzamid;
2,3-Difluor-N-[4-(2-trifluormethyl-indolizin-3-yl)-phenyl]-benzamid;
2,5-Difluor-N-[4-(2-trifluormethyl-indolizin-3-yl)-phenyl]-benzamid;
3,4-Dimethoxy-N-[4-(2-trifluormethyl-indolizin-3-yl)-phenyl]-benzamid;
N-[4-(5-Chlor-2-trifluormethyl-indolizin-3-yl)-phenyl]-2,3-difluor-benzamid;
5-Chlor-3-[4-(2,3-difluor-benzoylamino)-phenyl]-2-trifluormethyl-indolizin-6-carbonsäuremethylester;
3-[4-(2,3-Difluor-benzoylamino)-phenyl]-2-trifluormethyl-indolizin-6-carbonsäuremethylester;
2,3-Difluor-N-[4-(6-methoxy-2-trifluormethyl-indolizin-3-yl)-phenyl]-benzamid;
N-[4-(5-Fluor-2-trifluormethyl-indolizin-3-yl)-phenyl]-2,3-difluor-benzamid;
N-[4-(5-Methoxy-2-trifluormethyl-indolizin-3-yl)-phenyl]-2,3-difluor-benzamid;
N-[4-(5-Chlor-2,7-bis-trifluormethyl-indolizin-3-yl)-phenyl]-2,3-difluor-benzamid;
5-Methoxy-3-[4-(2,3-difluor-benzoylamino)-phenyl]-2-trifluormethyl-indolizin-6-carbonsäuremethylester;
2,3-Difluor-N-[4-(8-methoxy-2-trifluormethyl-indolizin-3-yl)-phenyl]-benzamid;
5-Chlor-3-[4-(2,3-difluor-benzoylamino)-phenyl]-2-trifluormethyl-indolizin-7-carbonsäuremethylester;
5-Chlor-3-[4-(2,6-difluor-benzoylamino)-phenyl]-2-trifluormethyl-indolizin-7-carbonsäuremethylester;
2,6-Difluor-N-[4-(8-methoxy-2-trifluormethyl-indolizin-3-yl)-phenyl]-benzamid;
5-Methoxy-3-[4-(2,6-difluor-benzoylamino)-phenyl]-2-trifluormethyl-indolizin-6-carbonsäuremethylester;
N-[4-(5-Chlor-2,7-bis-trifluormethyl-indolizin-3-yl)-phenyl]-2,6-difluor-benzamid;
N-[4-(5-Methoxy-2-trifluormethyl-indolizin-3-yl)-phenyl]-2,6-difluor-benzamid;
N-[4-(5-Fluor-2-trifluormethyl-indolizin-3-yl)-phenyl]-2,6-difluor-benzamid;
2,6-Difluor-N-[4-(6-methoxy-2-trifluormethyl-indolizin-3-yl)-phenyl]-benzamid;
3-[4-(2,6-Difluor-benzoylamino)-phenyl]-2-trifluormethyl-indolizin-6-carbonsäuremethylester;
5-Chlor-3-[4-(2,6-difluor-benzoylamino)-phenyl]-2-trifluormethyl-indolizin-6-carbonsäuremethylester;
N-[4-(5-Chlor-2-trifluormethyl-indolizin-3-yl)-phenyl]-2,6-difluor-benzamid;
N-[4-(5-Chlor-2-trifluormethyl-indolizin-3-yl)-phenyl]-2,4,5-trifluor-benzamid;
5-Chlor-3-[4-(2,4,5-trifluor-benzoylamino)-phenyl]-2-trifluormethyl-indolizin-6-carbonsäuremethylester;
3-[4-(2,4,5-Trifluor-benzoylamino)-phenyl]-2-trifluormethyl-indolizin-6-carbonsäuremethylester;
2,4,5-Trifluor-N-[4-(5-fluor-2-trifluormethyl-indolizin-3-yl)-phenyl]-benzamid;
2,4,5-Trifluor-N-[4-(6-methoxy-2-trifluormethyl-indolizin-3-yl)-phenyl]-benzamid;
2,4,5-Trifluor-N-[4-(5-methoxy-2-trifluormethyl-indolizin-3-yl)-phenyl]-benzamid;
N-[4-(5-Chlor-2,7-bis-trifluormethyl-indolizin-3-yl)-phenyl]-2,4,5-trifluor-benzamid;
5-Methoxy-3-[4-(2,4,5-trifluor-benzoylamino)-phenyl]-2-trifluormethyl-indolizin-6-carbonsäuremethylester;
2,4,5-Trifluor-N-[4-(8-methoxy-2-trifluormethyl-indolizin-3-yl)-phenyl]-benzamid;
5-Chlor-3-[4-(2,4,5-trifluor-benzoylamino)-phenyl]-2-trifluormethyl-indolizin-7-carbonsäuremethylester;
2,4,5-Trifluor-N-[4-(7-methoxy-2-trifluormethyl-indolizin-3-yl)-phenyl]-benzamid;
2,6-Difluor-N-[4-(7-methoxy-2-trifluormethyl-indolizin-3-yl)-phenyl]-benzamid;
2,3-Difluor-N-[4-(7-methoxy-2-trifluormethyl-indolizin-3-yl)-phenyl]-benzamid;
N-(2',5'-Dimethoxy-biphenyl-4-yl)-2,6-difluor-benzamid;
N-(2'-Trifluormethyl-5'-methyl-biphenyl-4-yl)-2,6-difluor-benzamid;
N-(2',5'-Bis-trifluormethyl-biphenyl-4-yl)-2,6-difluor-benzylamin;
N-(2',5'-Bis-trifluormethyl-biphenyl-4-yl)-2,6-difluor-benzylamin-HCl-Salz;
N-(2'-Methoxy-5'-chlor-biphenyl-4-yl)-2,6-difluor-benzylamin;
N-(2'-Methoxy-5'-chlor-biphenyl-4-yl)-2,6-difluor-benzylamin-HCl-Salz;
N',N'-Diethyl-N-(2',5'-bis-trifluormethyl-biphenyl-4-yl)-harnstoff;
2,3-Difluor-N-[4-(2-trifluor-methyl-indolizin-3-yl)-phenyl]-benzamid;
4-Methyl-N-[4-(2-methyl-indolizin-3-yl)-phenyl]-[1,2,3]thiadiazol-5-carbonsäureamid;
und pharmazeutisch akzeptablen Salzen, Solvaten oder
Clathraten derselben.

34. Verbindung der folgenden Strukturformel: 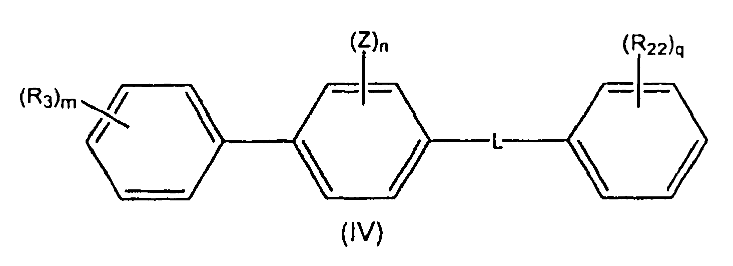 oder ein pharmazeutisch akzeptables Salz, Solvat oder Clathrat derselben,
wobei:
Z, R₃ und R₂₂ bei jedem Vorkommen unabhängig voneinander ausgewählt sind aus der Gruppe von einem optional substituierten Alkyl, einem optional substituierten Alkenyl, einem optional substituierten Alkinyl, einem optional substituierten Cycloalkyl, einem optional substituierten Cycloalkenyl, einem optional substituierten Heterocyclyl, einem optional substituierten Aryl, einem optional substituierten Heteroaryl, einem optional substituierten Aralkyl, einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für 0 oder eine ganze Zahl von 1 bis 4 steht;
m und q jeweils unabhängig voneinander für 0 oder eine ganze Zahl von 1 bis 5 stehen; und
p für 0, 1 oder 2 steht,
mit der Maßgabe, dass, wenn X für ein p-Halogenphenyl, p-Nitrophenyl, p-Cyanophenyl, p-(Methoxymethyl)phenyl, p-(Benzamido)phenyl, ein substituiertes p-(Benzamido)phenyl oder p-Carboxyphenyl steht, Y nicht für ein substituiertes oder unsubstituiertes Phenyl, ein unsubstituiertes Furyl, ein substituiertes oder
unsubstituiertes Thiophenyl, ein substituiertes Benzo[b]thiophenyl, ein unsubstituiertes Thiazolyl,
ein substituiertes 7,8-Dihydronaphthyl, ein substituiertes Pyrazinyl oder ein substituiertes oder unsubstituiertes Pyridinyl steht;
mit der Maßgabe, dass, wenn X m-Nitrophenyl oder m-(Trifluormethyl)phenyl ist, Y₁ nicht für ein substituiertes oder unsubstituiertes Phenyl steht; und mit der Maßgabe, dass X nicht für ein Phenyl, das in der ortho-Position mit -S(O)₂NH₂ substituiert ist, steht.

35. Verbindung der folgenden Strukturformel: 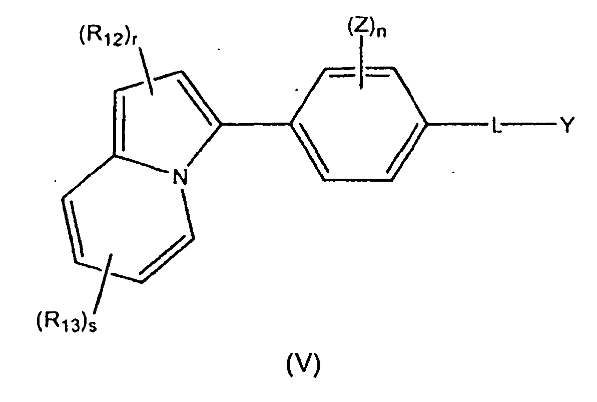 oder ein pharmazeutisch akzeptables Salz, Solvat oder Clathrat derselben,
wobei:
Y für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
L für einen Linker steht, der aus der Gruppe von -NRCH₂- und -NR-C(O)- ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
Z, R₁₂ und R₁₃ bei jedem Vorkommen unabhängig voneinander für ein optional substituiertes Alkyl, ein optional substituiertes Alkenyl, ein optional substituiertes Alkinyl, ein optional substituiertes Cycloalkyl,
ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteroaralkyl, ein Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
r für 0, 1 oder 2 steht;
n und s jeweils unabhängig voneinander für 0 oder eine ganze Zahl von 1 bis 4 stehen; und
p für 0, 1 oder 2 steht.

36. Verbindung nach Anspruch 35, wobei L -NHC(O)- ist und Y für ein optional substituiertes Phenyl steht.

37. Verbindung nach Anspruch 36, wobei die Verbindung eine Verbindung der folgenden Strukturformel: 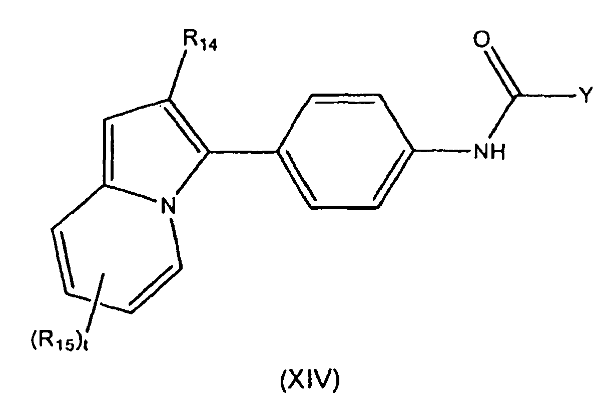 oder ein pharmazeutisch akzeptables Salz, Solvat oder Clathrat derselben ist,
wobei R₁₄ und R₁₅ jeweils unabhängig voneinander für -CF₃, -OCH₃, -F, -Cl oder -C(O)OCH₃ stehen; und
t für 0, 1 oder 2 steht.

38. Verbindung nach Anspruch 37, wobei die Verbindung eine Verbindung der folgenden Strukturformel: 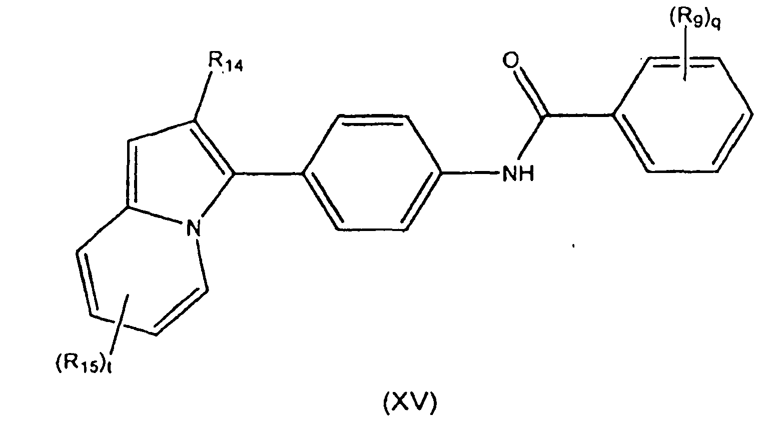 oder ein pharmazeutisch akzeptables Salz, Solvat oder Clathrat derselben ist,
wobei:
R₉ bei jedem Vorkommen unabhängig voneinander für Halogen, C₁-C₄-Alkyl, C₁-C₄-Halogenalkyl, C₁-C₄-Alkoxy, C₁-C₄-Halogenalkoxy oder Hydroxyl steht; und
q für 0 oder eine ganze Zahl von 1 bis 5 steht.

39. Verbindung nach Anspruch 38, wobei die Verbindung eine Verbindung der folgenden Strukturformel: 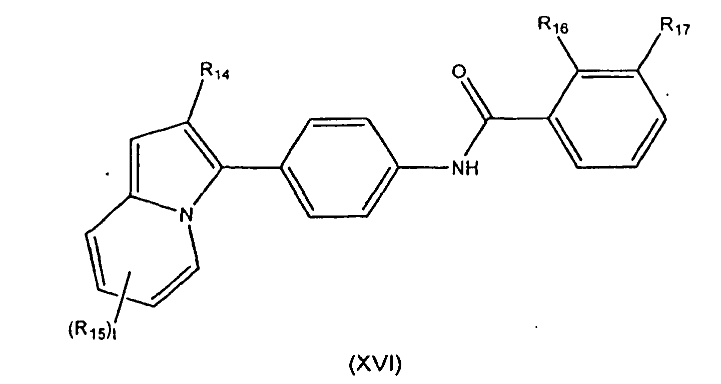 oder ein pharmazeutisch akzeptables Salz, Solvat oder Clathrat derselben ist,
wobei R₁₆ und R₁₇ jeweils unabhängig voneinander -F oder -OCH₃ sind.

40. Verbindung nach Anspruch 38, wobei die Verbindung eine Verbindung der folgenden Strukturformel: 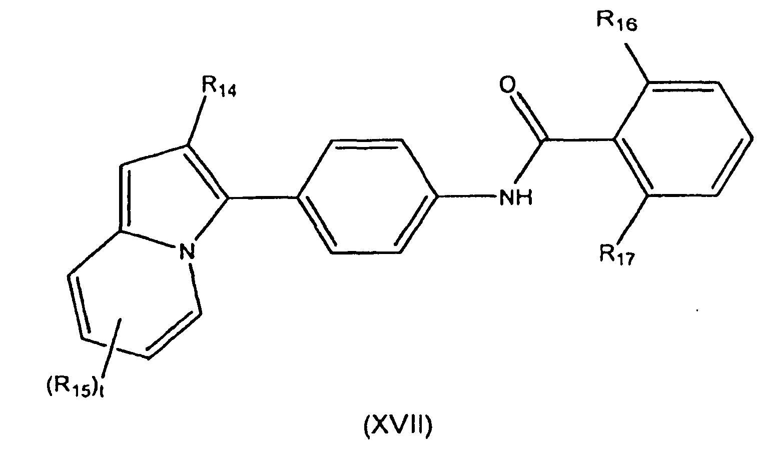 oder ein pharmazeutisch akzeptables Salz, Solvat oder
Clathrat derselben ist,
wobei R₁₆ und R₁₇ jeweils unabhängig voneinander -F oder -OCH₃ sind.

41. Verbindung der folgenden Strukturformel: 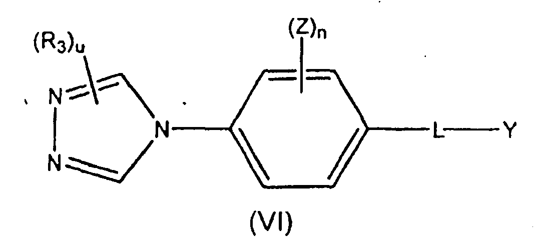 oder ein pharmazeutisch akzeptables Salz, Solvat oder Clathrat derselben,
wobei:
Y für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
Z und R₃ bei jedem Vorkommen unabhängig voneinander ausgewählt sind aus der Gruppe von einem optional substituierten Alkyl, einem optional substituierten Alkenyl, einem optional substituierten Alkinyl, einem optional substituierten Cycloalkyl, einem optional substituierten Cycloalkenyl, einem optional substituierten Heterocyclyl, einem optional substituierten Aryl, einem optional substituierten Heteroaryl, einem optional substituierten Aralkyl, einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für eine ganze Zahl steht, die aus 0-4 ausgewählt ist;
p für 0, 1 oder 2 steht; und
u für 0, 1 oder 2 steht.

42. Pharmazeutische Zusammensetzung, die einen pharmazeutisch akzeptablen Träger und eine oder mehrere Verbindungen der folgenden Strukturformel: 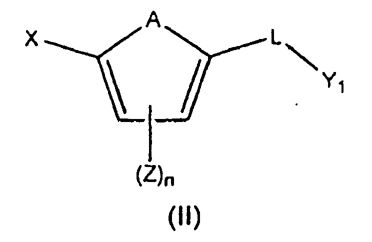 oder ein pharmazeutisch akzeptables Salz, Solvat oder Clathrat derselben umfasst,
wobei:
X für ein optional substituiertes Phenyl, ein optional substituiertes Triazolyl, ein optional substituiertes Pyridyl oder ein optional substituiertes Indolizinyl steht;
Y₁ für ein optional substituiertes Aryl oder ein optional substituiertes Heteroaryl steht;
A für -CH=CH-, -CZ=CH-, -CH=CZ- steht;
jedes Z unabhängig voneinander aus der Gruppe von einem optional substituierten Alkyl, einem optional substituierten Alkenyl, einem optional substituierten Alkinyl, einem optional substituierten Cycloalkyl,
einem optional substituierten Cycloalkenyl, einem optional substituierten Heterocyclyl, einem optional substituierten Aryl, einem optional substituierten Heteroaryl, einem optional substituierten Aralkyl,
einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O) OR₄, -OC(O)R₄, -NR₄C(O)NR₁R₂, -OC(O)NR₁R₂, -NR₄C(O)OR₅, -S(O)ₚR₄ oder -S(O)pNR₁R₂ ausgewählt ist;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für eine ganze Zahl steht, die aus 0-4 ausgewählt ist; und
p für 0, 1 oder 2 steht,
mit der Maßgabe, dass Y₁ nicht für ein Heteroaryl, das ferner mit einem substituierten oder unsubstituierten Aryl oder einem substituierten oder unsubstituierten Heteroaryl substituiert ist, steht;
mit der Maßgabe, dass, wenn X für ein p-Halogenphenyl, p-Nitrophenyl, p-Cyanophenyl, p-(Methoxymethyl)phenyl, p-(Benzamido)phenyl, substituiertes p-(Benzamido)phenyl oder p-Carboxyphenyl steht, Y₁ nicht für ein substituiertes oder unsubstituiertes Phenyl, ein unsubstituiertes Furyl, ein substituiertes oder unsubstituiertes Thiophenyl, ein substituiertes Benzo[b]thiophenyl, ein unsubstituiertes Thiazolyl, ein substituiertes 7,8-Dihydronaphthyl, ein substituiertes Pyrazinyl oder ein substituiertes oder unsubstituiertes Pyridinyl steht;
mit der Maßgabe, dass, wenn X m-Nitrophenyl oder m-(Trifluormethyl)phenyl ist, Y₁ nicht für ein substituiertes oder unsubstituiertes Phenyl steht;
mit der Maßgabe, dass X nicht für ein Phenyl, das in der ortho-Position mit -S(O)₂NH₂ substituiert ist, steht; und
mit der Maßgabe, dass X nicht für ein Nitrophenyl steht, wenn Y₁ für ein substituiertes 1H-Pyrazolyl steht.

43. Pharmazeutische Zusammensetzung nach Anspruch 42, wobei die Verbindung eine Verbindung der folgenden Strukturformel: 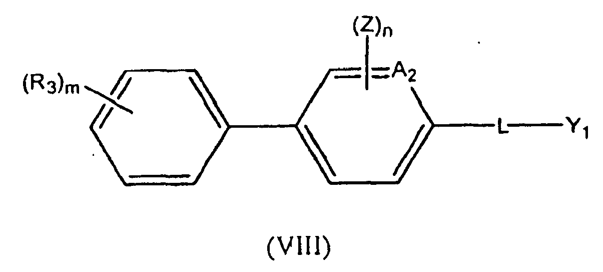 oder ein pharmazeutisch akzeptables Salz, Solvat oder Clathrat derselben ist,
wobei:
A₂ für CH oder CZ steht;
R₃ für ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl,
ein optional substituiertes Aralkyl, ein optional substituiertes Heteroaralkyl, ein Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ steht; und
m für 0 oder eine ganze Zahl von 1 bis 5 steht.

44. Pharmazeutische Zusammensetzung nach Anspruch 43, wobei A₂ CH ist.

45. Pharmazeutische Zusammensetzung nach Anspruch 43, wobei L -NHC(O)- oder -NHCH₂- ist.

46. Pharmazeutische Zusammensetzung nach Anspruch 45, wobei Y₁ für ein optional substituiertes Phenyl, ein optional substituiertes Pyridyl, ein optional substituiertes Thiophenyl, [1,2,3]Thiadiazolyl, ein optional substituiertes Isoxazolyl, 1H-Pyrazolyl, Chinolinyl, Imidazolyl oder 2,3-Dihydrobenzo[1,4]dioxin steht.

47. Pharmazeutische Zusammensetzung nach Anspruch 46, wobei Y₁ für ein optional substituiertes Phenyl, ein optional substituiertes Pyridyl oder ein optional substituiertes [1,2,3]Thiadiazolyl steht.

48. Pharmazeutische Zusammensetzung nach Anspruch 47, wobei die Verbindung eine Verbindung der folgenden Strukturformel: 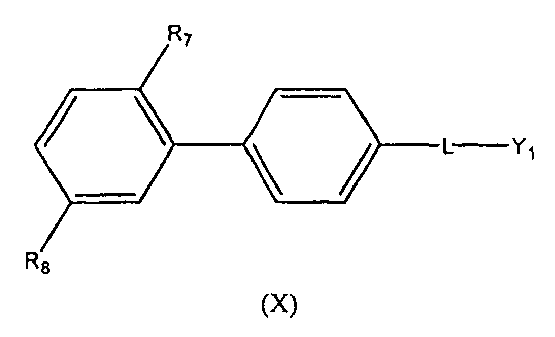 oder ein pharmazeutisch akzeptables Salz, Solvat oder Clathrat derselben ist,
wobei:
R₇ und R₈ jeweils unabhängig voneinander für -H, -CF₃, -CN, -C(O)CH₃, -F, -Cl, -OCH₃ , -OCH₂CH₃, -C(O)OCH₂CH₃, -SCH₃, -NHCH₃ oder C₁-C₄-Alkyl stehen, mit der Maßgabe, dass mindestens ein Rest von R₇ oder R₈ nicht -H ist.

49. Pharmazeutische Zusammensetzung, die einen pharmazeutisch akzeptablen Träger und eine oder mehrere Verbindungen der folgenden Strukturformel: 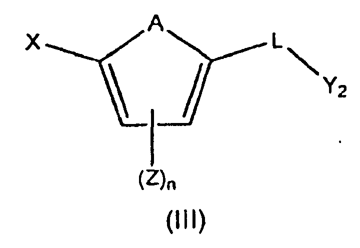 oder ein pharmazeutisch akzeptables Salz, Solvat oder Clathrat derselben umfasst,
wobei:
X für ein optional substituiertes Phenyl, ein optional substituiertes Triazolyl, ein optional substituiertes Pyridyl oder ein optional substituiertes Indolizinyl steht;
Y₂ für ein optional substituiertes Cycloalkyl, ein optional substituiertes Cycloalkenyl oder ein optional substituiertes Heterocyclyl steht;
A für -CH=CH-, -CZ=CH-, -CH=CZ- steht;
jedes Z unabhängig voneinander aus der Gruppe von einem optional substituierten Alkyl, einem optional substituierten Alkenyl, einem optional substituierten Alkinyl, einem optional substituierten Cycloalkyl,
einem optional substituierten Cycloalkenyl, einem optional substituierten Heterocyclyl, einem optional substituierten Aryl, einem optional substituierten Heteroaryl, einem optional substituierten Aralkyl,
einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ ausgewählt ist;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für 0 oder eine ganze Zahl von 1 bis 4 steht; und
p für 0, 1 oder 2 steht,
mit der Maßgabe, dass X nicht für ein Phenyl, das in der ortho-Position mit -CN oder -S(O)₂NH₂ substituiert ist, steht; und
mit der Maßgabe, dass Y₂ nicht für ein 4,5-Dihydroisoxazolyl, das ferner mit einem substituierten oder unsubstituierten Aryl oder einem substituierten oder unsubstituierten Heteroaryl substituiert ist, steht;
und
mit der Maßgabe, dass Y₂ nicht für ein substituiertes Cyclopentenyl steht.

50. Pharmazeutische Zusammensetzung, die einen pharmazeutisch akzeptablen Träger und eine oder mehrere Verbindungen umfasst, die ausgewählt sind aus der Gruppe von:
3-Fluor-N-(2'-trifluormethyl-biphenyl-4-yl)-isonicotinamid;
3-Fluor-N-(2'-methyl-biphenyl-4-yl)-isonicotinamid;
3-Fluor-N-(3'-trifluormethyl-biphenyl-4-yl)-isonicotinamid;
N-(2,2'-Bis-trifluormethyl-biphenyl-4-yl)-2,3-difluorbenzamid;
N-[4-(1,2-Dimethyl-but-1-enyl)-3-trifluormethylphenyl]-2,3-difluorbenzamid;
4'-(2,3-Difluor-benzoylamino)-biphenyl-2-carbonsäuredimethylamid;
N-(2'-Trifluormethyl-biphenyl-4-yl)-nicotinamid;
N-(2'-Trifluormethyl-biphenyl-4-yl)-isonicotinamid;
Thiophen-2-carbonsäure-(2'-trifluormethyl-biphenyl-4-yl)-amid;
4-Fluor-N-(2'-trifluormethyl-biphenyl-4-yl)-benzamid;
2,4-Dimethyl-thiazol-5-carbonsäure-(2'-trifluormethyl-biphenyl-4-yl)-amid;
4-Trifluormethyl-N-(2'-trifluormethyl-biphenyl-4-yl)-nicotinamid;
2-Methyl-5-trifluormethyl-oxazol-4-carbonsäure-(2'-trifluormethyl-biphenyl-4-yl)-amid;
2-Ethyl-5-methyl-2H-pyrazol-3-carbonsäure-(2'-trifluormethyl-biphenyl-4-yl)-amid;
2,3-Difluor-N-(2'-trifluormethyl-biphenyl-4-yl)-benzamid;
2,5-Difluor-N-(2'-trifluormethyl-biphenyl-4-yl)-benzamid;
2,3-Difluor-N-(3-fluor-2'-trifluormethyl-biphenyl-4-yl)-benzamid;
N-(2',5'-Bis-trifluormethyl-biphenyl-4-yl)-2,3-difluorbenzamid;
2,3-Difluor-N-(2'-fluor-5'-trifluormethyl-biphenyl-4-yl)-benzamid;
2,3-Difluor-N-(4'-fluor-2'-trifluormethyl-biphenyl-4-yl)-benzamid;
2,3-Difluor-N-[4-(2-trifluormethyl-indolizin-3-yl)-phenyl]-benzamid;
2,3-Difluor-N-(2'-fluor-6'-trifluormethyl-biphenyl-4-yl)-benzamid;
2,3-Difluor-N-(2'-chlor-5'-trifluormethyl-biphenyl-4-yl)-benzamid;
4-Methyl-[1,2,3]thiadiazol-5-carbonsäure-(2',5'-bis-trifluormethyl-biphenyl-4-yl)-amid;
Pyridin-2-carbonsäure-(2'-trifluormethyl-biphenyl-4-yl)-amid;
Pyrazin-2-carbonsäure-(2'-trifluormethyl-biphenyl-4-yl)-amid;
4-Methyl-[1,2,3]thiadiazol-5-carbonsäure-(2'-chlor-5'-trifluormethyl-biphenyl-4-yl)-amid;
N-(2',5'-Bis-trifluormethyl-biphenyl-4-yl)-2,5-difluorbenzamid;
N-(2',5'-Dichlor-biphenyl-4-yl)-2,3-difluorbenzamid;
N-(5'-Cyano-2'-methoxy-biphenyl-4-yl)-2,3-difluorbenzamid;
N-(2',5'-Dimethoxy-biphenyl-4-yl)-2,3-difluorbenzamid;
N-[4-(3,5-Bis-trifluormethyl-[1,2,4]triazol-4-yl)-phenyl]-2,3-difluorbenzamid;
3-Methyl-thiophen-2-carbonsäure-(4-(3,5-bis-trifluormethyl-[1,2,4]triazol-4-yl)-phenyl)-amid;
N-[4-(3-Trifluormethyl-5-(thiophen-4-yl)-[1,2,4]triazol-4-yl)-phenyl]-2,3-difluorbenzamid;
N-[4-(3-Trifluormethyl-5-(thiophen-4-yl)-[1,2,4]triazol-4-yl)-phenyl]-2,3-difluorbenzamid;
N-[4-(3-Trifluormethyl-indolizin-3-yl)-phenyl]-2,3-difluorbenzamid;
N-[4-(3-Cyano-5-trifluormethyl-pyrid-2-yl)-phenyl]-2,3-difluorbenzamid;
N-(2',5'-Bis-trifluormethyl-biphenyl-4-yl)-2-methoxybenzamid;
5-Methyl-isoxazol-3-carbonsäure-(2',5'-bis-trifluormethyl-biphenyl-4-yl)-amid;
1,3-Dimethyl-1H-pyrazol-5-carbonsäure-(2',5'-bis-trifluormethyl-biphenyl-4-yl)-amid;
[1,2,3]-Thiadiazol-4-carbonsäure-(2',5'-bis-trifluormethyl-biphenyl-4-yl)-amid;
Isoxazol-5-carbonsäure-(2',5'-bis-trifluormethyl-biphenyl-4-yl)-amid;
3,5-Dimethylisoxazol-4-carbonsäure-(2',5'-bis-trifluormethyl-biphenyl-4-yl)-amid;
N-(2'-Methoxy-5'-chlor-biphenyl-4-yl)-2,3-difluorbenzamid;
N-(2',5'-Bis-trifluormethyl-biphenyl-4-yl)-2-methoxybenzamid;
N-(2'-Methoxy-5'-methyl-biphenyl-4-yl)-2,3-difluorbenzamid;
N-(2',5'-Dimethyl-biphenyl-4-yl)-2,3-difluorbenzamid;
3-Methylisoxazol-4-carbonsäure-(2',5'-bis-trifluormethyl-biphenyl-4-yl)-amid;
N-(2',5'-Bis-trifluormethyl-biphenyl-4-yl)-2-hydroxybenzamid;
N-(2'-Methoxy-5'-acetyl-biphenyl-4-yl)-2,3-difluorbenzamid;
5-Methylisoxazol-4-carbonsäure-(2',5'-bis-trifluormethyl-biphenyl-4-yl)-amid;
N-(2',4',5'-Trimethyl-biphenyl-4-yl)-2,3-difluorbenzamid;
N-(2',5'-Bis-trifluormethyl-biphenyl-4-yl)-2,3-dimethylbenzamid;
N-(2',5'-Bis-trifluormethyl-biphenyl-4-yl)-2-methyl-3-chlorbenzamid;
N-(2',5'-Bis-trifluormethyl-biphenyl-4-yl)-2-methyl-3-fluorbenzamid;
N-(2',5'-Bis-trifluormethyl-biphenyl-4-yl)-2-methyl-3-methoxybenzamid;
4-Methyl-[1,2,3]-thiadiazol-5-carbonsäure-(2',5'-dimethoxy-biphenyl-4-yl)-amid;
N-(2',5'-Dimethoxy-biphenyl-4-yl)-2-methylbenzamid;
2-Methyl-pyridin-3-carbonsäure-(2',5'-bis-trifluormethyl-biphenyl-4-yl)-amid;
2,3-Dihydro-benzo[1,4]dioxin-5-carbonsäure-(2',5'-bis-trifluormethyl-biphenyl-4-yl)-amid;
1-Methyl-1H-imidazol-5-carbonsäure-(2',5'-bis-trifluormethyl-biphenyl-4-yl)-amid;
3-Methyl-pyridin-4-carbonsäure-(2',5'-dimethoxy-biphenyl-4-yl)-amid;
3-Methyl-pyridin-4-carbonsäure-(2',5'-bis-trifluormethyl-biphenyl-4-yl)-amid;
3-Methyl-pyridin-4-carbonsäure-(2'-methoxy-5'-chlorbiphenyl-4-yl)-amid;
3-Fluor-pyridin-4-carbonsäure-(2',5'-dimethoxybiphenyl-4-yl)-amid;
3-Fluor-pyridin-4-carbonsäure-(2'-methoxy-5'-chlorbiphenyl-4-yl)-amid;
3-Fluor-pyridin-4-carbonsäure-(2',5'-bis-trifluormethylbiphenyl-4-yl)-amid;
3-Methyl-pyridin-4-carbonsäure-(2'-methoxy-5'-methylbiphenyl-4-yl)-amid;
3-Methyl-pyridin-4-carbonsäure-(2',5'-dimethylbiphenyl-4-yl)-amid;
4-Methyl-[1,2,3]-thiadiazol-5-carbonsäure-(2'-methoxy-5'-acetylbiphenyl-4-yl)-amid;
3-Fluor-pyridin-4-carbonsäure-(2'-difluormethoxy-5'-chlorbiphenyl-4-yl)-amid;
4-Methyl-[1,2,3]-thiadiazol-S-carbonsäure-{2'-(N,N-dimethylamino)-5'-trifluormethoxybiphenyl-4-yl}-amid;
3-Methyl-pyridin-4-carbonsäure-(2'-chlor-5'-trifluormethylbiphenyl-4-yl)-amid;
3-Methyl-pyridin-4-carbonsäure-(2'-methylsulfanyl-biphenyl-4-yl)-amid;
3-Methyl-pyridin-4-carbonsäure-(2'-ethyl-biphenyl-4-yl)-amid;
3-Methyl-pyridin-4-carbonsäure-(2'-isopropyl-biphenyl-4-yl)-amid;
N-{5-(2',5'-Dimethoxyphenyl)-pyrid-2-yl}-2-methylbenzamid;
3-Methyl-pyridin-4-carbonsäure-(2',5'-diethylbiphenyl-4-yl)-amid;
3-Methyl-pyridin-4-carbonsäure-{2'-(N,N-dimethylamino)-5'-methoxybiphenyl-4-yl}-amid;
3-Methyl-pyridin-4-carbonsäure-{2'-(N-dimethylamino)-5'-carbethoxybiphenyl-4-yl}-amid;
3-Methyl-pyridin-4-carbonsäure-(2'-ethoxy-5'-chlorbiphenyl-4-yl)-amid;
N-(2'-Dimethoxy-5'-chlor-biphenyl-4-yl)-2,6-difluorbenzamid;
N-(2'-Methoxy-5'-chlor-biphenyl-4-yl)-2,4,5-trifluorbenzamid;
N-(2',5'-Bis-trifluormethyl-biphenyl-4-yl)-2,6-difluorbenzamid;
N-(2'-Chlor-5'-trifluormethyl-biphenyl-4-yl)-2,6-difluorbenzamid;
N-(2',5'-Dimethyl-biphenyl-4-yl)-2,6-difluorbenzamid;
N-(2',5'-Dichlor-biphenyl-4-yl)-2,6-difluorbenzamid;
2,3-Difluor-N-[4-(2-trifluormethyl-indolizin-3-yl)-phenyl]-benzamid;
2,5-Difluor-N-[4-(2-trifluormethyl-indolizin-3-yl)-phenyl]-benzamid;
3,4-Dimethoxy-N-[4-(2-trifluormethyl-indolizin-3-yl)-phenyl]-benzamid;
N-[4-(5-Chlor-2-trifluormethyl-indolizin-3-yl)-phenyl]-2,3-difluor-benzamid;
5-Chlor-3-[4-(2,3-difluor-benzoylamino)-phenyl]-2-trifluormethyl-indolizin-6-carbonsäuremethylester;
3-[4-(2,3-Difluor-benzoylamino)-phenyl]-2-trifluormethyl-indolizin-6-carbonsäuremethylester;
2,3-Difluor-N-[4-(6-methoxy-2-trifluormethyl-indolizin-3-yl)-phenyl]-benzamid;
N-[4-(5-Fluor-2-trifluormethyl-indolizin-3-yl)-phenyl]-2,3-difluor-benzamid;
N-[4-(5-Methoxy-2-trifluormethyl-indolizin-3-yl)-phenyl]-2,3-difluor-benzamid;
N-[4-(5-Chlor-2,7-bis-trifluormethyl-indolizin-3-yl)-phenyl]-2,3-difluor-benzamid;
5-Methoxy-3-[4-(2,3-difluor-benzoylamino)-phenyl]-2-trifluormethyl-indolizin-6-carbonsäuremethylester;
2,3-Difluor-N-[4-(8-methoxy-2-trifluormethyl-indolizin-3-yl)-phenyl]-benzamid;
5-Chlor-3-[4-(2,3-difluor-benzoylamino)-phenyl]-2-trifluormethyl-indolizin-7-carbonsäuremethylester;
5-Chlor-3-[4-(2,6-difluor-benzoylamino)-phenyl]-2-trifluormethyl-indolizin-7-carbonsäuremethylester;
2,6-Difluor-N-[4-(8-methoxy-2-trifluormethyl-indolizin-3-yl)-phenyl]-benzamid;
5-Methoxy-3-[4-(2,6-difluor-benzoylamino)-phenyl]-2-trifluormethyl-indolizin-6-carbonsäuremethylester;
N-[4-(5-Chlor-2,7-bis-trifluormethyl-indolizin-3-yl)-phenyl]-2,6-difluor-benzamid;
N-[4-(5-Methoxy-2-trifluormethyl-indolizin-3-yl)-phenyl]-2,6-difluor-benzamid;
N-[4-(5-Fluor-2-trifluormethyl-indolizin-3-yl)-phenyl]-2,6-difluor-benzamid;
2,6-Difluor-N-[4-(6-methoxy-2-trifluormethyl-indolizin-3-yl)-phenyl]-benzamid;
3-[4-(2,6-Difluor-benzoylamino)-phenyl]-2-trifluormethyl-indolizin-6-carbonsäuremethylester;
5-Chlor-3-[4-(2,6-difluor-benzoylamino)-phenyl]-2-trifluormethyl-indolizin-6-carbonsäuremethylester;
N-[4-(5-Chlor-2-trifluormethyl-indolizin-3-yl)-phenyl]-2,6-difluor-benzamid;
N-[4-(5-Chlor-2-trifluormethyl-indolizin-3-yl)-phenyl]-2,4,5-trifluor-benzamid;
5-Chlor-3-[4-(2,4,5-trifluor-benzoylamino)-phenyl]-2-trifluormethyl-indolizin-6-carbonsäuremethylester;
3-[4-(2,4,5-Trifluor-benzoylamino)-phenyl]-2-trifluormethyl-indolizin-6-carbonsäuremethylester;
2,4,5-Trifluor-N-[4-(5-fluor-2-trifluormethyl-indolizin-3-yl)-phenyl]-benzamid;
2,4,5-Trifluor-N-[4-(6-methoxy-2-trifluormethyl-indolizin-3-yl)-phenyl]-benzamid;
2,4,5-Trifluor-N-[4-(5-methoxy-2-trifluormethyl-indolizin-3-yl)-phenyl]-benzamid;
N-[4-(5-Chlor-2,7-bis-trifluormethyl-indolizin-3-yl)-phenyl]-2,4,5-trifluor-benzamid;
5-Methoxy-3-[4-(2,4,5-trifluor-benzoylamino)-phenyl]-2-trifluormethyl-indolizin-6-carbonsäuremethylester;
2,4,5-Trifluor-N-[4-(8-methoxy-2-trifluormethyl-indolizin-3-yl)-phenyl]-benzamid;
5-Chlor-3-[4-(2,4,5-trifluor-benzoylamino)-phenyl]-2-trifluormethyl-indolizin-7-carbonsäuremethylester;
2,4,5-Trifluor-N-[4-(7-methoxy-2-trifluormethyl-indolizin-3-yl)-phenyl]-benzamid;
2,6-Difluor-N-[4-(7-methoxy-2-trifluormethyl-indolizin-3-yl)-phenyl]-benzamid;
2,3-Difluor-N-[4-(7-methoxy-2-trifluormethyl-indolizin-3-yl)-phenyl]-benzamid;
N-(2',5'-Dimethoxy-biphenyl-4-yl)-2,6-difluor-benzamid;
N-(2'-Trifluormethyl-5'-methyl-biphenyl-4-yl)-2,6-difluor-benzamid;
N-(2',5'-Bis-trifluormethyl-biphenyl-4-yl)-2,6-difluor-benzylamin;
N-(2',5'-Bis-trifluormethyl-biphenyl-4-yl)-2,6-difluor-benzylamin-HCl-Salz;
N-(2'-Methoxy-5'-chlor-biphenyl-4-yl)-2,6-difluor-benzylamin;
N-(2'-Methoxy-5'-chlor-biphenyl-4-yl)-2,6-difluor-benzylamin-HCl-Salz;
N',N'-Diethyl-N-(2',5'-bis-trifluormethyl-biphenyl-4-yl)-harnstoff;
2,3-Difluor-N-[4-(2-trifluor-methyl-indolizin-3-yl)-phenyl]-benzamid;
4-Methyl-N-[4-(2-methyl-indolizin-3-yl)-phenyl]-[1,2,3]thiadiazol-5-carbonsäureamid;
und pharmazeutisch akzeptablen Salzen, Solvaten oder Clathraten derselben.

51. Pharmazeutische Zusammensetzung, die einen pharmazeutisch akzeptablen Träger und eine oder mehrere Verbindungen der folgenden Strukturformel: 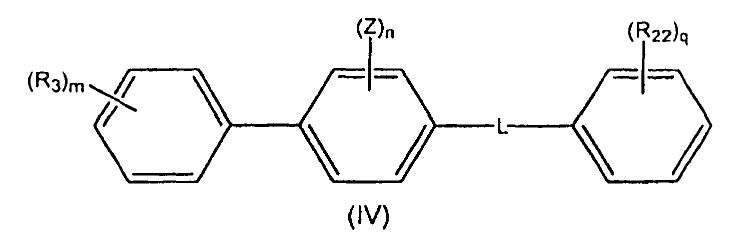 oder ein pharmazeutisch akzeptables Salz, Solvat oder Clathrat derselben umfasst,
wobei:
Z, R₃ und R₂₂ bei jedem Vorkommen unabhängig voneinander ausgewählt sind aus der Gruppe von einem optional substituierten Alkyl, einem optional substituierten Alkenyl, einem optional substituierten Alkinyl, einem optional substituierten Cycloalkyl, einem optional substituierten Cycloalkenyl, einem optional substituierten Heterocyclyl, einem optional substituierten Aryl, einem optional substituierten Heteroaryl, einem optional substituierten Aralkyl, einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für 0 oder eine ganze Zahl von 1 bis 4 steht;
m und q jeweils unabhängig voneinander für 0 oder eine ganze Zahl von 1 bis 5 stehen; und
p für 0, 1 oder 2 steht,
mit der Maßgabe, dass, wenn X für ein p-Halogenphenyl, p-Nitrophenyl, p-Cyanophenyl, p-(Methoxymethyl)phenyl, p-(Benzamido)phenyl, ein substituiertes p-(Benzamido)phenyl oder p-Carboxyphenyl steht, Y nicht für ein substituiertes oder unsubstituiertes Phenyl, ein unsubstituiertes Furyl, ein substituiertes oder
unsubstituiertes Thiophenyl, ein substituiertes Benzo[b]thiophenyl, ein unsubstituiertes Thiazolyl,
ein substituiertes 7,8-Dihydronaphthyl, ein substituiertes Pyrazinyl oder ein substituiertes oder unsubstituiertes Pyridinyl steht;
mit der Maßgabe, dass, wenn X m-Nitrophenyl oder m-(Trifluormethyl)phenyl ist, Y₁ nicht für ein substituiertes oder unsubstituiertes Phenyl steht; und
mit der Maßgabe, dass X nicht für ein Phenyl, das in der ortho-Position mit -S(O)₂NH₂ substituiert ist, steht.

52. Pharmazeutische Zusammensetzung, die einen pharmazeutisch akzeptablen Träger und eine oder mehrere Verbindungen der folgenden Strukturformel: 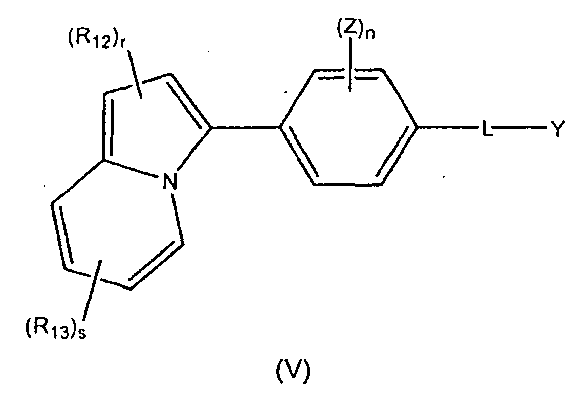 oder ein pharmazeutisch akzeptables Salz, Solvat oder Clathrat derselben umfasst,
wobei:
Y für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
L für einen Linker steht, der aus der Gruppe von -NRCH₂- und -NR-C(O)- ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
Z, R₁₂ und R₁₃ bei jedem Vorkommen unabhängig voneinander für ein optional substituiertes Alkyl, ein optional substituiertes Alkenyl, ein optional substituiertes Alkinyl, ein optional substituiertes Cycloalkyl,
ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteroaralkyl, ein Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂ stehen;
R₄ und R₅ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
r für 0, 1 oder 2 steht;
n und s jeweils unabhängig voneinander für 0 oder eine ganze Zahl von 1 bis 4 stehen; und
p für 0, 1 oder 2 steht.

53. Pharmazeutische Zusammensetzung nach Anspruch 52, wobei L -NHC(O)- ist und Y für ein optional substituiertes Phenyl steht.

54. Pharmazeutische Zusammensetzung nach Anspruch 53, wobei die Verbindung eine Verbindung der folgenden Strukturformel: 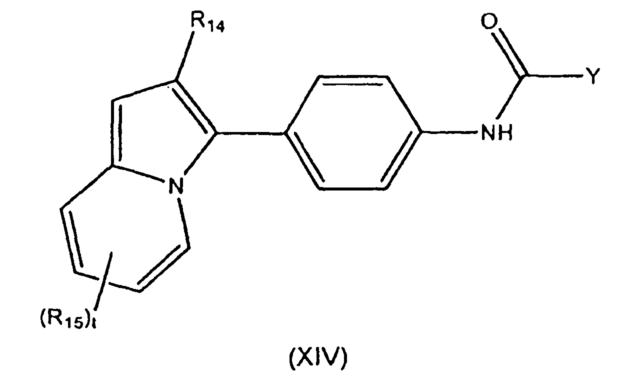 oder ein pharmazeutisch akzeptables Salz, Solvat oder Clathrat derselben ist,
wobei R₁₄ und R₁₅ jeweils unabhängig voneinander für -CF₃ , -OCH₃, -F, -Cl oder -C(O)OCH₃ stehen; und
t für 0, 1 oder 2 steht.

55. Pharmazeutische Zusammensetzung nach Anspruch 54, wobei die Verbindung eine Verbindung der folgenden Strukturformel: 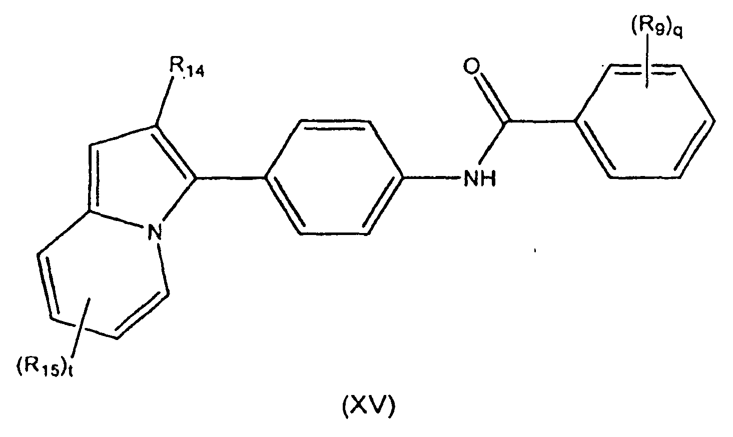 oder ein pharmazeutisch akzeptables Salz, Solvat oder Clathrat derselben ist,
wobei:
R₉ bei jedem Vorkommen unabhängig voneinander für Halogen, C₁-C₄-Alkyl, C₁-C₄-Halogenalkyl, C₁-C₄-Alkoxy, C₁-C₄-Halogenalkoxy oder Hydroxyl steht; und
q für 0 oder eine ganze Zahl von 1 bis 5 steht.

56. Pharmazeutische Zusammensetzung nach Anspruch 55, wobei die Verbindung eine Verbindung der folgenden Strukturformel: 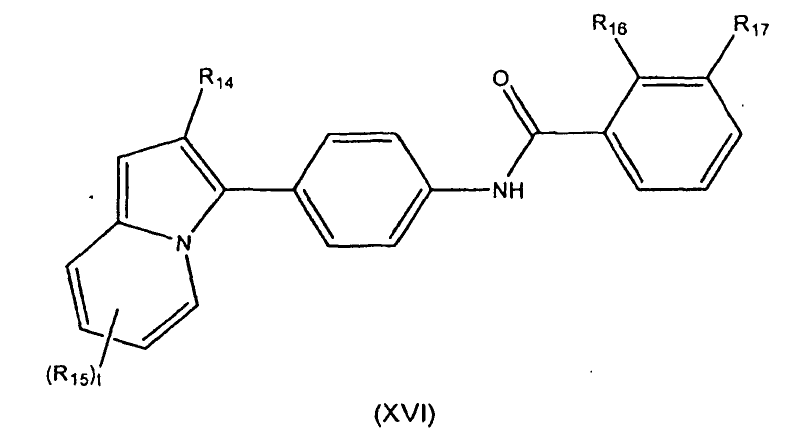 oder ein pharmazeutisch akzeptables Salz, Solvat oder Clathrat derselben ist,
wobei R₁₆ und R₁₇ jeweils unabhängig voneinander -F oder -OCH₃ sind.

57. Pharmazeutische Zusammensetzung nach Anspruch 55, wobei die Verbindung eine Verbindung der folgenden Strukturformel: 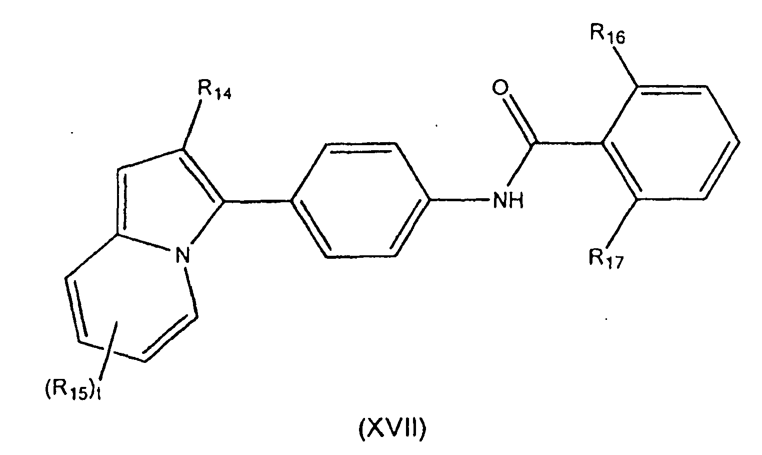 oder ein pharmazeutisch akzeptables Salz, Solvat oder Clathrat derselben ist,
wobei R₁₆ und R₁₇ jeweils unabhängig voneinander -F oder -OCH₃ sind.

58. Pharmazeutische Zusammensetzung, die einen pharmazeutisch akzeptablen Träger und eine oder mehrere Verbindungen der folgenden Strukturformel: 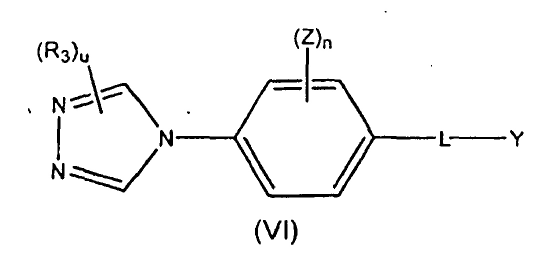 oder ein pharmazeutisch akzeptables Salz, Solvat oder Clathrat derselben umfasst,
wobei:
Y für ein optional substituiertes Aryl oder ein optional substituiertes Heteroaryl steht, die optional mit 1-3 Substituenten substituiert sein können, die unabhängig voneinander aus Halogen, C₁-C₄-Alkyl, halogeniertem C₁-C₄-Alkyl oder C₁-C₄-Alkoxy ausgewählt sind;
Z und R₃ bei jedem Vorkommen unabhängig voneinander ausgewählt sind aus der Gruppe von einem optional substituierten Alkyl, einem optional substituierten Alkenyl, einem optional substituierten Alkinyl, einem optional substituierten Cycloalkyl, einem optional substituierten Cycloalkenyl, einem optional substituierten Heterocyclyl, einem optional substituierten Aryl, einem optional substituierten Heteroaryl, einem optional substituierten Aralkyl, einem optional substituierten Heteroaralkyl, einem Halogenalkyl, -C(O)NR₁R₂, -NR₄C(O)R₅, Halogen, -OR₄, Cyano, Nitro, Halogenalkoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ oder -S(O)ₚNR₁R₂;
L für einen Linker steht, der aus der Gruppe von -NRCH₂- und -NR-C(O)- ausgewählt ist;
jedes R unabhängig voneinander aus -H, einem Alkyl, Acetyl, tert-Butoxycarbonyl, Benzyloxycarbonyl ausgewählt ist;
R₁ und R₂ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen; oder
R₁ und R₂ zusammengenommen mit dem Stickstoff, an den sie gebunden sind, für ein optional substituiertes Heterocyclyl oder optional substituiertes Heteroaryl stehen;
R₄ und R₄ bei jedem Vorkommen unabhängig voneinander für H, ein optional substituiertes Alkyl, ein optional substituiertes Alkenyl, ein optional substituiertes Alkinyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteroaralkyl stehen;
n für eine ganze Zahl steht, die aus 0-4 ausgewählt ist;
p für 0, 1 oder 2 steht; und
u für 0, 1 oder 2 steht.

59. Pharmazeutische Zusammensetzung nach Anspruch 40, 49, 50, 51, 52 oder 58, die ferner ein oder mehrere zusätzliche therapeutische Mittel umfasst.

60. Pharmazeutische Zusammensetzung nach Anspruch 59, wobei das zusätzliche therapeutische Mittel aus der Gruppe von Immunsuppressiva und entzündungshemmenden Mitteln und geeigneten Gemischen derselben ausgewählt ist.

61. Pharmazeutische Zusammensetzung nach Anspruch 60, wobei das zusätzliche therapeutische Mittel aus der Gruppe von Steroiden, nichtsteroidalen entzündungshemmenden Mitteln, Antihistaminika, Analgetika und geeigneten Gemischen derselben ausgewählt ist.

## Revendications

1. Procédé *in vitro* d'inhibition de l'activation d'une cellule immunitaire, comprenant l'administration à la cellule d'un composé de formule (I) : 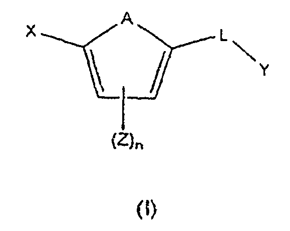 ou d'un sel, solvate ou clathrate pharmaceutiquement acceptable de celui-ci,
dans laquelle :
X est un phényle éventuellement substitué, un triazolyle éventuellement substitué, un pyridyle éventuellement substitué ou un indolizinyle éventuellement substitué ;
Y est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou alcoxy en C₁ à C₄ ;
A est -CH=CH-, -CZ=CH- ou -CH=CZ- ;
chaque Z est indépendamment choisi dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est 0 ou un entier de 1 à 4 ; et
p est 0, 1 ou 2.

2. Procédé *in vitro* d'inhibition de la production de cytokine dans une cellule, comprenant l'administration à la cellule d'un composé représenté par la formule structurale suivante : 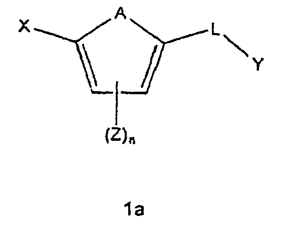 ou d'un sel, solvate ou clathrate pharmaceutiquement acceptable de celui-ci,
dans laquelle :
X est un phényle éventuellement substitué, un triazolyle éventuellement substitué, un pyridyle éventuellement substitué ou un indolizinyle éventuellement substitué ;
Y est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou un alcoxy en C₁ à C₄ ;
A est -CH=CH-, -CZ=CH- ou -CH=CZ-
chaque Z est indépendamment choisi dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est 0 ou un entier de 1 à 4 ; et
p est 0, 1 ou 2.

3. Procédé selon la revendication 2, dans lequel la cytokine qui est inhibée est choisie dans le groupe constitué par IL-2, IL-4, IL-5, IL-13, GM-CSF, TNF-α, IFN-γ et des combinaisons de ceux-ci.

4. Procédé *in vitro* de modulation d'un canal ionique dans une cellule, dans lequel le canal ionique intervient dans l'activation d'une cellule immunitaire, comprenant l'administration à la cellule d'un composé représenté par la formule structurale suivante : 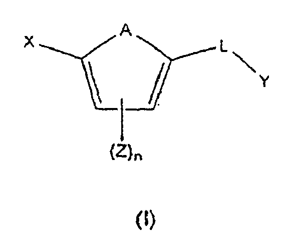 ou d'un sel, solvate ou clathrate pharmaceutiquement acceptable de celui-ci,
dans laquelle :
X est un phényle éventuellement substitué, un triazolyle éventuellement substitué, un pyridyle éventuellement substitué ou un indolizinyle éventuellement substitué ;
Y est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou alcoxy en C₁ à C₄ ;
A est -CH=CH-, -CZ=CH- ou -CH=CZ-
chaque Z est indépendamment choisi dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est un entier choisi parmi 0 à 4 ; et
p est 0, 1 ou 2.

5. Procédé selon la revendication 4, dans lequel le canal ionique est TRPM4.

6. Procédé *in vitro* d'inhibition de la prolifération de lymphocytes T et/ou de lymphocytes B en réponse à un antigène, comprenant l'administration à la cellule d'un composé représenté par la formule structurale suivante : 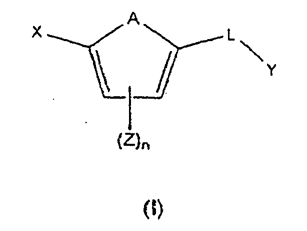 ou d'un sel, solvate ou clathrate pharmaceutiquement acceptable de celui-ci,
dans laquelle :
X est un phényle éventuellement substitué, un triazolyle éventuellement substitué, un pyridyle éventuellement substitué ou un indolizinyle éventuellement substitué ;
Y est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou alcoxy en C₁ à C₄ ;
A est -CH=CH-, -CZ=CH- ou -CH=CZ-
chaque Z est indépendamment choisi dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est 0 ou un entier de 1 à 4 ; et
p est 0, 1 ou 2.

7. Procédé selon la revendication 1, 2, 4 ou 6, dans lequel le composé est représenté par la formule structurale suivante : 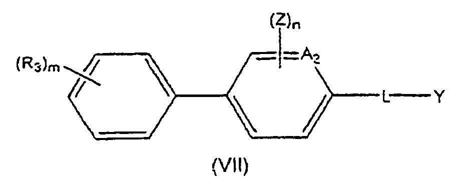 ou un sel, solvate ou clathrate pharmaceutiquement acceptable de celui-ci,
dans laquelle :
A₂ est CH ou CZ ;
R₃ est un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogène, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ; et
m est 0 ou un entier de 1 à 5.

8. Procédé selon la revendication 7, dans lequel A₂ est CH.

9. Procédé selon la revendication 7, dans lequel L est -NHC(O)- ou -NHCH₂-.

10. Procédé selon la revendication 9, dans lequel Y est un phényle éventuellement substitué, un pyridyle éventuellement substitué, un thiophényle éventuellement substitué, un [1,2,3]thiadiazolyle, un isoxazolyle éventuellement substitué, un 1H-pyrazolyle, un quinolinyle, un imidazolyle ou une 2,3-dihydrobenzo[1,4]-dioxine.

11. Procédé selon la revendication 10, dans lequel Y est un phényle éventuellement substitué, un pyridyle éventuellement substitué ou un [1,2,3]thiadiazolyle éventuellement substitué.

12. Procédé selon la revendication 11, dans lequel le composé est représenté par la formule structurale suivante : 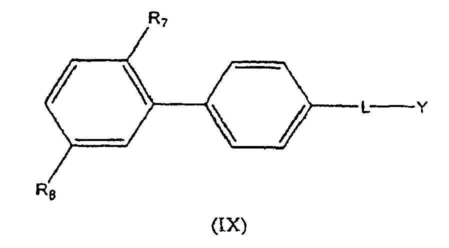 ou un sel, solvate ou clathrate pharmaceutiquement acceptable de celui-ci,
dans laquelle :
R₇ et R₈ sont chacun, indépendamment, -H, -CF₃, -CN, -C(O)CH₃, -F, -C1, -OCH₃, -OCH₂CH₃, -C(O)OCH₂CH₃, -SCH₃, -NHCH₃ ou un alkyle en C₁ à C₄, étant entendu qu'au moins l'un parmi R₇ ou R₈ n'est pas -H.

13. Procédé selon la revendication 12, dans lequel le composé est représenté par la formule structurale suivante : 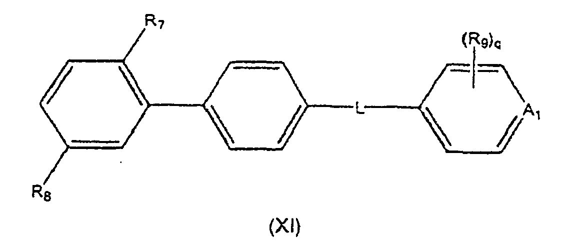 ou un sel, solvate ou clathrate pharmaceutiquement acceptable de celui-ci,
dans laquelle :
A₁ est CH, CR₉, N ou N→O ;
R₉, à chaque apparition, est, indépendamment, halogéno, alkyle en C₁ à C₄, halogénoalkyle en C₁ à C₄, alcoxy en C₁ à C₄, halogénoalcoxy en C₁ à C₄ ou hydroxyle ; et
q est 0 ou un entier de 1 à 5.

14. Procédé selon la revendication 13, dans lequel le composé est représenté par la formule structurale suivante : 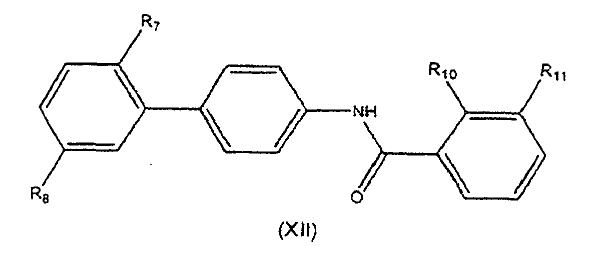 ou un sel, solvate ou clathrate pharmaceutiquement acceptable de celui-ci,
dans laquelle R₁₀ et R₁₁ sont chacun, indépendamment, -F, -C1, un alkyle en C₁ à C₄, un halogénoalkyle en C₁ à C₄, un alcoxy en C₁ à C₄ ou un halogénoalcoxy en C₁ à C₄.

15. Procédé selon la revendication 13, dans lequel le composé est représenté par la formule structurale suivante : 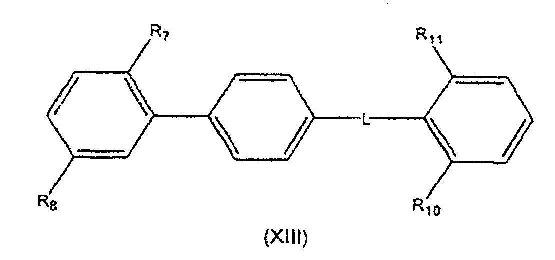 ou un sel, solvate ou clathrate pharmaceutiquement acceptable de celui-ci,
dans laquelle R₁₀ et R₁₁ sont chacun, indépendamment, -F, -Cl, un alkyle en C₁ à C₄, un halogénoalkyle en C₁ à C₄, un alcoxy en C₁ à C₄ et un halogénoalcoxy en C₁ à C₄.

16. Procédé selon la revendication 1, 2, 4 ou 6, dans lequel le composé est représenté par la formule structurale suivante : 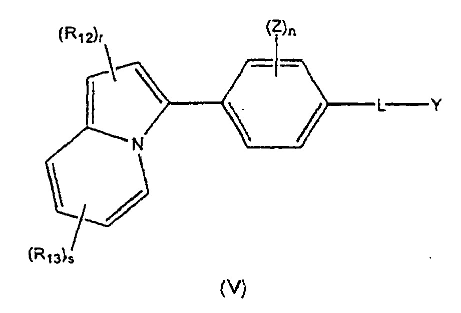 ou un sel, solvate ou clathrate pharmaceutiquement acceptable de celui-ci,
dans laquelle :
R₁₂ et R₁₃, à chaque apparition, sont, indépendamment, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogène, -OR₄, un cyano, un nitro, un halogénoalcoxy, -C(O)R₄, -NR₁R₂, -SR₄, -C(O)OR₄, -OC(O)R₄. -NR₄C(O)NR₁R₂, -OC(ONR₁R₂, -NR₄C(O)OR₅, -S(O)ₚR₄ ou -S(O)ₚNR₁R₂ ;
r est 0, 1 ou 2 ; et
s est 0 ou un entier de 1 à 4.

17. Procédé selon la revendication 16, dans lequel L est -NHC(O)- et Y est un phényle éventuellement substitué.

18. Procédé selon la revendication 17, dans lequel le composé est représenté par la formule structurale suivante : 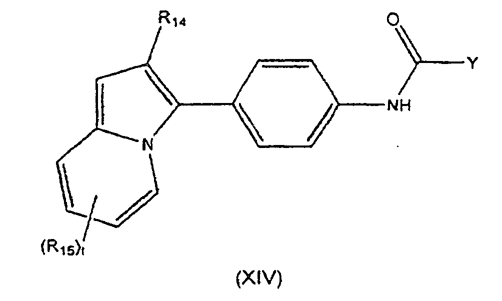 ou un sel, solvate ou clathrate pharmaceutiquement acceptable de celui-ci,
dans laquelle :
R₁₄ et R₁₅ sont chacun, indépendamment, -CF₃, -OCH₃, -F, -Cl ou -C(O)OCH₃ ; et
t est 0, 1 ou 2.

19. Procédé selon la revendication 18, dans lequel le composé est représenté par la formule structurale suivante : 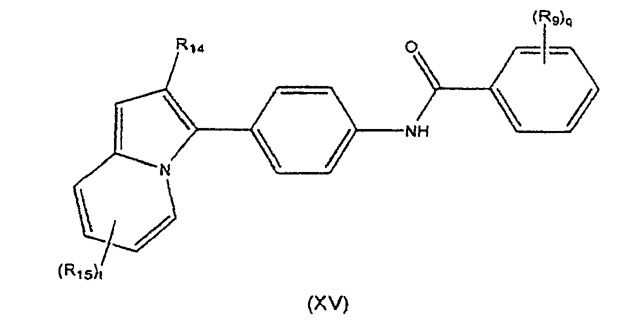 ou un sel, solvate ou clathrate pharmaceutiquement acceptable de celui-ci,
dans laquelle :
R₉, à chaque apparition, est, indépendamment, halogéno, alkyle en C₁ à C₄, halogénoalkyle en C₁ à C₄, alcoxy en C₁ à C₄, halogénoalcoxy en C₁ à C₄ ou hydroxyle ; et
q est 0 ou un entier de 1 à 5.

20. Procédé selon la revendication 19, dans lequel le composé est représenté par la formule structurale suivante : 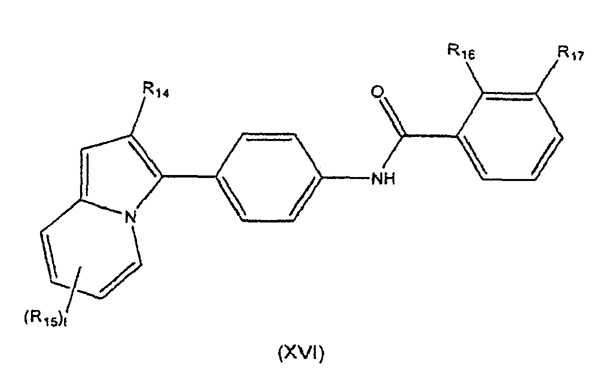 ou un sel, solvate ou clathrate pharmaceutiquement acceptable de celui-ci,
dans laquelle R₁₆ et R₁₇ sont chacun, indépendamment, -F ou -OCH₃.

21. Procédé selon la revendication 19, dans lequel le composé est représenté par la formule structurale suivante : 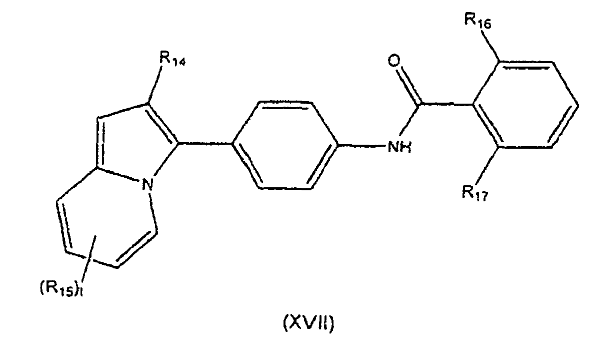 ou un sel, solvate ou clathrate pharmaceutiquement acceptable de celui-ci,
dans laquelle R₁₆ et R₁₇ sont chacun, indépendamment, -F ou -OCH₃.

22. Procédé selon la revendication 1, 2, 4 ou 6, dans lequel le composé est représenté par la formule structurale suivante : 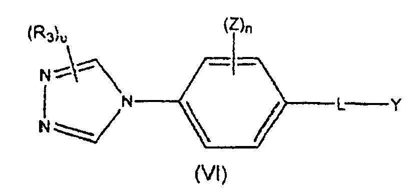 ou un sel, solvate ou clathrate pharmaceutiquement acceptable de celui-ci,
dans laquelle :
R₃ est un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ; et
u est 0, 1 ou 2.

23. Procédé selon la revendication 22, dans lequel le composé est représenté par la formule structurale suivante : 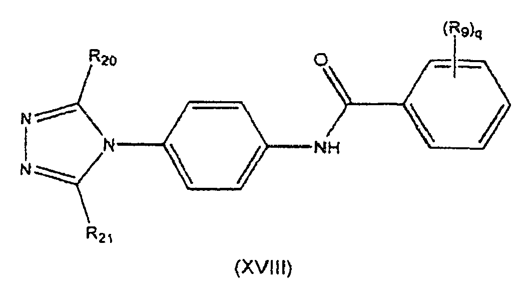 ou un sel, solvate ou clathrate pharmaceutiquement acceptable de celui-ci,
dans laquelle :
R₂₀ et R₂₁ sont chacun, indépendamment, -H, -F, -Cl, un alkyle en C₁ à C₄, un thiophényle, -OCH₃, -CF₃ ou -OCF₃ ;
R₉, à chaque apparition, est, indépendamment, halogéno, alkyle en C₁ à C₄, halogénoalkyle en C₁ à C₄, alcoxy en C₁ à C₄, halogénoalcoxy en C₁ à C₄ ou hydroxyle ; et
q est 0 ou un entier de 1 à 5.

24. Procédé selon la revendication 1, 2, 4 ou 6, dans lequel l'on administre à la cellule un ou plusieurs composés choisis dans le groupe constitué par :
le 3-fluoro-N-(2'-trifluorométhyl-biphényl-4-yl)-isonicotinamide ;
le 3-fluoro-N-(2'-méthyl-biphényl-4-yl)-isonicotinamide ;
le 3-fluoro-N-(3'-trifluorométhyl-biphényl-4-yl)-isonicotinamide ;
le N-(2,2'-bis-trifluorométhyl-biphényl-4-yl)-2,3-difluoro-benzamide ;
le N-[4-(1,2-diméthyl-but-1-ényl)-3-trifluorométhyl-phényl]-2,3-difluoro-benzamide ;
le diméthylamide de l'acide 4'-(2,3-difluoro-benzoylamino)-biphényl-2-carboxylique ;
le N-(2'-trifluorométhyl-biphényl-4-yl)-nicotinamide ;
le N-(2'-trifluorométhyl-biphényl-4-yl)-isonicotinamide ;
le (2'-trifluorométhyl-biphényl-4-yl)-amide de l'acide thiophène-2-carboxylique ;
le 4-fluoro-N-(2'-trifluorométhyl-biphényl-4-yl)-benzamide ;
le (2'-trifluorométhyl-biphényl-4-yl)-amide de l'acide 2,4-diméthyl-thiazole-5-carboxylique ;
le 4-trifluorométhyl-N-(2'-trifluorométhyl-biphényl-4-yl)-nicotinamide ;
le (2'-trifluorométhyl-biphényl-4-yl)-amide de l'acide 2-méthyl-5-trifluorométhyl-oxazole-4-carboxylique ;
le (2'-trifluorométhyl-biphényl-4-yl)-amide de l'acide 2-éthyl-5-méthyl-2H-pyrazole-3-carboxylique ;
le 2,3-difluoro-N-(2'-trifluorométhyl-biphényl-4-yl)-benzamide ;
le 2,5-difluoro-N-(2'-trifluorométhyl-biphényl-4-yl)-benzamide ;
le 2,3-difluoro-N-(3-fluoro-2'-trifluorométhyl-biphényl-4-yl)-benzamide ;
le N-(2',5'-bis-trifluorométhyl-biphényl-4-yl)-2,3-difluoro-benzamide ;
le 2,3-difluoro-N-(2'-fluoro-5'-trifluorométhyl-biphényl-4-yl)-benzamide ;
le 2,3-difluoro-N-(4'-fluoro-2'-trifluorométhyl-biphényl-4-yl)-benzamide ;
le 2,3-difluoro-N-[4-(2-trifluorométhyl-indolizin-3-yl)-phényl]-benzamide ;
le 2,3-difluoro-N-(2'-fluoro-6'-trifluorométhyl-biphényl-4-yl)-benzamide ;
le 2,3-difluoro-N-(2'-chloro-5'-trifluorométhyl-biphényl-4-yl)-benzamide ;
le (2',5'-bis-trifluorométhyl-biphényl-4-yl)-amide de l'acide 4-méthyl-[1,2,3]thiadiazole-5-carboxylique ;
le (2'-trifluorométhyl-biphényl-4-yl)-amide de l'acide pyridine-2-carboxylique ;
le (2'-trifluorométhyl-biphényl-4-yl)-amide de l'acide pyrazine-2-carboxylique ;
le (2'-chloro-5'-trifluoroméhyl-biphényl-4-yl)-amide de l'acide 4-méthyl-[1,2,3]thiadiazole-5-carboxylique ;
le N-(2',5'-bis-trifluorométhyl-biphényl-4-yl)-2,5-difluoro-benzamide ;
le N-(2',5'-dichloro-biphényl-4-yl)-2,3-difluorobenzamide ;
le N-(5'-cyano-2'-méthoxy-biphényl-4-yl)-2,3-difluoro-benzamide ;
le N-(2',5'-diméthoxy-biphényl-4-yl)-2,3-difluorobenzamide ;
le N-[4-(3,5-bis-trifluorométhyl-[1,2,4]triazol-4-yl)-phényl]-2,3-difluoro-benzamide ;
le (4-(3,5-bis-trifluorométhyl-[1,2,4]triazol-4-yl)-phényl)-amide de l'acide 3-méthyl-thiophène-2-carboxylique ;
le N-[4-(3-trifluorométhyl-5-(thiophén-4-yl)-[1,2,4]-triazol-4-yl)-phényl]-2,3-difluoro-benzamide ;
le N-[4-(3-trifluorométhyl-5-(thiophén-4-yl)-[1,2,4]-triazol-4-yl)-phényl]-2,3-difluoro-benzamide ;
le N-[4-(3-trifluorométhyl-indolizin-3-yl)-phényl]-2,3-difluoro-benzamide ;
le N-[4-(3-cyano-5-trifluorométhyl-pyrid-2-yl)-phényl]-2,3-difluoro-benzamide ;
le N-(2',5'-bis-trifluorométhyl-biphényl-4-yl)-2-méthoxy-benzamide ;
le (2',5'-bis-trifluorométhyl-biphényl-4-yl)-amide de l'acide 5-méthyl-isoxazol-3-carboxylique ;
le (2',5'-bis-trifluorométhyl-biphényl-4-yl)-amide de l'acide 1,3-diméthyl-1H-pyrazol-5-carboxylique ;
le (2',5'-bis-trifluorométhyl-biphényl-4-yl)-amide de l'acide [1,2,3]-thiadiazole-4-carboxylique ;
le (2',5'-bis-trifluorométhyl-biphényl-4-yl)-amide de l'acide isoxazole-5-carboxylique ;
le (2',5'-bis-trifluorométhyl-biphényl-4-yl)-amide de l'acide 3,5-diméthylisoxazole-4-carboxylique ;
le N-(2'-méthoxy-5'-chloro-biphényl-4-yl)-2,3-difluoro-benzamide ;
le N-(2',5'-bis-trifluorométhyl-biphényl-4-yl)-2-méthoxybenzamide ;
le N-(2'-méthoxy-5'-méthyl-biphényl-4-yl)-2,3-difluorobenzamide ;
le N-(2',5'-diméthyl-biphényl-4-yl)-2,3-difluorobenzamide ;
le (2',5'-bis-trifluorométhyl-biphényl-4-yl)-amide de l'acide 3-méthylisoxazole-4-carboxylique ;
le N-(2',5'-bis-trifluorométhyl-biphényl-4-yl)-2-hydroxybenzamide ;
le N-(2'-méthoxy-5'-acétyl-biphényl-4-yl)-2,3-difluorobenzamide ;
le (2',5'-bis-trifluorométhyl-biphényl-4-yl)-amide de l'acide 5-méthylisoxazole-4-carboxylique ;
le N-(2',4',5'-triméthyl-biphényl-4-yl)-2,3-difluorobenzamide ;
le N-(2',5'-bis-trifluorométhyl-biphényl-4-yl)-2,3-diméthylbenzamide ;
le N-(2',5'-bis-trifluorométhyl-biphényl-4-yl)-2-méthyl-3-chlorobenzamide ;
le N-(2',5'-bis-trifluorométhyl-biphényl-4-yl)-2-méthyl-3-fluorobenzamide ;
le N-(2',5'-bis-trifluorométhyl-biphényl-4-yl)-2-méthyl-3-méthoxybenzamide ;
le (2',5'-diméthoxy-biphényl-4-yl)-amide de l'acide 4-méthyl-[1,2,3]thiadiazole-5-carboxylique ;
le N-(2',5'-diméthoxy-biphényl-4-yl)-2-méthylbenzamide ;
le (2',5'-bis-trifluorométhyl-biphényl-4-yl)-amide de l'acide 2-méthyl-pyridine-3-carboxylique ;
le (2',5'-bis-trifluorométhyl-biphényl-4-yl)-amide de l'acide 2,3-dihydro-benzo[1,4]dioxine-5-carboxylique ;
le (2',5'-bis-trifluorométhyl-biphényl-4-yl)-amide de l'acide 1-méthyl-1H-imidazole-5-carboxylique ;
le (2',5'-diméthoxy-biphényl-4-yl)-amide de l'acide 3-méthyl-pyridine-4-carboxylique ;
le (2',5'-bis-trifluorométhyl-biphényl-4-yl)-amide de l'acide 3-méthyl-pyridine-4-carboxylique ;
le (2'-méthoxy-5'-chlorobiphényl-4-yl)-amide de l'acide 3-méthyl-pyridine-4-carboxylique ;
le (2',5'-diméthoxybiphényl-4-yl)-amide de l'acide 3-fluoro-pyridine-4-carboxylique ;
le (2'-méthoxy-5'-chlorobiphényl-4-yl)-amide de l'acide 3-fluoro-pyridine-4-carboxylique ;
le (2',5'-bis-trifluorométhylbiphényl-4-yl)-amide de l'acide 3-fluoro-pyridine-4-carboxylique ;
le (2'-méthoxy-5'-méthylbiphényl-4-yl)-amide de l'acide 3-méthyl-pyridine-4-carboxylique ;
le (2',5'-diméthylbiphényl-4-yl)-amide de l'acide 3-méthyl-pyridine-4-carboxylique ;
le (2'-méthoxy-5'-acétylbiphényl-4-yl)-amide de l'acide 4-méthyl-[1,2,3]-thiadiazole-5-carboxylique ;
le (2'-difluorométhoxy-5'-chlorobiphényl-4-yl)-amide de l'acide 3-fluoro-pyridine-4-carboxylique ;
le {2'-(N,N-diméthylamino)-5'-trifluorométhoxybiphényl-4-yl}-amide de l'acide 4-méthyl-[1,2,3]-thiadiazole-5-carboxylique ;
le (2'-chloro-5'-trifluorométhylbiphényl-4-yl)-amide de l'acide 3-méthyl-pyridine-4-carboxylique ;
le (2'-méthylsulfanyl-biphényl-4-yl)-amide de l'acide 3-méthyl-pyridine-4-carboxylique ;
le (2'-éthyl-biphényl-4-yl)-amide de l'acide 3-méthyl-pyridine-4-carboxylique ;
le (2'-isopropyl-biphényl-4-yl)-amide de l'acide 3-méthyl-pyridine-4-carboxylique ;
le N-{5-(2',5'-diméthoxyphényl)-pyrid-2-yl}-2-méthyl-benzamide ;
le (2',5'-diéthylbiphényl-4-yl)-amide de l'acide 3-méthyl-pyridine-4-carboxylique ;
le {2'-(N,N-diméthylamino)-5'-méthoxybiphényl-4-yl}-amide de l'acide 3-méthyl-pyridine-4-carboxylique ;
le {2'-(N-diméthylamino)-5'-carbéthoxybiphényl-4-yl}-amide de l'acide 3-méthyl-pyridine-4-carboxylique ;
le (2'-éthoxy-5'-chlorobiphényl-4-yl)-amide de l'acide 3-méthyl-pyridine-4-carboxylique ;
le N-(2'-diméthoxy-5'-chloro-biphényl-4-yl)-2,6-difluorobenzamide ;
le N-(2'-méthoxy-5'-chloro-biphényl-4-yl)-2,4,5-trifluorobenzamide ;
le N-(2',5'-bis-trifluorométhyl-biphényl-4-yl)-2,6-difluorobenzamide ;
le N-(2'-chloro-5'-trifluorométhyl-biphényl-4-yl)-2,6-difluorobenzamide ;
le N-(2',5'-diméthyl-biphényl-4-yl)-2,6-difluorobenzamide ;
le N-(2',5'-dichloro-biphényl-4-yl)-2,6-difluorobenzamide ;
le 2,3-difluoro-N-[4-(2-trifluorométhyl-indolizin-3-yl)-phényl]-benzamide ;
le 2,5-difluoro-N-[4-(2-trifluorométhyl-indolizin-3-yl)-phényl]-benzamide ;
le 3,4-diméthoxy-N-[4-(2-trifluorométhyl-indolizin-3-yl)-phényl]-benzamide ;
le N-[4-(5-chloro-2-trifluorométhyl-indolizin-3-yl)-phényl]-2,3-difluorobenzamide ;
l'ester méthylique de l'acide 5-chloro-3-[4-(2,3-difluoro-benzoylamino)-phényl]-2-trifluorométhyl-indolizine-6-carboxylique ;
l'ester méthylique de l'acide 3-[4-(2,3-difluorobenzoylamino)-phényl]-2-trifluorométhyl-indolizine-6-carboxylique ;
le 2,3-difluoro-N-[4-(6-méthoxy-2-trifluorométhyl-indolizin-3-yl)-phényl]-benzamide ;
le N-[4-(5-fluoro-2-trifluorométhyl-indolizin-3-yl)-phényl]-2,3-difluorobenzamide ;
le N-[4-(5-méthoxy-2-trifluorométhyl-indolizin-3-yl)-phényl]-2,3-difluorobenzamide ;
le N-[4-(5-chloro-2,7-bis-trifluorométhyl-indolizin-3-yl)-phényl]-2,3-difluorobenzamide ;
l'ester méthylique de l'acide 5-méthoxy-3-[4-(2,3-difluoro-benzoylamino)-phényl]-2-trifluorométhyl-indolizine-6-carboxylique ;
le 2,3-difluoro-N-[4-(8-méthoxy-2-trifluorométhyl-indolizin-3-yl)-phényl]-benzamide ;
l'ester méthylique de l'acide 5-chloro-3-[4-(2,3-difluoro-benzoylamino)-phényl]-2-trifluorométhyl-indolizine-7-carboxylique ;
l'ester méthylique de l'acide 5-chloro-3-[4-(2,6-difluoro-benzoylamino)-phényl]-2-trifluorométhyl-indolizine-7-carboxylique ;
le 2,6-difluoro-N-[4-(8-méthoxy-2-trifluorométhyl-indolizin-3-yl)-phényl]-benzamide ;
l'ester méthylique de l'acide 5-méthoxy-3-[4-(2,6-difluoro-benzoylamino)-phényl]-2-trifluorométhyl-indolizine-6-carboxylique ;
le N-[4-(5-chloro-2,7-bis-trifluorométhyl-indolizin-3-yl)-phényl]-2,6-difluorobenzamide ;
le N-[4-(5-méthoxy-2-trifluorométhyl-indolizin-3-yl)-phényl]-2,6-difluorobenzamide ;
le N-[4-(5-fluoro-2-trifluorométhyl-indolizin-3-yl)-phényl]-2,6-difluorobenzamide ;
le 2,6-difluoro-N-[4-(6-méthoxy-2-trifluorométhyl-indolizin-3-yl)-phényl]-benzamide ;
l'ester méthylique de l'acide 3-[4-(2,6-difluoro-benzoylamino)-phényl]-2-trifluorométhyl-indolizine-6-carboxylique ;
l'ester méthylique de l'acide 5-chloro-3-[4-(2,6-difluoro-benzoylamino)-phényl]-2-trifluorométhyl-indolizine-6-carboxylique ;
le N-[4-(5-chloro-2-trifluorométhyl-indolizin-3-yl)-phényl]-2,6-difluorobenzamide ;
le N-[4-(5-chloro-2-trifluorométhyl-indolizin-3-yl)-phényl]-2,4,5-trifluorobenzamide ;
l'ester méthylique de l'acide 5-chloro-3-[4-(2,4,5-trifluoro-benzoylamino)-phényl]-2-trifluorométhyl-indolizine-6-carboxylique ;
l'ester méthylique de l'acide 3-[4-(2,4,5-trifluoro-benzoylamino)-phényl]-2-trifluorométhyl-indolizine-6-carboxylique ;
le 2,4,5-trifluoro-N-[4-(5-fluoro-2-trifluorométhyl-indolizin-3-yl)-phényl]benzamide ;
le 2,4,5-trifluoro-N-[4-(6-méthoxy-2-trifluorométhyl-indolizin-3-yl)-phényl]benzamide ;
le 2,4,5-trifluoro-N-[4-(5-méthoxy-2-trifluorométhyl-indolizin-3-yl)-phényl]benzamide ;
le N-[4-(5-chloro-2,7-bis-trifluorométhyl-indolizin-3-yl)-phényl]-2,4,5-trifluorobenzamide ;
l'ester méthylique de l'acide 5-méthoxy-3-[4-(2,4,5-trifluoro-benzoylamino)-phényl]-2-trifluorométhyl-indolizine-6-carboxylique ;
le 2,4,5-trifluoro-N-[4-(8-méthoxy-2-trifluorométhyl-indolizin-3-yl)-phényl]-benzamide ;
l'ester méthylique de l'acide 5-chloro-3-[4-(2,4,5-trifluoro-benzoylamino)-phényl]-2-trifluorométhyl-indolizine-7-carboxylique ;
le 2,4,5-trifluoro-N-[4-(7-méthoxy-2-trifluorométhyl-indolizin-3-yl)-phényl]-benzamide ;
le 2,6-difluoro-N-[4-(7-méthoxy-2-trifluorométhyl-indolizin-3-yl)-phényl]-benzamide ;
le 2,3-difluoro-N-[4-(7-méthoxy-2-trifluorométhyl-indolizin-3-yl)-phényl]-benzamide ;
le N-(2',5'-diméthoxy-biphényl-4-yl)-2,6-difluoro-benzamide ;
le N-(2'-trifluorométhyl-5'-méthyl-biphényl-4-yl)-2,6-difluoro-benzamide ;
la N-(2',5'-bis-trifluorométhyl-biphényl-4-yl)-2,6-difluoro-benzyl amine ;
le sel N-(2',5'-bis-trifluorométhyl-biphényl-4-yl)-2,6-difluoro-benzyl amine HCl ;
la N-(2'-méthoxy-5'-chloro-biphényl-4-yl)-2,6-difluoro-benzyl amine ;
le sel N-(2'-méthoxy-5'-chloro-biphényl-4-yl)-2,6-difluoro-benzyl amine HCl ;
la N',N'-diéthyl-N-(2',5'-bis-trifluorométhyl-biphényl-4-yl)urée ;
le 2,3-difluoro-N-[4-(2-trifluoro-méthyl-indolizin-3-yl)-phényl]-benzamide ;
un amide de l'acide 4-méthyl-N-[4-(2-méthyl-indolizin-3-yl)-phényl]-[1,2,3]thiadiazole-5-carboxylique ; et
les sels, solvates ou clathrates pharmaceutiquement acceptables de ceux-ci.

25. Composé représenté par la formule structurale (II) suivante : 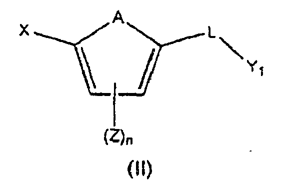 ou un sel, solvate ou clathrate pharmaceutiquement acceptable de celui-ci,
dans laquelle :
X est un phényle éventuellement substitué, un triazolyle éventuellement substitué, un pyridyle éventuellement substitué ou un indolizinyle éventuellement substitué ;
Y₁ est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou alcoxy en C₁ à C₄ ;
A est -CH=CH-, -CZ=CH- ou -CH=CZ- ;
chaque Z est indépendamment choisi dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est entier choisi parmi 0 à 4 ; et
p est 0, 1 ou 2,
étant entendu que Y₁ n'est pas un hétéroaryle qui est plus encore substitué par un aryle substitué ou non substitué ou un hétéroaryle substitué ou non substitué ;
étant entendu que lorsque X est un p-halogénophényle, un p-nitrophényle, un p-cyanophényle, un p-(méthoxyméthyl)phényle, un p-(benzamido)phényle, un p-(benzamido)phényle substitué ou un p-carboxyphényle, Y₁ n'est pas un phényle substitué ou non substitué, un furyle non substitué, un thiophényle substitué ou non substitué, un benzo[b]thiophényle substitué, un thiazolyle non substitué, un 7,8-dihydronaphtyle substitué, un pyrazinyle substitué ou un pyridinyle substitué ou non substitué,
étant entendu que lorsque X est un m-nitrophényle ou un m-(trifluorométhyl)phényle, Y₁ n'est pas un phényle substitué ou non substitué ;
étant entendu que X n'est pas un phényle qui est substitué en position ortho par -S(O)₂NH₂ ; et
étant entendu que X n'est pas un nitrophényle lorsque Y₁ est un 1H-pyrazolyle substitué.

26. Composé selon la revendication 25, dans lequel le composé est représenté par la formule structurale suivante : 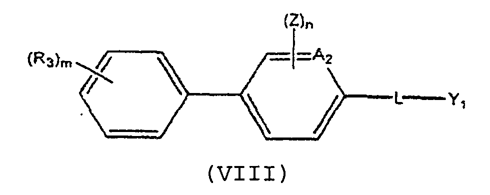 ou un sel, solvate ou clathrate pharmaceutiquement acceptable de celui-ci,
dans laquelle :
A₂ est CH ou CZ ;
R₃ est un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ; et
m est 0 ou un entier de 1 à 5.

27. Composé selon la revendication 26, dans lequel A₂ est CH.

28. Composé selon la revendication 26, dans lequel L est -NHC(O)- ou -NHCH₂-.

29. Composé selon la revendication 28, dans lequel Y₁ est un phényle éventuellement substitué, un pyridyle éventuellement substitué, un thiophényle éventuellement substitué, un [1,2,3]thiadiazolyle, un isoxazolyle éventuellement substitué, un 1H-pyrazolyle, un quinolinyle, un imidazolyle ou une 2,3-dihydrobenzo[1,4]-dioxine.

30. Composé selon la revendication 29, dans lequel Y₁ est un phényle éventuellement substitué, un pyridyle éventuellement substitué ou un [1,2,3]thiadiazolyle éventuellement substitué.

31. Composé selon la revendication 30, dans lequel le composé est représenté par la formule structurale suivante : 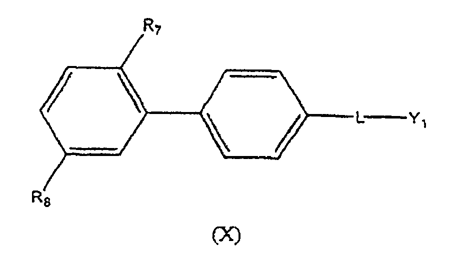 ou un sel, solvate ou clathrate pharmaceutiquement acceptable de celui-ci,
dans laquelle :
R₇ et R₈ sont chacun, indépendamment, -H, -CF₃, -CN, -C(O)CH₃, -F, -Cl, -OCH₃, -OCH₂CH₃, -C(O)OCH₂CH₃, -SCH₃, -NHCH₃ ou un alkyle en C₁ à C₄, étant entendu qu'au moins l'un parmi R₇ ou R₈ n'est pas -H.

32. Composé représenté par la formule structurale suivante : 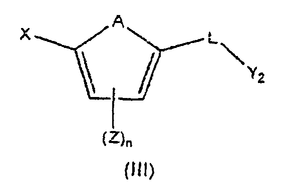 ou un sel, solvate ou clathrate pharmaceutiquement acceptable de celui-ci,
dans laquelle :
X est un phényle éventuellement substitué, un triazolyle éventuellement substitué, un pyridyle éventuellement substitué ou un indolizinyle éventuellement substitué ;
Y₂ est un cycloalkyle éventuellement substitué, un cycloalcényle éventuellement substitué ou un hétérocyclyle éventuellement substitué ;
A est -CH=CH-, -CZ=CH- ou -CH=CZ- ;
chaque Z est indépendamment choisi dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogène, -OR₄, un cyano, un nitro, un halogénoalcoxy, -C(O)R₄, -NR₁R₂, - SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est 0 ou un entier de 1 à 4 ; et
p est 0, 1 ou 2,
étant entendu que X n'est pas un phényle qui est substitué en position ortho par -CN ou -S(O)₂NH₂ ;
étant entendu que Y₂ n'est pas un 4,5-dihydro-isoxazolyle qui est plus encore substitué par un aryle substitué ou non substitué ou un hétéroaryle substitué ou non substitué ; et
étant entendu que Y₂ n'est pas un cyclopentényle substitué.

33. Composé choisi dans le groupe constitué par :
le 3-fluoro-N-(2'-trifluorométhyl-biphényl-4-yl)-isonicotinamide ;
le 3-fluoro-N-(2'-méthyl-biphényl-4-yl)-isonicotinamide ;
le 3-fluoro-N-(3'-trifluorométhyl-biphényl-4-yl)-isonicotinamide ;
le N-(2,2'-bis-trifluorométhyl-biphényl-4-yl)-2,3-difluoro-benzamide ;
le N-[4-(1,2-diméthyl-but-1-ényl)-3-trifluorométhyl-phényl]-2,3-difluoro-benzamide ;
le diméthylamide de l'acide 4'-(2,3-difluoro-benzoylamino)-biphényl-2-carboxylique ;
le N-(2'-trifluorométhyl-biphényl-4-yl)-nicotinamide ;
le N-(2'-trifluorométhyl-biphényl-4-yl)-isonicotinamide ;
le (2'-trifluorométhyl-biphényl-4-yl)-amide de l'acide thiophène-2-carboxylique ;
le 4-fluoro-N-(2'-trifluorométhyl-biphényl-4-yl)-benzamide ;
le (2'-trifluorométhyl-biphényl-4-yl)-amide de l'acide 2,4-diméthyl-thiazole-5-carboxylique ;
le 4-trifluorométhyl-N-(2'-trifluorométhyl-biphényl-4-yl)-nicotinamide ;
le (2'-trifluorométhyl-biphényl-4-yl)-amide de l'acide 2-méthyl-5-trifluorométhyl-oxazole-4-carboxylique ;
le (2'-trifluorométhyl-biphényl-4-yl)-amide de l'acide 2-éthyl-5-méthyl-2H-pyrazole-3-carboxylique ;
le 2,3-difluoro-N-(2'-trifluorométhyl-biphényl-4-yl)-benzamide ;
le 2,5-difluoro-N-(2'-trifluorométhyl-biphényl-4-yl)-benzamide ;
le 2,3-difluoro-N-(3-fluoro-2'-trifluorométhyl-biphényl-4-yl)-benzamide ;
le N-(2',5'-bis-trifluorométhyl-biphényl-4-yl)-2,3-difluoro-benzamide ;
le 2,3-difluoro-N-(2'-fluoro-5'-trifluorométhyl-biphényl-4-yl)-benzamide ;
le 2,3-difluoro-N-(4'-fluoro-2'-trifluorométhyl-biphényl-4-yl)-benzamide ;
le 2,3-difluoro-N-[4-(2-trifluorométhyl-indolizin-3-yl)-phényl]-benzamide ;
le 2,3-difluoro-N-(2'-fluoro-6'-trifluorométhyl-biphényl-4-yl)-benzamide ;
le 2,3-difluoro-N-(2'-chloro-5'-trifluorométhyl-biphényl-4-yl)-benzamide ;
le (2',5'-bis-trifluorométhyl-biphényl-4-yl)-amide de l'acide 4-méthyl-[1,2,3]thiadiazole-5-carboxylique ;
le (2'-trifluorométhyl-biphényl-4-yl)-amide de l'acide pyridine-2-carboxylique ;
le (2'-trifluorométhyl-biphényl-4-yl)-amide de l'acide pyrazine-2-carboxylique ;
le (2'-chloro-5'-trifluoroméhyl-biphényl-4-yl)-amide de l'acide 4-méthyl-[1,2,3]thiadiazole-5-carboxylique ;
le N-(2',5'-bis-trifluorométhyl-biphényl-4-yl)-2,5-difluoro-benzamide ;
le N-(2',5'-dichloro-biphényl-4-yl)-2,3-difluoro-benzamide ;
le N-(5'-cyano-2'-méthoxy-biphényl-4-yl)-2,3-difluoro-benzamide ;
le N-(2',5'-diméthoxy-biphényl-4-yl)-2,3-difluoro-benzamide ;
le N-[4-(3,5-bis-trifluorométhyl-[1,2,4]triazol-4-yl)-phényl]-2,3-difluoro-benzamide ;
le (4-(3,5-bis-trifluorométhyl-[1,2,4]triazol-4-yl)-phényl)-amide de l'acide 3-méthyl-thiophène-2-carboxylique ;
le N-[4-(3-trifluorométhyl-5-(thiophén-4-yl)-[1,2,4]-triazol-4-yl)-phényl]-2,3-difluoro-benzamide ;
le N-[4-(3-trifluorométhyl-5-(thiophén-4-yl)-[1,2,4]-triazol-4-yl)-phényl]-2,3-difluoro-benzamide ;
le N-[4-(3-trifluorométhyl-indolizin-3-yl)-phényl]-2,3-difluoro-benzamide ;
le N-[4-(3-cyano-5-trifluorométhyl-pyrid-2-yl)-phényl]-2,3-difluoro-benzamide ;
le N-(2',5'-bis-trifluorométhyl-biphényl-4-yl)-2-méthoxy-benzamide ;
le (2',5'-bis-trifluorométhyl-biphényl-4-yl)-amide de l'acide 5-méthyl-isoxazol-3-carboxylique ;
le (2',5'-bis-trifluorométhyl-biphényl-4-yl)-amide de l'acide 1,3-diméthyl-1H-pyrazol-5-carboxylique ;
le (2',5'-bis-trifluorométhyl-biphényl-4-yl)-amide de l'acide [1,2,3]-thiadiazole-4-carboxylique ;
le (2',5'-bis-trifluorométhyl-biphényl-4-yl)-amide de l'acide isoxazole-5-carboxylique ;
le (2',5'-bis-trifluorométhyl-biphényl-4-yl)-amide de l'acide 3,5-diméthylisoxazole-4-carboxylique ;
le N-(2'-méthoxy-5'-chloro-biphényl-4-yl)-2,3-difluoro-benzamide ;
le N-(2',5'-bis-trifluorométhyl-biphényl-4-yl)-2-méthoxybenzamide ;
le N-(2'-méthoxy-5'-méthyl-biphényl-4-yl)-2,3-difluorobenzamide ;
le N-(2',5'-diméthyl-biphényl-4-yl)-2,3-difluoro-benzamide ;
le (2',5'-bis-trifluorométhyl-biphényl-4-yl)-amide de l'acide 3-méthylisoxazole-4-carboxylique ;
le N-(2',5'-bis-trifluorométhyl-biphényl-4-yl)-2-hydroxybenzamide ;
le N-(2'-méthoxy-5'-acétyl-biphényl-4-yl)-2,3-difluorobenzamide ;
le (2',5'-bis-trifluorométhyl-biphényl-4-yl)-amide de l'acide 5-méthylisoxazole-4-carboxylique ;
le N-(2',4',5'-triméthyl-biphényl-4-yl)-2,3-difluoro-benzamide ;
le N-(2',5'-bis-trifluorométhyl-biphényl-4-yl)-2,3-diméthylbenzamide ;
le N-(2',5'-bis-trifluorométhyl-biphényl-4-yl)-2-méthyl-3-chlorobenzamide ;
le N-(2',5'-bis-trifluorométhyl-biphényl-4-yl)-2-méthyl-3-fluorobenzamide ;
le N-(2',5'-bis-trifluorométhyl-biphényl-4-yl)-2-méthyl-3-méthoxybenzamide ;
le (2',5'-diméthoxy-biphényl-4-yl)-amide de l'acide 4-méthyl-[1,2,3]thiadiazole-5-carboxylique ;
le N-(2',5'-diméthoxy-biphényl-4-yl)-2-méthyl-benzamide ;
le (2',5'-bis-trifluorométhyl-biphényl-4-yl)-amide de l'acide 2-méthyl-pyridine-3-carboxylique ;
le (2',5'-bis-trifluorométhyl-biphényl-4-yl)-amide de l'acide 2,3-dihydro-benzo[1,4]dioxine-5-carboxylique ;
le (2',5'-bis-trifluorométhyl-biphényl-4-yl)-amide de l'acide 1-méthyl-1H-imidazole-5-carboxylique ;
le (2',5'-diméthoxy-biphényl-4-yl)-amide de l'acide 3-méthyl-pyridine-4-carboxylique ;
le (2',5'-bis-trifluorométhyl-biphényl-4-yl)-amide de l'acide 3-méthyl-pyridine-4-carboxylique ;
le (2'-méthoxy-5'-chlorobiphényl-4-yl)-amide de l'acide 3-méthyl-pyridine-4-carboxylique ;
le (2',5'-diméthoxybiphényl-4-yl)-amide de l'acide 3-fluoro-pyridine-4-carboxylique ;
le (2'-méthoxy-5'-chlorobiphényl-4-yl)-amide de l'acide 3-fluoro-pyridine-4-carboxylique ;
le (2',5'-bis-trifluorométhylbiphényl-4-yl)-amide de l'acide 3-fluoro-pyridine-4-carboxylique ;
le (2'-méthoxy-5'-méthylbiphényl-4-yl)-amide de l'acide 3-méthyl-pyridine-4-carboxylique ;
le (2',5'-diméthylbiphényl-4-yl)-amide de l'acide 3-méthyl-pyridine-4-carboxylique ;
le (2'-méthoxy-5'-acétylbiphényl-4-yl)-amide de l'acide 4-méthyl-[1,2,3]-thiadiazole-5-carboxylique ;
le (2'-difluorométhoxy-5'-chlorobiphényl-4-yl)-amide de l'acide 3-fluoro-pyridine-4-carboxylique ;
le {2'-(N,N-diméthylamino)-5'-trifluorométhoxy-biphényl-4-yl}-amide de l'acide 4-méthyl-[1,2,3]-thiadiazole-5-carboxylique ;
le (2'-chloro-5'-trifluorométhylbiphényl-4-yl)-amide de l'acide 3-méthyl-pyridine-4-carboxylique ;
le (2'-méthylsulfanyl-biphényl-4-yl)-amide de l'acide 3-méthyl-pyridine-4-carboxylique ;
le (2'-éthyl-biphényl-4-yl)-amide de l'acide 3-méthyl-pyridine-4-carboxylique ;
le (2'-isopropyl-biphényl-4-yl)-amide de l'acide 3-méthyl-pyridine-4-carboxylique ;
le N-{5-(2',5'-diméthoxyphényl)-pyrid-2-yl}-2-méthyl-benzamide ;
le (2',5'-diéthylbiphényl-4-yl)-amide de l'acide 3-méthyl-pyridine-4-carboxylique ;
le {2'-(N,N-diméthylamino)-5'-méthoxybiphényl-4-yl}-amide de l'acide 3-méthyl-pyridine-4-carboxylique ;
le {2'-(N-diméthylamino)-5'-carbéthoxybiphényl-4-yl}-amide de l'acide 3-méthyl-pyridine-4-carboxylique ;
le (2'-éthoxy-5'-chlorobiphényl-4-yl)-amide de l'acide 3-méthyl-pyridine-4-carboxylique ;
le N-(2'-diméthoxy-5'-chloro-biphényl-4-yl)-2,6-difluorobenzamide ;
le N-(2'-méthoxy-5'-chloro-biphényl-4-yl)-2,4,5-trifluorobenzamide ;
le N-(2',5'-bis-trifluorométhyl-biphényl-4-yl)-2,6-difluorobenzamide ;
le N-(2'-chloro-5'-trifluorométhyl-biphényl-4-yl)-2,6-difluorobenzamide ;
le N-(2',5'-diméthyl-biphényl-4-yl)-2,6-difluoro-benzamide ;
le N-(2',5'-dichloro-biphényl-4-yl)-2,6-difluoro-benzamide ;
le 2,3-difluoro-N-[4-(2-trifluorométhyl-indolizin-3-yl)-phényl]-benzamide ;
le 2,5-difluoro-N-[4-(2-trifluorométhyl-indolizin-3-yl)-phényl]-benzamide ;
le 3,4-diméthoxy-N-[4-(2-trifluorométhyl-indolizin-3-yl)-phényl]-benzamide ;
le N-[4-(5-chloro-2-trifluorométhyl-indolizin-3-yl)-phényl]-2,3-difluorobenzamide ;
l'ester méthylique de l'acide 5-chloro-3-[4-(2,3-difluoro-benzoylamino)-phényl]-2-trifluorométhyl-indolizine-6-carboxylique ;
l'ester méthylique de l'acide 3-[4-(2,3-difluoro-benzoylamino)-phényl]-2-trifluorométhyl-indolizine-6-carboxylique ;
le 2,3-difluoro-N-[4-(6-méthoxy-2-trifluorométhyl-indolizin-3-yl)-phényl]-benzamide ;
le N-[4-(5-fluoro-2-trifluorométhyl-indolizin-3-yl)-phényl]-2,3-difluorobenzamide ;
le N-[4-(5-méthoxy-2-trifluorométhyl-indolizin-3-yl)-phényl]-2,3-difluorobenzamide ;
le N-[4-(5-chloro-2,7-bis-trifluorométhyl-indolizin-3-yl)-phényl]-2,3-difluorobenzamide ;
l'ester méthylique de l'acide 5-méthoxy-3-[4-(2,3-difluoro-benzoylamino)-phényl]-2-trifluorométhyl-indolizine-6-carboxylique ;
le 2,3-difluoro-N-[4-(8-méthoxy-2-trifluorométhyl-indolizin-3-yl)-phényl]-benzamide ;
l'ester méthylique de l'acide 5-chloro-3-[4-(2,3-difluoro-benzoylamino)-phényl]-2-trifluorométhyl-indolizine-7-carboxylique ;
l'ester méthylique de l'acide 5-chloro-3-[4-(2,6-difluoro-benzoylamino)-phényl]-2-trifluorométhyl-indolizine-7-carboxylique ;
le 2,6-difluoro-N-[4-(8-méthoxy-2-trifluorométhyl-indolizin-3-yl)-phényl]-benzamide ;
l'ester méthylique de l'acide 5-méthoxy-3-[4-(2,6-difluoro-benzoylamino)-phényl]-2-trifluorométhyl-indolizine-6-carboxylique ;
le N-[4-(5-chloro-2,7-bis-trifluorométhyl-indolizin-3-yl)-phényl]-2,6-difluorobenzamide ;
le N-[4-(5-méthoxy-2-trifluorométhyl-indolizin-3-yl)-phényl]-2,6-difluorobenzamide ;
le N-[4-(5-fluoro-2-trifluorométhyl-indolizin-3-yl)-phényl]-2,6-difluorobenzamide ;
le 2,6-difluoro-N-[4-(6-méthoxy-2-trifluorométhyl-indolizin-3-yl)-phényl]-benzamide ;
l'ester méthylique de l'acide 3-[4-(2,6-difluoro-benzoylamino)-phényl]-2-trifluorométhyl-indolizine-6-carboxylique ;
l'ester méthylique de l'acide 5-chloro-3-[4-(2,6-difluoro-benzoylamino)-phényl]-2-trifluorométhyl-indolizine-6-carboxylique ;
le N-[4-(5-chloro-2-trifluorométhyl-indolizin-3-yl)-phényl]-2,6-difluorobenzamide ;
le N-[4-(5-chloro-2-trifluorométhyl-indolizin-3-yl)-phényl]-2,4,5-trifluorobenzamide ;
l'ester méthylique de l'acide 5-chloro-3-[4-(2,4,5-trifluoro-benzoylamino)-phényl]-2-trifluorométhyl-indolizine-6-carboxylique ;
l'ester méthylique de l'acide 3-[4-(2,4,5-trifluoro-benzoylamino)-phényl]-2-trifluorométhyl-indolizine-6-carboxylique ;
le 2,4,5-trifluoro-N-[4-(5-fluoro-2-trifluorométhyl-indolizin-3-yl)-phényl]benzamide ;
le 2,4,5-trifluoro-N-[4-(6-méthoxy-2-trifluorométhyl-indolizin-3-yl)-phényl]benzamide ;
le 2,4,5-trifluoro-N-[4-(5-méthoxy-2-trifluorométhyl-indolizin-3-yl)-phényl]benzamide ;
le N-[4-(5-chloro-2,7-bis-trifluorométhyl-indolizin-3-yl)-phényl]-2,4,5-trifluorobenzamide ;
l'ester méthylique de l'acide 5-méthoxy-3-[4-(2,4,5-trifluoro-benzoylamino)-phényl]-2-trifluorométhyl-indolizine-6-carboxylique ;
le 2,4,5-trifluoro-N-[4-(8-méthoxy-2-trifluorométhyl-indolizin-3-yl)-phényl]-benzamide ;
l'ester méthylique de l'acide 5-chloro-3-[4-(2,4,5-trifluoro-benzoylamino)-phényl]-2-trifluorométhyl-indolizine-7-carboxylique ;
le 2,4,5-trifluoro-N-[4-(7-méthoxy-2-trifluorométhyl-indolizin-3-yl)-phényl]-benzamide ;
le 2,6-difluoro-N-[4-(7-méthoxy-2-trifluorométhyl-indolizin-3-yl)-phényl]-benzamide ;
le 2,3-difluoro-N-[4-(7-méthoxy-2-trifluorométhyl-indolizin-3-yl)-phényl]-benzamide ;
le N-(2',5'-diméthoxy-biphényl-4-yl)-2,6-difluoro-benzamide ;
le N-(2'-trifluorométhyl-5'-méthyl-biphényl-4-yl)-2,6-difluoro-benzamide ;
la N-(2',5'-bis-trifluorométhyl-biphényl-4-yl)-2,6-difluoro-benzyl amine ;
le sel N-(2',5'-bis-trifluorométhyl-biphényl-4-yl)-2,6-difluoro-benzyl amine HCl ;
la N-(2'-méthoxy-5'-chloro-biphényl-4-yl)-2,6-difluoro-benzyl amine ;
le sel N-(2'-méthoxy-5'-chloro-biphényl-4-yl)-2,6-difluoro-benzyl amine HCl ;
la N',N'-diéthyl-N-(2',5'-bis-trifluorométhyl-biphényl-4-yl)urée ;
le 2,3-difluoro-N-[4-(2-trifluoro-méthyl-indolizin-3-yl)-phényl]-benzamide ;
un amide de l'acide 4-méthyl-N-[4-(2-méthyl-indolizin-3-yl)-phényl]-[1,2,3]thiadiazole-5-carboxylique ; et
les sels, solvates ou clathrates pharmaceutiquement acceptables de celui-ci.

34. Composé représenté par la formule structurale suivante : 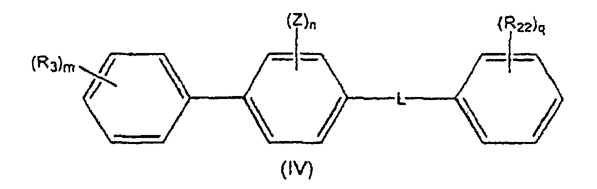 ou un sel, solvate ou clathrate pharmaceutiquement acceptable de celui-ci,
dans laquelle :
Z, R₃ et R₂₂, à chaque apparition, sont, indépendamment, choisis dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogène, -OR₄, un cyano, un nitro, un halogénoalcoxy, -C(O)R₄, -NR₁R₂, -SR₄, -C(O)OR₄, -OC(O)R₄, -NR₄C (O) NR₁R₂, -OC(O) NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est 0 ou un entier de 1 à 4 ;
m et q sont chacun indépendamment 0 ou un entier de 1 à 5 ; et
p est 0, 1 ou 2,
étant entendu que lorsque X est un p-halogénophényle, un p-nitrophényle, un p-cyanophényle, un p-(méthoxy-méthyl)phényle, un p-(benzamido)phényle, un p-(benzamido)phényle substitué ou un p-carboxyphényle, Y n'est pas un phényle substitué ou non substitué, un furyle non substitué, un thiophényle substitué ou non substitué, un benzo[b]thiophényle substitué, un thiazolyle non substitué, un 7,8-dihydronaphtyle substitué, un pyrazinyle substitué ou un pyridinyle substitué ou non substitué,
étant entendu que lorsque X est un m-nitrophényle ou un m-(trifluorométhyl)phényle, Y₁ n'est pas un phényle substitué ou non substitué ; et
étant entendu que X n'est pas un phényle qui est substitué en position ortho par -S(O)₂NH₂.

35. Composé représenté par la formule structurale suivante : 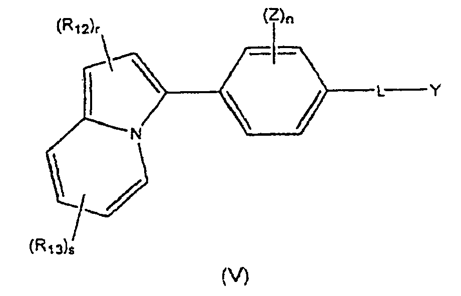 ou un sel, solvate ou clathrate pharmaceutiquement acceptable de celui-ci,
dans laquelle :
Y est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou alcoxy en C₁ à C₄ ;
L est un lieur choisi dans le groupe constitué par -NRCH₂- et -NR-C(O)- ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
Z, R₁₂ et R₁₃, à chaque apparition, sont, indépendamment, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogène, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
r est 0, 1 ou 2 ;
n et s sont chacun, indépendamment, 0 ou un entier de 1 à 4 ; et
p est 0, 1 ou 2.

36. Composé selon la revendication 35, dans lequel L est -NHC(O)- et Y est un phényle éventuellement substitué.

37. Composé selon la revendication 36, dans lequel le composé est représenté par la formule structurale suivante : 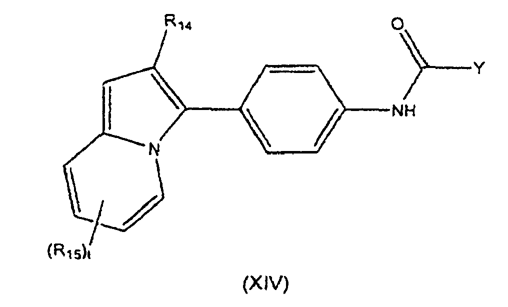 ou un sel, solvate ou clathrate pharmaceutiquement acceptable de celui-ci,
dans laquelle R₁₄ et R₁₅ sont chacun, indépendamment, -CF₃ , -OCH₃ , -F, -Cl ou -C(O)OCH₃ ; et
t est 0, 1 ou 2.

38. Composé selon la revendication 37, dans lequel le composé est représenté par la formule structurale suivante : 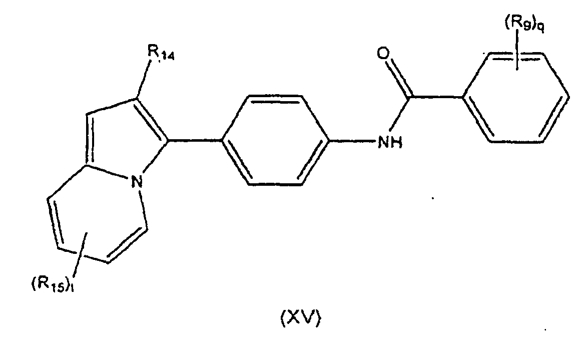 ou un sel, solvate ou clathrate pharmaceutiquement acceptable de celui-ci,
dans laquelle :
R₉, à chaque apparition, est, indépendamment, halogéno, alkyle en C₁ à C₄, halogénoalkyle en C₁ à C₄, alcoxy en C₁ à C₄, halogénoalcoxy en C₁ à C₄ ou hydroxyle ; et
q est 0 ou un entier de 1 à 5.

39. Composé selon la revendication 38, dans lequel le composé est représenté par la formule structurale suivante : 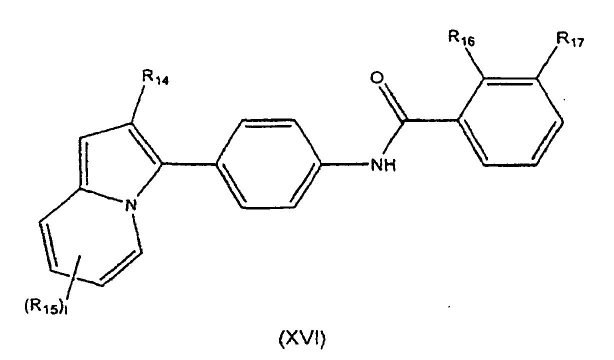 ou un sel, solvate ou clathrate pharmaceutiquement acceptable de celui-ci,
dans laquelle R₁₆ et R₁₇ sont chacun, indépendamment, -F ou -OCH₃.

40. Composé selon la revendication 38, dans lequel le composé est représenté par la formule structurale suivante : 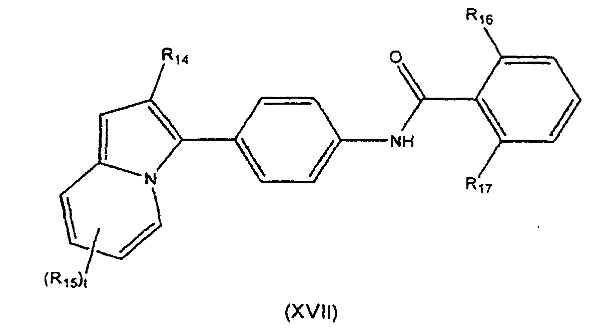 ou un sel, solvate ou clathrate pharmaceutiquement acceptable de celui-ci,
dans laquelle R₁₆ et R₁₇ sont chacun, indépendamment, -F ou -OCH₃.

41. Composé représenté par la formule structurale suivants : 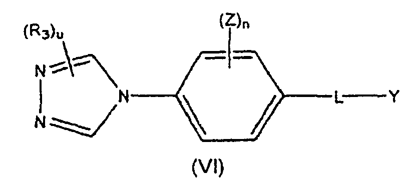 ou un sel, solvate ou clathrate pharmaceutiquement acceptable de celui-ci,
dans laquelle :
Y est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou alcoxy en C₁ à C₄ ;
Z et R₃, à chaque apparition, sont, indépendamment, choisis dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est un entier choisi parmi 0 à 4 ;
p est 0, 1 ou 2 ; et
u est 0, 1 ou 2.

42. Composition pharmaceutique, comprenant un support pharmaceutiquement acceptable et un ou plusieurs composés représentés par la formule structurale suivante : 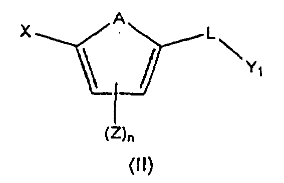 ou un sel, solvate ou clathrate pharmaceutiquement acceptable de celui-ci,
dans laquelle :
X est un phényle éventuellement substitué, un triazolyle éventuellement substitué, un pyridyle éventuellement substitué ou un indolizinyle éventuellement substitué ;
Y₁ est un aryle éventuellement substitué ou un hétéroaryle éventuellement substitué ;
A est -CH=CH-, -CZ=CH- ou -CH=CZ- ;
chaque Z est indépendamment choisi dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est entier choisi parmi 0 à 4 ; et
p est 0, 1 ou 2,
étant entendu que Y₁ n'est pas un hétéroaryle qui est plus encore substitué par un aryle substitué ou non substitué ou un hétéroaryle substitué ou non substitué ;
étant entendu que lorsque X est un p-halogénophényle, un p-nitrophényle, un p-cyanophényle, un p-(méthoxyméthyl)phényle, un p-(benzamido)phényle, un p-(benzamido)phényle substitué ou un p-carboxyphényle, Y₁ n'est pas un phényle substitué ou non substitué, un furyle non substitué, un thiophényle substitué ou non substitué, un benzo[b]thiophényle substitué, un thiazolyle non substitué, un 7,8-dihydronaphtyle substitué, un pyrazinyle substitué ou un pyridinyle substitué ou non substitué,
étant entendu que lorsque X est un m-nitrophényle ou un m-(trifluorométhyl)phényle, Y₁ n'est pas un phényle substitué ou non substitué ;
étant entendu que X n'est pas un phényle qui est substitué en position ortho par -S(O)₂NH₂ ; et
étant entendu que X n'est pas un nitrophényle lorsque Y₁ est un 1H-pyrazolyle substitué.

43. Composition pharmaceutique selon la revendication 42, dans laquelle le composé est représenté par la formule structurale suivante : 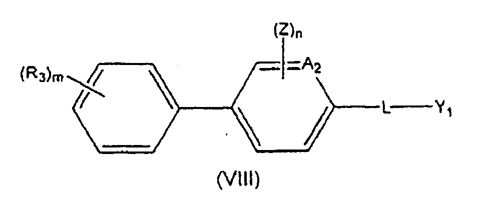 ou un sel, solvate ou clathrate pharmaceutiquement acceptable de celui-ci,
dans laquelle :
A₂ est CH ou CZ ;
R₃ est un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ; et
m est 0 ou un entier de 1 à 5.

44. Composition pharmaceutique selon la revendication 43, dans laquelle A₂ est CH.

45. Composition pharmaceutique selon la revendication 43, dans laquelle L est -NHC(O)- ou -NHCH₂-.

46. Composition pharmaceutique selon la revendication 45, dans laquelle Y₁ est un phényle éventuellement substitué, un pyridyle éventuellement substitué, un thiophényle éventuellement substitué, un [1,2,3]-thiadiazolyle, un isoxazolyle éventuellement substitué, un 1H-pyrazolyle, un quinolinyle, un imidazolyle ou une 2,3-dihydrobenzo[1,4]dioxine.

47. Composition pharmaceutique selon la revendication 46, dans laquelle Y₁ est un phényle éventuellement substitué, un pyridyle éventuellement substitué ou un [1,2,3]thiadiazolyle éventuellement substitué.

48. Composition pharmaceutique selon la revendication 47, dans laquelle le composé est représenté par la formule structurale suivants : 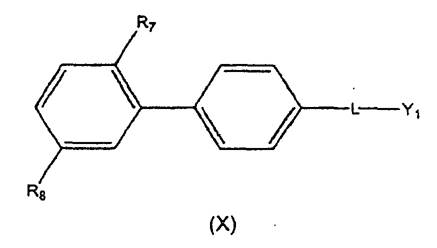 ou un sel, solvate ou clathrate pharmaceutiquement acceptable de celui-ci,
dans laquelle :
R₇ et R₈ sont chacun, indépendamment, -H, -CF₃, -CN, -C(O)CH₃, -F, -Cl, -OCH₃ , -OCH₂CH₃, -C(O)OCH₂CH₃, -SCH₃, -NHCH₃ ou un alkyle en C₁ à C₄, étant entendu qu'au moins l'un parmi R₇ ou R₈ n'est pas -H.

49. Composition pharmaceutique, comprenant un support pharmaceutiquement acceptable et un ou plusieurs composés représentés par la formule structurale suivante : 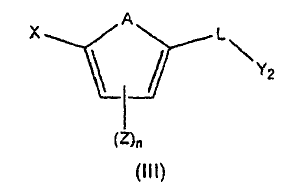 ou un sel, solvate ou clathrate pharmaceutiquement acceptable de celui-ci,
dans laquelle :
X est un phényle éventuellement substitué, un triazolyle éventuellement substitué, un pyridyle éventuellement substitué ou un indolizinyle éventuellement substitué ;
Y₂ est un cycloalkyle éventuellement substitué, un cycloalcényle éventuellement substitué ou un hétérocyclyle éventuellement substitué ;
A est -CH=CH-, -CZ=CH- ou -CH=CZ- ;
chaque Z est indépendamment choisi dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogène, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C (O) OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est 0 ou un entier de 1 à 4 ; et
p est 0, 1 ou 2,
étant entendu que X n'est pas un phényle qui est substitué en position ortho par -CN ou -S(O)₂NH₂ ;
étant entendu que Y₂ n'est pas un 4,5-dihydro-isoxazolyle qui est plus encore substitué par un aryle substitué ou non substitué ou un hétéroaryle substitué ou non substitué ; et
étant entendu que Y₂ n'est pas un cyclopentényle substitué.

50. Composition pharmaceutique, comprenant un support pharmaceutiquement acceptable et un ou plusieurs composés choisis dans le groupe constitué par :
le 3-fluoro-N-(2'-trifluorométhyl-biphényl-4-yl)-isonicotinamide ;
le 3-fluoro-N-(2'-méthyl-biphényl-4-yl)-isonicotinamide ;
le 3-fluoro-N-(3'-trifluorométhyl-biphényl-4-yl)-isonicotinamide ;
le N-(2,2'-bis-trifluorométhyl-biphényl-4-yl)-2,3-difluoro-benzamide ;
le N-[4-(1,2-diméthyl-but-1-ényl)-3-trifluorométhyl-phényl]-2,3-difluoro-benzamide ;
le diméthylamide de l'acide 4'-(2,3-difluoro-benzoylamino)-biphényl-2-carboxylique ;
le N-(2'-trifluorométhyl-biphényl-4-yl)-nicotinamide ;
le N-(2'-trifluorométhyl-biphényl-4-yl)-isonicotinamide ;
le (2'-trifluorométhyl-biphényl-4-yl)-amide de l'acide thiophène-2-carboxylique ;
le 4-fluoro-N-(2'-trifluorométhyl-biphényl-4-yl)-benzamide ;
le (2'-trifluorométhyl-biphényl-4-yl)-amide de l'acide 2,4-diméthyl-thiazole-5-carboxylique ;
le 4-trifluorométhyl-N-(2'-trifluorométhyl-biphényl-4-yl)-nicotinamide ;
le (2'-trifluorométhyl-biphényl-4-yl)-amide de l'acide 2-méthyl-5-trifluorométhyl-oxazole-4-carboxylique ;
le (2'-trifluorométhyl-biphényl-4-yl)-amide de l'acide 2-éthyl-5-méthyl-2H-pyrazole-3-carboxylique ;
le 2,3-difluoro-N-(2'-trifluorométhyl-biphényl-4-yl)-benzamide ;
le 2,5-difluoro-N-(2'-trifluorométhyl-biphényl-4-yl)-benzamide ;
le 2,3-difluoro-N-(3-fluoro-2'-trifluorométhyl-biphényl-4-yl)-benzamide ;
le N-(2',5'-bis-trifluorométhyl-biphényl-4-yl)-2,3-difluoro-benzamide ;
le 2,3-difluoro-N-(2'-fluoro-5'-trifluorométhyl-biphényl-4-yl)-benzamide ;
le 2,3-difluoro-N-(4'-fluoro-2'-trifluorométhyl-biphényl-4-yl)-benzamide ;
le 2,3-difluoro-N-[4-(2-trifluorométhyl-indolizin-3-yl)-phényl]-benzamide ;
le 2,3-difluoro-N-(2'-fluoro-6'-trifluorométhyl-biphényl-4-yl)-benzamide ;
le 2,3-difluoro-N-(2'-chloro-5'-trifluorométhyl-biphényl-4-yl)-benzamide ;
le (2',5'-bis-trifluorométhyl-biphényl-4-yl)-amide de l'acide 4-méthyl-[1,2,3]thiadiazole-5-carboxylique ;
le (2'-trifluorométhyl-biphényl-4-yl)-amide de l'acide pyridine-2-carboxylique ;
le (2'-trifluorométhyl-biphényl-4-yl)-amide de l'acide pyrazine-2-carboxylique ;
le (2'-chloro-5'-trifluorométhyl-biphényl-4-yl)-amide de l'acide 4-méthyl-[1,2,3]thiadiazole-5-carboxylique ;
le N-(2',5'-bis-trifluorométhyl-biphényl-4-yl)-2,5-difluoro-benzamide ;
le N-(2',5'-dichloro-biphényl-4-yl)-2,3-difluoro-benzamide ;
le N-(5'-cyano-2'-méthoxy-biphényl-4-yl)-2,3-difluoro-benzamide ;
le N-(2',5'-diméthoxy-biphényl-4-yl)-2,3-difluoro-benzamide ;
le N-[4-(3,5-bis-trifluorométhyl-[1,2,4]triazol-4-yl)-phényl]-2,3-difluoro-benzamide ;
le (4-(3,5-bis-trifluorométhyl-[1,2,4]triazol-4-yl)-phényl)-amide de l'acide 3-méthyl-thiophène-2-carboxylique ;
le N-[4-(3-trifluorométhyl-5-(thiophén-4-yl)-[1,2,4]-triazol-4-yl)-phényl]-2,3-difluoro-benzamide ;
le N-[4-(3-trifluorométhyl-5-(thiophén-4-yl)-[1,2,4]-triazol-4-yl)-phényl]-2,3-difluoro-benzamide ;
le N-[4-(3-trifluorométhyl-indolizin-3-yl)-phényl]-2,3-difluoro-benzamide ;
le N-[4-(3-cyano-5-trifluorométhyl-pyrid-2-yl)-phényl]-2,3-difluoro-benzamide ;
le N-(2',5'-bis-trifluorométhyl-biphényl-4-yl)-2-méthoxy-benzamide ;
le (2',5'-bis-trifluorométhyl-biphényl-4-yl)-amide de l'acide 5-méthyl-isoxazol-3-carboxylique ;
le (2',5'-bis-trifluorométhyl-biphényl-4-yl)-amide de l'acide 1,3-diméthyl-1H-pyrazol-5-carboxylique ;
le (2',5'-bis-trifluorométhyl-biphényl-4-yl)-amide de l'acide [1,2,3]-thiadiazole-4-carboxylique ;
le (2',5'-bis-trifluorométhyl-biphényl-4-yl)-amide de l'acide isoxazole-5-carboxylique ;
le (2',5'-bis-trifluorométhyl-biphényl-4-yl)-amide de l'acide 3,5-diméthylisoxazole-4-carboxylique ;
le N-(2'-méthoxy-5'-chloro-biphényl-4-yl)-2,3-difluoro-benzamide ;
le N-(2',5'-bis-trifluorométhyl-biphényl-4-yl)-2-méthoxybenzamide ;
le N-(2'-méthoxy-5'-méthyl-biphényl-4-yl)-2,3-difluorobenzamide ;
le N-(2',5'-diméthyl-biphényl-4-yl)-2,3-difluoro-benzamide ;
le (2',5'-bis-trifluorométhyl-biphényl-4-yl)-amide de l'acide 3-méthylisoxazole-4-carboxylique ;
le N-(2',5'-bis-trifluorométhyl-biphényl-4-yl)-2-hydroxybenzamide ;
le N-(2'-méthoxy-5'-acétyl-biphényl-4-yl)-2,3-difluorobenzamide ;
le (2',5'-bis-trifluorométhyl-biphényl-4-yl)-amide de l'acide 5-méthylisoxazole-4-carboxylique ;
le N-(2',4',5'-triméthyl-biphényl-4-yl)-2,3-difluoro-benzamide ;
le N-(2',5'-bis-trifluorométhyl-biphényl-4-yl)-2,3-diméthylbenzamide ;
le N-(2',5'-bis-trifluorométhyl-biphényl-4-yl)-2-méthyl-3-chlorobenzamide ;
le N-(2',5'-bis-trifluorométhyl-biphényl-4-yl)-2-méthyl-3-fluorobenzamide ;
le N-(2',5'-bis-trifluorométhyl-biphényl-4-yl)-2-méthyl-3-méthoxybenzamide ;
le (2',5'-diméthoxy-biphényl-4-yl)-amide de l'acide 4-méthyl-[1,2,3]thiadiazole-5-carboxylique ;
le N-(2',5'-diméthoxy-biphényl-4-yl)-2-méthyl-benzamide ;
le (2',5'-bis-trifluorométhyl-biphényl-4-yl)-amide de l'acide 2-méthyl-pyridine-3-carboxylique ;
le (2',5'-bis-trifluorométhyl-biphényl-4-yl)-amide de l'acide 2,3-dihydro-benzo[1,4]dioxine-5-carboxylique ;
le (2',5'-bis-trifluorométhyl-biphényl-4-yl)-amide de l'acide 1-méthyl-1H-imidazole-5-carboxylique ;
le (2',5'-diméthoxy-biphényl-4-yl)-amide de l'acide 3-méthyl-pyridine-4-carboxylique ;
le (2',5'-bis-trifluorométhyl-biphényl-4-yl)-amide de l'acide 3-méthyl-pyridine-4-carboxylique ;
le (2'-méthoxy-5'-chlorobiphényl-4-yl)-amide de l'acide 3-méthyl-pyridine-4-carboxylique ;
le (2',5'-diméthoxybiphényl-4-yl)-amide de l'acide 3-fluoro-pyridine-4-carboxylique ;
le (2'-méthoxy-5'-chlorobiphényl-4-yl)-amide de l'acide 3-fluoro-pyridine-4-carboxylique ;
le (2',5'-bis-trifluorométhylbiphényl-4-yl)-amide de l'acide 3-fluoro-pyridine-4-carboxylique ;
le (2'-méthoxy-5'-méthylbiphényl-4-yl)-amide de l'acide 3-méthyl-pyridine-4-carboxylique ;
le (2',5'-diméthylbiphényl-4-yl)-amide de l'acide 3-méthyl-pyridine-4-carboxylique ;
le (2'-méthoxy-5'-acétylbiphényl-4-yl)-amide de l'acide 4-méthyl-[1,2,3]-thiadiazole-5-carboxylique ;
le (2'-difluorométhoxy-5'-chlorobiphényl-4-yl)-amide de l'acide 3-fluoro-pyridine-4-carboxylique ;
le {2'-(N,N-diméthylamino)-5'-trifluorométhoxy-biphényl-4-yl}-amide de l'acide 4-méthyl-[1,2,3]-thiadiazole-5-carboxylique ;
le (2'-chloro-5'-trifluorométhylbiphényl-4-yl)-amide de l'acide 3-méthyl-pyridine-4-carboxylique ;
le (2'-méthylsulfanyl-biphényl-4-yl)-amide de l'acide 3-méthyl-pyridine-4-carboxylique ;
le (2'-éthyl-biphényl-4-yl)-amide de l'acide 3-méthyl-pyridine-4-carboxylique ;
le (2'-isopropyl-biphényl-4-yl)-amide de l'acide 3-méthyl-pyridine-4-carboxylique ;
le N-{5-(2',5'-diméthoxyphényl)-pyrid-2-yl}-2-méthylbenzamide ;
le (2',5'-diéthylbiphényl-4-yl)-amide de l'acide 3-méthyl-pyridine-4-carboxylique ;
le {2'-(N,N-diméthylamino)-5'-méthoxybiphényl-4-yl}-amide de l'acide 3-méthyl-pyridine-4-carboxylique ;
le {2'-(N-diméthylamino)-5'-carbéthoxybiphényl-4-yl}-amide de l'acide 3-méthyl-pyridine-4-carboxylique ;
le (2'-éthoxy-5'-chlorobiphényl-4-yl)-amide de l'acide 3-méthyl-pyridine-4-carboxylique ;
le N-(2'-diméthoxy-5'-chloro-biphényl-4-yl)-2,6-difluorobenzamide ;
le N-(2'-méthoxy-5'-chloro-biphényl-4-yl)-2,4,5-trifluorobenzamide ;
le N-(2',5'-bis-trifluorométhyl-biphényl-4-yl)-2,6-difluorobenzamide ;
le N-(2'-chloro-5'-trifluorométhyl-biphényl-4-yl)-2,6-difluorobenzamide ;
le N-(2',5'-diméthyl-biphényl-4-yl)-2,6-difluorobenzamide ;
le N-(2',5'-dichloro-biphényl-4-yl)-2,6-difluorobenzamide ;
le 2,3-difluoro-N-[4-(2-trifluorométhyl-indolizin-3-yl)-phényl]-benzamide ;
le 2,5-difluoro-N-[4-(2-trifluorométhyl-indolizin-3-yl)-phényl]-benzamide ;
le 3,4-diméthoxy-N-[4-(2-trifluorométhyl-indolizin-3-yl)-phényl]-benzamide ;
le N-[4-(5-chloro-2-trifluorométhyl-indolizin-3-yl)-phényl]-2,3-difluorobenzamide ;
l'ester méthylique de l'acide 5-chloro-3-[4-(2,3-difluoro-benzoylamino)-phényl]-2-trifluorométhyl-indolizine-6-carboxylique ;
l'ester méthylique de l'acide 3-[4-(2,3-difluoro-benzoylamino)-phényl]-2-trifluorométhyl-indolizine-6-carboxylique ;
le 2,3-difluoro-N-[4-(6-méthoxy-2-trifluorométhyl-indolizin-3-yl)-phényl]-benzamide ;
le N-[4-(5-fluoro-2-trifluorométhyl-indolizin-3-yl)-phényl]-2,3-difluorobenzamide ;
le N-[4-(5-méthoxy-2-trifluorométhyl-indolizin-3-yl)-phényl]-2,3-difluorobenzamide ;
le N-[4-(5-chloro-2,7-bis-trifluorométhyl-indolizin-3-yl)-phényl]-2,3-difluorobenzamide ;
l'ester méthylique de l'acide 5-méthoxy-3-[4-(2,3-difluoro-benzoylamino)-phényl]-2-trifluorométhyl-indolizine-6-carboxylique ;
le 2,3-difluoro-N-[4-(8-méthoxy-2-trifluorométhyl-indolizin-3-yl)-phényl]-benzamide ;
l'ester méthylique de l'acide 5-chloro-3-[4-(2,3-difluoro-benzoylamino)-phényl]-2-trifluorométhyl-indolizine-7-carboxylique ;
l'ester méthylique de l'acide 5-chloro-3-[4-(2,6-difluoro-benzoylamino)-phényl]-2-trifluorométhyl-indolizine-7-carboxylique ;
le 2,6-difluoro-N-[4-(8-méthoxy-2-trifluorométhyl-indolizin-3-yl)-phényl]-benzamide ;
l'ester méthylique de l'acide 5-méthoxy-3-[4-(2,6-difluoro-benzoylamino)-phényl]-2-trifluorométhyl-indolizine-6-carboxylique ;
le N-[4-(5-chloro-2,7-bis-trifluorométhyl-indolizin-3-yl)-phényl]-2,6-difluorobenzamide ;
le N-[4-(5-méthoxy-2-trifluorométhyl-indolizin-3-yl)-phényl]-2,6-difluorobenzamide ;
le N-[4-(5-fluoro-2-trifluorométhyl-indolizin-3-yl)-phényl]-2,6-difluorobenzamide ;
le 2,6-difluoro-N-[4-(6-méthoxy-2-trifluorométhyl-indolizin-3-yl)-phényl]-benzamide ;
l'ester méthylique de l'acide 3-[4-(2,6-difluoro-benzoylamino)-phényl]-2-trifluorométhyl-indolizine-6-carboxylique ;
l'ester méthylique de l'acide 5-chloro-3-[4-(2,6-difluoro-benzoylamino)-phényl]-2-trifluorométhyl-indolizine-6-carboxylique ;
le N-[4-(5-chloro-2-trifluorométhyl-indolizin-3-yl)-phényl]-2,6-difluorobenzamide ;
le N-[4-(5-chloro-2-trifluorométhyl-indolizin-3-yl)-phényl]-2,4,5-trifluorobenzamide ;
l'ester méthylique de l'acide 5-chloro-3-[4-(2,4,5-trifluoro-benzoylamino)-phényl]-2-trifluorométhyl-indolizine-6-carboxylique ;
l'ester méthylique de l'acide 3-[4-(2,4,5-trifluoro-benzoylamino)-phényl]-2-trifluorométhyl-indolizine-6-carboxylique ;
le 2,4,5-trifluoro-N-[4-(5-fluoro-2-trifluorométhyl-indolizin-3-yl)-phényl]benzamide ;
le 2,4,5-trifluoro-N-[4-(6-méthoxy-2-trifluorométhyl-indolizin-3-yl)-phényl]benzamide ;
le 2,4,5-trifluoro-N-[4-(5-méthoxy-2-trifluorométhyl-indolizin-3-yl)-phényl]benzamide ;
le N-[4-(5-chloro-2,7-bis-trifluorométhyl-indolizin-3-yl)-phényl]-2,4,5-trifluorobenzamide ;
l'ester méthylique de l'acide 5-méthoxy-3-[4-(2,4,5-trifluoro-benzoylamino)-phényl]-2-trifluorométhyl-indolizine-6-carboxylique ;
le 2,4,5-trifluoro-N-[4-(8-méthoxy-2-trifluorométhyl-indolizin-3-yl)-phényl]-benzamide ;
l'ester méthylique de l'acide 5-chloro-3-[4-(2,4,5-trifluoro-benzoylamino)-phényl]-2-trifluorométhyl-indolizine-7-carboxylique ;
le 2,4,5-trifluoro-N-[4-(7-méthoxy-2-trifluorométhyl-indolizin-3-yl)-phényl]-benzamide ;
le 2,6-difluoro-N-[4-(7-méthoxy-2-trifluorométhyl-indolizin-3-yl)-phényl]-benzamide ;
le 2,3-difluoro-N-[4-(7-méthoxy-2-trifluorométhyl-indolizin-3-yl)-phényl]-benzamide ;
le N-(2',5'-diméthoxy-biphényl-4-yl)-2,6-difluoro-benzamide ;
le N-(2'-trifluorométhyl-5'-méthyl-biphényl-4-yl)-2,6-difluoro-benzamide ;
la N-(2',5'-bis-trifluorométhyl-biphényl-4-yl)-2,6-difluoro-benzyl amine ;
le sel N-(2',5'-bis-trifluorométhyl-biphényl-4-yl)-2,6-difluoro-benzyl amine HCl ;
la N-(2'-méthoxy-5'-chloro-biphényl-4-yl)-2,6-difluoro-benzyl amine ;
le sel N-(2'-méthoxy-5'-chloro-biphényl-4-yl)-2,6-difluoro-benzyl amine HCl ;
la N',N'-diéthyl-N-(2',5'-bis-trifluorométhyl-biphényl-4-yl)urée ;
le 2,3-difluoro-N-[4-(2-trifluoro-méthyl-indolizin-3-yl)-phényl]-benzamide ;
un amide de l'acide 4-méthyl-N-[4-(2-méthyl-indolizin-3-yl)-phényl]-[1,2,3]thiadiazole-5-carboxylique ; et
les sels, solvates ou clathrates pharmaceutiquement acceptables de ceux-ci.

51. Composition pharmaceutique, comprenant un support pharmaceutiquement acceptable et un ou plusieurs composés représentés par la formule structurale suivante : 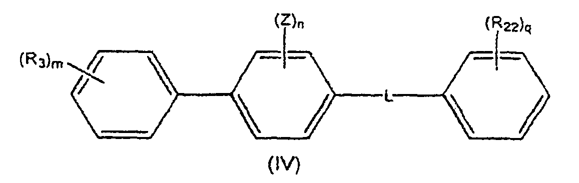 ou un sel, solvate ou clathrate pharmaceutiquement acceptable de ceux-ci,
dans laquelle :
Z, R₃ et R₂₂, à chaque apparition, sont, indépendamment, choisis dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R_{2,} -NR₄C(O)R₅, un halogène, -OR₄ , un cyano , un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C (O) OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est 0 ou un entier de 1 à 4 ;
m et q sont chacun indépendamment 0 ou un entier de 1 à 5 ; et
p est 0, 1 ou 2,
étant entendu que lorsque X est un p-halogénophényle, un p-nitrophényle, un p-cyanophényle, un p-(méthoxy-méthyl)phényle, un p-(benzamido)phényle, un p-(benzamido)phényle substitué ou un p-carboxyphényle, Y n'est pas un phényle substitué ou non substitué, un furyle non substitué, un thiophényle substitué ou non substitué, un benzo[b]thiophényle substitué, un thiazolyle non substitué, un 7,8-dihydronaphtyle substitué, un pyrazinyle substitué ou un pyridinyle substitué ou non substitué,
étant entendu que lorsque X est un m-nitrophényle ou un m-(trifluorométhyl)phényle, Y₁ n'est pas un phényle substitué ou non substitué ; et
étant entendu que X n'est pas un phényle qui est substitué en position ortho par -S(O)₂NH₂.

52. Composition pharmaceutique, comprenant un support pharmaceutiquement acceptable et un ou plusieurs composés représentés par la formule structurale suivante : 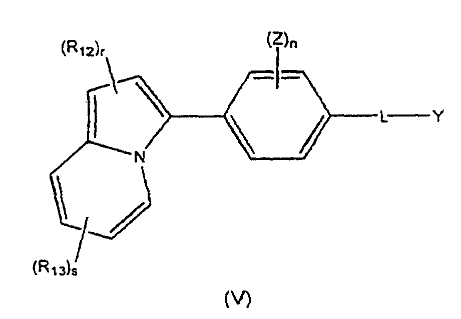 ou un sel, solvate ou clathrate pharmaceutiquement acceptable de ceux-ci,
dans laquelle :
Y est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou alcoxy en C₁ à C₄ ;
L est un lieur choisi dans le groupe constitué par -NRCH₂- et -NR-C(O)- ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
Z, R₁₂ et R₁₃, à chaque apparition, sont, indépendamment, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C(O)R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
r est 0, 1 ou 2 ;
n et s sont chacun, indépendamment, 0 ou un entier de 1 à 4 ; et
p est 0, 1 ou 2.

53. Composition pharmaceutique selon la revendication 52, dans laquelle L est -NHC(O)- et Y est un phényle éventuellement substitué.

54. Composition pharmaceutique selon la revendication 53, dans laquelle le composé est représenté par la formule structurale suivante : 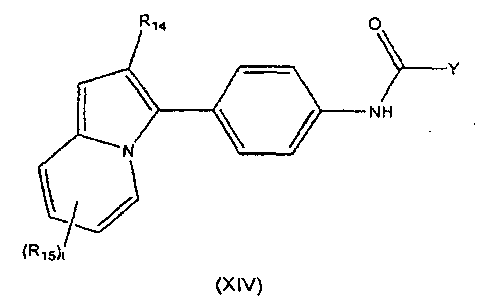 ou un sel, solvate ou clathrate pharmaceutiquement acceptable de celui-ci,
dans laquelle R₁₄ et R₁₅ sont chacun, indépendamment, -CF₃, -OCH₃, -F, -Cl ou -C(O)OCH₃ ; et
t est 0, 1 ou 2.

55. Composition pharmaceutique selon la revendication 54, dans laquelle le composé est représenté par la formule structurale suivants : 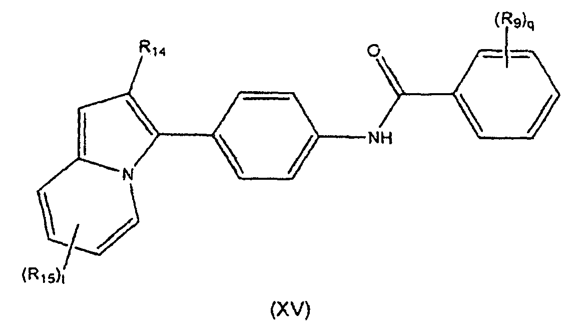 ou un sel, solvate ou clathrate pharmaceutiquement acceptable de celui-ci,
dans laquelle :
R₉, à chaque apparition, est, indépendamment, halogéno, alkyle en C₁ à C₄, halogénoalkyle en C₁ à C₄, alcoxy en C₁ à C₄, halogénoalcoxy en C₁ à C₄ ou hydroxyle ; et
q est 0 ou un entier de 1 à 5.

56. Composition pharmaceutique selon la revendication 55, dans laquelle le composé est représenté par la formule structurale suivante : 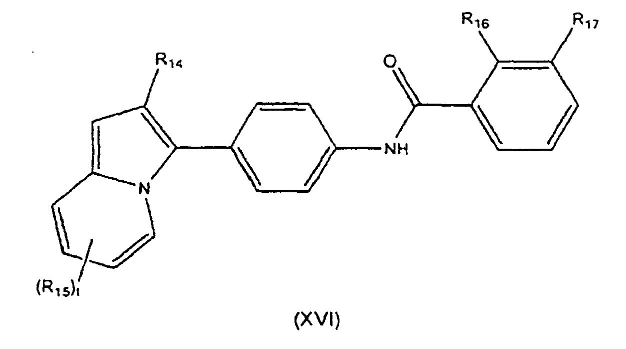 ou un sel, solvate ou clathrate pharmaceutiquement acceptable de celui-ci,
dans laquelle R₁₆ et R₁₇ sont chacun, indépendamment, -F ou -OCH₃.

57. Composition pharmaceutique selon la revendication 55, dans laquelle le composé est représenté par la formule structurale suivante : 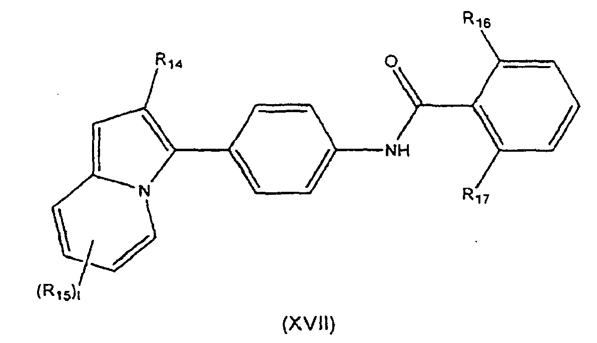 ou un sel, solvate ou clathrate pharmaceutiquement acceptable de celui-ci,
dans laquelle R₁₆ et R₁₇ sont chacun, indépendamment, -F ou -OCH₃.

58. Composition pharmaceutique, comprenant un support pharmaceutiquement acceptable et un ou plusieurs composés représentés par la formule structurale suivante : 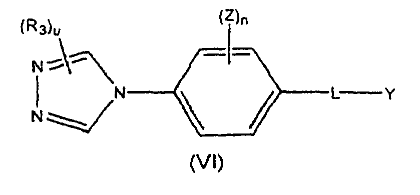 ou un sel, solvate ou clathrate pharmaceutiquement acceptable de ceux-ci,
dans laquelle :
Y est un aryle éventuellement substitué ou un hétéroaryle éventuellement substitué qui peut être éventuellement substitué par 1 à 3 substituants indépendamment choisis parmi halogéno, alkyle en C₁ à C₄, alkyle en C₁ à C₄ halogéné ou alcoxy en C₁ à C₄ ;
Z et R₃, à chaque apparition, sont, indépendamment, choisis dans le groupe constitué par un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué, un hétéroaralkyle éventuellement substitué, un halogénoalkyle, -C(O)NR₁R₂, -NR₄C (O) R₅, un halogéno, -OR₄, un cyano, un nitro, un halogénoalcoxy, -C(O)R₄, -NR₁R₂, -SR₄, -C(O)OR₄, -OC(O)R₄, -NR₄C(O)NR₁R₂, -OC(O)NR₁R₂, -NR₄C(O)OR₅, -S(O)ₚR₄ ou -S(O)ₚNR₁R₂ ;
L est un lieur choisi dans le groupe constitué par -NRCH₂- et -NR-C(O)- ;
chaque R est indépendamment choisi parmi -H, un alkyle, un acétyle, un tert-butoxycarbonyle, un benzyloxycarbonyle ;
R₁ et R₂, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ; ou R₁ et R₂ pris ensemble avec l'azote auquel ils sont attachés sont un hétérocyclyle éventuellement substitué ou un hétéroaryle éventuellement substitué ;
R₄ et R₅, à chaque apparition, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocyclyle éventuellement substitué, un aryle éventuellement substitué, un hétéroaryle éventuellement substitué, un aralkyle éventuellement substitué ou un hétéroaralkyle éventuellement substitué ;
n est un entier choisi parmi 0 à 4 ;
p est 0, 1 ou 2 ; et
u est 0, 1 ou 2.

59. Composition pharmaceutique selon la revendication 40, 49, 50, 51, 52 ou 58, comprenant en outre un ou plusieurs agents thérapeutiques supplémentaires.

60. Composition pharmaceutique selon la revendication 59, dans laquelle l'agent thérapeutique supplémentaire est choisi dans le groupe constitué par les agents immunosuppresseurs et les agents anti-inflammatoires et des mélanges appropriés de ceux-ci.

61. Composition pharmaceutique selon la revendication 60, dans laquelle l'agent thérapeutique supplémentaire est choisi dans le groupe constitué par les stéroïdes, les agents anti-inflammatoires non stéroïdiens, les antihistaminiques, les analgésiques, et des mélanges appropriés de ceux-ci.
